(19) Europäisches Patentamt
European Patent Office
Office européen des brevets

(11) EP 1 526 178 B1

(12) **EUROPEAN PATENT SPECIFICATION**

(45) Date of publication and mention of the grant of the patent:
**11.11.2009 Bulletin 2009/46**

(21) Application number: **03292673.5**

(22) Date of filing: **24.10.2003**

(51) Int Cl.:
*C12N 15/30* (2006.01)
*A61K 39/015* (2006.01)
*A61K 39/395* (2006.01)
*A61K 48/00* (2006.01)
*C07K 14/445* (2006.01)
*C07K 16/20* (2006.01)
*G01N 33/569* (2006.01)
*C12N 15/63* (2006.01)

(54) **MSP-3-like family of genes**

MSP-3-ähnliche Genfamilie

Famille de gènes du type MSP-3

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(43) Date of publication of application:
**27.04.2005 Bulletin 2005/17**

(60) Divisional application:
**09156218.1**

(73) Proprietor: **INSTITUT PASTEUR**
**75724 Paris Cedex 15 (FR)**

(72) Inventor: **Druilhe, Pierre**
**75015 Paris (FR)**

(74) Representative: **Desaix, Anne et al**
**Ernest Gutmann - Yves Plasseraud S.A.S.**
**3, rue Auber**
**75009 Paris (FR)**

(56) References cited:

- **CARLOTTA TRUCCO ET AL.: "The merozoite surface protein 6 gene codes for a 36 kDa protein associated with the Plasmodium falciparum merozoite surface protein-1 complex" MOLECULAR AND BIOCHEMICAL PARASITOLOGY, vol. 112, 2001, pages 91-101, XP002283182**
- **CLAUDE OEUVRAY ET AL.: "Merozoite surface protein - 3: A malaria protein inducing antibodies that promote Plasmodium falciparum killing by cooperation with blood monocytes" BLOOD, vol. 84, no. 5, 1 September 1994 (1994-09-01), pages 1594-1602, XP002283183**
- **MICHAEL THEISEN ET AL.: "Identification of a major B-cell epitope of the Plasmodium falciparum glutamate-rich protein (GLURP), targeted by human antibodies mediating parasite killing" VACCINE, vol. 19, 2001, pages 204-212, XP004228829**
- **VIKKI M. MARSHALL ET AL.: "Close linkage of three merozoite surface protein genes on chromosome 2 of Plasmodium falciparum" MOLECULAR AND BIOCHEMICAL PARASITOLOGY, no. 94, 1998, pages 13-25, XP002283184**
- **DATABASE EMBL Entry BI815359 5 October 2001 (2001-10-05), TANG, K. ET AL.: "PfESToaa17f04.y1 Plasmodium falciparum 3D7 asexual cDNA" XP002283186 accession no. BI815359 Database accession no. BI815359**
- **DATABASE EMBL Entry AU086276 11 January 2001 (2001-01-11), WATANABE, J. ET AL.: "Plasmodium falciparum 3D7 cDNA clone from Sugano malaria cDNA library: XPFn2229" XP002283187 Database accession no. AU086276 -& WATANABE, J. ET AL.: "FULL-malaria: a database for a full-length cDNA enriched cDNA library from human malaria parasite Plasmodium falciparum" NUCLEIC ACID RESEARCH, vol. 29, 2001, pages 70-71, XP002283185**
- **DATABASE UNIPROT Entry Q8IJ53 1 March 2003 (2003-03-01), GARDNER, M:J: ET AL.: "Hypothetical protein" XP002283188 Database accession no. Q8IJ53 -& GARDNER, M.J. ET AL.: "Genome sequence of the human malaria parasite Plasmodium falciparum" NATURE, vol. 419, 2002, pages 498-511, XP002283190**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BLACKMAN M.J. ET AL: 'Processing of the Plasmodium falciparum major merozoite surface protein-1: identification of a 33-kilodalton secondary processing product which is shed prior to erythrocyte invasion' MOLECULAR AND BIOCHEMICAL PARASITOLOGY vol. 49, 1991, pages 35 - 44
- SANJAY SINGH ET AL: 'Biochemical and immunological characterization of bacterially expressed and refolded Plasmodium falciparum 42-kilodalton C-terminal Merozoite Surface Protein 1' INFECTION AND IMMUNITY vol. 71, no. 12, December 2003, pages 6766 - 6774
- BLACKMAN M.J. ET AL: 'Antibodies inhibit the protease-mediated processing of a malaria Merozoite Surface Protein' JOURNAL OF EXPERIMENTAL MEDICINE vol. 180, July 1994, pages 389 - 393
- JOSÉ A. GUEVARA PATIÑO ET AL: 'Antibodies that inhibit malaria Merozoite Surface Protein-1 processing and erythrocyte invasion are blocked by naturally acquired huan antibodies' JOURNAL OF EXPERIMENTAL MEDICINE vol. 186, no. 10, 17 November 1997, pages 1689 - 1699
- GRAS-MASSE H. ET AL: 'Synthetic vaccines and HIV-1 hypervariability: a "mixotope" approach' PEPT. RES. vol. 5, no. 4, 1992, pages 211 - 216
- ESTAQUIER J. ET AL: 'The mixotope: a combinatorial peptide library as a T cell and B cell immunogen' EUR. J. IMMUNOL. vol. 24, no. 11, 1994, pages 2789 - 2795
- PERDOMO M.F.: 'Neutralization of HIV-1 by redirection of natural antibodies' PNAS vol. 105, no. 34, 26 August 2008, pages 12515 - 12520
- RABALAIS G.P. ET AL: 'Rapid diagnosis of respiratory viral infections by using a shell vial assay and a monoclonal antibody pool' JOURNAL OF CLINICAL MICROBIOLOGY vol. 30, no. 6, June 1992, pages 1505 - 1508
- CHRISTIAN ROUSSILHON ET AL: 'Long-term clinical protection from Falciparum Malaria is strongly associated with IgG3 antibodies to Merozoite Surface Protein 3' PLOS MEDICINE vol. 4, no. 11, November 2007, pages 1 - 13


Remarks:

The file contains technical information submitted after the application was filed and not included in this specification

## Description

The present invention relates to the protection against malaria. More particularly, the application describes a novel family of genes encompassing the already known MSP-3 gene, and showing exceptional redundancy of exposed epitopes, hence suggesting that this family of genes plays an important part in the immunogenicity of the parasite. The characterization of this gene family enables the definition of novel immunogenic and vaccine compositions against *P. falciparum.*

The parasites responsible for malaria in human, including especially *Plasmodium falciparum*, exhibit different morphologies in the human host and express different antigens as a function of their localization in the organism of the infected host. The morphological and antigenic differences of these parasites during their life cycle in man enable at least four distinct stages of development to be defined.

The very first stage of development of the parasite in man corresponds to the sporozoite form introduced into the blood of the host by bites of insect vectors of the parasite. The second stage corresponds to the passage of the parasite into the liver and to the infection of the hepatic cells in which the parasites develop to form the hepatic schizonts which, when they are mature (for example, in the case of *P. falciparum* on the 6$^{th}$ day after penetration of the sporozoites) release hepatic merozoites by bursting. The third stage is characterized by the infection of the blood erythrocytes by the asexual forms (merozoites) of the parasite; this erythrocytic stage of development corresponds to the pathogenic phase of the disease. The fourth stage corresponds to the formation of the forms with sexual potential (or gametocytes) which will become extracellular sexual forms or gametes in the mosquito.

Antibodies have been repeatedly shown to play an important part in the development of clinical immunity to *Plasmodium falciparum* malaria.

Numerous immunological studies now suggest that human antibodies of the cytophilic subclasses (IgG1 and IgG3) are particularly critical to the state of premunition. This anti-parasite immunity is a strain-independent, non-sterilizing type of immunity which is acquired after lengthy exposure (15-20 years) to the parasite. It is commonly observed in Africa and in Papua-New Guinea but it has only recently been documented in S-E Asia (Soe, Khin Saw et al. 2001). Although antibodies can act directly upon merozoite invasion of red blood cells, the most efficient *in vivo* mechanism for antibody-mediated parasite control in endemic areas requires the participation of monocytes (Khusmith and Druilhe 1983); (Lunel and Druilhe 1989). The antibody-dependent cellular inhibition (ADCI) assay mimics this cooperation between monocytes and cytophilic parasite-specific antibodies and appears today as the best *in vitro* surrogate marker of acquired immunity against *P. falciparum* blood stages.

Two molecules have so far been identified as targets of ADCI-effective human antibodies, namely the 48-kDa Merozoite surface-protein 3, - hereafter designated as *MSP-3* - (Oeuvray, Bouharoun-Tayoun et al. 1994) and the 220-kDa Glutamate-rich protein, - hereafter designated as *GLURP* - (Theisen, Soe et al. 1998). It has also been shown that GLURP and MSP-3 can inhibit parasite growth *in vivo* by passive transfer in *P. falciparum*-humanized SCID mice (Badell, Oeuvray et al. 2000). The association of human antibodies against MSP-3 with clinical protection is also indicated by a number of immuno-epidemiological studies, which demonstrate that the levels of MSP-3 specific cytophilic antibodies (IgG1 and IgG3) are significantly associated with a reduced risk of malaria attacks (Roussillon 1999). These studies have further shown that cytophilic IgG3 antibodies play a major part in protection against malaria, hence bringing epidemiological support to the concept that antibodies against MSP-3 can actively control parasite multiplication *in vivo* by cooperation with cells bearing Fcγ II receptors (Bouharoun-Tayoun, Oeuvray et al. 1995). These receptors display higher affinity for the IgG3 subclass than for the IgG1 subclass (Pleass and Woof 2001). The major B-cell epitopes recognized by these human IgG antibodies have been localized to conserved sequences in the MSP-$3_{212-257}$ region (Oeuvray, Bouharoun-Tayoun et al. 1994; Theisen, Soe et al. 2000; Theisen, Dodoo et al. 2001). Nucleotide-sequencing have demonstrated that these important epitopes are highly conserved among a number of *P. falciparum* laboratory lines and field isolates from Africa and Asia (Huber, Felger et al. 1997); (McColl and Anders 1997).

Trucco et al. (2001) discloses the proteins sequence of the MSP6 (now called MSP3.2) and the MSP3 (now called MSP3.1) proteins and their sequence alignment. Oeuvray et al. (1994) discloses the identification of an antigenic epitope (MSP-3b) on the MSP3 protein and describes its nucleotide and protein sequences. This antigenic epitope was found by using MoAb 2-45 antibody and confirmed by testing antibody directed against a synthetic MSP-3b peptide. The Q8IJ53 Accession Number describes a 424 amino acid hypothetical protein, hypothetically expressed from an *in silico* predicted Open Reading Frame from the sequence of the genome of *Plasmodium falciparum.*

The inventors have now characterized a series of 9 *P. falciparum* genes, all clustered at the 3' terminus of chromosome 10, which encode proteins and epitopes within, which are all targets for naturally occuring antibodies in malaria exposed individuals, mediating *P. falciparum* erythrocytic stage killing by cooperation with blood monocytes, and which exhibit an unusual degree of sequence conservation among various *P. falciparum* isotates.

The present application discloses a family of isolated genes, called the MSP-3-like family, the products of which having common structural and immunological features, as well as to some of these genes and the corresponding proteins, taken individually.

Antigenic polypeptides as disclosed in the claims comprising epitopes from said novel proteins, as well as antigenic polypeptidic compositions comprising at least two of said epitopes, are also part of the invention.

Other important aspects of the invention are immunogenic compositions and vaccines against malaria, comprising as an immunogen a polypeptide or a polypeptidic composition as mentioned above.

Recombinant antibodies and part thereof, which cross-react with several products of the MSP-3-like gene family, are also disclosed, either taken as such or in a medicament for passive immunotherapy or in a kit for the *in vitro* diagnosis of malaria.

The application discloses methods for the *in vitro* diagnosis of malaria in an individual, either by using an antigenic polypeptide, or by using an antibody as defined above, as well as kits comprising at least part of the necessary reagents (polypeptides, antibodies ...) for performing these methods.

Nucleotide sequences encoding at least one of the novel *P. falciparum* antigens, and their use in a medicament or a nucleic acid vaccine against *P. falciparum,* are also disclosed.

Throughout the present text, a number of terms are used, that should be understood according to the following definitions :

In what follows, the term "gene" is synonymous to either a "naturally occurring sequence" including a coding sequence, or to a recombinant or synthetic sequence including a coding sequence. In the present text, a "gene" does also not necessarily contain regulatory elements, contrarily to the acceptation of this word which is often used in the scientific literature. Accordingly the gene according to the invention is any nucleotide sequence which comprises the Open Reading Frame of the naturally occurring sequence of *Plasmodium* or which comprises the same and further contains all or part of the regulatory sequences for expression of said naturally occurring sequence. According to the present definition, the gene is an isolated nucleic acid molecule, i.e, a nucleotide sequence which is not in its natural environment.

In the present text, the expression "family of genes" has the same meaning as in the scientific literature, *i.e.*, it designates a group of several genes which have a number of features or characteristics (structural or functional) in common.

According to the present application, a "MSP-3-c/d-like motif" is an amino acids sequence of 20 amino acids, which is identical to any of the sequences of SEQ ID Nos: 25 to 30, or which is obtained by shuffling of at least two of these sequences. For example, a sequence having the amino acids 1 to 5 of SEQ ID No:25, followed by the amino acids 6 to 12 of SEQ ID No:29 and the amino acids 13 to 20 of SEQ ID No:27, is a MSP-3-c/d-like motif. In other words, a "MSP-3-c/d-like motif" is an amino acids sequence of 20 amino acids, wherein the amino acids are chosen among the following :

Table 1

| a.a. position | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| a.a. | L | E<br>S | L<br>H<br>S<br>Q | I<br>V<br>L | K<br>N<br>Y<br>P | L<br>I<br>V | T<br>S<br>P | S<br>L | K<br>W<br>S | D | E<br>K<br>R<br>I | E<br>N | D<br>N<br>Q | I | I<br>V<br>S<br>P<br>A | K<br>D<br>N | H<br>E | N<br>S | E<br>D | D<br>Q |

Several of these aminoacids have a similar charge and will unlikely change the overall structure of the molecule or the recognition by antibodies, *e.g.*, valine, isoleucine, leucine.

Any amino acids sequence of 20 amino acids, which comprises the most conserved amino acids indicated above (*i.e.*, amino acids at positions 1, 2, 8, 10, 12, 14 and 17 to 20), and wherein the amino acid residues at other positions are different from the above one and which is recognized by an antibody directed against any of the MSP-3-c/d motifs of SEQ ID Nos: 25 to 30, will also be considered as a "MSP-3-c/d-like motif", according to the present invention. This latter functional property can be tested by any immunoassay such as those known by the skilled artisan and/or described below.

In the present text, a "MSP-3-b-like motif" designates an amino acids sequence of 11 to 14 amino acids, which is identical to any of the sequences of SEQ ID Nos: 17 to 24, or which is obtained by shuffling of at least two of these sequences. For example, a sequence having the amino acids 1 to 5 of SEQ ID No: 17, followed by the amino acids 6 to 11 of SEQ ID No:22, is a MSP-3-b-like motif. In other words, a "MSP-3-b-like motif" is an amino acids sequence of 11 to 14 amino acids, wherein the amino acids are chosen among the following, wherein "-" means "no amino acid" :

Table 2

| a.a. position | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| a.a. | I<br>Y<br>- | L<br>F<br>- | E<br>D<br>P<br>- | R<br>D<br>- | G<br>A<br>L<br>- | W<br>G<br>S<br>I<br>E | E<br>L<br>A | F<br>I<br>G<br>L<br>S | G<br>S<br>A | G | G<br>S<br>A | V<br>A<br>L<br>I<br>S | P<br>Y<br>L | E<br>F<br>- |

Several of these aminoacids have a similar charge and will unlikely change the overall structure of the molecule or the recognition by antibodies, *e.g.*, valine, isoleucine, leucine.

A subgroup of MSP-3-b-like motifs corresponds to the sequences of SEQ ID Noes:17, 18 and 22 and their combination, *i.e.*, the sequences of 11 amino-acids selected as follows:

Table 3

| a.a. position | 1 | 2 | 3 | 4 | 5 | 6 | 7 | 8 | 9 | 10 | 11 |
|---|---|---|---|---|---|---|---|---|---|---|---|
| a.a. | I<br>Y | L<br>F | G<br>A | W | E | F<br>I | G | G | G | V<br>A | P |

Any amino acids sequence of 11 to 14 amino acids, which comprises the most conserved amino acids indicated above (*i.e.*, the amino acids indicated in table 3, which correspond to particular amino acids at positions 1, 2, and 5 to 13 of Table 2), and wherein the amino acid residues at other positions are different from the above one, and which is recognized by an antibody directed against any of the MSP-3-b motifs of SEQ ID Nos: 17 to 24, will also be considered as a "MSP-3-b-like motif", according to the present invention. This latter functional property can be tested by any immunoassay such as those known by the skilled artisan and/or described below.

In what follows, reference is sometimes made to a gene or a protein which is an "homologue" of a particular gene or protein the sequence of which is disclosed. This word herein designates close sequences in different *Plasmodium* strains (in particular, *P. falciparum* strains), *i.e.*, sequences exhibiting at least 70%, and preferably at least 90% of sequence identity, with the sequence of reference.

A "conservative Substitution means, in an amino acid sequence, a substitution of one amino acid residue by another one which has similar properties having regard to hydrophobicity and/or steric hindrance, so that the tertiary structure of the polypeptide is not dramatically changed. For example, replacing a guanine by an alanine or *vice-versa,* is a conservative substitution. Valine, leucine and isoleucine are also amino acids that can be conservatively substituted by each other. Other groups of conservative substitution are, without being limitative, (D, E), (K, R), (N, Q), and (F, W, Y). A variant of a polypeptide, obtained by conservative substitution of at least one amino acid of said polypeptide, will be designated here as a "conservative variant" of said polypeptide. '

Further definitions are provided in the following text, when necessary.

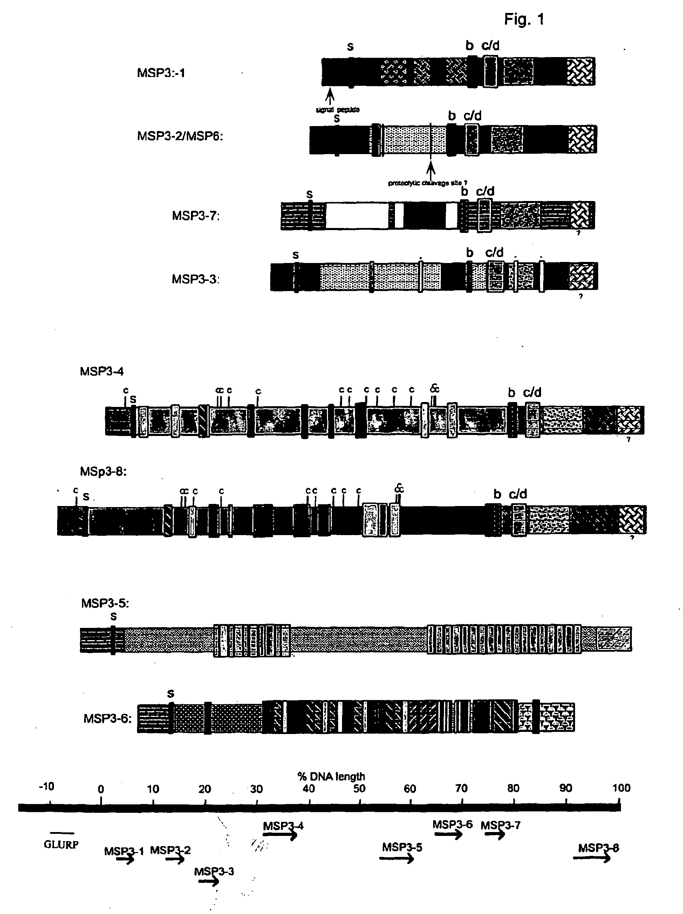

The inventors herein describe a group of 9 genes, 6 of which have never been described, and which are all clustered in the same region of chromosome 10. This chromosome indeed contains a series of 9 open reading frames, separated by non coding regions, and comprises in a row (5' - 3') genes encoding proteins denominated first GLURP, followed at 1300 base-pairs by MSP-3 (now denominated MSP-3-1) followed by 7 other genes denominated MSP-3-2, MSP-3-3, MSP-3-4, MSP-3-5, MSP-3-6, MSP-3-7, MSP-3-8. This organisation is shown in Figure 1.

Besides being clustered in the same chromosomal region, those 9 genes have outstanding features, that indicate that they are privileged products for vaccine development against *P. falciparum* blood stage infection:

It was shown that all 9 genes were expressed simultaneously in all parasites studied, *i.e.*, that the corresponding proteins could be detected in *P. falciparum* erythrocytic stages and are all located on the merozoite surface. This was previously shown for GLURP, MSP-3-1 and MSP-3-2 (designated "MSP6" by (Trucco, Fernandez-Reyes et al. 2001) and has been further demonstrated for the remaining by the construction of particular sequences in the N-terminus of those genes which are unique for each of them, which do not share cross-reactive epitopes, and which corresponding antibodies all react with the merozoite surface. Moreover, transcription was demonstrated by RT-PCR with unique primers specific of each.

Moreover, the 8 MSP-3-like genes share the same general gene organisation, which is illustrated in Figure 1, with an initial N-terminus "signature" of 4 aminoacids (indicated "s" in Figure 1) identical in each of them and identical to similar MSP-3 homologous proteins described in *Plasmodium vivax* and *Plasmodium Knowlesi.*

A first object disclosed in the application is hence a family (or group) of isolated genes which have the following properties :

- they are located on chromosome 10 of *Plasmodium falciparum;*
- they are highly conserved in *Plasmodium falciparum* strains;
- they are expressed in *Plasmodium falciparum* at the erythrocytic stages;
- they encode proteins which have a NLRN or NLRK signature at their N-terminal extremity and which are located at the merozoite surface,

wherein said family comprises at least 3 genes.

The NLRN or NLRK signature is most often followed by A or G, in the proteins of the MSP-3 family according to the invention.

The genes of the family preferably also share the same general organization, as shown in Figure 1.

An example of such a family is the whole MSP-3-like family, comprising the genes of sequences SEQ ID Nos: 1, 3, 5, 7, 9, 11, 13, and 15. Any group of at least 3 genes selected amongst these genes is also considered as a gene family.

Except from the N-terminal signature mentioned above, the remaining of the N-terminus part is highly variable from one gene product to the other, whereas, in contrast, the C-terminus is identical in its organisation for all genes, except 2 (MSP-3-5 and MSP-3-6) including the "b" epitope-like stretch ("b"), the "c/d" epitope-like ("c/d"), the Glutamic-rich region, and at the extreme C-term a leucine zipper.

Particular families of genes are hence families as described above, wherein said genes further have the following property : they encode proteins which have a MSP-3-b-like motif and/or a MSP-3-c/d-like motif. An example of such a family is the family encompassing the genes of sequences SEQ ID Nos: 1, 3, 5, 7, 13, and 15, or any group of at least 3 genes selected amongst these sequences.

All 7 proteins (GLURP + the 6 homologous MSP-3-like molecules) elicit antibodies in individual exposed to malaria.

For those gene products in which it has been investigated, particularly GLURP, MSP-3-1 and MSP-3-2, the immune responses elicited are associated with clinical protection against malaria attacks under field conditions. This association is highly statistical significant, particularly with antibodies made of the IgG3 isotype, and was confirmed in three settings, Dielmo and Ndiop in Africa, Oo-Do in Burma. For reasons of homology described below, it is extremely likely that the same finding will be made for the remaining 5 genes.

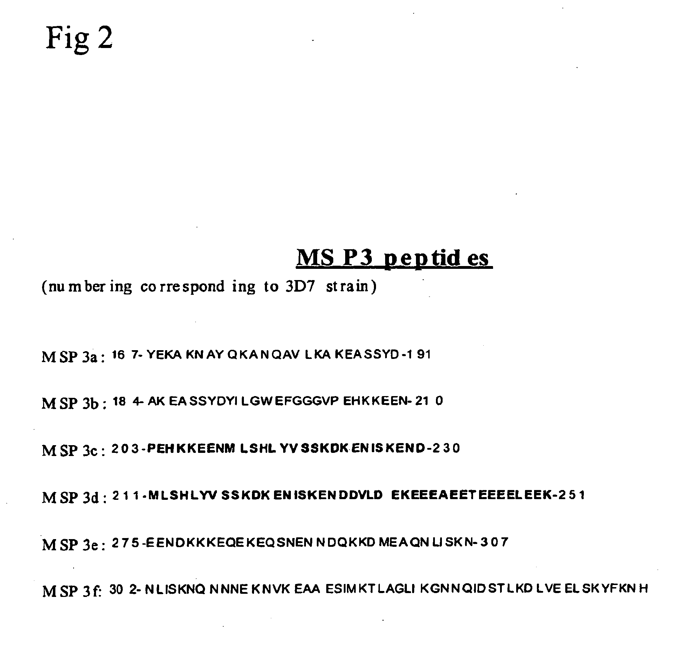

In the case of GLURP, MSP-3-1 and MSP-3-2, the regions targeted by antibodies associated with protection are the non repeat region R0 of GLURP and the C-terminus non repeated region of MSP- and MSP-3-2. The various peptides derived from MSP-3-1 are shown in Fig 2. Protection was associated with antibodies to peptides MSP-3-b, c and d.

Antibodies to the 7 gene products are all effective at mediating *P. falciparum* blood stage killing, in the monocyte-dependent, antibody-mediated ADCI mechanism, under *in vitro* conditions. These results, described in Example 1, show that antibodies to each of those regions are equally effective at achieving *P. falciparum* erythrocytic stage growth inhibition under *in vitro* conditions.

Preferred family of genes therefore further have the following property : antibodies to the products of said genes mediate *Plasmodium falciparum* blood stage killing, in the monocyte-dependent, antibody-mediated ADCI mechanism, under *in vitro* conditions.

According two another preferred embodiment, a family of genes therefore further have the following property : antibodies to the products of said genes mediate *Plasmodium falciparum* growth inhibition in mice infected by *P. fafciparum.*

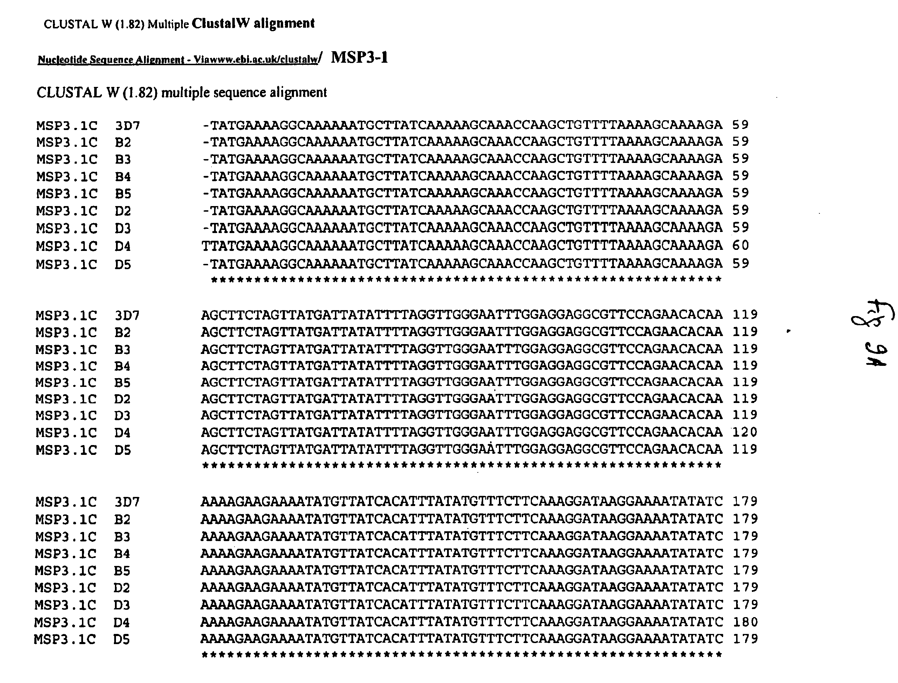

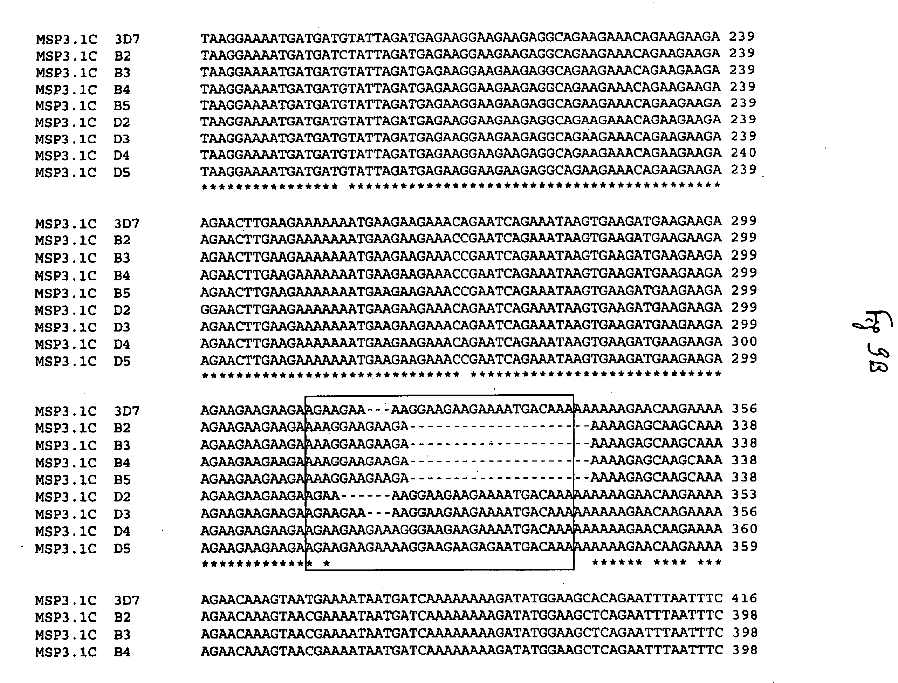

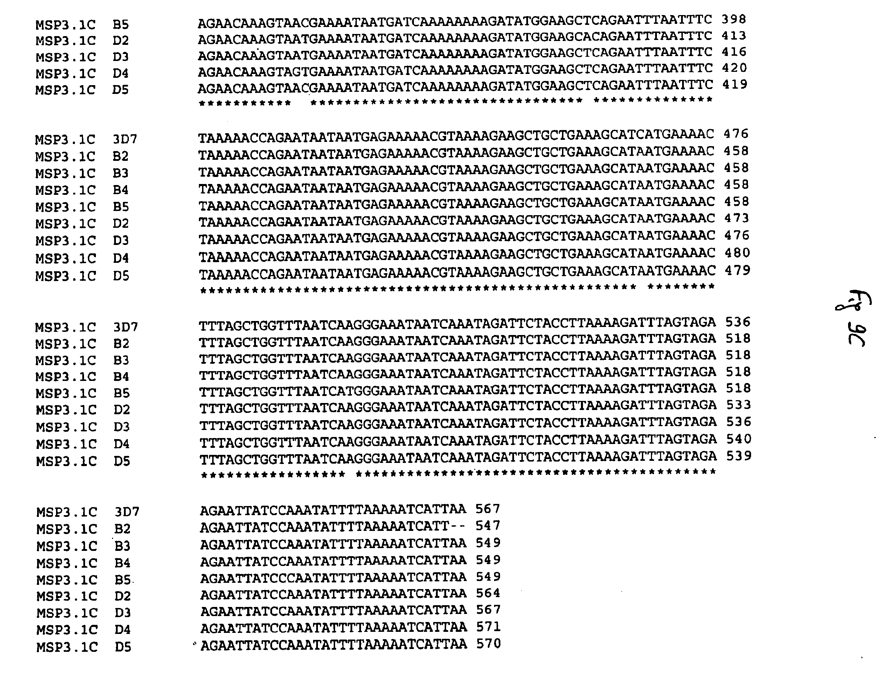

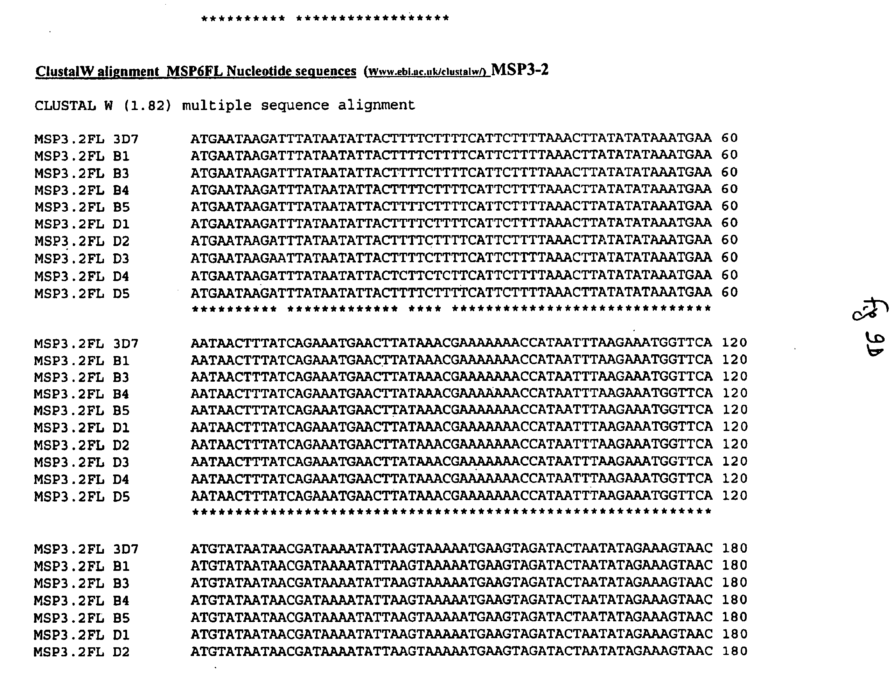

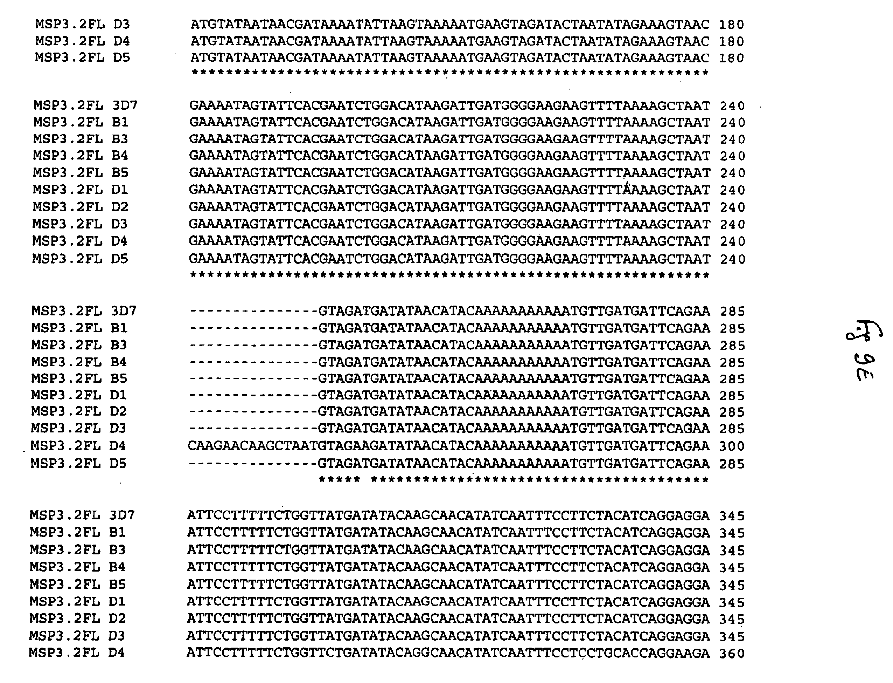

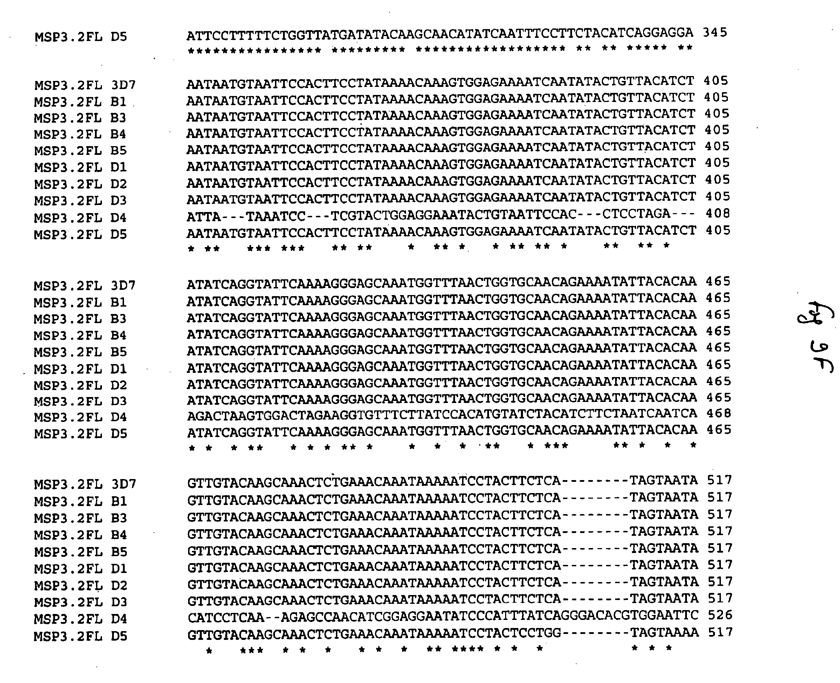

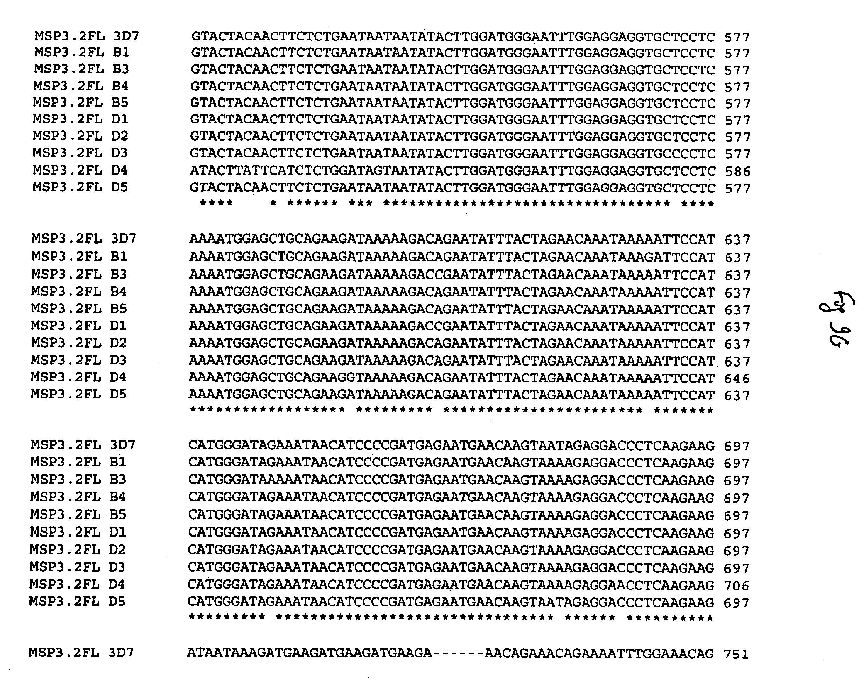

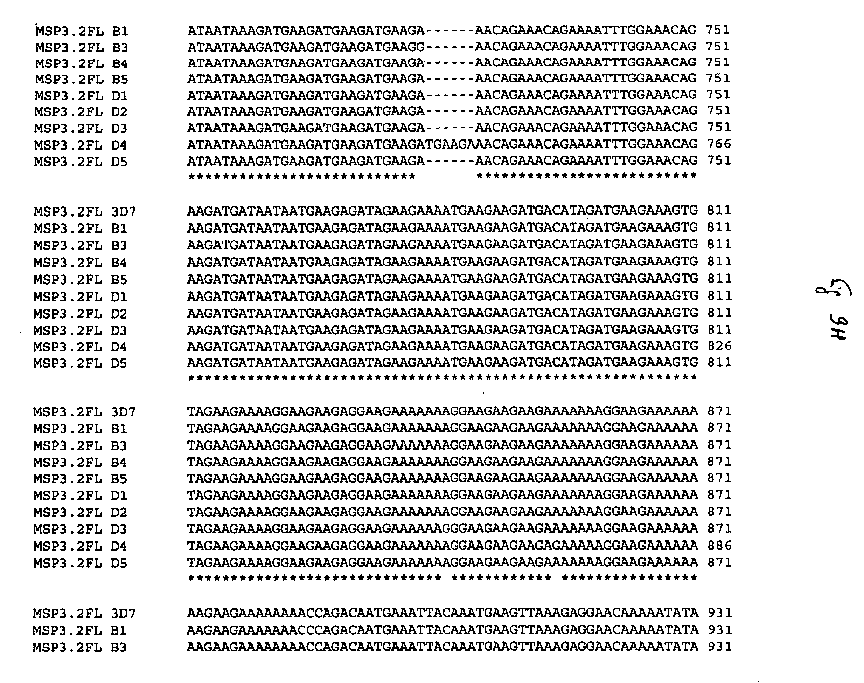

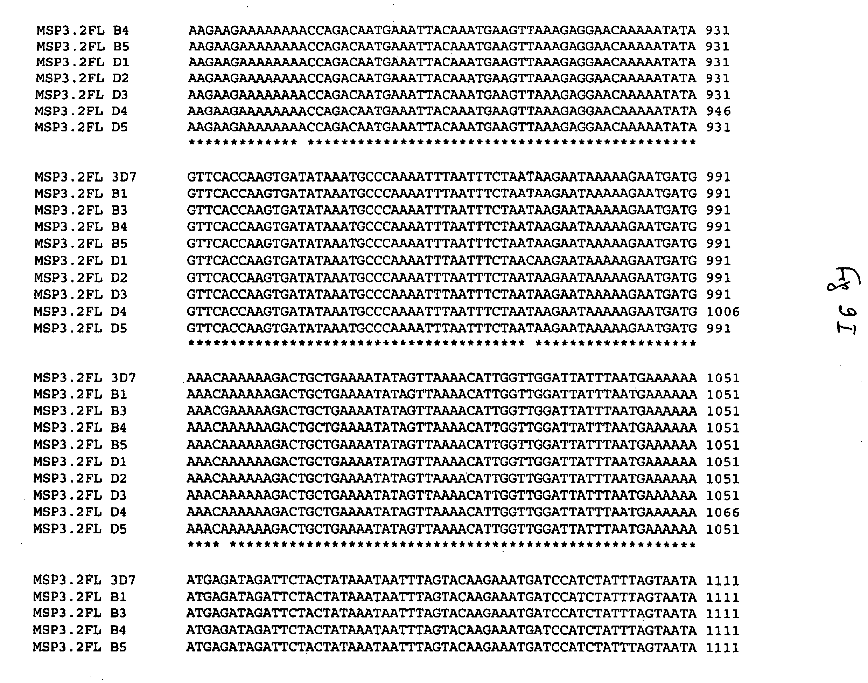

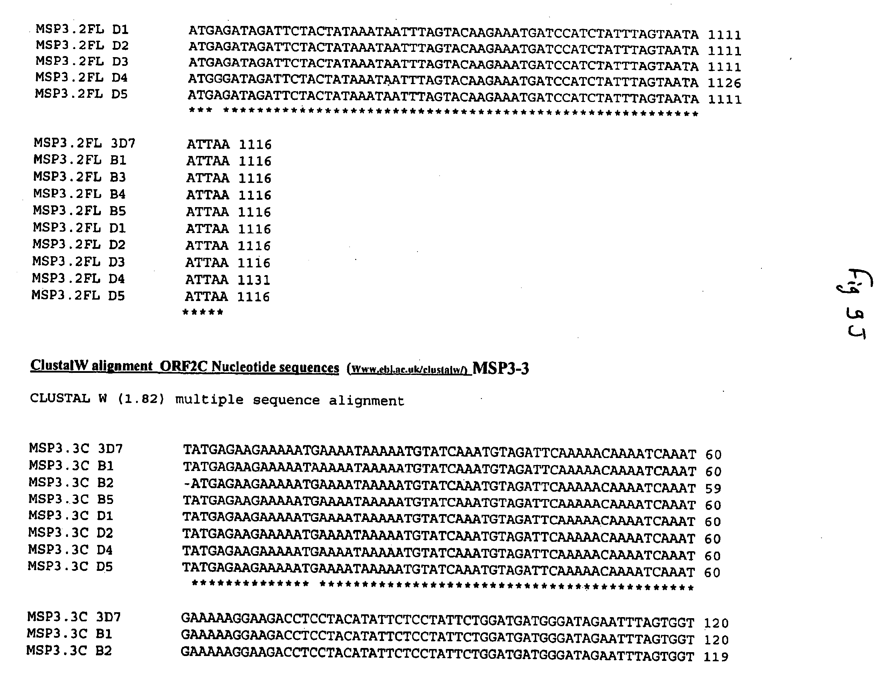

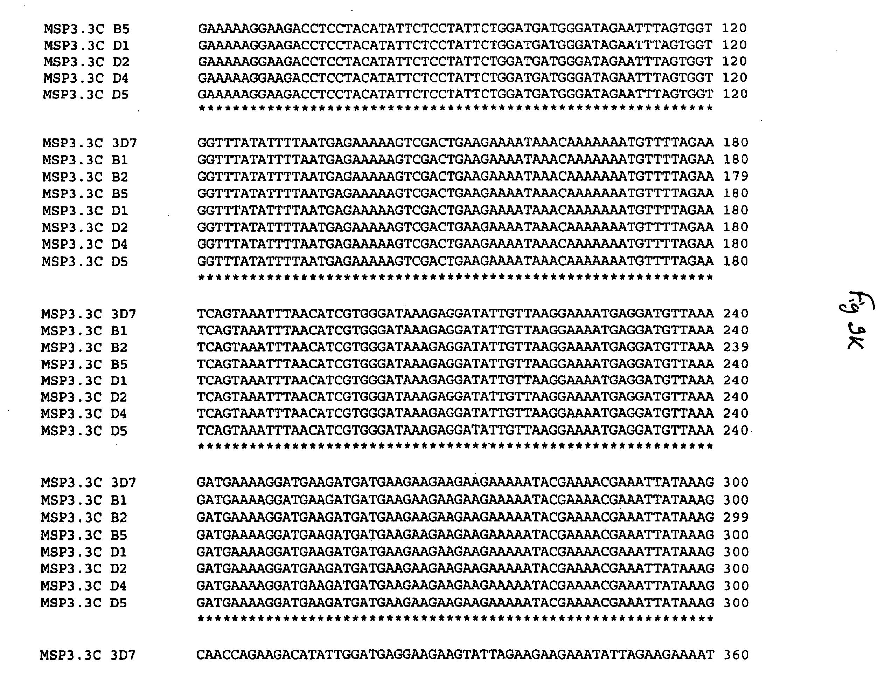

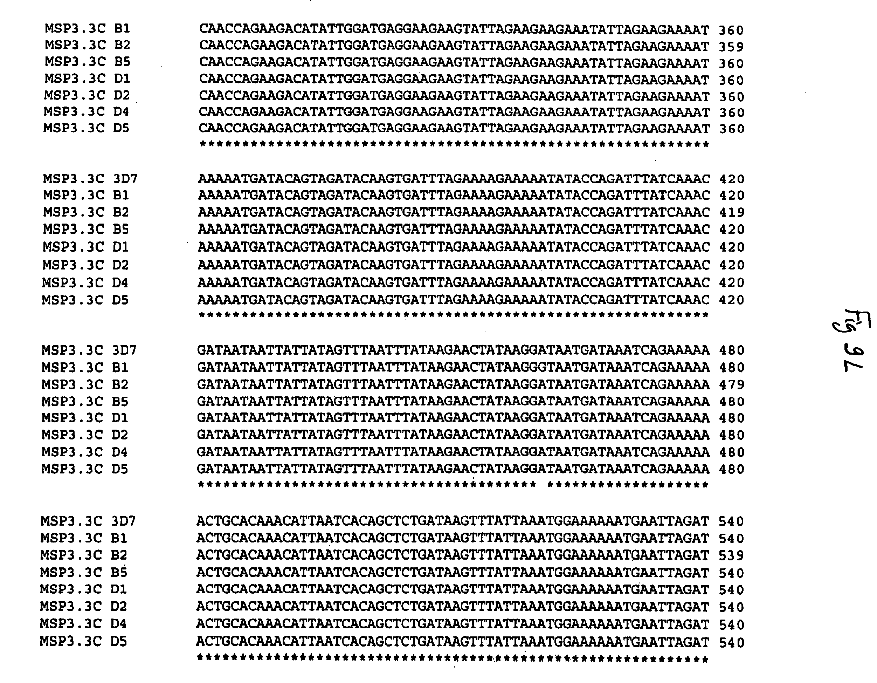

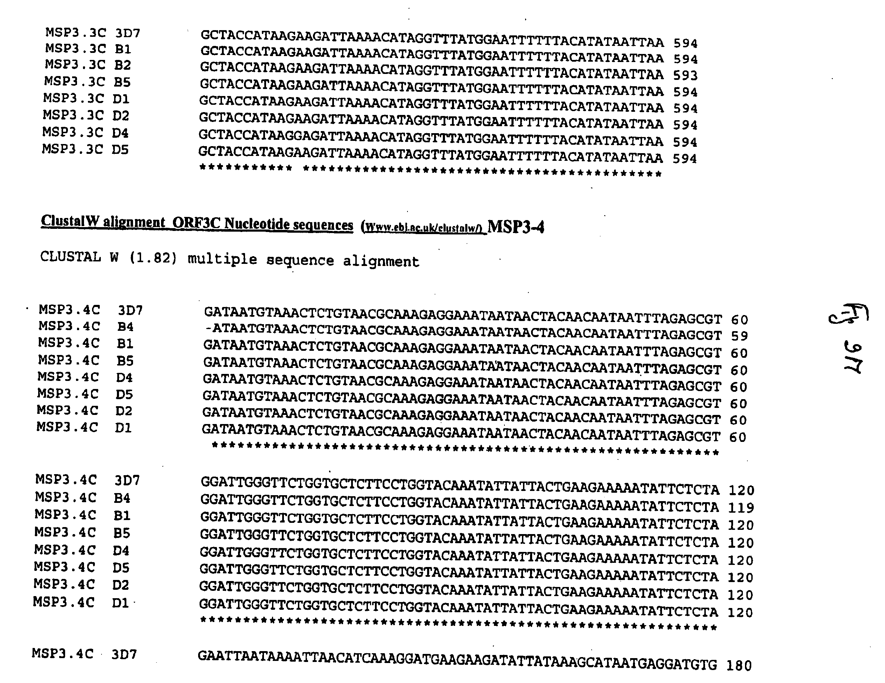

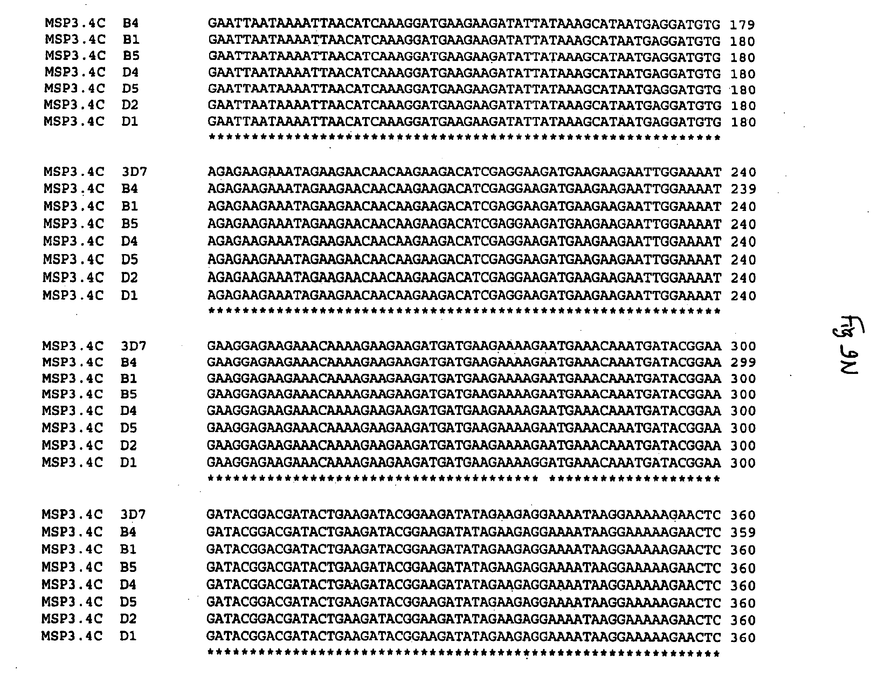

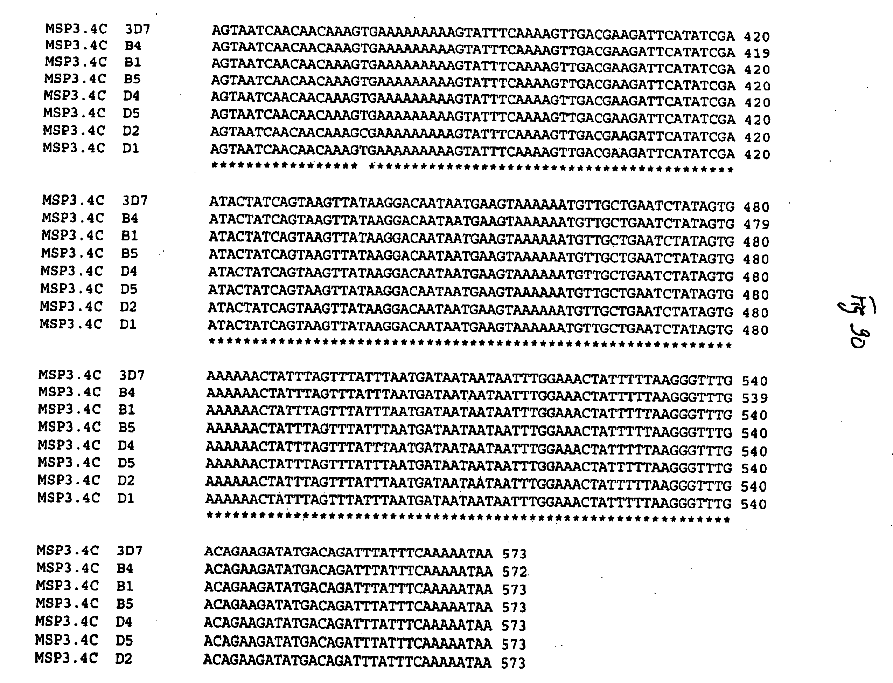

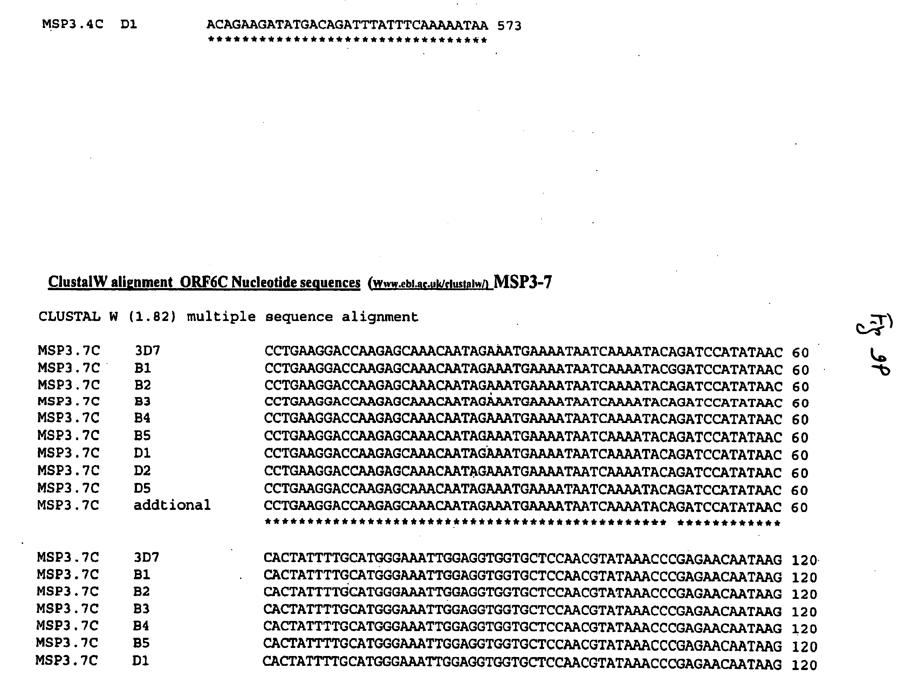

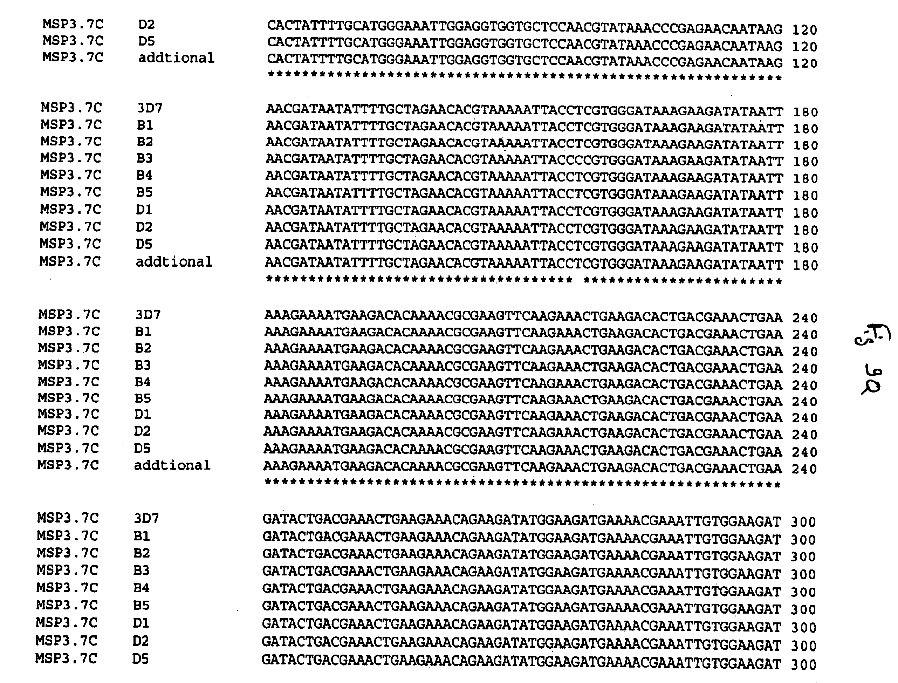

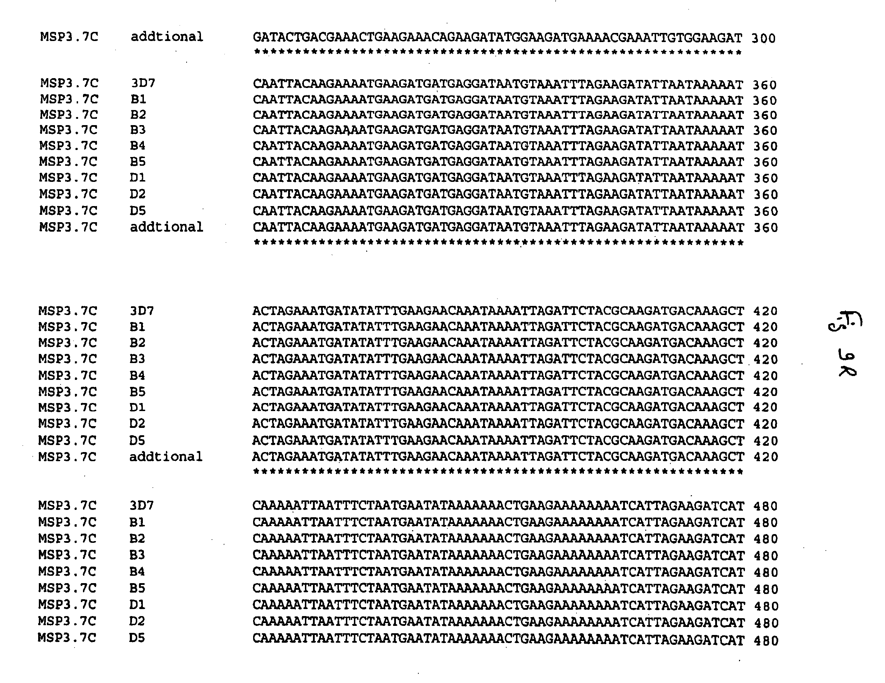

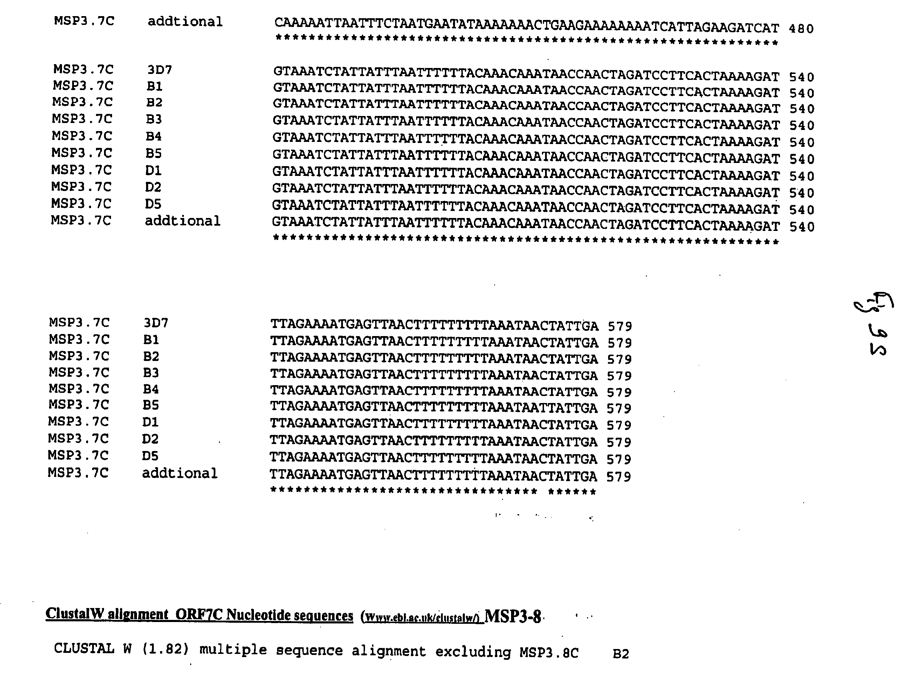

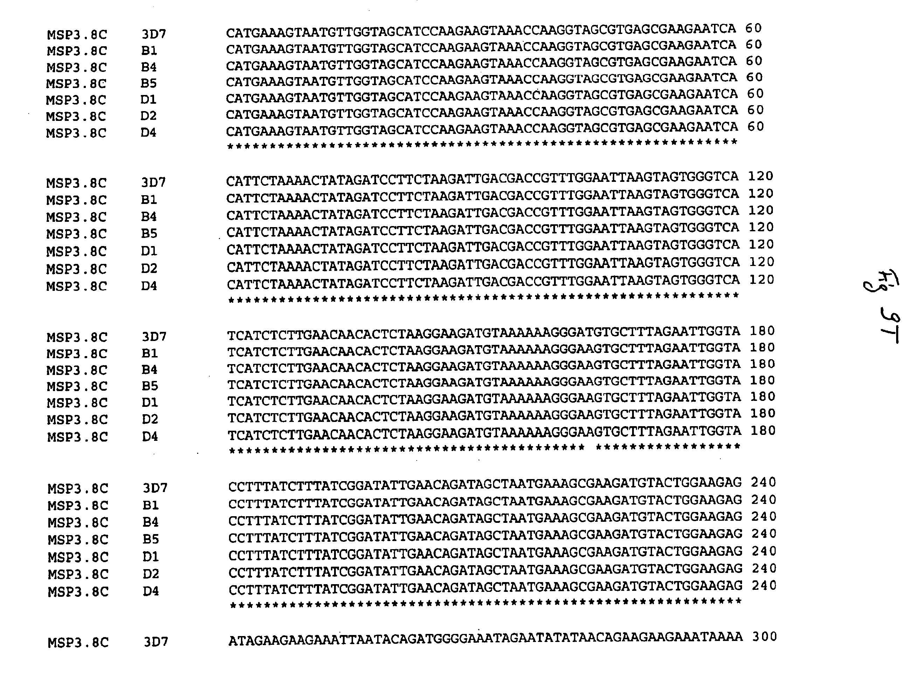

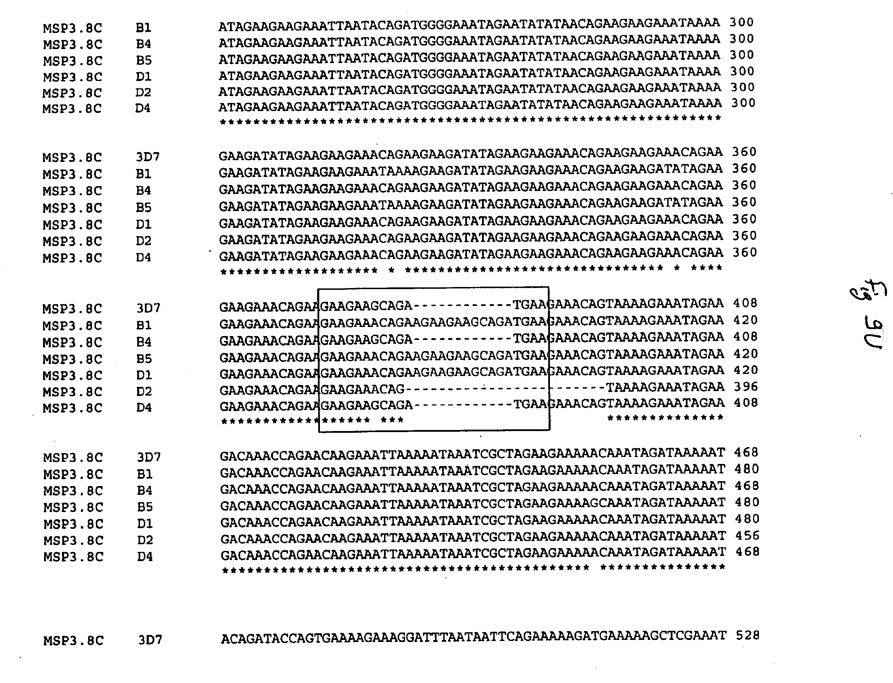

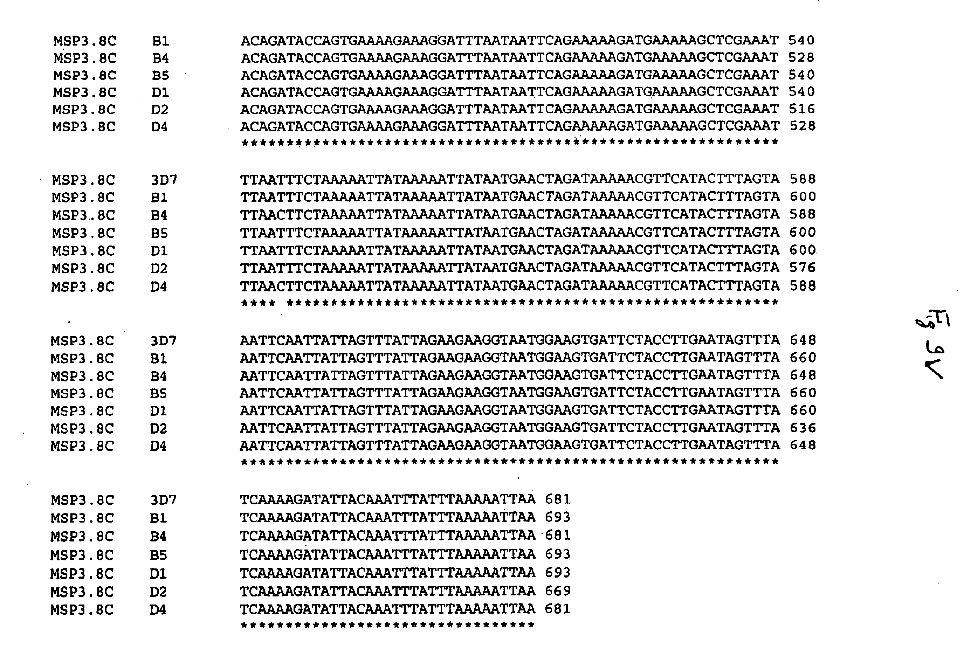

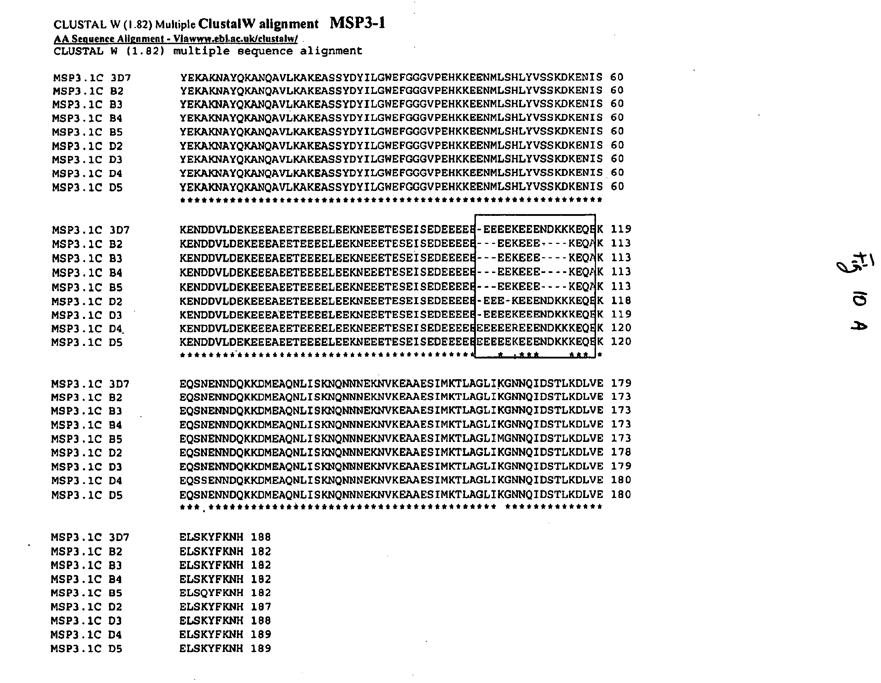

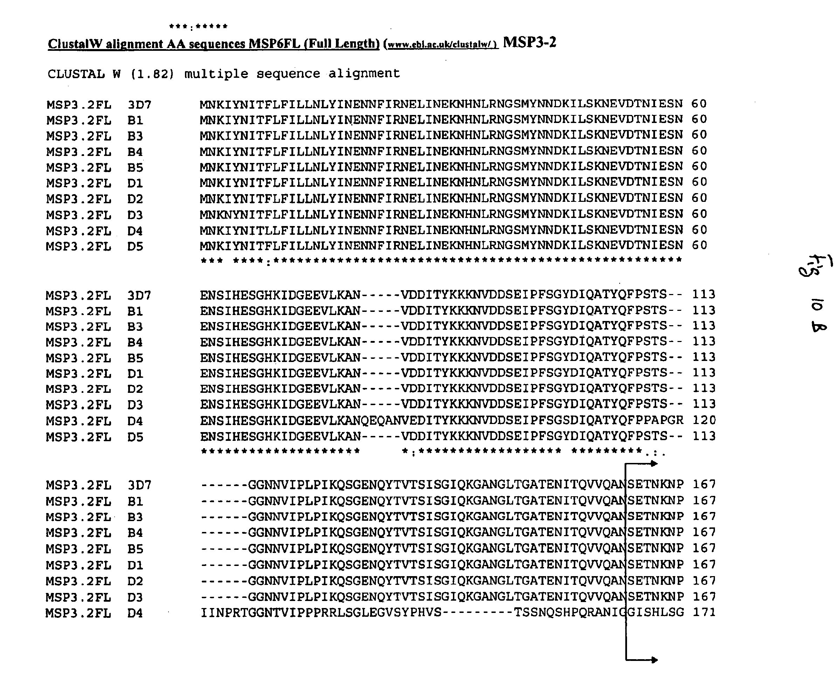

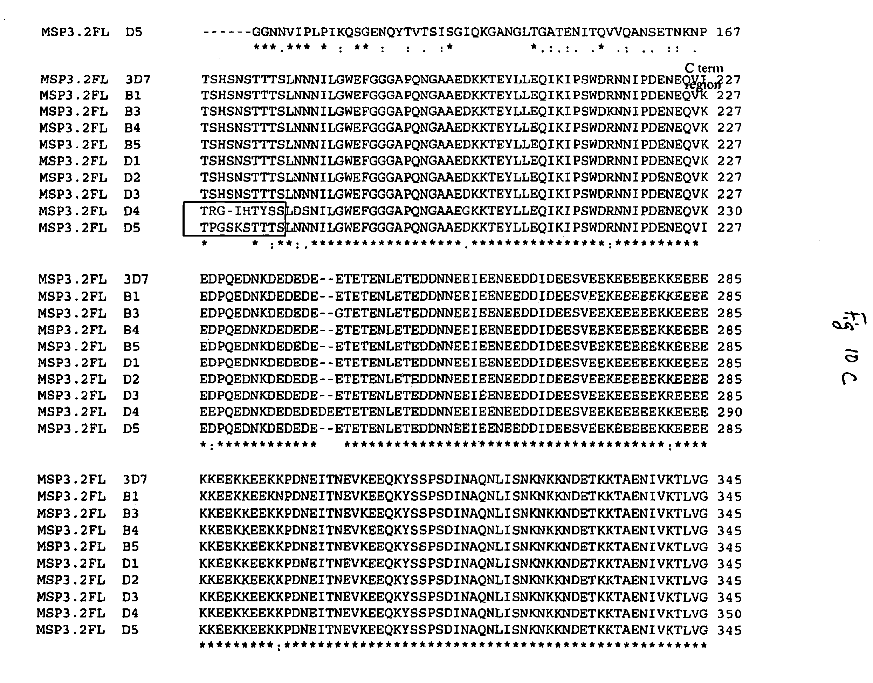

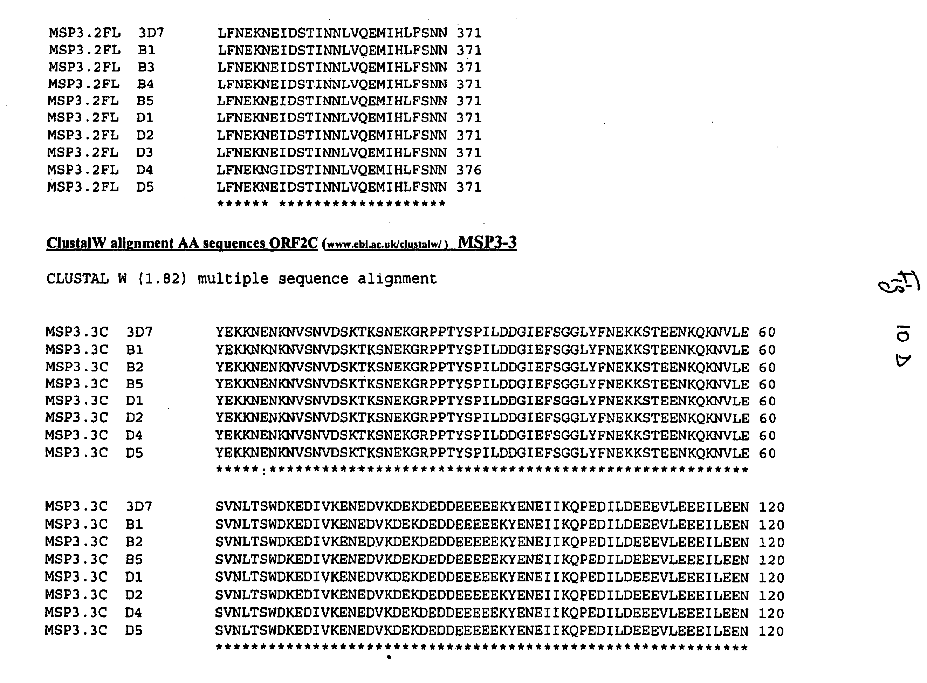

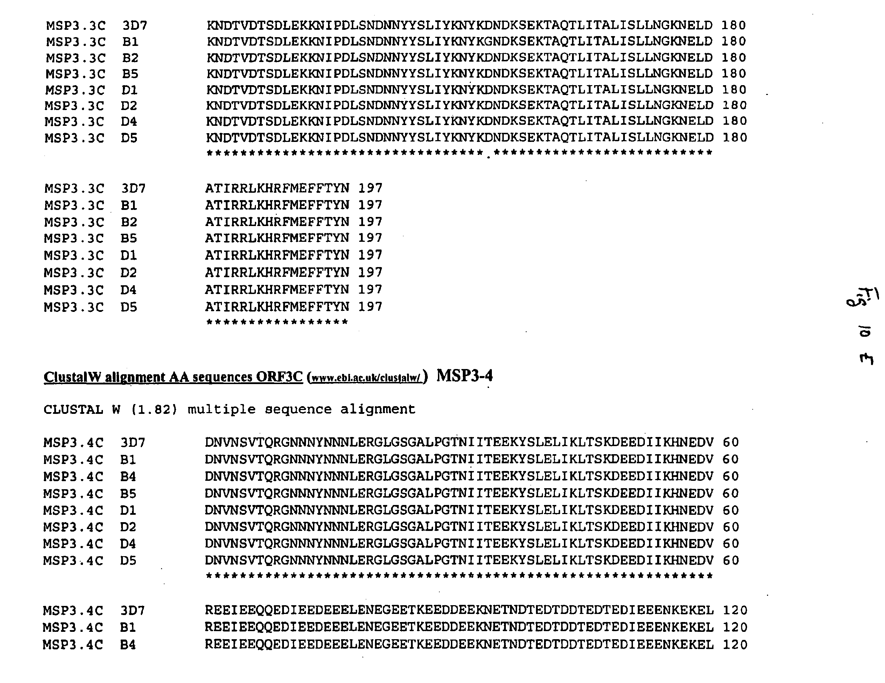

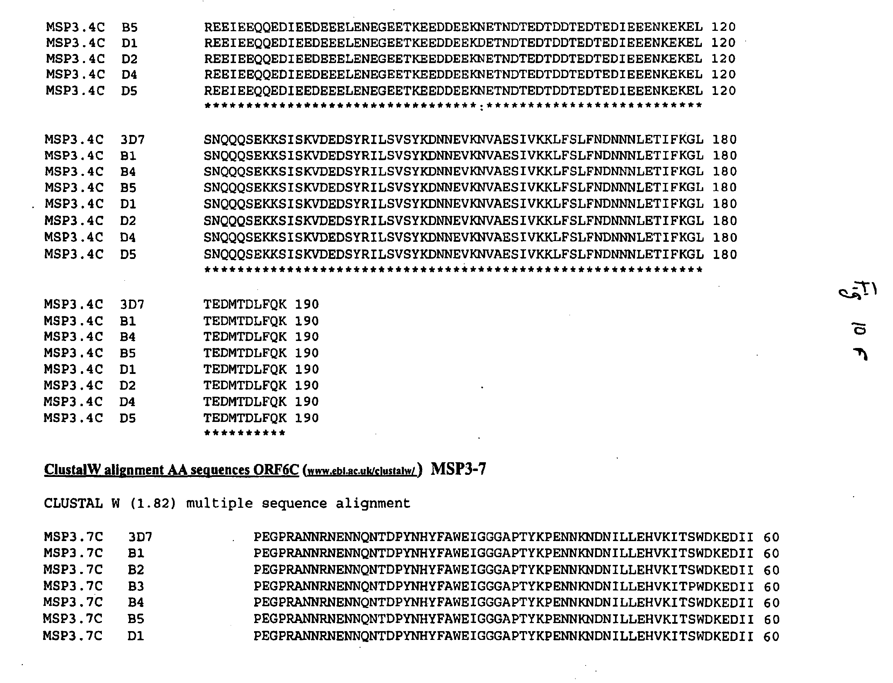

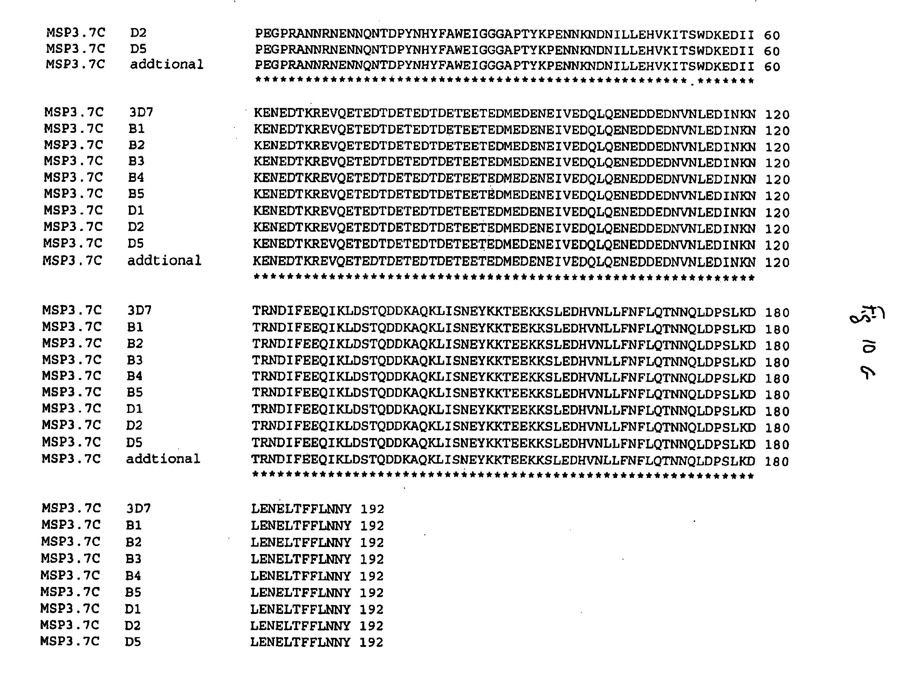

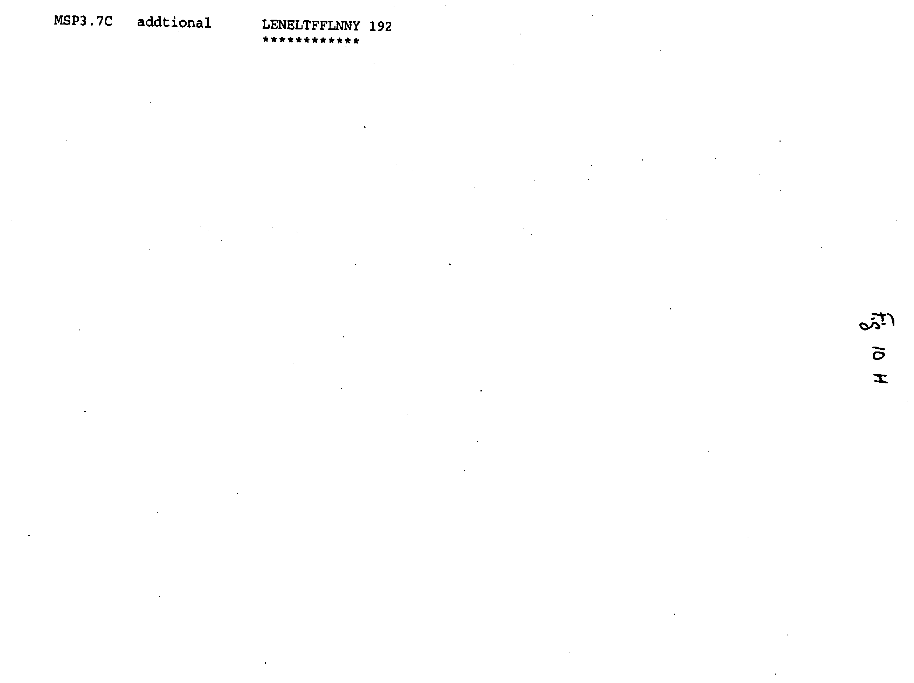

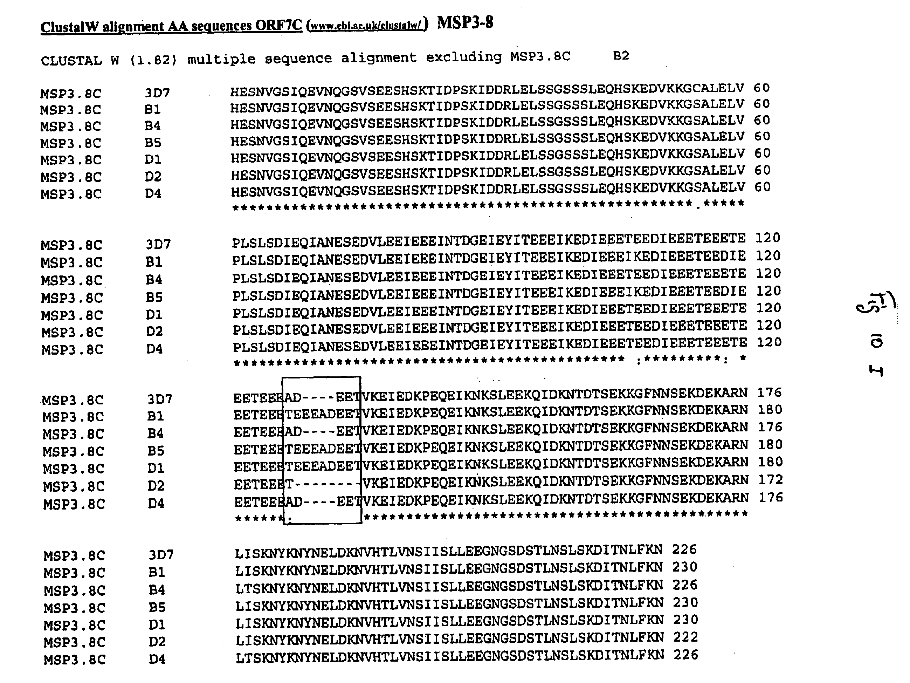

The inventors have also demonstrated that there is a very unusual high degree of sequence conservation of each of the 7 genes, among various *P. falciparum* isolates. This had been previously shown for GLURP and led to choose the R0 non-repetitive region which has the highest conservation among various isolates, yet has some aminoacid substitutions. This was also shown for MSP1-3-1 whose sequence was found to be outstandingly conserved among 111 isolates for the region covering peptides MSP-3-a, b, c and d, *i.e.*, the region used for immunisation of volunteers, where no single aminoacid substitution and therefore no aminoacid change was found whatsoever. This was recently further confirmed for the remaining of the C-terminus of MSP-3-1 and the whole C-term conserved region of MSP-3-2, MSP-3-3, MSP-3-4, MSP-3-7, and MSP-3-8 (Figures 9 and 10). This remarkable degree of sequence conservation of this gene family is in marked contrast with the relatively large polymorphism observed for most of the other vaccine candidates currently studied, and is obviously an important criterium that strengthens the potential of this gene family for vaccine development.

Gene families as described above, comprising at least 3 genes selected amongst the genes of sequences SEQ ID Nos: 1, 3, 5, 7, 9, 11, 13, and 15, or their homologues in *Plasmodium,* particularly *Plasmodium falciparum* strains are therefore also preferred gene families.

Another aspect disclosed in the application is an isolated *Plasmodium falciparum* gene which has the sequence of SEQ ID No:5, 7, 13 or 15, or an isolated gene corresponding to an homologue of a *Plasmodium falciparum* gene of sequence of SEQ ID No:5, 7, 9, 11, 13 or 15 in a *Plasmodium* strain.

These genes, which are non described MSP-3-like genes, can be very useful for the skilled artisan in a number of applications in the research, diagnostic and vaccinations fields, for the reasons described above and hereafter. In particular, they can be used to produce recombinant MSP-3- like proteins. Accordingly, recombinant proteins of SEQ ID Nos: 6, 8, 10, 12, 14 and 16, are also disclosed, as well as any recombinant protein having the sequence of a protein which is an homologue of a protein of SEQ ID Nos: 6, 8, 10, 12, 14 or 16 in a *Plasmodium* strain different from the 3D7 strain.

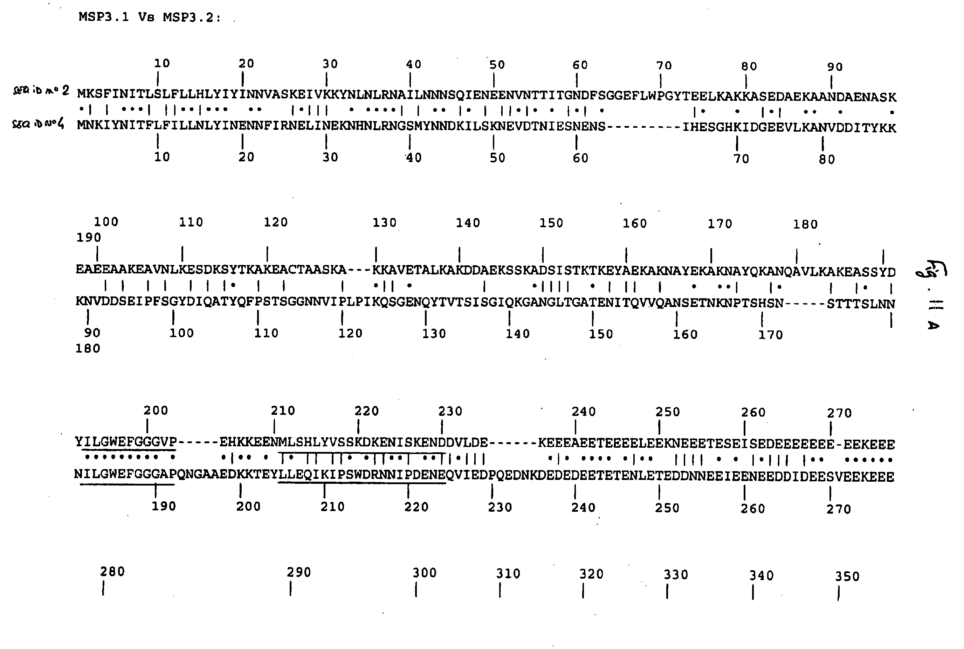

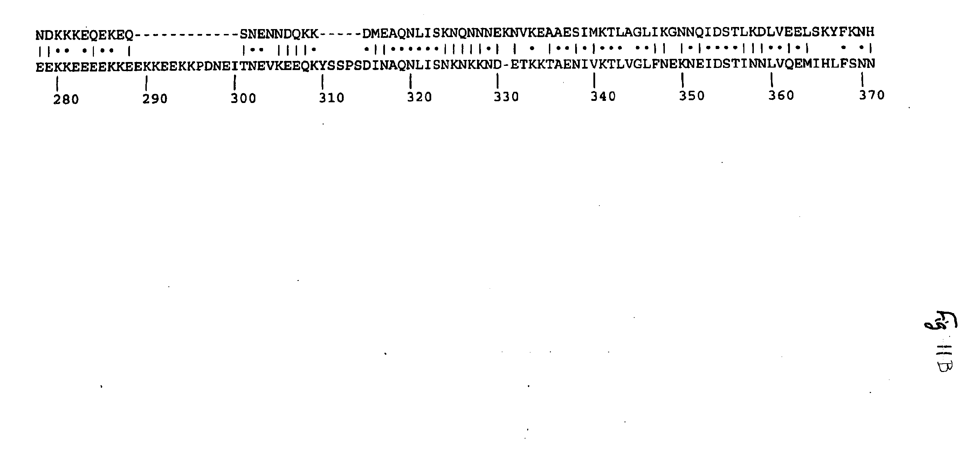

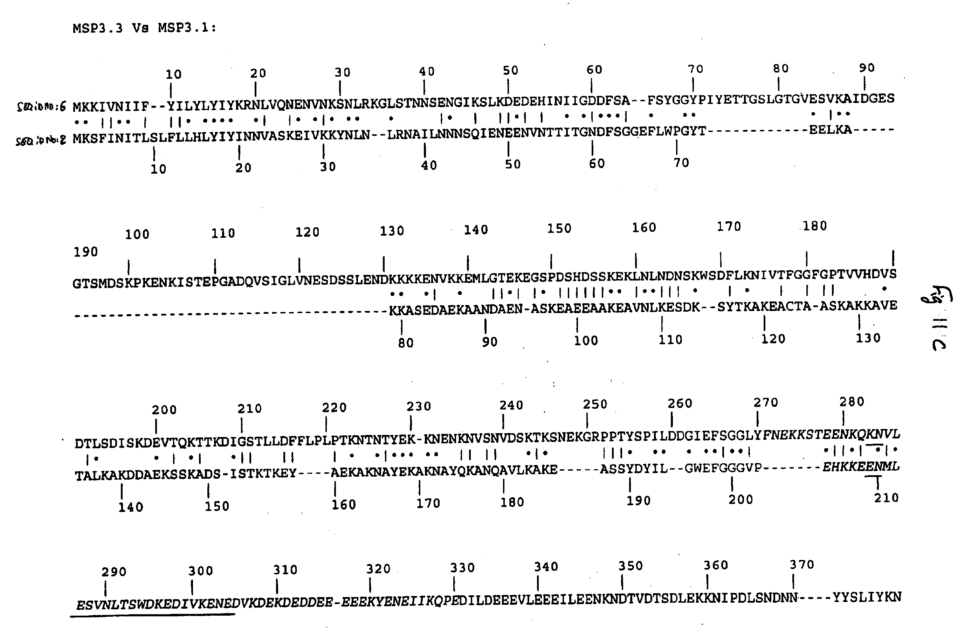

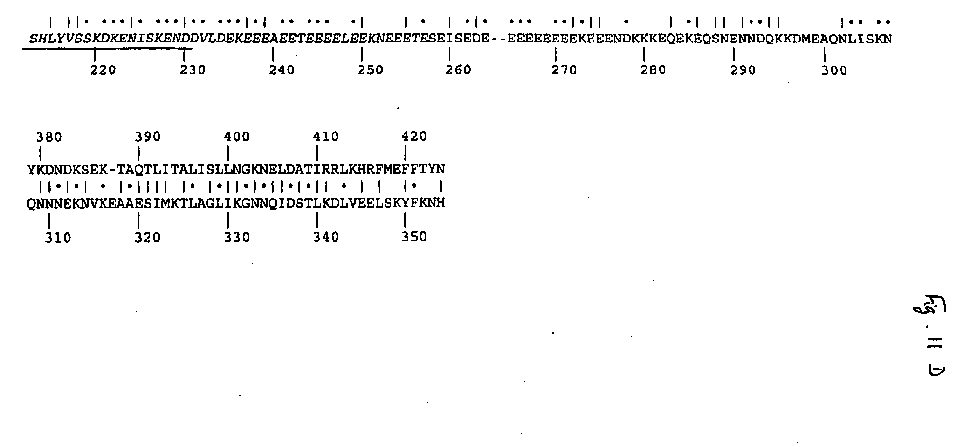

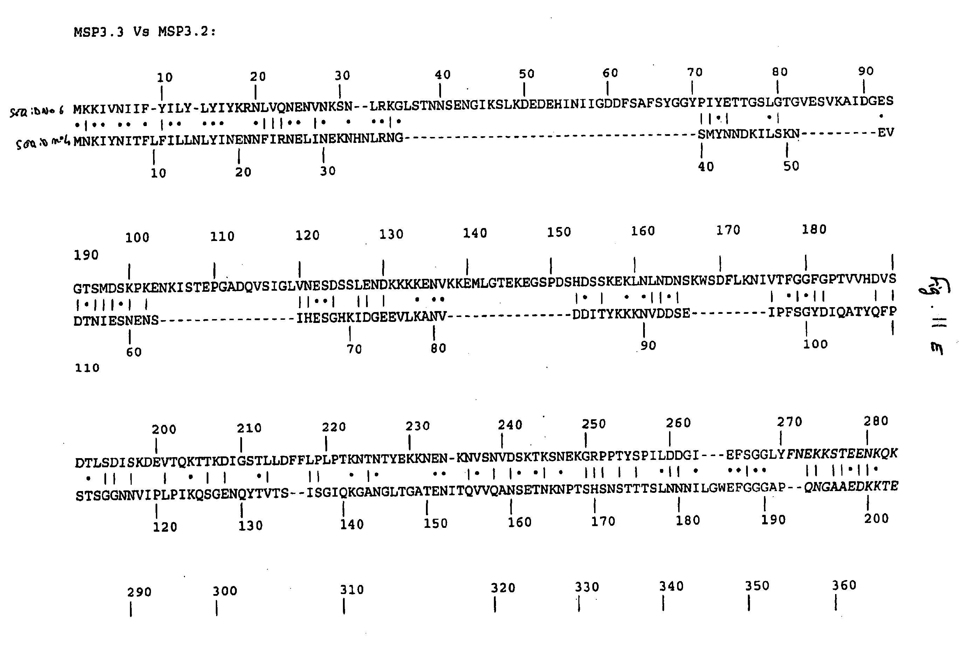

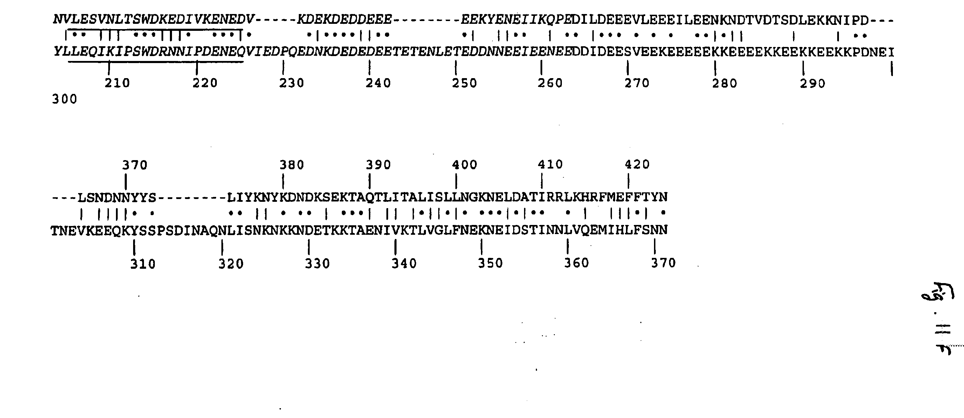

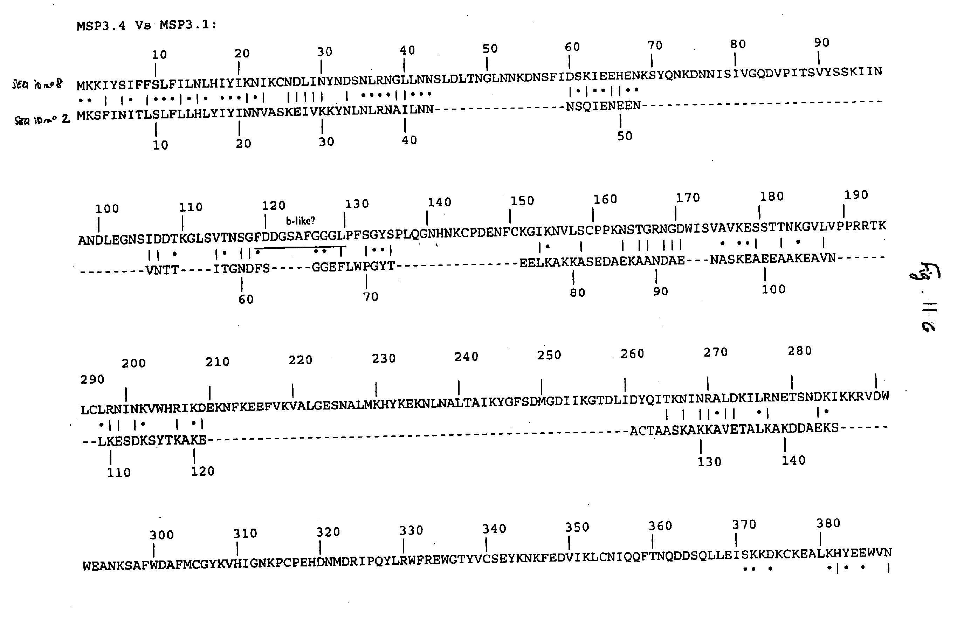

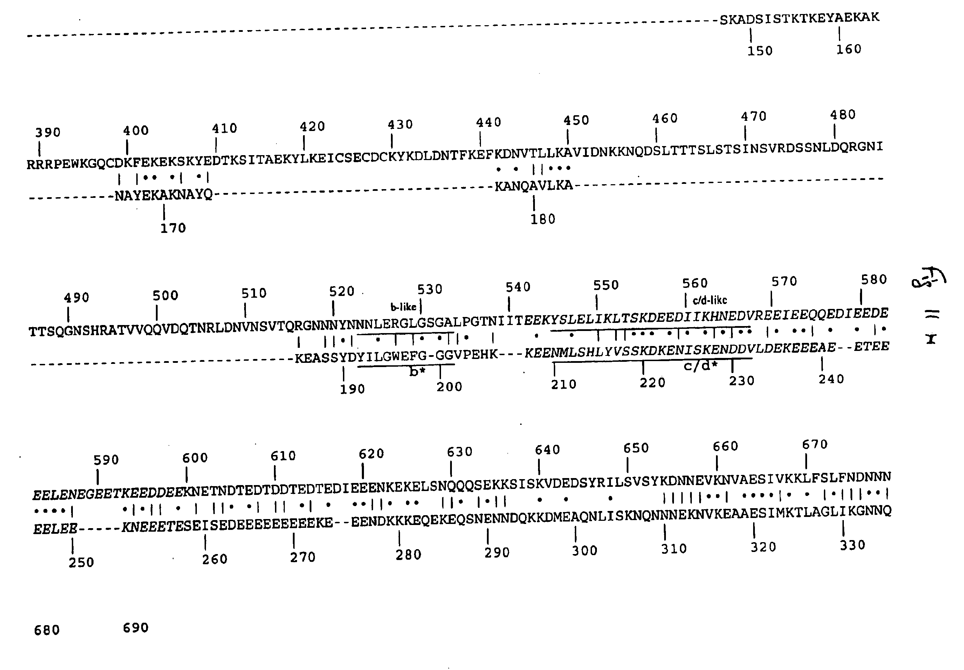

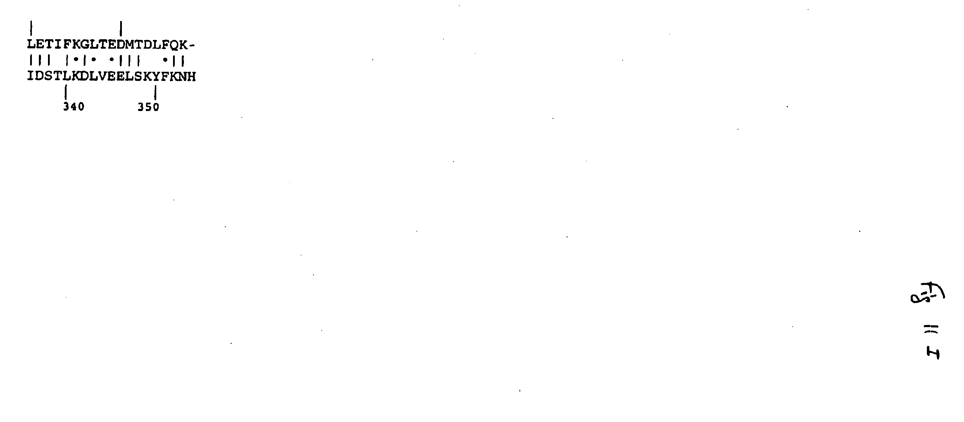

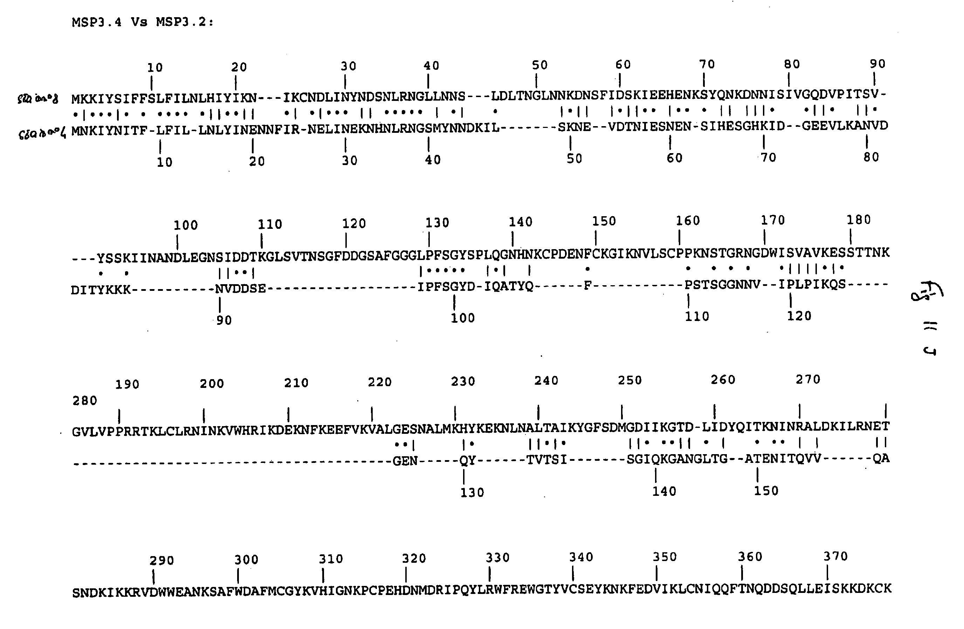

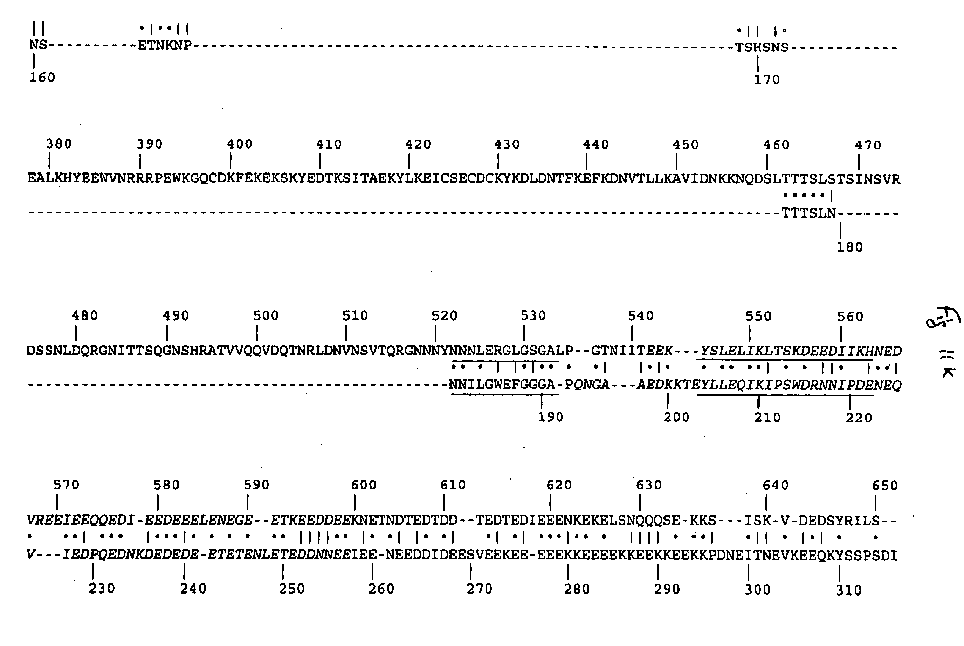

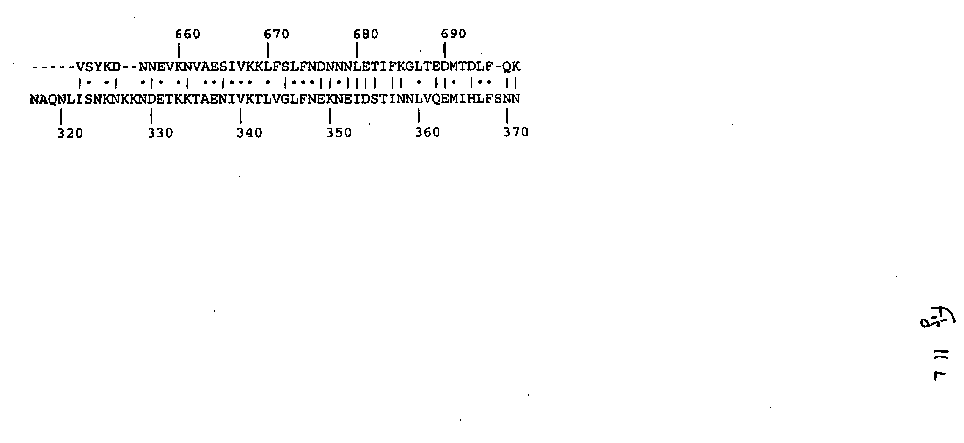

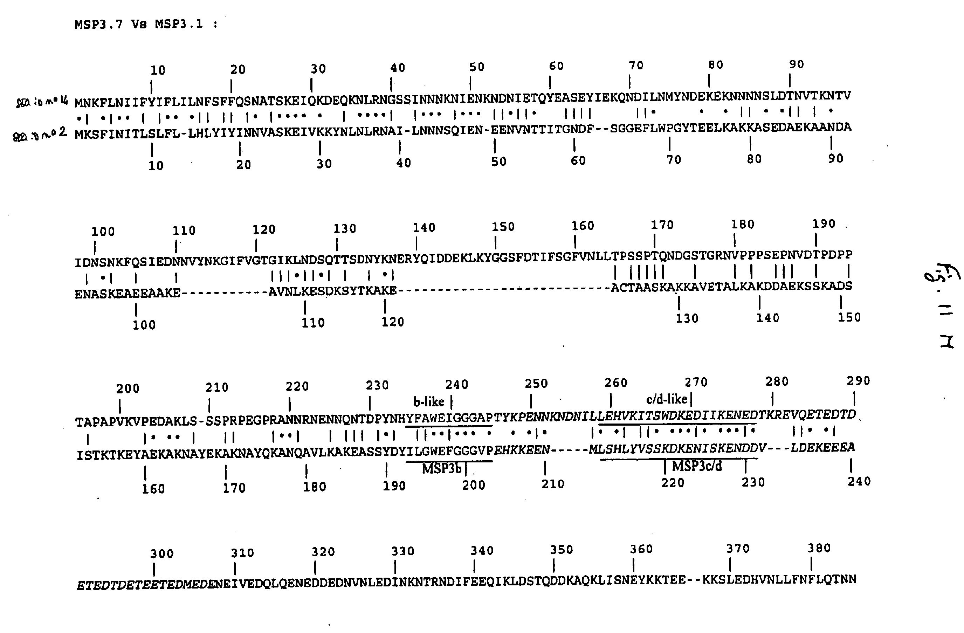

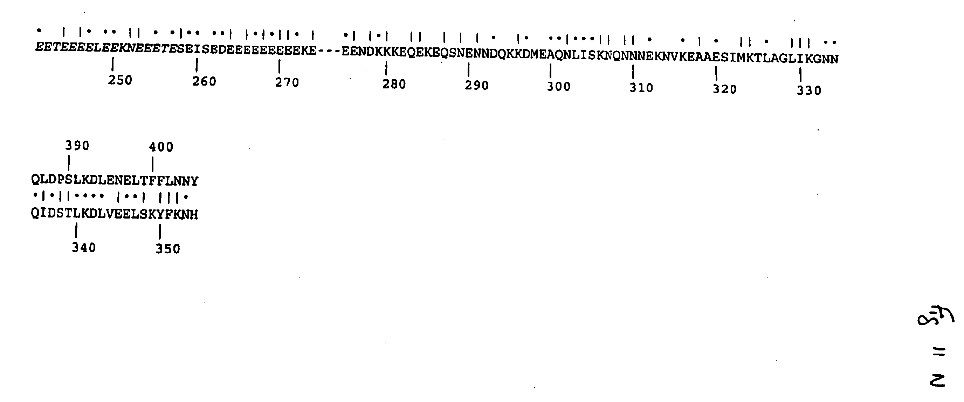

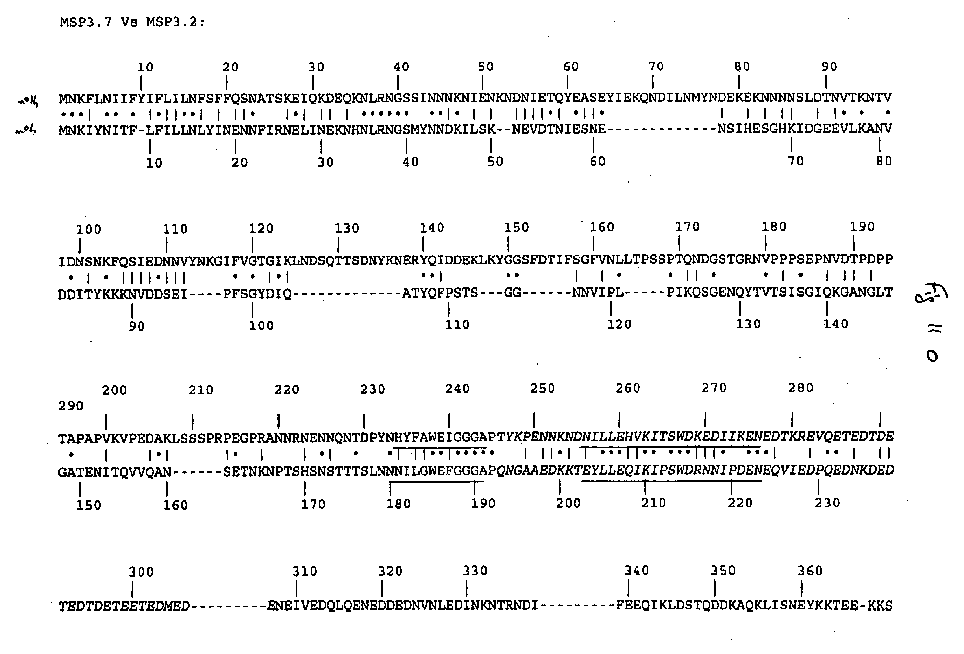

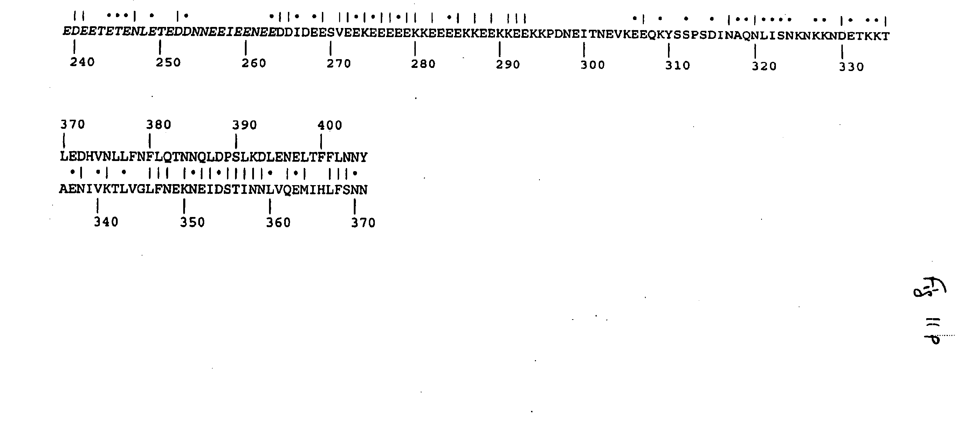

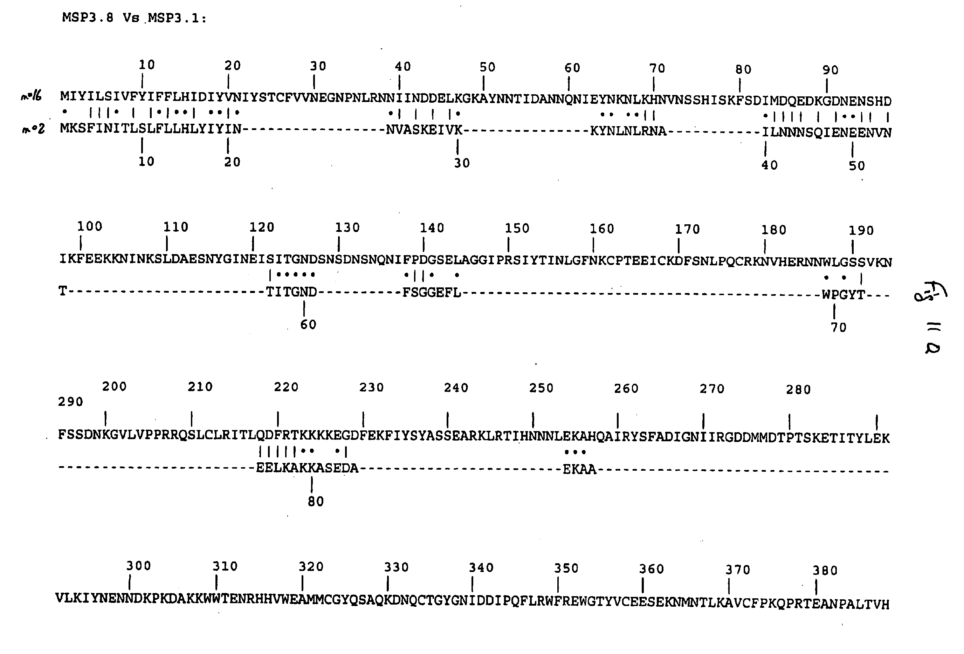

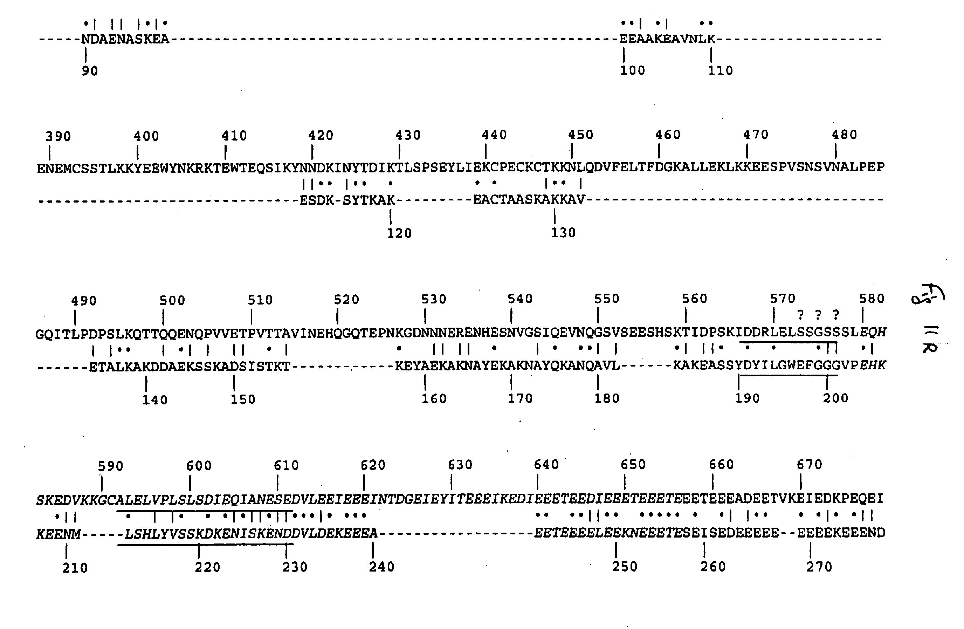

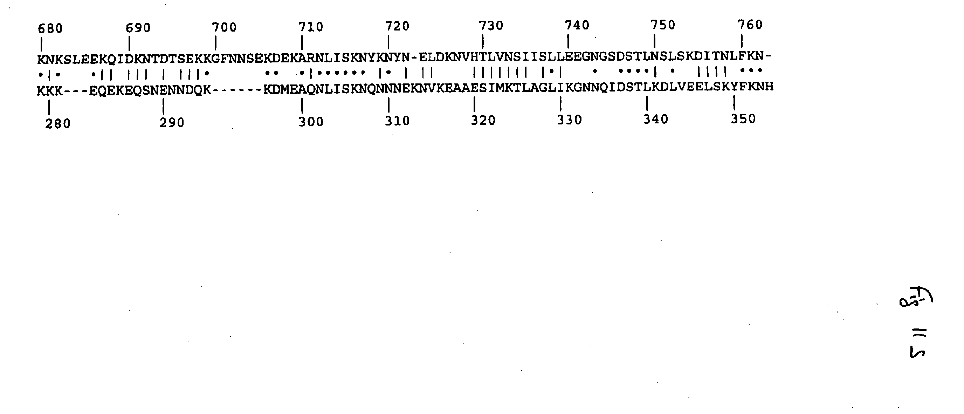

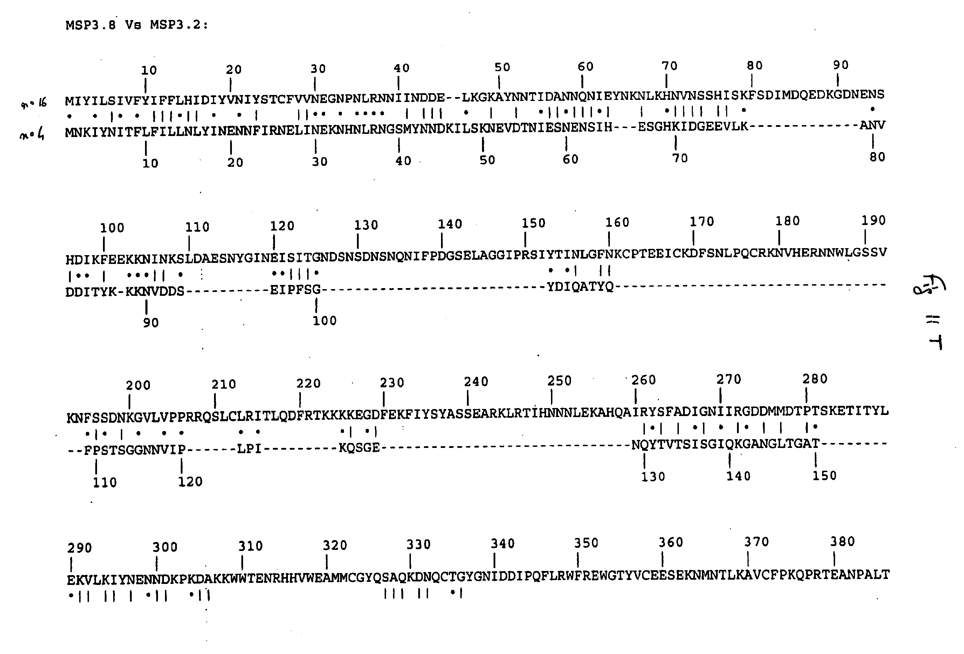

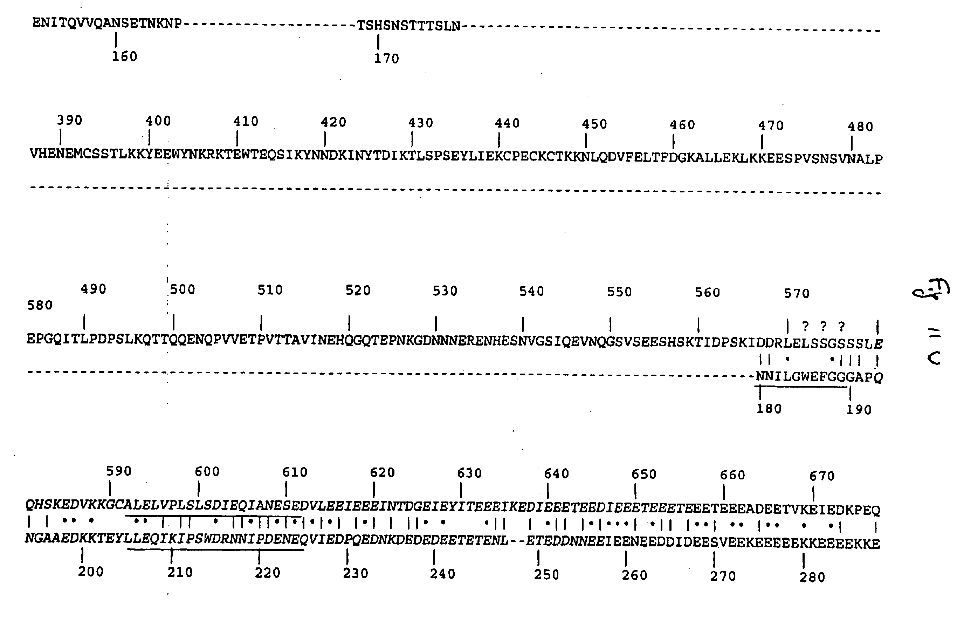

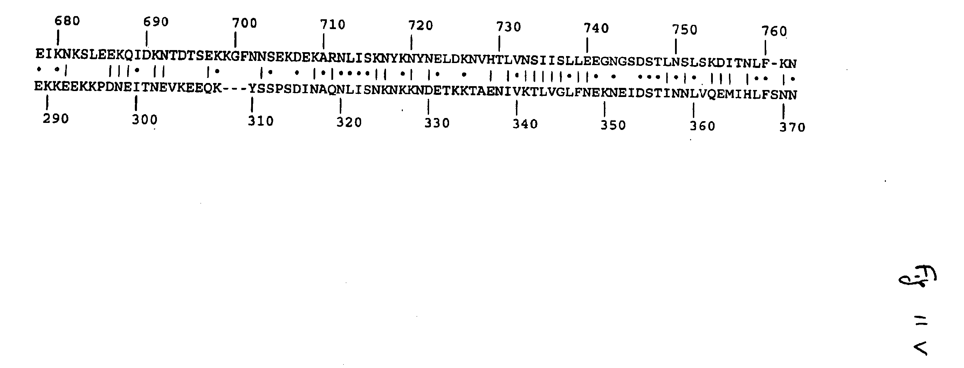

The comparison of sequences between genes of the MSP-3 family shows a very unusual conservation of the epitopes between members of the family, especially of those targeted by biologically active antibodies, which is critical for protection. The comparison of the sequences are summarised in Figure 11.

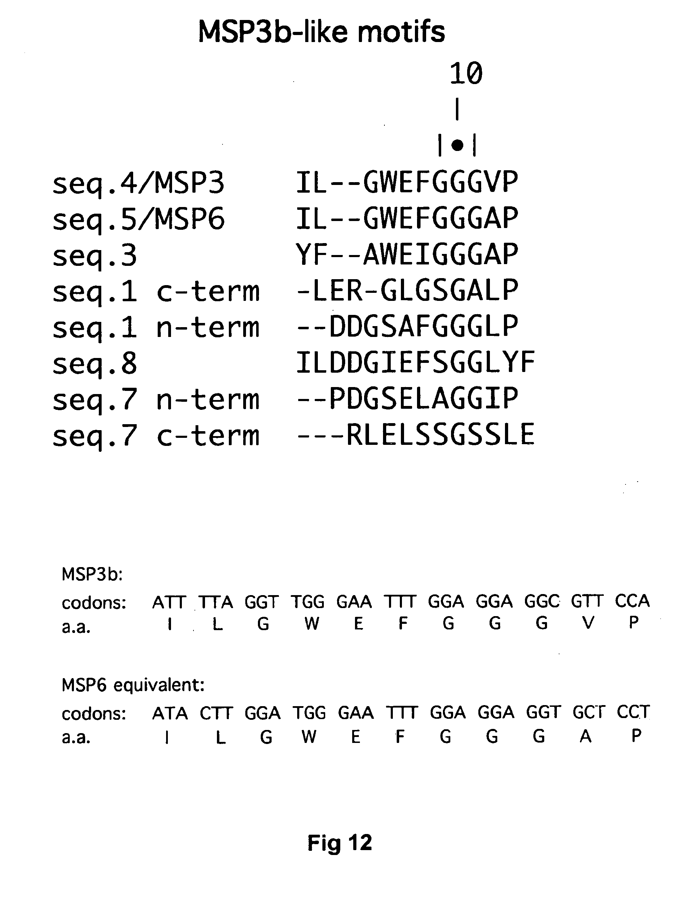

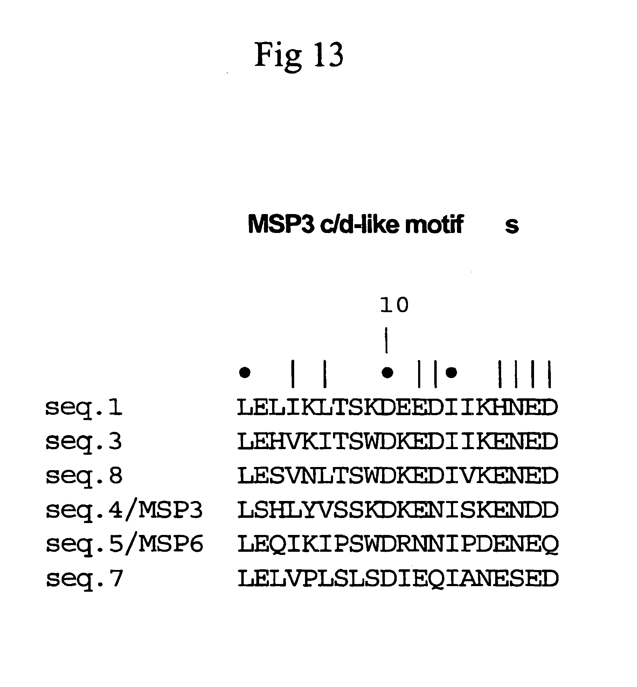

The inventors have identified 2 regions which are very similar, if not totally identical, between members of the family and concern one critical region in the MSP-3-b peptide and one in the MSP-3-c and d peptides (region that is covered by both peptides MSP-3-c and MSP-3-d). The small differences between these very conserved epitopes among the various genes is summarised in Figures 12 and 13. It is noteworthy and highly significant that the most conserved regions across the various genes are those two that are the target of biologically active antibodies in the AOCI - assay in vitro and by passive transfer in SCID mice (see above).

The inventors have also demonstrated that there exists immunological cross-reactivity between the different proteins of the MSP-3 family, as a consequence of those structural homologies between members of the gene family (example 5).

The practical consequence at immunological and vaccine development level is that immunisation by any of the members of the gene family will induce antibodies reactive to the same and to all of the remaining gene products.

Therefore, the present application describes a very particular type of multi-gene family where, instead of epitope polymorphism, which is usually the feature of multi-gene families described to-date, epitope conservation is the main characteristic and where, in case of deletion, mutation in one given gene, another or all other members of the family can take over the antigenic function. In addition, all genes are simultaneously expressed by one given parasite.

The application thus also discloses a protein which is encoded by a gene among those disclosed here above. In a particular embodiment, the protein is a recombinant protein.

An antigenic polypeptide comprising a fragment of at least 10, preferably at least 15, consecutive amino acids from a protein according to the invention, is therefore disclosed. Of course, polypeptides limited to fragments of MSP-3-1 or MSP-3-2, are not considered here.

Preferred antigenic polypeptides according to the invention are antigenic polypeptides from MSP3-3 that comprise at least the MSP-3-c/d-like motif as defined in SEQ ID NO: 27. An antigenic polypeptide of the invention further comprises at least one motif selected amongst the sequences SEQ ID Nos: 17 to 24 and is part of the invention.

Another aspect of the present invention is an antigenic polypeptidic composition comprising a MSP3 c/d like motif of MSP3.3 as defined above and, at least another MSP-3-b-like motif. By "polypeptidic composition" is meant a composition comprising polypeptidic components, *i.e.*, polypeptides or molecules comprising a polypeptidic moiety, such as lipopolypeptides, conjugates consisting of polypeptides bound to a support, etc. The polypeptidic compositions according to the invention can be solutions, caplets, etc.

In a particular embodiment of the antigenic polypeptidic composition according to the invention, the at least another MSP-3-c/d-like motif is selected amongst the sequences of SEQ ID Nos: 25 26, 28, 29 and 30 and conservative variants thereof.

In the antigenic polypeptidic composition of the invention, the at least two different MSP-3-c/d-like motifs can be carried by distinct molecules (*i.e.*, the composition can comprise a diversity of molecules each containing only one motif); alternatively, each polypeptidic component of these composition can carry at least two motifs. An antigenic composition as described above, which contains molecules that comprise at least two different MSP-3-c/d-like motifs, is hence an object of the present invention. These molecules can be complex molecules, in which the at least two motifs are part of distinct peptides covalently linked to a common carrier; preferably, their polypeptidic moiety is constituted by a unique polypeptide comprising said motifs. Fusion proteins, comprising several parts coming from different MSP-3 proteins, can be included in these compositions.

In view of the conservation of the epitopes, the inventors have investigated whether cytophilic antibodies against GLURP and MSP-3 are involved in the development of immunity to clinical malaria in an Asian population of Myanmar, as they have been reported to be in Africa, *i.e.*, in a different human and parasite genetic background. Results, disclosed in Example 7 below, show that levels of cytophilic IgG3 antibodies against conserved regions of MSP-3-1 and GLURP are significantly correlated with clinical protection against *P. falciparum* malaria. In contrast, levels of non-cytophilic IgG4 antibodies against GLURP increased with the number of malaria attacks. Most importantly, there was a complementary effect of the MSP-3-1- and GLURP-specific IgG3 antibodies in malaria protection. In those individuals not responding to one of the antigens, a strong response to the other was consistently detected and associated with protection, suggesting that the induction of antibodies against both MSP3 and GLURP could be important for the development of protective immunity.

According to another embodiment of the invention, the antigenic polypeptidic composition hence further comprises an antigenic polypeptidic molecule comprising at least 10 consecutive amino acid residues from the R0 region of GLURP.

As mentioned above, an antigenic polypeptidic composition according to the invention can comprise a limited number of molecules each comprising a variety of epitopes, or a variety of molecules each comprising a limited number of epitopes. According to a particular embodiment, the composition of antigenic polypeptides comprises from 2, preferably from 3 to less than 9 polypeptides encoded by the genes of the invention.

An example of antigenic polypeptidic composition according to the latter possibility is a mixotope comprising a variety of synthetic peptides comprising the sequence:

X1-X2-X3-X4-X5-X6-X7-X8-X9-G-X9-X10-X11-X12 (SEQ ID No:31),

wherein:

X1 = I, Y or none;
X2 = L, F or none;
X3 = E, D, P or none;
X4 = R, D or none;
X5 = G, A, L or none;
X6 = W, G, S, I or E;
X7 = E, L or A;
X8 = F, I, G, L or S;
X9=G, SorA;
X10 = V, A, L, I or S;
X11 =P, Y or L;
X12 = E, F or none.

A "mixotope" is a combinatorial library of peptides which can be obtained in a single synthesis, as described by (Gras-Masse, Georges et al. 1999).

Another mixotope derived from MSP-3-b and which can be included in an antigenic polypeptidic composition according to the invention is a mixotope comprising a variety of synthetic peptides comprising the sequence

$X_1$-$X_2$-$X_3$-W-E- $X_4$-G-G-G-$X_5$-P (SEQ ID No:32),

wherein:

$X_1$ = I or Y:
$X_2$ = L or F;
$X_3$ = G or A;
$X_4$ = F or I; and
$X_5$ = V or A.

Similarly, another antigenic polypeptidic composition according to the invention is a mixotope comprising a variety of synthetic peptides comprising the sequence
L-X1-X2-X3-X4-X3-X5-X6-X7-D-XS-X9-X10-I-X11-X12-X13-X14-X15-X16 (SEQ ID No:33),
wherein:

X1 = E, or S;
X2 = L, H, S or Q;
X3 = I, V or L;
X4 = K, N, Y or P;
X5 = T, S or P;
X6 = S or L;
X7 = K, W or S;
X8 = E, K, R or I;
X9 = E or N;
X10 = D, N or Q;

X11 = I, V, S, P or A;
X12 = K, D or N;
X13 = H or E;
X14 = N or S;
X15 = E or D;
X16 = D or Q.

The above antigenic mixotope compositions can be a mix of at least 50, at least 100, or at least 500 peptides of different sequences. They can also comprise a combinatorial library of synthetic peptides corresponding to each of the observed and potential substitutions An antigenic composition, comprising a mix of the two above-described mixotopes, is also included in the present invention.

In any of the above-described antigenic polypeptides or antigenic polypeptidic compositions a lipidic molecule can be linked to at least part of the polypeptidic molecules. An example of lipidic molecule that can be used therefore is a C-terminal palmitoylysylamide residue.

As already mentioned above, at least part of the polypeptides or polypeptidic molecules in the antigenic polypeptide according to the invention, can be bound to a support, thereby constituting conjugates. Preferred supports in this embodiment of the invention are viral particles, nitrocellulose or polystyrene beads, and biodegradable polymers such as lipophosphoglycanes or poly-L lactic acid.

Another aspect of the present invention concerns an immunogenic composition comprising as an immunogen a polypeptide or a polypeptidic composition especially prepared by recombination as any of those described above.

As discussed in Example 5, the gene family described herein presents a remarkable characteristic, which is the epitope conservation between the various members of the family, which leads to immunogenic cross-reactivity between the various products of the gene family. The vaccination potential of MSP-3-1 and its fragments, illustrated in Examples 2 and 3, together with the epitope conservation and the cross-reactivity mentioned above, are remarkable features that make this gene family and the polypeptidic compositions derived therefrom particularly interesting candidates for vaccination against malaria. Another aspect of the present invention is hence the use of a polypeptide or a polypeptidic composition as described above, for the preparation of a vaccine against malaria, as well as such a vaccine, comprising as an immunogen said polypeptide or polypeptidic composition, in association with a suitable pharmaceutical vehicle.

An immunogenic composition and a vaccine according to the invention can further comprise at least one antigen selected amongst LSA-1 (Guerin-Marchand, Druilhe et al. 1987), LSA-3 (Daubersies, Thomas et al. 2000), LSA-5, SALSA (Bottius, BenMohamed et al. 1996), STARP (Fidock, Bottius et al. 1994), TRAP (Robson, Hall et al. 1988), PfEXPI (Simmons, Woollett et al. 1987), CS (Dame, Williams et al. 1984), MSP1 (Miller, Roberts et al. 1993), MSP2 (Thomas, Carr et al. 1990), MSP4 (Marshall, Tieqiao et al. 1998), MSP5 (Marshall, Tieqiao et al. 1998), AMA-1 (Peterson, Marshall et al. 1989; Escalante, Grebert et al. 2001), SERP (Knapp, Hundt et al. 1989) and GLURP (supra).

According to one particular embodiment of the invention, the immunogenic composition or the vaccine is formulated for intradermal or intramuscular injection. In that case, said immunogenic composition or vaccine preferably comprises between 1 and 100 μg of immunogen per injection dose, more preferably between 2 and 50 μg. Alternatively, the immunogenic composition or the vaccine can be formulated for oral administration, as described by (BenMohamed, Belkaid et al. 2002).

The immunogenic composition or vaccine of the invention can also further comprise SBAS2 and/or Alum and/or Montanide as an adjuvant.

Other aspects of the present invention relate to antibodies and fragments of antibodies directed against the antigens disclosed herein. As described above and in Example 5, the epitope conservation in the MSP-3 family leads to cross-reactivity of the antibodies obtained against one antigen. For example, a synthetic or recombinant antibody which cross-reacts with MSP-3-3 and which mediates *Plasmodium falciparum* blood stage growth inhibition or killing, in the monocyte-dependent, antibody-mediated ADCI mechanism, under *in vitro* conditions, is a particularly interesting antibody according to the invention.

A pool of antibodies and/or fragments of antibodies directed against polypeptides according to the invention, is also part of the invention.

Another pool of antibodies and/or fragments of antibodies according to the invention is directed against a polypeptidic composition as described above.

Preferred antibodies (or fragments) according to the invention are human or humanized antibodies. These antibodies or fragments of antibodies can be produced for example in *Lemna,* as well as in maize, tobacco, CHO cells, and the like. When produced in CHO cells, they can be obtained for example by using the method described in WO 03/016354.

The present invention also pertains to the use of a composition comprising an antibody or a pool of antibodies or fragments thereof as described above, for the preparation of a medicament against malaria. Of course, a medicament for passive immunotherapy of malaria, comprising such an antibody or a pool of antibodies, is also considered as part of the invention. Such medicament can further comprise antibodies directed against at least one antigen selected amongst LSA-1, LSA-3, LSA-5, SALSA, STARP, TRAP, PfEXP1, CS, MSP1, MSP2, MSP4, MSP5, AMA-1, SERP and GLURP.

Methods for the prophylaxis, the attenuation or the treatment of malaria, by administering to a patient in need thereof, an immunogenic composition, a vaccine, or a medicament comprising antibodies, as described above, are also enclosed in the invention.

The invention also concerns a method for the *in vitro* diagnosis of malaria in an individual likely to be infected by *P. falciparum,* which comprises the bringing of a biological sample from said individual into contact with an antigenic polypeptide of the invention, under conditions enabling the formation of antigen/antibody complexes between said antigenic peptide or polypeptide and the antibodies possibly present in the biological sample, and the *in vitro* detection of the antigen/antibody complexes possibly formed. In this method, the *in vitro* diagnosis can be performed by an ELISA assay. It is also possible to bring the biological sample into contact with one or several antigenic peptides originating from other antigens selected amongst LSA-1, LSA-3, LSA-5, SALSA, STARP, TRAP, PfEXP1, CS, MSP-3-1, MSP-3-2, MSP-3-5, MSP-3-6, MSP1, MSP2, MSP4, MSP5, AMA-1, SERP and GLURP, as an additional step of the method.

An alternative method for the *in vitro* diagnosis of malaria in an individual likely to be infected by *P. falciparum* comprises the bringing of a biological sample from said individual into contact with antibodies according to the invention, under conditions enabling the formation of antigen/antibody complexes between said antibodies and the antigens specific for *P. falciparum* possibly present in the biological sample, and the *in vitro* detection of the antigen/antibody complexes possibly formed.

Kits for the *in vitro* diagnosis of malaria, based on the particular features of the MSP-3 family, are also contemplated. For example, they can comprise at least one peptide or polypeptide according to the invention, possibly bound to a support. Such a kit can further comprise reagents for enabling the formation of antigen/antibody complexes between said antigenic peptide or polypeptide and the antibodies possibly present in a biological sample, and reagents enabling the *in vitro* detection of the antigen/antibody complexes possibly formed.

Another kit for the *in vitro* diagnosis of malaria, according to the invention, comprises antibodies as described above, and, if necessary, reagents for enabling the formation of antigen/antibody complexes between said antibodies and antigens from the proteins of the MSP-3 family possibly present in a biological sample, and reagents enabling the *in vitro* detection of the antigen/antibody complexes possibly formed.

Also part of the present invention is a recombinant nucleotide sequence coding for an antigenic polypeptide according to the invention. Particular sequences according to the invention are nucleotide sequences of the invention comprising a sequence encoding at least two MSP-3-b-like and/or MSP-3-c/d-like motifs, wherein at least one of said motifs is selected amongst the motifs of SEQ ID Nos: 19 to 24 and 27 to 30, or their conservative variants. An example is a recombinant nucleotide sequence comprising a sequence encoding a fusion protein comprising several MSP-3-c/d-like motifs, wherein at least two of said motifs are selected amongst the motifs of SEQ ID Nos: 25 to 30 and their conservative variants.

The invention also pertains to a recombinant cloning and/or expression vector, comprising a nucleotide sequence of the invention as described above, which can be, for example, under the control of a promoter and regulatory elements homologous or heterologous vis-à-vis a host cell, for expression in the host cell.

An expression vector as described in the above paragraph can advantageously be used for the preparation of a medicament for genetic immunisation against *Plasmodium falciparum.*

The invention also pertains to a nucleic acid vaccine comprising a nucleotide sequence of the invention.

A recombinant host cell, for example a bacterium, a yeast, an insect cell, or a mammalian cell, which is transformed by an expression vector as described above, is also part of the present invention.

Several aspects and advantages of the present invention are illustrated in the following figures and experimental data.

## LEGENDS TO THE FIGURES :

Figure 1 : Organisation of nine genes clustered in the same region of chromosome 10. Nine open reading frames are separated by non coding regions, and encode in a row (5'-3') genes encoding proteins denominated first GLURP, followed at 1300 base-pairs by MSP-3 (now denominated MSP-3-1) followed by 7 other genes denominated MSP-3-2, MSP-3-3, MSP-3-4, MSP-3-5, MSP-3-6, MSP-3-7, MSP-3-8.

Figure 2 : Various peptides derived from MSP-3-1. Protection was associated with antibodies to peptides MSP-3b, c and d.

Figures 3, 4 and 5 : *In vivo* studies. Passive transfer experiments of specific antibodies into *P. falciparum*-infected, human RBCs-grafted, immunocompromised mice.
Antibodies to MSP 3-b peptide, MSP-3-d peptide and to GLURP-R0 region were all found able, under passive transfer conditions *in vivo,* to clear a *P. falciparum* parasitemia established in immunocompromised SCID mice.

Figure 6: *In vivo* studies. Confirmation results.
A human recombinant antibody directed to the MSP-3-b epitope, cross-reactive with MSP-3-2 recombinant protein which, upon passive transfer, can clear the parasitemia in *P. falciparum* SCID mice.

Figure 7 : Results obtained using antibodies elicited by artificial immunisation of human volunteers using a Long Synthetic Peptide covering the region MSP-3-b, c, d peptides.
The same effect is observed, both under *in vitro* conditions and under *in vivo* conditions, in the *P. falciparum* SCID mouse model.

Figure 8 : Comparison between the biological effect of total African IgG with purified anti-MSP-3-b antibodies adjusted at the same concentration as in the total African IgG.
A stronger and more complete effect of the anti-MSP-3-b antibodies alone is observed, which stresses their vaccine potential.

Figure 9: ClustalW alignment of the nucleotide sequences of the MSP-3 family

Figure 10 : ClustalW alignment of the peptide sequences of the MSP-3 family

Figure 11 : Comparison of sequences between genes MSP3 family.
This comparison shows a very unusual conservation of the epitopes between members of the family, those targeted by biologically active antibodies, which is critical for protection.

Figure 12 : MSP-3-b - like motifs

Figure 13 : MSP-3-c-d - like motifs

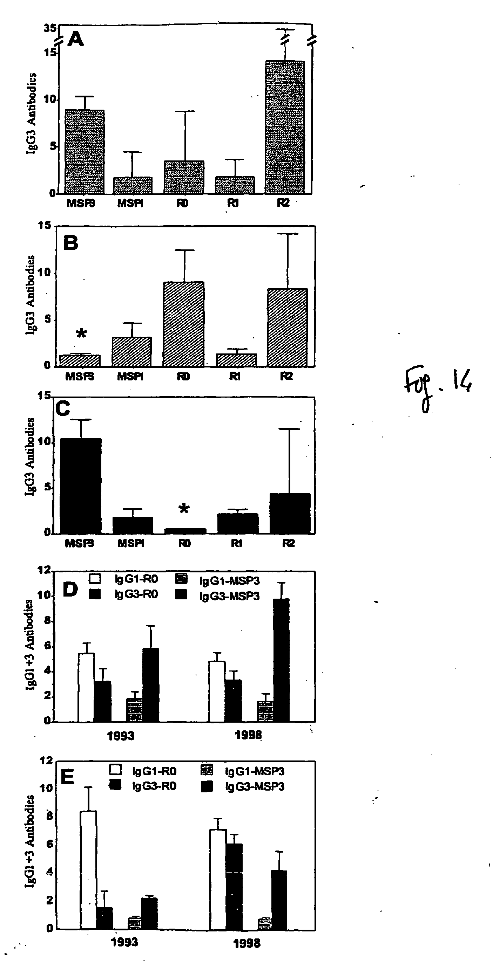

Figure 14 : **A**. Pattern of IgG3 antibody responses against each of the antigens in the 30 protected individuals of OoDo (means and standard errors of the ratios of IgG3-specific responses). **B**. Pattern of IgG3 responses in 7 protected OoDo inhabitants with low IgG3 anti-MSP3 response (low IgG3 cut off values were defined as those under the lower 95% confidence Interval limits of the mean, ie.anti-MSP3b IgG3 ratios <2.30). **C** Pattern of IgG3 responses in 15 protected OoDo inhabitants with low IgG3 anti-RO response (low IgG3 cut off values were defined as those under the lower 95% confidence interval limits of the mean IgG3 ratios, ie. IgG3 ratios of anti-GLURP R0 <1.38). **D.** Changes at 5 years interval in 7 protected individuals with high IgG3 MSP3 responses in 1998. **E.** Changes at 5 years interval in 6 protected individuals with high IgG3 anti-GLURP R0 responses in 1998.

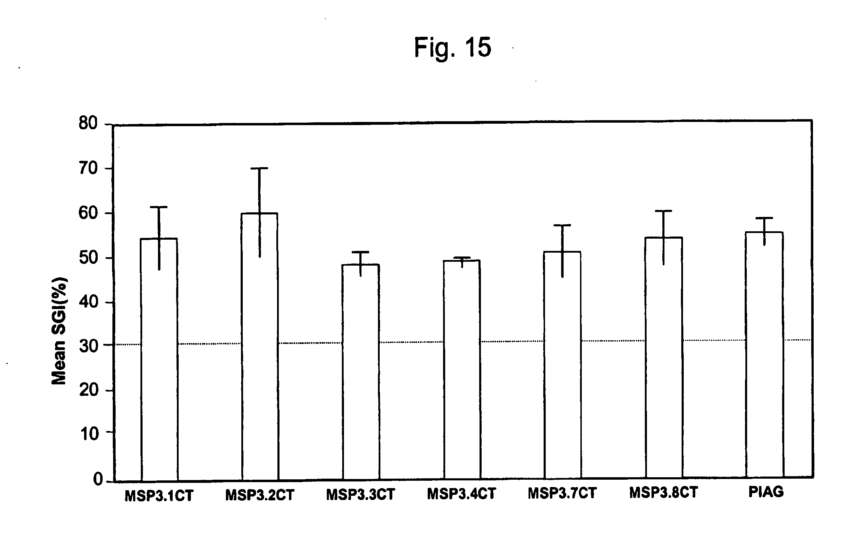

Figure 15 : ADCI activity of antibodies affinity purified on various constructs derived from the MSP-3 gene family. The results are expressed as the mean SGI (specific growth inihibitory index) as compared to a positive control, the pool of the immune African immunoglobulins (PIAG) which has been used for passive transfer into Thai children. The sequences used for affinity purification correspond to the C-terminus region, which is the most homologous part between the genes and the only one very well conserved and are indicated by a line below the C-term region in figure 18.
Results show that all antibodies specific to each region to each of the 6 genes are strongly active in the ADCI mechanism as much as the pool of African immunoglobulins shown to be effective at clearing *P. falciparum* by passive transfer in infected individuals.

Figure 16 : pattern of cross-reactivity, of antibodies affinity purified on the C-terminus region of each of the members of the MSP-3 family, with other members of the MSP-3 family. GLURP, 571 and BSA serve as negative controls.
Results show that antibodies affinity purified on a given C-terminus region of one member of the family cross react, to various extent, to all other members of the MSP-3 family. The strongest cross-reactive pattern is obtained with MSP-3-4 which shows a strong positive signal with all other members followed by MSP-3-8. However, this dot-blot merely shows cross-reactive epitopes in each of the member of the MSP-3 family.

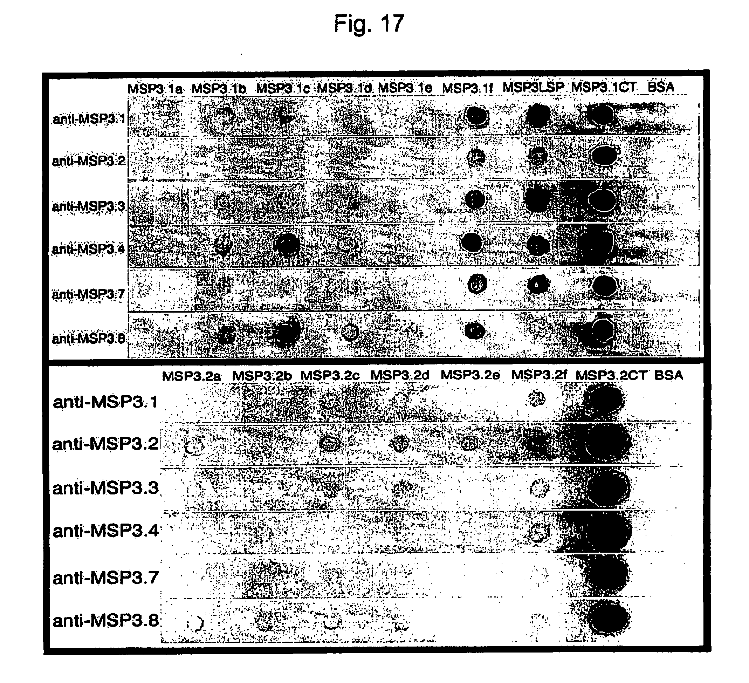

Figure 17 : patterns of cross reactivity, of antibodies affinity purified on the C-terminues region of each fo the members of the MSP-3 family, with peptides derived from the MSP-3-1 and the MSP-3-2 members of the MSP-3 family. The peptides are peptides a, b, c, d, and f, from MSP-3-1 and from MSP-3-2. The recombinant MSP-3 C-term and BSA serve as positive and negative controls respectively.
Results show that antibodies to the C-terminus regions of the various members of the family react, to various extents, with various regions of the C-terminus of MSP-3-1, particularly MSP-3b and c, and the strongest response being obtained on MSP-3-f. The cross reactivity with various peptides of MSP3-2 is not as strong as that obtained with MSP-3-1. Finally, the very strong cross-reactivity obtained with MSP-3-1 CT, the C terminus recombinant, also suggests a cross-reactivity with an epitope not defined by any of the individual peptides but most likely a conformational epitope generated by the longer C-term recombinant. In this case, the extent of cross-reactivity of any given affinity purified antibody to any given member of the family demonstrate the structural homology of the various members of that family and the existence of cross-reactive epitopes, including those generated by 3-dimensional conformation. The same holds true for MSP-3-2.

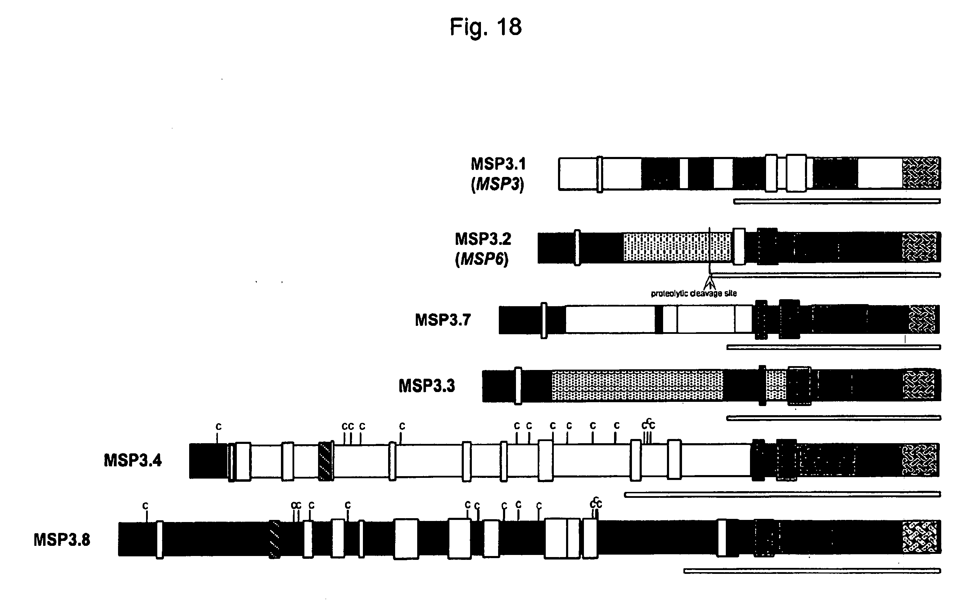

Figure 18 : A schematic representation of the various members of the MSP-3 family. Underlined is the C-terminus region which was used to build up recombinant, antigens which were used in the immunoassays.

**EXAMPLES :**

**Example 1 : *In vitro* blood stage killing of *P. falciparum* by antibodies to the gene products, by the ADCI mechanism**

**2.A. Materials and methods : the ADCI assay**

2.A.1. Introduction

The Antibody Dependent Cellular Inhibition (ADCI) assay is designed to assess the capability of antibodies to inhibit the in vitro growth of *Plasmodium falciparum* in the presence of monocytes. Studies have shown that antibodies that proved protective against *P. falciparum* blood stages by passive transfer in humans are unable to inhibit the parasite in vitro unless they are able to cooperate with blood monocytes. It was also shown that antibodies that were not protective in vivo had no effect on *P. falciparum* growth in the ADCI assay. The ADCI is therefore an in vitro assay the results of which reflect the protective effect of anti-malarial antibodies observed under in vivo conditions in humans.
The antibodies able to cooperate with monocytes should be obviously cytophilic: IgG1 and IgG3 isotypes are efficient in ADCI while IgG2, IgG4 and IgM are not efficient. This is consistent with the findings that in sera from protected individuals, cytophilic anti-*P.falciparum* antibodies are predominant, while in non-protected patients the antibodies produced against the parasite are mostly non-cytophilic.
The results suggest that ADCI likely involves the following succession of events: at the time of schizonts rupture, the contact between some merozoite surface component and cytophilic antibodies bound to monocytes via their Fc fragment triggers the release of soluble mediators which diffuse in the culture medium and block the division of surrounding intra-erythrocytic parasites.
The major steps involved in the ADCI protocol are:

(i). Serum IgG preparation using ion exchange chromatography
(ii). Monocyte isolation from a healthy blood donor
(iii). Preparation of *P.falciparum* parasites including synchronization and schizont enrichment.
(iv). Parasite culture, for 96 hrs, in the presence of antibodies and monocytes.
(v). Inhibition effect assessed by microscopic observation and parasite counting.

2.A.2. Materials

***IgG Preparation***

1. Tris buffer: 0.025 *M* Tris-HCl, 0.035 M NaCl, pH 8.8.
2. Phosphate Buffer Saline (PBS), pH 7.4.
3. GF-05-Trisacryl filtration column (IBF, Biothecnics, Villeneuve La Garenne, France).
4. DEAE-Trisacryl ion exchange chromatography column (IBF).
5. G25 Filtration column.

6. Amicon filters and tubes for protein concentration (Mol. Wt. cut off: 50,000 Da).
7. Sterile Millex filters, 0.22 μm pore size (Millipore Continental Water Systems, Bedford MA).
8. Spectrophotometer equipped with Ultra Violet lamp.

***Monocyte Preparation***

1. Heparinized blood collected from a healthy donor, 20-40 mL volume.
2. Ficoll-Hypaque density gradient (Pharmacia LKB Uppsala, Sweden).
3. Hank's solution supplemented with $NaHCO_3$, pH 7.0.
4. RPMI 1640 culture medium supplemented with 35 m*M* Hepes and 23 m*M* $NaHCO_3$,; prepare with mineral water; store at 4°C.
5. Reagents for non-specific esterase (NSE) staining: fixing solution, nitrite, dye, buffer and substrate
6. 96-well sterile plastic plates (TPP, Switzerland).
7. Refrigerated centrifuge.
8. $CO_2$ incubator.
9. Inverted microscope.

***Parasite Preparation***

1. RPMI 1640 culture medium (*see* above).
2. 10% Albumax stock solution; store at 4°C for up to 1 month.
3. 5% Sorbitol for parasite synchronization.
4. Plasmagel for schizont enrichment.
5. Reagents for fixing and staining of thin smears: methanol, eosine, methylene blue.

2.A.3. Methods

***IgG preparation***

IgGs are extracted from human sera (*see* Note 1) as follows:

1. Dilute the serum at a ratio of 1 to 3 in Tris buffer.
2. Filter the diluted serum through a GF-05 Trisacryl gel filtration column previously equilibrated in the Tris buffer. Ensure that the ratio of serum to filtration gel is 1 volume of undiluted serum to 4 volumes of GF-05 gel.
3. Pool the protein-containing fractions
4. Load over a DEAE-Trisacryl ion exchange chromatography column previously equilibrated with Tris buffer. Ensure that the ratio of serum to filtration gel is 1 volume of undiluted serum to 4 volumes of DEAE gel.
5. Collect fractions of 1 mL volume.
6. Measure the optical density (OD) of each fraction using a 280 nm filter.
7. Calculate the IgG concentration as follows:

$$\text{IgG concentration (mg/mL)} = \frac{\text{OD 280 nm}}{1.4}$$

8. Pool the fractions containing IgGs.
9. Concentrate the IgG solution using Amicon filters. Amicon filters are first soaked in distilled water for 1 hour and than adapted to special tubes in which the IgG solution is added.
10. Centrifuge the tubes at 876*g* for 2 hr at 4°C. This usually leads to a 25-fold concentration.
11. Perform a final step of gel filtration using a G25 column previously equilibrated in RPMI culture medium.
12. Collect the IgG fractions in RPMI.
13. Measure the optical density (OD) of each fraction using a 280 nm filter.
14. Calculate the IgG concentration.
15. Pool the fractions containing IgGs.

16.sterilize the IgG fractions by filtration through 0.22 μm pore size filters.
17. Store the sterile IgG solution at 4°C for up to 1 month (or add Albumax for longer storage- but not recommended-).

***Monocyte Preparation***

The procedure for monocyte preparation is based on that described by Boyum (Scand J. Clin. Lab. Invest. 1968, 21, 77-89) and includes the following steps:

1. Dilute the heparinized blood 3-fold in Hank's solution.
2. Carefully layer two volumes of diluted blood onto 1 volume of Ficoll-Hypaque (maximum volume of 20 mL of diluted blood per tube).
3. Centrifuge at 560 g for 20 min at 20°C.
4. Remove the mononuclear cell layer at the Ficoll/plasma interface.
5. Add 45 mL of Hank's solution to the mononuclear cell suspension.
6. Centrifuge at 1000 g for 15 min at 20°C.
7. Carefully resuspend the pelleted cells in 45 mL of Hank's solution.
8. Centrifuge again at 1000 g for 15 min at 20°C. Repeat this washing step twice more.
9. Finally, centrifuge at 180 g for 6 min at 20°C, to remove any platelets that remains in the supernatant.
10. Resuspend the mononoclear cells in 2 mL of RPMI.
11. Calculate the mononuclear cell concentration (i.e. lymphocytes plus monocytes) in the cell suspension: dilute a 20 μL aliquot of the cell suspension 3-fold in RPMI and count cell numbers using a hemocytometer (Malassez type for example).
12. Determine the number of monocytes using the Non Specific Esterase (NSE) staining technique:

    (i). In microtube A, add 40 μL of mononuclear cell suspension to 40 μL of fixing solution.
    (ii). In microtube B, mix the NSE staining reagents in the following order: 60 μL of nitrite, 60 μL of dye, 180 μL of buffer, and 30 μL of substrate
    (iii). Add the mixture in microtube B to the cells in microtube A.
    (iv). Take a 20 μL sample of the stained cells and measure the proportion of monocytes : lymphocytes: monocytes will be colored in brown whereas the lymphocytes will be uncolored. Usually the proportion of monocytes is 10-20% of the total mononuclear cells.

13. Adjust the cell suspension to a concentration of 2 x $10^5$ monocytes per 100 μL, with RPMI.
14. Aliquot the cell suspension in a 96-well plate at 100 μL/well.
15. Incubate for 90 min at 37°C, 5% $CO_2$. During this incubation, monocytes will adhere to the plastic.
16. Remove the non-adherent cells and wash the monocytes by adding, and thoroughly removing, 200 μL of RPMI in each well.
17. Repeat this washing procedure 3 times in order to remove all the non-adherent cells.
18. At least 95% of the recovered cells will be monocytes. Control for the cell appearance and the relative homogeneity of cell distribution in the different wells by observation using an inverted microscope (*see* Notes 2, 3, and 4)

***Parasite Preparation***

*P. falciparum* strains are cultivated in RPMI 1640 supplemented with 0.5% Albumax.
Parasites are synchronized by Sorbitol treatments as follows:

1. Dilute the sorbitol stock to 5% in mineral water.
2. Centrifuge the asynchronous parasite culture suspension at 1200 rpm for 10 min at 20°C.
3. Resuspend the pellet in the 5% sorbitol solution. This will lead to the selective lysis of schizont infected RBC without any effect on the rings and young trophozoites.

When required, schizonts are enriched by flotation on plasmagel as follows:

1. Centrifuge cultures containing asynchronous parasites at 250 g for 10 min at 20°C
2. Resuspend the pellet at a final concentration of 20% red blood cells (RBC), 30% RPMI, 50% plasmagel.
3. Incubate at 37°C for 30 min. Schizont-infected RBC will remain in the supernatant, whereas young trophozoite-infected and uninfected RBC will sediment.
4. Collect carefully the supernatant, by centrifugation at 250 g for 10 min at 20°C.

5. Prepare a thin smear from the pelleted cells, stain, and determine the parasitemia by microscopic examination.
6. Usually, using this method, synchronous schizont infected RBC are recovered at ~ 70 % parasitemia.

For the ADCI assay, synchronized early schizont parasites are used. Usually the parasitemia is 0.5-1.0% and the hematocrit 4%.

***The ADCI Assay***

1. After the last washing step, add in each monocyte containing well:

(i). 40 μLof RPMI supplemented with 0.5% Albumax (culture medium).
(ii). 10 μL of the antibody solution to be tested. Usually the IgGs are used at 10% of their original concentration in the serum (~ 20 mg/mL for adults from hyperendemic areas, and ~ 12 mg/mL for children from endemic area and primary attack patients). (*see* Note 5).
(iii). 50 μL of parasite culture, at 0.5% parasitemia and 4% hematocrit.

2. Control wells consist of the following;

(i). Monocytes (MN) and parasites with normal IgG (N IgG) prepared from the serum of a donor with no history of malaria.
(ii). Parasite culture with IgG to be tested without MN.

3. Maintain the culture at 37°C for 96 hrs in a candle-jar (or a low $O_2$, 5% $CO_2$ incubator).
4. Add 50 μL of culture medium to each well after 48 and 72 hrs.
5. Remove the supernatant after 96 hrs. Prepare thin smears from each well, stain, and determine the parasitemia by microscopic examination. In order to ensure a relative precision in the parasite counting, a minimum of 50,000 red blood cells (RBC) should be counted and the percentage of infected RBC calculated (*see* Notes 6 and 7).
6. Calculate the specific Growth Inhibitory Index (SGI), taking into account the possible inhibition induced by monocytes or antibodies alone:

**SGI = 100 X (1- [Percent parasitemia with MN and Abs / Percent parasitemia with Abs] / [Percent parasitemia with MN+ N IgG / Percent parasitemia with N IgG])**

<u>2.A.4. notes</u>

1. IgG preparation from sera to be tested is an essential step because a non-antibody dependent inhibition of parasite growth has frequently been observed when unfractionated sera were used, probably due to oxidized lipids.
2. Monocyte (MN) function in ADCI is dependent upon several factors such as water used to prepare RPMI 1640. Highly purified water, such as Millipore water, although adequate for parasite culturing, leads to a poor yield in the number of MN recovered after adherence to the plastic wells. On the other hand, water which contains traces of minerals, such as commercially available Volvic water, or glass-distilled water, provide consistently a good monocyte function.
3. Improved monocyte adherence can be obtained by coating the culture wells with fibronectin i.e. coating with autologous plasma from the MN donor, followed by washing with RPMI 1640, prior to incubation with mononuclear cells.
4. MN from subjects with a viral infection (e.g. influenza) are frequently able to induce a non IgG dependent inhibition of parasite growth. This non-specific inhibition effect could prevent the observation of the IgG-dependent inhibition in ADCI. Therefore, MN donors suspected of having a viral infection, or who have had fever in the past 8 days, should be avoided. The results from ADCI are not reliable when the direct effect of MN alone is greater than 50% inhibition. The preparation of MN in medium containing heterologous serum, such as FCS, results in the differenciation of MN, their progressive transformation into macrophages which have lost their ADCI promoting effect.
5. If required, murine IgG can be tested in ADCI with Human MN. The IgG2a isotype is able to bind to the human Fc y receptor II present on monocytes shown to be involved in the ADCI mechanism.
6. A possible variation of the ADCI assay is the assessment of a competition effect between protective cytophilic antibodies (adults from hyperendemic area) directed to the merozoite surface antigens, and non-protective antibodies (children from endemic area and primary attack patients) which recognize the same antigens but are not able to trigger the monocyte activation because they do not bind to Fc gamma receptors. Therefore non-cytophilc Ig directed to the "critical" antigens may block the ADCI effect of protective antibodies. Each IgG fraction should be used at 10% of its original concentration in the serum.
7. The ADCI assay protocol can be modified and performed as a two-step ADCI with short-term activation of monocytes according to the following procedure:

(i). Incubate MN for 12-18 hrs with test Ig and synchronous mature schizonts infected RBC, at 5-10% parasitemia. During this first culture time, infected RBC rupture occurs and merozoites are released.
(ii). Collect supernatants from each well and centrifuge them at 700 g.
(iii). Distribute the supernatants in a 96-well plate, at 100 μL/well
(iv). Add to each well 100μL of *P.falciparum* asynchronous culture containing fresh medium, at 0.5-1% parasitemia, 5% hematocrit (particular care is taken to reduce to a minimum the leucocyte contamination of the RBC preparation used for this second culture).
(v). At 36 hr of culture, add 1 mCi of $^3$H hypoxanthine to each well.
(vi). At 48 hr of culture, harvest cells and estimate $^3$H uptake by counting in a liquid scintillation counter.

**2.B. Results**

Table 1 :

| ADCI conditions | | antibody | volume | MN(-) | MN(+) | SGI % | Adj SGII% | Direct |
|---|---|---|---|---|---|---|---|---|
| **ADCI 02/03** | 17/1/03 | RPMI | 10μl | 7.8 | 7,1 | 0% | | 0% |
| **Parasite strain** | 3D7 | NIG dialysed | 5μl | 7,5 | 6,3 | 8% | | 4% |
| MN **preparation** | attach | PIAG (IFA 35,000)Ali | 10μl | 11,8 | 5 | 53% | 100% | -51% |
| **Initial para.(%)** | 0.5% asynchro in AB+ RBC | anti-MSP3.1 CT (MSP3 Simon) | 10μl | 11.8 | 5 | 53% | **100 %** | -51% |
| **Final. para.(%)** | 7.8% | anti-MSP3.4 CT | 10μl | 12,6 | 5.1 | 56% | **104 %** | -62% |
| **Duration of ADCI** | 72 h | | 15μl | 9,4 | 5 | 42% | **78%** | -21% |
| **MN inhibition** | 9% | anti-MSP3.7 CT | 10μl | 9,9 | 5 | 45% | **83%** | -27% |
| **%** MN donor | Frozen cyto 18/10/02 | | 15μl | 11,4 | 10 | 4% | **7%** | -46% |
| ADCI | standard | anti-MSP3.1 CT (MSP3 old) | 10μl | 11 | 4,6 | 54% | **101 %** | -41% |
| | | | 15μl | 11,3 | 4,6 | 55% | **103%** | -45% |
| plate 1 | | anti-MSP3.2 CT (MSP6) | 10μl | 12,5 | 3,4 | 70% | **131 %** | 60% |
| All antibodies were adjusted | | | 15μl | 9,8 | 4,5 | 50% | **93%** | -26% |

(continued)

| ADCI conditions | | antibody | volume | MN(-) | MN(+) | SGI % | Adj SGII% | Direct |
|---|---|---|---|---|---|---|---|---|
| to IFA titre of 1: 200 | | anti-MSP3.8 CT | 10µl | 12 | 4,4 | 60% | **112** | -54% |
| | | | 15µl | 8,2 | 7,7 | -3% | **-6%** | -5% |
| | | anti-MSP3.3 CT | 10µl | 9,1 | 4,1 | 51% | **94%** | -17% |
| | | | 15µl | 9,1 | 4,5 | 46% | **85%** | -17% |

The C-terminal regions used to produce the antibodies are indicated in Figure 18, and correspond to the horizontal lines below each of the proteins. They have been cloned in *E.coli* using the PTCR-His vector.

**Example 2 : *In vivo* assays by passive transfer of antibodies in mice**

The *in vitro* results shown in example 1 were confirmed under *in vivo* conditions by passive transfer experiments of specific antibodies into *P. falciparum*-infected, human RBCs-grafted, immunocompromised mice.

The materials and methods used to perform the experiments described in the present example are described in (Brahimi, Perignon et al. 1993; Badell, Oeuvray et al. 2000). In particular, the methods to obtain the antibodies have been described by Brahimi *et al.*

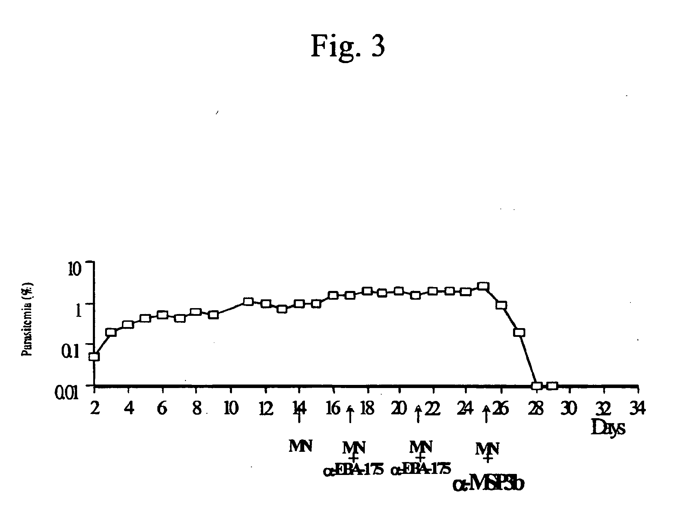

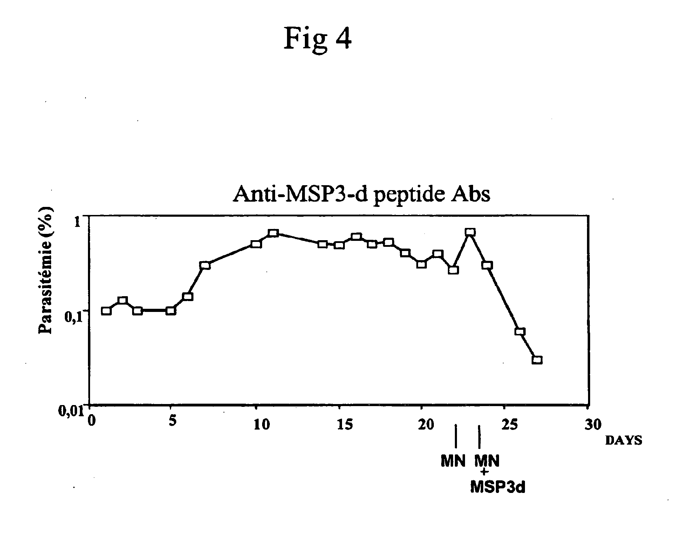

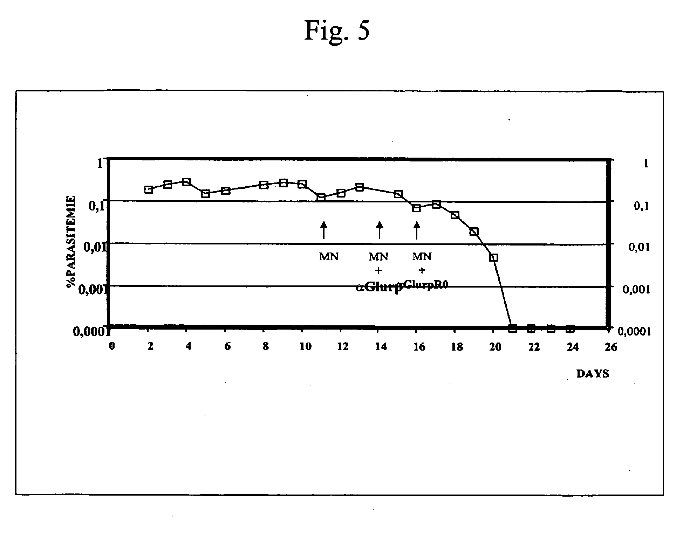

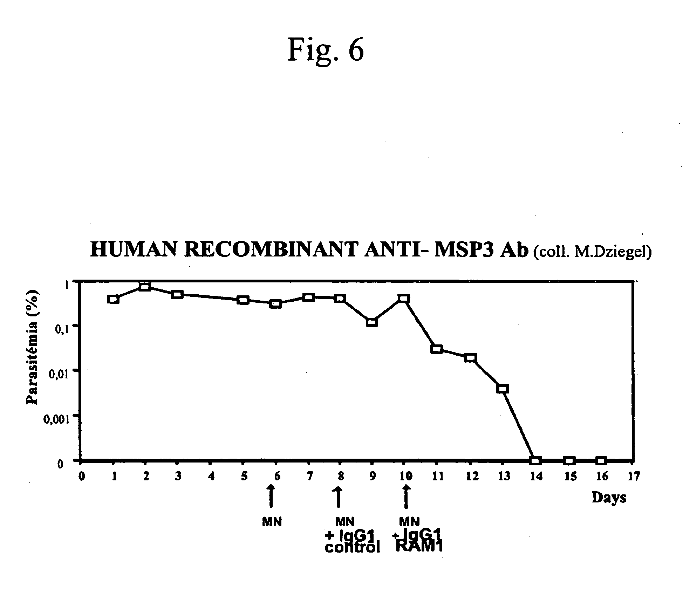

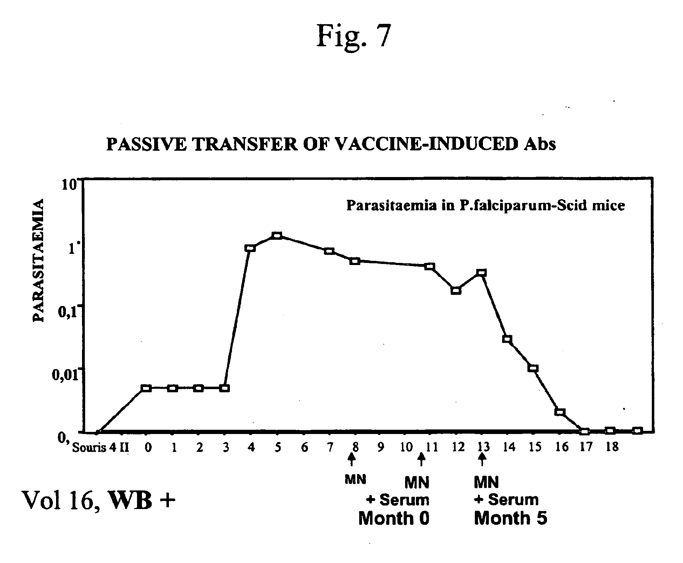

Due to the complexity of the handling of this model, all antibodies could not be tested so far but antibodies to MSP-3-b peptide, MSP-3-d peptide and to GLURP-R0 region were all found able, under passive transfer conditions *in vivo,* to clear a *P. falciparum* parasitemia established in immunocompromised SCID mice (Figures 3, 4 and 5). It can be seen that the clearance effect of anti-MSP-3-b and MSP-3-d antibodies is extremely strong and, conversely that the clearance induced by anti-GLURP antibodies, adjusted to the same antibody concentration, is less effective: the time to clearance of parasites following transfer is about twice as long with anti-GLURP as that obtained with anti-MSP-3 antibodies. Again here, for reasons described herein, the cross-reactivity network between the 6 genes described in detail implies that antibodies directed to the other genes will most likely have the same biological effect if transferred in *P. falciparum* infected mice. Finally, this *in vivo* effect was further confirmed by using a human recombinant antibody directed to the MSP-3-b epitope (Figure 6), cross-reactive with MSP-3-2 recombinant protein and which, upon passive transfer, can clear the parasitemia in *P. falciparum* SCID mice. Essentially similar results were also obtained using antibodies elicited by artificial immunisation of human volunteers using a Long Synthetic Peptide covering the region MSP-3-b, c, d peptides which showed the same effect, both under *in vitro* conditions and under *in vivo* conditions, in the *P. falciparum* SCID. mouse model (Figure 7).

**Example 3 : Immunization experiments in monkeys**

The protective data gathered under *in vitro* and *in vivo* conditions was further confirmed independently by showing that aotus monkeys immunised by MSP-3-1 in recombinant form adjuvated by Freund complete adjuvant, produced antibodies effective in the ADCI mechanism and that the monkeys, when challenged by a virulent *P. falciparum* blood stage inoculation, were able to control and to clear their *P. falciparum* parasitemia, whereas control monkeys did not.

**Example 4 : comparison of the biological effects obtained with total African IgG and with purified anti-MSP-3-b antibodies**

The comparison of the biological effect obtained with total African IgG and with purified anti-MSP-3-b antibodies adjusted at the same concentration as in the-total African IgG shows a stronger and more complete effect of the anti-MSP-3-b antibodies alone, which stresses their vaccine potential. In the course of previous and present studies, the inventors observed that affinity-purified antibodies to MSP-3-b peptide had apparently a faster and stronger effect than total African IgG, from which they were extracted. This observation was extremely intriguing, since *P. falciparum* being made of nearly 6000 different proteins, and peptide MSP-3-b being only a small region of one of them, one can compute that antt-MSP-3-b antibodies would correspond to less than 1 / 10,000 of the total antibodies raised by exposure to the parasite.

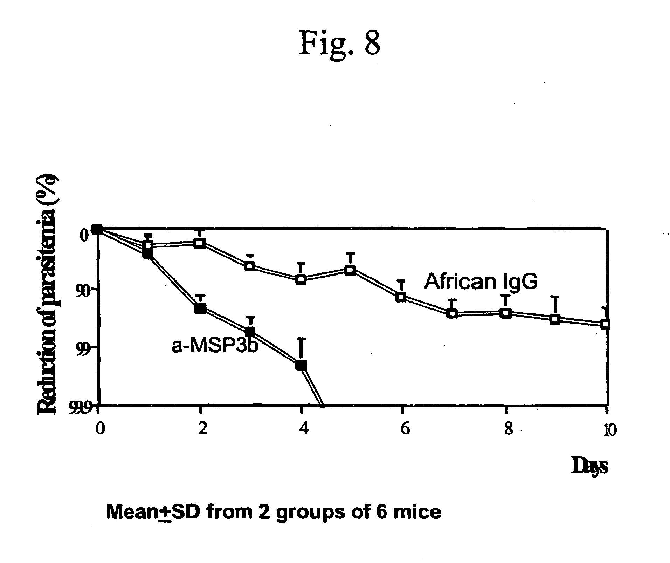

Further studies were conducted either with total IgG or with anti-MSP-3-b antibodies and are summarised in figure 8. It is noteworthy that in these experiments, the amount of anti-MSP-3-b antibodies in the total IgG or in the purified preparation was exactly the same. These experiments, which correspond to the mean ± SD of 6 mice treated by anti-MSP-3-b antibodies and 6 mice treated by total African purified IgG clearly confirmed that there was:

- a much faster effect of anti-MSP-3-b antibodies,
- a more complete effect of anti-MSP-3-b antibodies, since they led to a full clearance of the parasitemia in mice, whereas immune IgG led to a decrease but without sterilising effect, as was the case with the same preparation when injected into human volunteers (and, as is the case, in African adults donors who keep a chronic, low-grade parasitemia).

This observation implies that there are other antibodies present in the immune African IgG which compete or block the inhibitory effect of anti-MSP-3 antibodies. This negative interaction between different antibodies is reminiscent of that reported, for instance, by Blackman and collaborators for anti-MSP-1 antibodies. It can also be related to the interference of non-cytophylic antibodies directed to other merozoite surface antigens and which could act indirectly, for instance, by steric hindrance, reducing the access to MSP-3 antigens of anti-MSP-3 antibodies.

Anyhow, this observation has also very important implications for vaccine development: it can be taken as an indication that immunisation by selected malarial antigens may elicit stronger protective responses than those resulting from exposure to all malarial proteins, or at least to several of them.

In other words, the immunisation, by molecules which are identified as targets of protective mechanisms may lead to induce a stronger protection than that developed by natural exposure, which is already the strongest Protection known against asexual blood stages in human beings. It is thus extremely promising for the development of a future, efficient, malarial vaccine.

**<u>Example 5 : epitope conservation in the MSP-3 family</u>**

The materials and methods used to perform the experiments described in the present example are described in (Brahimi, Perignon et al. 1993; Badell, Oeuvray et al. 2000). In particular, the methods to obtain the antibodies have been described by Brahimi *et al.*

As a consequence of the structural homologies between members of the gene family, the existence of immunological cross-reactivity between the corresponding proteins was confirmed : human antibodies were affinity purified on the product of each gene and reacted to all of the remaining.

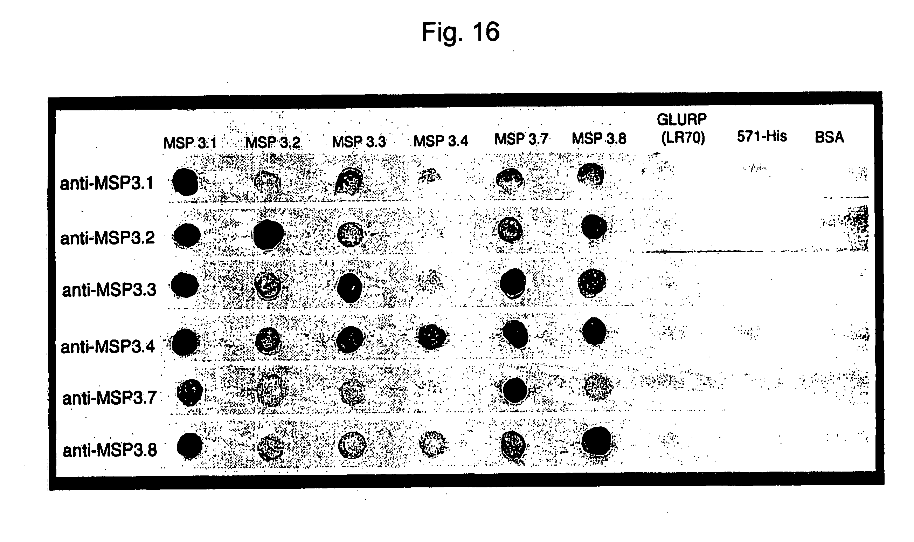

The results show that antibodies induced against one single protein of the MSP-3 family also react with other antigens, in immunoblot (Figures 16 and 17), and in ELISA (Table 2 below).

Table 2 : Antibodies to the seven gene products are all effective at mediating *P. falciparum* blood stage killing, in the monocyte-dependent, antibody-mediated ADCI mechanism, under *in vitro* conditions. Results show that antibodies to each of those regions are equally effective at achieving *P. falciparum* erythrocytic stage growth inhibition under *in vitro* conditions.

| | **MSP3.1 CT** | **MSP3.2 CT** | **MSP3.3 CT** | **MSP3.4 CT** | **MSP3.7 CT** | **MSP3.8 CT** | **571-His** | **BSA** |
|---|---|---|---|---|---|---|---|---|
| Anti-***MSP3.1***CT | **100** | 6 | 33 | 4 | 35 | 23 | 3 | 4 |
| Anti-***MSP3.2***CT | 54 | **100** | 22 | 4 | 39 | 37 | 4 | 4 |
| Anti-***MSP3.3***CT | **117** | 45 | **100** | 5 | **100** | 47 | 5 | 5 |
| Anti-***MSP3.4***CT | **216** | 44 | **130** | **100** | **147** | **103** | 10 | 10 |
| Anti-***MSP3.7***CT | 32 | 4 | 3 | 3 | **100** | 5 | 4 | 4 |

(continued)

| | MSP3.1 CT | MSP3.2 CT | MSP3.3 CT | MSP3.4 CT | MSP3.7 CT | MSP3.8 CT | 571-His | BSA |
|---|---|---|---|---|---|---|---|---|
| Anti-***MSP3.8*** CT | 73 | 23 | 26 | 8 | 53 | **100** | 6 | 5 |

This study, which is still ongoing, showed that antibodies affinity purified on the product of one gene cross-reacted with the products of the other genes and *vice-versa* for each of them, which is also indirectly shown by the results obtained in ADCI (Example 1).

The practical consequence at immunological and vaccine development level is that immunisation by any of the members of the gene family will induce antibodies reactive to the same and to all of the remaining gene products.

Therefore, this constitutes a very particular type of multi-gene family where, instead of epitope polymorphism, which is usually the feature of multi-gene families described to-date, epitope conservation is here the main characteristic and where, in case of deletion, mutation in one given gene, another or all other members of the family can take over the antigenic function. In addition all genes are simultaneously expressed by one given parasite.

It is herein proposed that this constitutes not only a preferential vaccine family but also a mechanism developed by the parasite to ensure its survival. The parasite can only survive provided it does not kill its host : by inducing antibodies that reduce parasitemia through the ADCI mechanism, the parasite ensures a sufficient degree of protection of the immune host and therefore ensures its own survival. The epitope duplication provided by the gene family ensures that more than one gene product can fulfil this essential task.

## Example 6 : Results obtained In ADCI with MSP-3-2 peptides

The results obtained in ADCI with MSP-3-2 peptides a, b, c, d, e and F are the same as those obtained with the same peptides from MSP-3-1, *i.e.*, the antibodies directed against the peptides MSP-3-2 b, c, d and e have an ADCI activity, whereas those directed against the peptides MSP-3-2 a and f do not.

## Example 7 : complementarity between responses to MSP3 and GLURP shown in a longitudinal clinical and parasitological follow-up study

### 7.A. MATERIALS AND METHODS

### 7.A.1. Study area, population and clinical surveillance

OoDo village is a re-seftled forested region of Myanmar with a tropical climate characterized by hot dry, monsoon and cool dry seasons. In this area, malaria was found to be stable and hyper-endemic with seasonal variation, the majority of infections were due to *Plasmodium falciparum* (98%) and *Plasmodium vivax* was responsible for the remaining 2%. A malaria attack was defined according to 4 concomitant criteria: i)- corrected axillary temperature ≥ 38.0°C, ii)- absence of other clinical diseases, iii)- presence of asexual *P. falciparum* forms in thick-films, and iv)- clinical and parasitological improvement after chloroquine treatment. Two febrile attacks were regarded as two different malaria episodes if they were separated by ≥72 h. The results of the first 33 months of follow-up have recently been published (Soe, Khin Saw et al. 2001). The same study population was followed-up for one additional year, up to 31st December 1998, using the same protocol. Venous blood samples were drawn during September 1998, and malarial attack rates recorded from January 1st to December 31st 1998 were used to analyze the relationship with clinical protection.

### 7.A.2. Blood sampling and parasitological study

Surveillance of malarial infection was carried out by systematic monthly examination of thick and thin blood films from finger-prick. A slide was regarded as negative if no parasite was visualized in 200 oil-fields in Giemsa stained thick film. For febrile cases two finger-prick films before and after chloroquine treatment were examined. Venous samples were collected in vacutainers, sera aliquoted aseptically, and stored at -20°C until tested. Samples taken from a representative subgroup of 116 villagers from whom more than 60% of the monthly blood films were available for parasitological data were selected from the larger cohort of 292 residents.

### 7.A.3. Antigens

The three recombinant GLURP antigens were derived from the N-terminal non-repeat region R0 ($GLURP_{27-500}$), the central repeat region R1 ($GLURP_{489-705}$), and the C-terminal repeat region R2 ($GLURP_{705-1178}$) of *P. falciparum* F32 (Oeuvray, Theisen et al. 2000). The C-terminal 19-kDa fragment of MSP1, $MSP1_{19}$, from the Wellcome strain (MSP1-W-19) was produced as a recombinant GST-fusion protein in *Escherichia coli* and was a kind gift from Dr. A. Holder, UK. The GST-tag was removed by enzymatic cleavage and subsequent affinity chromatography before use. The MSP3b synthetic peptide (184-AKEASSYDYILGWEFGGGVPEHKKEEN-210, SEQ ID No:5) contained the MSP3b B-cell epitope which reacts with ADCI-effective human antibodies (Oeuvray, Bouharoun-Tayoun et al. 1994).

### 7.A.4. Antibody Assays

The levels of antibodies to the three *P. falciparum*-derived antigens were measured by enzyme-linked immunosorbent assay (ELISA) as previously described (Oeuvray, Theisen et al. 2000). Briefly, microtiter plates (Maxisorb, Nunc, Denmark) were coated overnight at 4°C with recombinant proteins or synthetic peptide at the following concentrations: 0.5μg/ml (R0 and R2), 1 μg/ml (R1 and MSP1) and 5 μg/ml (MSP3b). For GLURP antigens 0.05 M $Na_2CO_3$, pH 9.6 and for MSP1 and MSP3 phosphate buffered saline (PBS) pH 7.4 were used as coating buffers. The next day the plates were washed with PBS plus 0.05% Tween 20 (PBST) and blocked with 2.5% non-fat milk in PBS for 2 h. Sera diluted in PBST containing 1.25 % (w/v) non-fat milk, were added to duplicate wells and incubated for 1 h at room temperature. Various dilutions of sera were made for each antigen: 1:200 for GLURP, 1:100 for MSP1 and 1:20 for MSP3. These dilutions were selected after preliminary pilot studies, which revealed more than a 10-fold difference between control and test samples. Bound antibody was detected by peroxidase-conjugated goat anti-human immunoglobulin (Caltag Laboratories), diluted 1:3000. Color was revealed by O-phenylenediamine (Sigma, St. Louis, Mo.) and $H_2O_2$ in citrate buffer pH 5 for 30 min. The optical density (OD) at 492 nm was determined in a plate reader (Titertek Multiskan MCC 1340). The plates were washed extensively with PBST between each incubation step. All ELISA tests included 6 control sera, randomly selected among 100 French blood donors never exposed to malaria.

For subclasses determination of IgG1-4, monoclonal mouse anti-human subclasses (clones NL16=IgG1 (Boehringer®), HP6002=IgG2 (Sigma®), Zg4=IgG3, and RJ4=IgG4 (both from Immunotech®)) were used. They were diluted 1:2,000, 1:10,000, 1:10,000, and 1:1,000, respectively in 1.25% (w/v) non-fat milk in PBST and incubated for 1 h at room temperature. Goat anti-mouse IgG conjugated to peroxidase (Caltag Laboratories®), diluted 1:3000 in 1.25% (w/v) non-fat milk in PBST was added and incubated for 1 h. Bound labeled antibody was revealed as described above. The dilutions of each isotype-specific monoclonal antibody (MAb) had been determined previously as those discriminating between human Ig subclasses, i.e. yielding no cross-reactions between subclasses (Oeuvray, Theisen et al. 2000). The results for total IgG as well as subclass antibody levels were expressed as ratios of antibody response which were calculated by dividing the mean OD of test with the mean plus 3 SD of the 6 normal controls run simultaneously. A sample with a ratio of ≥1 was considered positive.

### 7.A.5. Statistical analysis

The Mann-Whitney U-test and Spearman's rank-order correlation coefficient were used for the calculations of *P*-values. The association between the risk of malaria attack during 1998 and the levels of antibodies (expressed in ratios) were tested with JMP® software, using either a Poisson regression model where the effect of confounding factors such as age, gender, time spent in the village and transmission were controlled or a logistic regression analysis (with or without occurrence of malaria attack).

### 7.8. RESULTS

### 7.B.1. *P. falciparum* infections in the study cohort

All 116 subjects in the study cohort were from OoDo village situated in Myanmar, South-East Asia, where malaria is hyper-endemic (Soe, Khin Saw et al. 2001). The prevalence of *P. falciparum* parasitemia fluctuated around 40% from January to July and dropped to around 20% from August to December in 1998. The incidence of clinical malaria, which was calculated as the average number of attacks per month in the study cohort and expressed in percentage varied considerably over the year, peaking in June. The infective inoculation rate has not been determined for OoDo, however, Tun-Lin, W et al (Tun-Lin, Thu et al. 1995) found 13.7 infective bites per person per year in a village, which is located 15 km East of OoDo village. The finding agrees well with an estimated number of 11 infective bites per person per year as calculated by the method of (Beier, Killeen et al. 1999). Most infections (98 %) were due to *P. falciparum* (Soe, Khin Saw et al. 2001). During the 12-month period of continuous clinical surveillance, 86 (74%) of the 116 villagers had at least one malaria attack as defined in the Material and Method section and these individuals were considered to be susceptible to malaria. During the same 12-month period, 30 (26%) of the villagers had no episode of clinical malaria, and these individuals were regarded as clinically protected.

### 7.B.2. Antibody recognition of *P. falclparum*-derived MSP3, GLURP, and MSP1 antigens

Levels of IgG, and IgG subclasses against the $MSP3b_{184-210}$ peptide (MSP3b) and the four recombinant proteins representing the $GLURP_{27-500}$ (R0), $GLURP_{489-705}$(R1), $GLURP_{705-1179}$ (R2), and MSP1-19-kDa C-terminal regions were determined in the 116 sera collected during September 1998. R2 was the most frequently recognized antigen by IgG antibodies (67.2%) followed by R1, MSP3b and MSP1 (all at 62%), and R0 (58.6%). The highest OD values were obtained against R0 and R2, whereas MSP3b yielded lower OD values. Levels of IgG against all three GLURP regions and MSP1 were significantly associated with age (Spearman's rank-order correlation coefficient, R=0.51, 0.26, 0.41, and 0.43 for R0, R1. R2, and MSP1, respectively, $P<0.05$) while the IgG response against MSP3 was independent of age (R= 0.16, P=0.17). As for the subclass responses, IgG1 and IgG4 against MSP1, IgG2 against MSP3, IgG1 and IgG3 against R0, IgG2 and IgG3 against R1 and IgG4 against R2, were found significantly associated with age (Table 3). Neither level nor prevalence of positive antibody response varied with gender for any of the antigens tested.

**Table 3.** Relationship between age and the level of subclass antibody responses to each of the antigens studied. *P* and R-values were calculated by the Sperman's Rank correlation Coefficient.

| Antigens | IgG Subclasses | *P* | R |
|---|---|---|---|
| MSP1 | IgG1 | 0.0002 | 0.344 |
| | IgG4 | 0.0009 | 0.309 |
| MSP3 | IgG2 | 0.0090 | 0.242 |
| GLURP antigens | | | |
| R0 | IgG1 | 0.0220 | 0.213 |
| | IgG3 | 0.0040 | 0.268 |
| R1 | IgG2 | 0.0040 | 0.268 |
| | IgG3 | 0.0008 | 0.313 |
| R2 | IgG4 | 0.0140 | 0.231 |

### 7.B.3. Antibody responses and clinical protection

A striking difference between IgG subclass responses and protection was observed for the three different antigens (Table 4). For example, the IgG response against the C-terminal 19-kDa fragment of MSP1 was almost exclusively of the IgG1 subclass with a median value 8.6 times higher in the protected than in the susceptible group whereas, IgG3 antibodies predominated against the MSP3b epitope in protected individuals with a median value 6.5 times higher than that found in susceptible individuals. Although less pronounced, a similar dissimilarity in the cytophilic IgG subclasses response was also observed for different regions of GLURP, IgG1 antibodies predominating against the non-repeat R0-region and IgG3 antibodies prevailing against the R2 repeat-region.

**Table 4.** Median levels (and interquartile range) of IgG subclass antibodies to MSP1, MSP3 and GLURP antigens found in villagers from OoDo considered as either protected from, or susceptible to, *P. falciparum* malaria attacks over 1 year of active and continuous follow-up. Given the number of statistical tests carried out, the Bonferroni's correction factor was applied to determine the level of significance and only *P* values < .0025 were considered significant (*). *P* values were determined from the non-parametric Mann-Whitney U-test. Fold difference refers to the ratio of median values from the two groups. # Values marginally different.

| Antigen | IgG Subclass | Protected group (n=30) | Susceptible group (n=86) | *P* values | Fold difference |
|---|---|---|---|---|---|
| MSP1 | IgG1 | 9.5 (0.8-25.03) | 1.1 (0.5-8.22) | .003 # | 8.6 |
| | IgG2 | 0.9 (0.8-1.26) | 0.9 (0.8-1.09) | >.05 | |
| | IgG3 | 0.9 (0.1-2.63) | 0.4 (0.0-0.81) | >.05 | |
| | IgG4 | 1.2 (0.8-3.01) | 1.0 (0.7-1.47) | .01 | |
| MSP3 | IgG1 | 1.4 (0.8-2.4) | 0.7 (0.4-7.0) | <.001* | 2.0 |
| | IgG2 | 1.1 (0.9-1.57) | 0.8 (0.7-1.0) | >.05 | |
| | IgG3 | 6.5 (2.5-14.03) | 1.0 (0.6-1.49) | <.001* | 6.5 |
| | IgG4 | 1.3 (1.0-1.82) | 1.0 (0.9-1.35) | <.001* | 1.3 |
| R0 | IgG1 | 3.9 (2.4-7.62) | 1.8 (1.0-3.15) | <.001* | 2.2 |
| | IgG2 | 0.9 (0.4-2.5) | 0.8 (0.4-1.33) | >.05 | |
| | IgG3 | 1.3 (0.6-3.76) | 0.9 (0.3-1.44) | .019 | |
| | IgG4 | 0.2 (0.2-2.05) | 0.6 (0.2-1.07) | >.05 | |
| R1 | IgG1 | 0.3 (0.1-0.9) | 0.2 (0.1-0.4) | >.05 | |
| | IgG2 | 0.9 (0.4-1.64) | 0.6 (0.4-0.91) | >.05 | |
| | IgG3 | 1.2 (0.4-2.64) | 0.5 (0.2-0.91) | .039 | |
| | IgG4 | 0.2 (0.2-0.52) | 0.7 (0.2-0.94) | .021 | |
| R2 | IgG1 | 2.0 (0.9-5.4) | 1.1 (0.3-3.11) | .01 | |
| | IgG2 | 2.0 (1.0-4.02) | 0.9 (0.2-1.73) | <.001* | 2.2 |
| | IgG3 | 6.4 (1.7-12.01) | 0.9 (0.3-2.83) | <.001* | 7.1 |
| | IgG4 | 1.0 (0.6-1.2) | 0.6 (0.4-0.85) | .003 | |

Since the antibody titers against GLURP and MSP1 increased as a function of age, the correlation of clinical status of the villagers with various antibodies was reexamined in a logistic-regression model considering age and all the antibody responses (log transformed) as explanatory variables. When testing these parameters in the model and in particular when age was controlled for, among all antibody responses, the strongest predictors of malaria protection identified were increased levels of IgG3 against MSP3b (F ratio= 67.5; *P* < 0.0001) and against GLURP-R0 (F ratio = 23.1; *P* < 0.0001). Other antibodies were not significantly associated with protection. In contrast, the analysis indicated that levels of IgG4 against R0 (F ratio = 4.4; *P* = 0.038) and R1 (F ratio = 3.9; *P* = 0.051) increased with the number of malaria attacks, i.e. they were to some extent predictive of susceptibility to malaria.

**7.B.4. Antigen specificity of IgG3 responses in protected villagers**

Figure 14A shows the general pattern of IgG3 antibody responses found against the different blood stage antigens in OoDo. The range of values was large for most antigen-specific antibody responses and this suggested that different subgroups of "responders" might exist. Sera of villagers who were protected from clinical malaria did not all show high IgG3 values against both MSP3b and GLURP-R0. Some individuals displayed an unexpectedly low IgG3 reactivity against either one of these 2 antigens. In an attempt to understand how these villagers were protected against malaria attacks, two sub-groups were identified, characterized by : a)- low IgG3 responses against MSP3 (7 out of 30 cases) or b)- low IgG3 responses against R0 (15 out of 30 cases). The levels of IgG3 antibodies against the other three antigens were estimated (Fig. 14B). The 7 protected individuals (mean age ± 1std error = 33.9 ± 7.0 years) with a low IgG3 response to MSP3b (IgG3 ratio= 1.26 ± .22) were found to have a strong IgG3 response to R0 (IgG3 ratio= 9.09 ± 3.41) and to R2 (IgG3 ratio= 8.36 ± 5.86). In the 2nd subgroup of 15 other individuals (24.7 ± 4.3 years of age) also protected despite a low IgG3 response to GLURP-R0 (IgG3 ratio = 0.55 ± .08), the reverse situation was found (Figure 14C ) : a high IgG3 antibody response against MSP3b was observed (IgG3 ratio=10.45 ± 2.07) and to a lesser extent against GLURP-R2 (IgG3 ratio=4.43 ± .80). The titers for those responding to only one antigen tended to be higher than those responding to both antigens (Table 4). The number of years spent in OoDo village did not significantly differ between the groups of low-responders to MSP3 (20.43 ± 4.70 years of residence) and R0 (18.7 ± 10.6 years of residence).

Sera from 13 of the 30 individuals considered as protected in 1998 had also been sampled in 1993, and therefore were used to compare at 5 years interval the relative levels of anti-MSP3 and anti-RO IgG1 and IgG3 antibodies. As shown in Fig 14D and 14E, there was no major change detectable in the levels of specific IgG1 against the two antigens. In contrast, levels of IgG3 antibodies to both MSP3 and GLURP increased from 1993 to 1998. In the subgroup of 7 individuals with elevated IgG3 against MSP3 in 1998 (Fig 14D), the difference corresponded to a 1.67 times increase (*P*=.11). In the subgroup of 6 subjects with elevated IgG3 against R0 in 1998 (Fig 14E), the difference corresponded to a more important, ie. 3.93 times increase (*P*=.05). The 6 individuals with high IgG3 responses against MSP3 in 1998 also had high titers 5 years earlier, suggesting that they were already protected via a sustained anti-MSP3 IgG3 response in 1993, when they were 18.2±9.8 years of age. In contrast, for GLURP the 7 individuals with a strong anti-R0 IgG3 response detectable in 1998 had substantially lower anti-R0 IgG3 responses 5 years earlier (*P*=0.0157), when they were 23.7±6.9 years of age. Thus, there was a drastic change in those 7 individuals protected via IgG3 to R0 in 1998 and their protection 5 years earlier was possibly related to IgG3 against MSP3.

## 7.C. DISCUSSION

The present study is the first one to show an association between antigen-specific antibody responses and protection from clinical malaria in S-E Asia. The prevalence of positive antibody responses against GLURP and MSP3 was high in OoDo, ranging from 58.6 % (R0) to 67.2% (R2). This observation is in-keeping with the finding that B-cell epitopes within GLURP and MSP3 are highly conserved among *P. falciparum* laboratory lines and field isolates from Africa and Asia (Huber, Felger et al. 1997),(McColl and Anders 1997; de Stricker, Vuust et al. 2000). The prevalence of antibodies to MSP1-W-19 was also high, being almost twice the values found in the Gambia and Sierra Leone (Egan, Morris et al. 1996) and in Ghana (Dodoo, Theander et al. 1999) suggesting that strains related to the Wellcome strain might be prevalent in OoDo.

The highest ELISA-titers were found against the recombinant GLURP RO- and R2-regions and MSP1. The differences in GLURP-R0, -R1, -R2 and MSP1 ELISA-titers very likely reflected differences in serum antibody reactivity. In contrast, the MSP3-ELISA gave comparatively lower signals, however this discrepancy might be at least in part, related to the use of a short synthetic peptide defining a single or limited number of epitopes, as compared to recombinant proteins in the case of GLURP and MSP1 which are known to define several epitopes (Theisen, Soe et al. 2000).

Levels of IgG against all GLURP regions and MSP1 were significantly associated with age ($P<0.05$) while in contrast to this situation, the IgG response against MSP3 was found independent of age. Regarding IgG subclass responses, several of them were also found significantly associated with age and these variations could reflect the duration of exposure to the malaria parasites as well as the gradual maturation of the immune system over time.

There was a statistically significant increase in the levels of IgG3 against R0 and MSP3 among the protected individuals living in OoDo as compared to the non-protected ones. These results are in agreement with those of Dodoo et al. (Dodoo, Theisen et al. 2000), who found that cytophilic antibody responses against R0 and R2 were strong predictors of protection in Ghanaian children, and those of Oeuvray et al., who found a consistent correlation between protection and elevated IgG3 against both GLURP-R0 and R2 in Dielmo, West Africa (Oeuvray, Theisen et al. 2000). Similarly MSP3-specific IgG3 responses have previously been associated with protection against clinical malaria in Dielmo. Altogether, these results suggest that the same subclass of IgG response to the same critical epitopes are involved in the gradual development of protection against *P. falciparum* malaria in African as well as in Asian populations living in malaria endemic areas. In addition, the present study found a significant negative correlation between the levels of non-cytophilic antibodies against R0 and R1 and clinical protection. Therefore, on the one hand, there is a positive association between cytophilic IgG subclass responses and protection and on the other hand, a negative association between non-cytophilic subclass responses with the same epitope specificity and protection. This epidemiological result is in agreement with the *in vitro* observation that non-cytophilic antibodies can inhibit the bridging of merozoites and human monocytes by cytophilic antibodies against the same antigenic target and thereby reduce the ability of the latter to control parasite multiplication by the ADCI mechanism (Bouharoun-Tayoun and Druilhe 1992).

Whereas most of the protected residents of OoDo had high IgG3 responses against both MSP3 and GLURP, a number of individuals with low or almost no IgG3 responses against either one of these antigens also appeared to be protected. The inventors found that all the protected individuals with low GLURP-R0 specific IgG3 response had significantly elevated levels of specific anti-MSP3 IgG3 antibodies, and vice versa. This observation suggests that antibodies against GLURP and MSP3 may act in a complementary manner to control parasite multiplication in immune individuals. This is relevant in consideration of the role of these antibodies in ADCI mechanism. Indeed, only the simultaneous assessment of several antigens disclosed this complementary effect. This finding is in favor of testing simultaneously several antigens for complementary as for possible antagonistic effects that could have consequences on the design of combined vaccines.

In conclusion, the present study shows that (1)- the critical epitopes in the MSP3 and GLURP antigens which are most conserved, are targets of protective antibodies in geographically distant endemic areas of the world. (2)- IgG3 antibodies to MSP3 and GLURP-R0 are the strongest predictors of protection from clinical malaria in an African and also an Asian setting. (3)- To reach a state of premunition in Asia as well as in Africa, it is needed to produce a cytophilic subclass of antibody against critical antigens (namely, MSP3b and GLURP which both induce antibodies active in ADCI). (4)- There appears to be a complementation effect between these two antigens. IgG3 responses might have similar effects against the risk of malarial attacks, provided they are present against one antigen when responses to the other are low or almost absent. (5) - The responses to different B cell epitopes on a given antigen appear to evolve independantly and the level of recognition can change over time.

The complementarity of responses observed to the two main targets of ADCI identified to date provide the first rational basis for combining these two antigens in a hybrid vaccine formulation. Moreover, immunogenicity studies performed in pre-clinical animal models with the hybrid vaccine lend further support to this antigen combination by showing improved immunogenicity with well-balanced, equilibrated responses to each molecule.

## REFERENCES

Badell, E., C. Oeuvray, et al. (2000). "Human malaria in immunocompromised mice: an in vivo model to study defense mechanisms against Plasmodium falciparum." J Exp Med 192(11): 1653-60.

Beier, J. C., G. F. Killeen, et al. (1999). "Short report: entomologic inoculation rates and Plasmodium falciparum malaria prevalence in Africa." Am J Trop Med Hyg 61(1): 109-13.

BenMohamed, L., Y. Belkaid, et al. (2002). "Systemic immune responses induced by mucosal administration of lipopeptides without adjuvant." Eur J Immunol 32(8): 2274-81.

Bottius, E., L. BenMohamed, et al. (1996). "A novel Plasmodium falciparum sporozoite and liver stage antigen (SALSA) defines major B, T helper, and CTL epitopes." J Immunol 156(8): 2874-84.

Bouharoun-Tayoun, H. and P. Druilhe (1992). "Antibodies in falciparum malaria: what matters most, quantity or quality?" Mem Inst Oswaldo Cruz 87 Suppl 3: 229-34.

Bouharoun-Tayoun, H., C. Oeuvray, et al. (1995). "Mechanisms underlying the monocyte-mediated antibody-dependent killing of Plasmodium falciparum asexual blood stages:" J Exp Med 182(2): 409-18.

Brahimi, K., J. L. Perignon, et al. (1993). "Fast immunopurification of small amounts of specific antibodies on peptides bound to ELISA plates." J Immunol Methods 162(1): 69-75.

Dame, J. B., J. L. Williams, et al. (1984). "Structure of the gene encoding the immunodominant surface antigen on the sporozoite of the human malaria parasite Plasmodium falciparum." Science 225(4662): 593-9.

Daubersies, P., A. W. Thomas, et al. (2000). "Protection against Plasmodium falciparum malaria in chimpanzees by immunization with the conserved pre-erythrocytic liver-stage antigen 3." Nat Med 6(11): 1258-63.

de Stricker, K., J. Vuust, et al. (2000). "Conservation and heterogeneity of the glutamate-rich protein (GLURP) among field isolates and laboratory lines of Plasmodium falciparum." Mol Biochem Parasitol 111(1): 123-30.

Dodoo, D., T. G. Theander, et al. (1999). "Levels of antibody to conserved parts of Plasmodium falciparum merozoite surface protein 1 in Ghanaian children are not associated with protection from clinical malaria." Infect Immun 67(5): 2131-7.

Dodoo, D., M. Theisen, et al. (2000). "Naturally acquired antibodies to the glutamate-rich protein are associated with protection against Plasmodium falciparum malaria." J Infect Dis 181(3): 1202-5.

Egan, A. F., J. Morris, et al. (1996). "Clinical immunity to Plasmodium falciparum malaria is associated with serum antibodies to the 19-kDa C-terminal fragment of the merozoite surface antigen, PfMSP-1." J Infect Dis 173(3): 765-9.

Escalante, A. A., H. M. Grebert, et al. (2001). "Polymorphism in the gene encoding the apical membrane antigen-1 (AMA-1) of Plasmodium falciparum. X. Asembo Bay Cohort Project." Mol Biochem Parasitol 113(2): 279-87.

Fidock, D. A., E. Bottius, et al. (1994). "Cloning and characterization of a novel Plasmodium falciparum sporozoite surface antigen, STARP." Mol Biochem Parasitol 64(2): 219-32.

Gras-Masse, H., B. Georges, et al. (1999). "Convergent peptide libraries, or mixotopes, to elicit or to identify specific immune responses." Curr Opin Immunol 11(2): 223-8.

Guerih-Marchand, C., P. Druilhe, et al. (1987). "A liver-stage-specific antigen of Plasmodium falciparum characterized by gene cloning." Nature 329(6135): 164-7.

Huber, W., I. Felger, et al. (1997). "Limited sequence polymorphism in the Plasmodium falciparum merozoite surface protein 3." Mol Biochem Parasitol 87(2): 231-4.
Khusmith, S. and P. Druilhe (1983). "Cooperation between antibodies and monocytes that inhibit in vitro proliferation of Plasmodium falciparum." Infect Immun 41(1): 219-23.
Knapp, B., E. Hundt, et al. (1989). "Molecular cloning, genomic structure and localization in a blood stage antigen of Plasmodium falciparum characterized by a serine stretch." Mol Biochem Parasitol 32(1): 73-83.
Lunel, F. and P. Druilhe (1989). "Effector cells involved in nonspecific and antibody-dependent mechanisms directed against Plasmodium falciparum blood stages in vitro." Infect Immun 57(7): 2043-9.
Marshall, V. M., W. Tieqiao, et al. (1998). "Close linkage of three merozoite surface protein genes on chromosome 2 of Plasmodium falciparum." Mol Biochem Parasitol 94(1): 13-25.
McColl, D. J. and R. F. Anders (1997). "Conservation of structural motifs and antigenic diversity in the Plasmodium falciparum merozoite surface protein-3 (MSP-3)." Mol Biochem Parasitol 90(1): 21-31.
Miller, L. H., T. Roberts, et al. (1993). "Analysis of sequence diversity in the Plasmodium falciparum merozoite surface protein-1 (MSP-1)." Mol Biochem Parasitol 59(1): 1-14.
Oeuvray, C., H. Bouharoun-Tayoun, et al. (1994). "Merozoite surface protein-3: a malaria protein inducing antibodies that promote Plasmodium falciparum killing by cooperation with blood monocytes." Blood 84(5): 1594-602.
Oeuvray, C., M. Theisen, et al. (2000). "Cytophilic immunoglobulin responses to Plasmodium falciparum glutamate-rich protein are correlated with protection against clinical malaria in Dielmo, Senegal." Infect Immun 68(5): 2617-20.
Peterson, M. G., V. M. Marshall, et al. (1989). "Integral membrane protein located in the apical complex of Plasmodium falciparum." Mol Cell Biol 9(7): 3151-4.
Pleass, R. J. and J. M. Woof (2001). "Fc receptors and immunity to parasites." Trends Parasitol 17(11): 545-51.
Robson, K. J., J. R. Hall, et al. (1988). "A highly conserved amino-acid sequence in thrombospondin, properdin and in proteins from sporozoites and blood stages of a human malaria parasite." Nature 335(6185): 79-82.
Roussillon, C. (1999). "Correlates of Immune protection in malaria." MIM African Malaria Conference, 14-19 March, Durban South Africa.
Simmons, D., G. Woollett, et al. (1987). "A malaria protein exported into a new compartment within the host erythrocyte." Embo J 6(2): 485-91.
Soe, S., A. Khin Saw, et al. (2001). "Premunition against Plasmodium falciparum in a malaria hyperendemic village in Myanmar." Trans R Soc Trop Med Hyg 95(1): 81-4.
Theisen, M., D. Dodoo, et al. (2001). "Selection of glutamate-rich protein long synthetic peptides for vaccine development: antigenicity and relationship with clinical protection and immunogenicity." Infect Immun 69(9): 5223-9.
Theisen, M., S. Soe, et al. (2000). "Identification of a major B-cell epitope of the Plasmodium falciparum glutamate-rich protein (GLURP), targeted by human antibodies mediating parasite killing." Vaccine 19(2-3): 204-12.
Theisen, M., S. Soe, et al. (1998). "The glutamate-rich protein (GLURP) of Plasmodium falciparum is a target for antibody-dependent monocyte-mediated inhibition of parasite growth in vitro." Infect Immun 66(1): 11-7.
Thomas, A. W., D. A. Carr, et al. (1990). "Sequence comparison of allelic forms of the Plasmodium falciparum merozoite surface antigen MSA2." Mol Biochem Parasitol 43(2): 211-20.
Trucco, C., D. Fernandez-Reyes, et al. (2001). "The merozoite surface protein 6 gene codes for a 36 kDa protein associated with the Plasmodium falciparum merozoite surface protein-1 complex." Mol Biochem Parasitol 112(1): 91-101.
Tun-Lin, W., M. M. Thu, et al. (1995). "Hyperendemic malaria in a forested, hilly Myanmar village." J Am Mosq Control Assoc 11(4): 401-7.

## Claims

1. An antigenic Polypeptide from the MSP3.3 protein of SEQ ID NO:6 comprising at least the MSP-3-c/d-like motif as defined in SEQ ID NO:27.

2. An antigenic polypeptide from the MSP3.3 protein of SEQ ID NO:6 comprising at least one MSP-3-c/d-like motif obtained by conservative substitution of at least one amino-acid of SEQ ID NO:27, said motif being recognized by an antibody directed against the MSP-3-c/d-like motif as defined in SEQ ID NO:27, provided that said antigenic polypeptide is not a fragment of MSP-3-1 of SEQ ID NO: 2 or a fragment of MSP-3-2 of SEQ ID NO: 4.

3. An antigenic polypeptide according to claim 2, comprising at least one MSP-3-c/d-like motif exhibiting at least 90% identity with the motif as defined in SEQ ID NO:27.

4. An antigenic polypeptide according to any one of claims 1 to 3 further comprising at least a MSP-3-b-like motif having a sequence selected amongst the sequences of SEQ ID Nos: 17 to 24.

5. An antigenic polypeptidic composition comprising a MSP-3-c/d-like motif as defined in any one of claims 1 to 4 and at least another MSP-3-c/d-like motif selected in the group consisting of:

   a. SEQ ID NO:25, SEQ ID NO:26, SEQ ID NO:28, SEQ ID NO:29, SEQ ID NO:30;
   b. a MSP-3-c/d-like motif obtained by conservative substitution of at least one amino-acid in a sequence selected in the group consisting having a sequence of SEQ ID NO:25, SEQ ID NO:26, SEQ ID NO:28, SEQ ID NO:29 or SEQ ID NO:30, said motif being recognized by an antibody directed against any of the MSP-3-c/d-like motif as defined in SEQ ID NO:25 to SEQ 10 NO:30; and
   c. a MSP-3-c/d-like motif exhibiting at least 90% identity with SEQ ID NO:25, SEQ ID NO:26. SEQ ID NO:28, SEQ ID NO:29 and SEQ ID NO:30;

6. An antigenic polypeptide composition according to claim 5 comprising a MSP-3-c/d-like motif as defined in SEQ ID NO:27 and at least another MSP-3-c/d-like motif selected in the group consisting of SEQ ID NO:25, SEQ ID NO: 26, SEQ ID NO:28, SEQ ID NO:29 and SEQ ID NO:30.

7. An antigenic polypeptide composition according to claim 5 or 6. wherein said at least two different MSP-3-c/d-like motifs are carried by distinct molecules.

8. An antigenic polypeptide composition according to claim 5 or 6, wherein said at least two different MSP-3-c/d-like motifs are carried by a unique molecule.

9. The antigenic polypeptidic composition according to any one of claims 5 to 8, further comprising an antigenic polypeptide comprising at least 10 consecutive amino acid residues from the R0 region of GLURP.

10. The antigenic polypeptidic composition according to any one of claims 5 to 7, which is a mixotope comprising synthetic peptides comprising the sequence:

    $L\text{-}X_1X_2X_3\text{-}X_4\text{-}X_3\text{-}X_5\text{-}X_6\text{-}X_7\text{-}D\text{-}X_8\text{-}X_9\text{-}X_{10}\text{-}I\text{-}X_{11}\text{-}X_{12}X_{13}\text{-}X_{14}\text{-}X_{15}\text{-}X_{16}$

    (SEQ ID No:33), wherein:

$X_1$ = E, or S;
$X_2$ = L, H, S or Q;
$X_3$ = I, V or L;
$X_4$ = K, N, Y or P;
$X_5$ = T, S or P;
$X_6$ = S or L;
$X_7$ = K, W or S;
$X_8$ = E, K, R or I;
$X_9$ = E or N;
$X_{10}$ = D, N or Q;
$X_{11}$ = I, V, S, P or A;
$X_{12}$ = K, D or N;
$X_{13}$ = H or E;
$X_{14}$ = N or S;
$X_{15}$ = E or D; and
$X_{16}$ = D or Q.

**11.** The antigenic polypeptidic composition according to claim 10, which is a mix of at least 50, at least 100, or at least 500 peptides of different sequences.

**12.** The antigenic polypeptide according to any one of claims 1 to 4, or the antigenic polypeptidic composition according to any one of claims 5 to 11, wherein a lipidic molecule is linked to at least part of the polypeptidic molecules.

**13.** The antigenic polypeptide or polypeptidic composition according to claim 12, wherein the lipidic molecule is a C-terminal palmitoylysylamide residue.

**14.** The antigenic polypeptide according to any of claims 1 to 4, or the antigenic polypeptidic composition according to any of claims 5 to 13, wherein at least part of the polypeptidic molecules are bound to a support.

**15.** The antigenic polypeptide or polypeptidic composition according to claim 14, wherein the support is viral particles, or nitrocellulose or polystyrene beads, or a biodegradable polymer

**16.** The antigenic polypeptide or polypeptidic composition according to claim 15, wherein said biodegradable polymer is lipophosphoglycanes or poly-L lactic acid.

**17.** An immunogenic composition comprising, as an immunogen, an antigenic polypeptide according to any one of claims 1 to 4 and 12 to 16, or a polypeptidic composition according to any one of claims 5 to 15.

**18.** A vaccine against malaria comprising, as an immunogen, an antigenic polypeptide according to any one of claims 1 to 4 and 12 to 16, or a polypeptidic composition according to any one of claims 5 to 15, in association with a suitable pharmaceutical vehicle.

**19.** The immunogenic composition of claim 17 or the vaccine of claim 18, further comprising at least one antigen selected amongst LSA-1, LSA-3, LSA-5, SALSA, STARP, TRAP, PfEXP1, CS, MSP1, MSP2, MSP4, MSP5, AMA-1, SERP and GLURP.

**20.** The immunogenic composition or the vaccine according to any of claims 17 to 20, which is formulated for intradermal or intramuscular injection.

**21.** The immunogenic composition or vaccine of claim 20, comprising between 1 and 100 μg of immunogen per injection dose.

**22.** The immunogenic composition or vaccine of claim 20, comprising between 2 and 50 μg of immunogen per injection dose

**23.** The immunogenic composition or vaccine of any one of claims 17 to 22, further comprising SBAS2 and/or Alum and/or Montanide as an adjuvant.

**24.** Use of an antigenic polypeptide according to any one of claims 1 to 4 and 12 to 16, or a polypeptidic composition according to any one of claims 5 to 16, for the preparation of a vaccine composition against malaria.

**25.** A synthetic or recombinant antibody directed against the MSP-3-c/d-like motif of an antigenic polypeptide as defined in any one of claims 1 to 3.

**26.** A pool of antibodies directed against a polypeptidic composition according to any one of claims 5 to 16.

**27.** An antibody according to claim 25, or a pool of antibodies according to claim 26, wherein said antibodies are human or humanized antibodies.

**28.** Use of a composition comprising an antibody or a pool of antibodies according to any one of claims 25 to 27, for the preparation of a medicament against malaria.

**29.** A medicament for passive immunotherapy of malaria, comprising an antibody or a pool of antibodies according to any one of claims 25 to 27.

**30.** The medicament of claim 29, further comprising antibodies directed against at least one antigen selected amongst LSA-1, LSA-3, LSA-5, SALSA, STARP, TRAP, PfEXP1, CS, MSP1, MSP2, MSP4, MSP5, AMA-1, SERP and GLURP.

**31.** A method for the *in vitro* diagnosis of malaria in an individual likely to be infected by *P. falciparum*, which comprises the bringing of a biological sample from said individual into contact with an antigenic polypeptide of any one of claims 1 to 4, under conditions enabling the formation of antigen/antibody complexes between said antigenic peptide or polypeptide and the antibodies possibly present in the biological sample, and the *in vitro* detection of the antigen/antibody complexes possibly formed.

**32.** The method of claim 31, wherein the in vitro diagnosis is performed by an ELISA assay.

**33.** The method of claim 31 or 32, wherein the biological sample is further brought into contact with one or several antigenic peptide originating from other antigens selected amongst LSA-1, LSA-3, LSA-5, SALSA, STARP, TRAP, PfEXP1, CS, MSP-3-1, MSP-3-2, MSP-3-5, MSP-3-6, MSP1, MSP2, MSP4, MSP5, AMA-1, SERP and GLURP.

**34.** A kit for the *in vitro* diagnosis of malaria, comprising at least one antigenic polypeptide as defined in any of claims 1 to 4.

**35.** The kit of claim 34, wherein the antigenic peptide or polypeptide is bound to a support.

**36.** The kit of claim 34 or 35, further comprising reagents for enabling the formation of antigen/antibody complexes between said antigenic peptide or polypeptide and the antibodies possibly present in a biological sample, and reagents enabling the *in vitro* detection of the antigen/antibody complexes possibly formed.

**37.** A method for the *in vitro* diagnosis of malaria in an individual likely to be infected by *P, falciparum*, which comprises the bringing of a biological sample from said individual into contact with antibodies as defined in any one of claims 25 to 27, under conditions enabling the formation of antigen/antibody complexes between said antibodies and the antigens specific for *P. falciparum* possibly present in the biological sample, and the *in vitro* detection of the antigen/antibody complexes possibly formed.

**38.** A kit for the *in vitro* diagnosis of malaria, comprising antibodies as defined in any one of claims 25 to 27.

**39.** The kit of claim 38, further comprising reagents for enabling the formation of antigen/antibody complexes between said antibodies and antigens from the proteins of the MSP-3 family possibly present in a biological sample, and reagents enabling the *in vitro* detection of the antigen/antibody complexes possibly formed.

**40.** A recombinant nucleotide sequence encoding an antigenic polypeptide as defined in any of claims 1 to 4.

**41.** The recombinant nucleotide sequence according to claim 40, comprising a sequence encoding a fusion protein comprising several MSP-3-c/d-like motifs, wherein at least two of said motifs are selected amongst the motifs of SEQ ID NOs: 25 to 30.

**42.** A recombinant cloning and/or expression vector, comprising a nucleotide sequence according to any one of claims 40 to 41.

**43.** The recombinant cloning and/or expression vector of claim 42, wherein the nucleotide sequence is under the control of a promoter and regulatory elements for expression in a host cell, said promoter and regulatory elements being homologous or heterologous vis-à-vis the host cell.

**44.** Use of an expression vector according to claim 43, for the preparation of a medicament for genetic immunisation against *Plasmodium falciparum.*

**45.** A DNA vaccine comprising a nucleotide sequence according to any one of claims 40 to 41.

**46.** A recombinant host cell, which is transformed by the vector of claim 42 or 43.

**47.** The host cell of claim 46, which is a bacterium, a yeast, an insect cell, or a mammalian cell.

**48.** An antigenic polypeptide according to any one of claims 1 to 4 and 12 to 16, or a polypeptidic composition according to any one of claims 5 to 16, for use as a vaccine composition against malaria.

**49.** A composition comprising an antibody or a pool of antibodies according to any one of claims 25 to 27, for use as a medicament against malaria

**50.** An expression vector according to claim 42 or 43, for use as a medicament for genetic immunisation against *Plasmodium falciparum*.

**Patentansprüche**

**1.** Antigenes Polypeptid des MSP3.3 Proteins aus SEQ ID Nr. 6, umfassend mindestens das in SEQ ID Nr. 27 definierte MSP-3-c/d-ähnliche Motiv.

**2.** Antigenes Polypeptid des MSP3.3 Proteins aus SEQ ID Nr. 6, umfassend mindestens ein MSP-3-c/d-ähnliches Motiv, welches durch konservative Substitution mindestens einer Aminosäure der SEQ ID Nr. 27 erhalten wurde, wobei das Motiv durch einen Antikörper erkannt wird, der gegen das in SEQ ID Nr. 27 definierte MSP-3-c/d-ähnliche Motiv gerichtet ist, vorausgesetzt, dass das antigene Polypeptid nicht ein Fragment von MSP-3-1 der SEQ ID Nr. 2 oder ein Fragment von MSP-3-2 der SEQ ID Nr. 4 ist.

**3.** Antigenes Polypeptid nach Anspruch 2, umfassend mindestens ein MSP-3-c/d-ähnliches Motiv, das mindestens 90 % Übereinstimmung mit dem in SEQ ID Nr. 27 definierten Motiv aufweist.

**4.** Antigenes Polypeptid nach einem der Ansprüche 1 bis 3, weiter umfassend mindestens ein MSP-3-b-ähnliches Motiv mit einer Sequenz ausgewählt unter den Sequenzen der SEQ ID Nr. 17 bis 24.

**5.** Antigene Polypeptidzusammensetzung, umfassend ein in einem der Ansprüche 1 bis 4 definiertes MSP-3-c/d-ähnliches Motiv und mindestens ein weiteres MSP-3-c/d-ähnliches Motiv ausgewählt aus der Gruppe bestehend aus:

a. SEQ ID Nr. 25, SEQ ID Nr. 26, SEQ ID Nr. 28, SEQ ID Nr. 29, SEQ ID Nr. 30;
b. einem MSP-3-c/d-ähnlichen Motiv, welches durch konservative Substitution mindestens einer Aminosäure in einer Sequenz erhalten wurde, die ausgewählt ist aus der Gruppe bestehend aus SEQ ID Nr. 25, SEQ ID Nr. 26, SEQ ID Nr. 28, SEQ ID Nr. 29, SEQ ID Nr. 30, wobei das Motiv durch einen Antikörper erkannt wird, der gegen jegliches der in SEQ ID Nr. 25 bis SEQ ID Nr. 30 definierten MSP-3-c/d-ähnlichen Motive gerichtet ist; und
c. ein MSP-3-c/d-ähnliches Motiv, das mindestens 90 % Übereinstimmung mit SEQ ID Nr. 25, SEQ ID Nr. 26, SEQ ID Nr. 28, SEQ ID Nr. 29, SEQ ID Nr. 30 aufweist.

**6.** Antigene Polypeptidzusammensetzung nach Anspruch 5, umfassend ein wie in SEQ ID Nr. 27 definiertes MSP-3-c/d-ähnliches Motiv und mindestens ein weiteres MSP-3-c/d-ähnliches Motiv ausgewählt aus der Gruppe bestehend aus SEQ ID Nr. 25, SEQ ID Nr. 26, SEQ ID Nr. 28, SEQ ID Nr. 29 und SEQ ID Nr. 30.

**7.** Antigene Polypeptidzusammensetzung nach Anspruch 5 oder 6, worin die mindestens zwei unterschiedlichen MSP-3-c/d-ähnlichen Motive durch verschiedene Moleküle getragen werden.

**8.** Antigene Polypeptidzusammensetzung nach Anspruch 5 oder 6, worin die mindestens zwei unterschiedlichen MSP-3-c/d-ähnlichen Motive durch ein einzelnes Molekül getragen werden.

**9.** Antigene Polypeptidzusammensetzung nach einem der Ansprüche 5 bis 8, weiter umfassend ein antigenes Polypeptid, umfassend mindestens 10 aufeinander folgende Aminosäurereste der R0 Region von GLURP.

**10.** Antigene Polypeptidzusammensetzung nach einem der Ansprüche 5 bis 7, die ein Mixotop ist, umfassend synthetische Peptide, die die Sequenz umfassen:

$$L\text{-}X_1\text{-}X_2\text{-}X_3\text{-}X_4\text{-}X_3\text{-}X_5\text{-}X_6\text{-}X_7\text{-}D\text{-}X_8\text{-}X_9\text{-}X_{10}\text{-}I\text{-}X_{11}\text{-}X_{12}\text{-}X_{13}\text{-}X_{14}\text{-}X_{15}\text{-}X_{16}$$

(SEQ ID Nr. 33), worin

$X_1$= E oder S;
$X_2$= L, H, S oder Q;
$X_3$= I, V oder L;
$X_4$= K, N, Y oder P;
$X_5$= T, S oder P;
$X_6$= S oder L;
$X_7$= K, W oder S;
$X_8$= E, K, R oder I;
$X_9$= E oder N;
$X_{10}$= D, N oder Q;
$X_{11}$= I, V, S, P oder A;
$X_{12}$= K, D oder N;
$X_{13}$= H oder E;
$X_{14}$= N oder S;
$X_{15}$= E oder D; und
$X_{16}$= D oder Q.

**11.** Antigene Polypeptidzusammensetzung nach Anspruch 10, welche ein Gemisch aus mindestens 50, mindestens 100 oder mindestens 500 Peptiden unterschiedlicher Sequenz ist.

**12.** Antigenes Polypeptid nach einem der Ansprüche 1 bis 4 oder antigene Polypeptidzusammensetzung nach einem der Ansprüche 5 bis 11, worin ein lipidisches Molekül mit mindestens einem Teil der polypeptidischen Moleküle vernetzt ist.

**13.** Antigenes Polypeptid oder antigene Polypeptidzusammensetzung nach Anspruch 12, worin das lipidische Molekül ein C-terminaler Palmitoylysylamidrest ist.

**14.** Antigenes Polypeptid nach einem der Ansprüche 1 bis 4 oder antigene Polypeptidzusammensetzung nach einem der Ansprüche 5 bis 13, worin mindestens ein Teil der polypeptidischen Moleküle an einen Träger gebunden sind.

**15.** Antigenes Polypeptid oder antigene Polypeptidzusammensetzung nach Anspruch 14, worin der Träger virale Partikel oder Nitrozellulose oder Polystyrolkügelchen oder ein biologisch abbaubares Polymer ist.

**16.** Antigenes Polypeptid oder antigene Polypeptidzusammensetzung nach Anspruch 15, worin das biologisch abbaubare Polymer Lipophosphoglycane oder poly-L Milchsäure ist.

**17.** Immunogene Zusammensetzung, umfassend ein antigenes Polypeptid nach einem der Ansprüche 1 bis 4 und 12 bis 16 oder eine Polypeptidzusammensetzung nach einem der Ansprüche 5 bis 15 als ein Immunogen.

**18.** Impfstoff gegen Malaria, umfassend ein antigenes Polypeptid nach einem der Ansprüche 1 bis 4 und 12 bis 16 oder eine Polypeptidzusammensetzung nach einem der Ansprüche 5 bis 15 als ein Immunogen in Verbindung mit einem geeigneten pharmazeutischen Träger.

**19.** Immunogene Zusammensetzung nach Anspruch 17 oder Impfstoff nach Anspruch 18, weiter umfassend mindestens ein Antigen ausgewählt unter LSA-1, LSA-3, LSA-5, SALSA, STARP, TRAP, PfEXP1, CS, MSP1, MSP2, MSP4, MSP5, AMA-1, SERP und GLURP.

**20.** Immunogene Zusammensetzung oder Impfstoff nach einem der Ansprüche 17 bis 20, formuliert für intradermale oder intramuskuläre Injektion.

**21.** Immunogene Zusammensetzung oder Impfstoff nach Anspruch 20, umfassend zwischen 1 und 100 μg Immunogen pro Injektionsdosis.

**22.** Immunogene Zusammensetzung oder Impfstoff nach Anspruch 20, umfassend zwischen 2 und 50 μg Immunogen pro Injektionsdosis.

**23.** Immunogene Zusammensetzung oder Impfstoff nach einem der Ansprüche 17 bis 22, weiter umfassend SBAS2 und/oder Alum und/oder Montanid als Zusatz.

**24.** Verwendung eines antigenen Polypeptids nach einem der Ansprüche 1 bis 4 und 12 bis 16 oder einer Polypeptidzusammensetzung nach einem der Ansprüche 5 bis 16 zur Herstellung einer Impfstoffzusammensetzung gegen Malaria.

**25.** Synthetischer oder rekombinanter Antikörper, gerichtet gegen das MSP-3-c/d-ähnliche Motiv eines in einem der Ansprüche 1 bis 3 definierten antigenen Polypeptids.

**26.** Pool von Antikörpern, gerichtet gegen eine Polypeptidzusammensetzung nach einem der Ansprüche 5 bis 16.

**27.** Antikörper nach Anspruch 25 oder Pool von Antikörpern nach Anspruch 26, worin die Antikörper humane oder humanisierte Antikörper sind.

**28.** Verwendung einer Zusammensetzung umfassend einen Antikörper oder einen Pool von Antikörpern nach einem der Ansprüche 25 bis 27 zur Herstellung eines Medikaments gegen Malaria.

**29.** Medikament für die passive Immuntherapie von Malaria, umfassend einen Antikörper oder einen Pool von Antikörpern nach einem der Ansprüche 25 bis 27.

**30.** Medikament nach Anspruch 29, weiter umfassend Antikörper gerichtet gegen mindestens ein Antigen ausgewählt unter LSA-1, LSA-3, LSA-5, SALSA, STARP, TRAP, PfEXP1, CS, MSP1, MSP2, MSP4, MSP5, AMA-1, SERP und GLURP.

**31.** Verfahren zur in vitro Diagnose von Malaria in einem Individuum, das wahrscheinlich mit *P. falciparum* infiziert ist, umfassend

Inkontaktbringen einer biologischen Probe des Individuums mit einem antigenen Polypeptid nach einem der Ansprüche 1 bis 4 unter Bedingungen, die die Bildung von Antigen/Antikörper Komplexen zwischen dem antigenen Peptid oder Polypeptid und den möglicherweise in der biologischen Probe enthaltenen Antikörpern ermöglichen, und
in vitro Erfassen der möglicherweise gebildeten Antigen/Antikörper Komplexen.

**32.** Verfahren nach Anspruch 31, wobei die in vitro Diagnose mittels eines ELISA Assays erfolgt.

**33.** Verfahren nach Anspruch 31 oder 32, wobei die biologische Probe weiterhin mit einem oder mehreren antigenen Peptiden in Kontakt gebracht wird, die aus anderen Antigenen ausgewählt unter LSA-1, LSA-3, LSA-5, SALSA, STARP, TRAP, PfEXP1, CS, MSP-3-1, MSP-3-2, MSP-3-5, MSP-3-6, MSP1, MSP2, MSP4, MSP5, AMA-1, SERP und GLURP hervorgehen.

**34.** Kit für die in vitro Diagnose von Malaria, umfassend mindestens ein antigenes Polypeptid wie in einem der Ansprüche 1 bis 4 definiert.

**35.** Kit nach Anspruch 34, worin das antigene Peptid oder Polypeptid an einen Träger gebunden ist.

**36.** Kit nach Anspruch 34 oder 35, weiter umfassend Reagenzien, die die Bildung von Antigen/Antikörper Komplexen zwischen dem antigenen Peptid oder Polypeptid und den möglicherweise in der biologischen Probe enthaltenen Antikörpern ermöglichen, und Reagenzien, die die in vitro Erfassung der möglicherweise gebildeten Antigen/Antikörper Komplexe ermöglichen.

**37.** Verfahren zur in vitro Diagnose von Malaria in einem Individuum, das wahrscheinlich mit *P. falciparum* infiziert ist, umfassend

Inkontaktbringen einer biologischen Probe des Individuums mit Antikörpern nach einem der Ansprüche 25 bis 27 unter Bedingungen, die die Bildung von Antigen/Antikörper Komplexen zwischen den Antikörpern und den möglicherweise in der biologischen Probe enthaltenen und für *P. falciparum* spezifischen Antigenen ermöglichen, und
in vitro Erfassen der möglicherweise gebildeten Antigen/Antikörper Komplexen.

**38.** Kit zur in vitro Diagnose von Malaria, umfassend Antikörper wie in einem der Ansprüche 25 bis 27 definiert.

**39.** Kit nach Anspruch 38, weiter umfassend Reagenzien, die die Bildung von Antigen/Antikörper Komplexen zwischen den Antikörpern und den möglicherweise in der biologischen Probe enthaltenen Antigenen der Proteine der MSP-3 Familie ermöglichen, und Reagenzien, die die in vitro Erfassung der möglicherweise gebildeten Antigen/Antikörper Komplexe ermöglichen.

**40.** Rekombinante Nukleotidsequenz, die ein antigenes Polypeptid wie in einem der Ansprüche 1 bis 4 definiert codiert.

**41.** Rekombinante Nukleotidsequenz nach Anspruch 40, umfassend eine ein Fusionsprotein codierende Sequenz, das mehrere MSP-3-c/d-ähnliche Motive umfasst, wobei mindestens zwei der Motive ausgewählt sind unter den Motiven der SEQ ID Nr. 25 bis 30.

**42.** Rekombinanter Klonierungs- und/oder Expressionsvektor, umfassend eine Nukleotidsequenz nach einem der Ansprüche 40 bis 41.

**43.** Rekombinanter Klonierungs- und/oder Expressionsvektor nach Anspruch 42, worin die Nukleotidsequenz unter Kontrolle eines Promotors und regulatorischen Elementen zur Expression in einer Wirtszelle steht, wobei der Promotor und die regulatorischen Elemente gegenüber der Wirtszelle homolog oder heterolog sind.

**44.** Verwendung eines Expressionsvektors nach Anspruch 43 zur Herstellung eines Medikaments für genetische Immunisierung gegen *Plasmodium falciparum*.

**45.** DNA-Impfstoff umfassend eine Nukleotidsequenz nach einem der Ansprüche 40 bis 41.

**46.** Rekombinante Wirtszelle, die mit dem Vektor nach Anspruch 42 oder 43 transformiert wurde.

**47.** Wirtszelle nach Anspruch 46, die ein Bakterium, eine Hefe, eine Insektenzelle oder eine Säugetierzelle ist.

**48.** Antigenes Polypeptid nach einem der Ansprüche 1 bis 4 und 12 bis 16 oder polypeptidische Zusammensetzung nach einem der Ansprüche 5 bis 16 zur Verwendung als Impfstoffzusammensetzung gegen Malaria.

**49.** Zusammensetzung umfassend einen Antikörper oder einen Antikörperpool nach einem der Ansprüche 25 bis 27 zur Verwendung als Medikament gegen Malaria.

**50.** Expressionsvektor nach Anspruch 42 oder 43 zur Verwendung als Medikament zur genetischen Immunisierung gegen *Plasmodium falciparum.*

## Revendications

**1.** Polypeptide antigénique de la protéine MSP3.3 de SEQ ID NO: 6, comprenant au moins le motif MSP-3-c/d-like tel que défini dans SEQ ID NO: 27.

**2.** Polypeptide antigénique de la protéine MSP3.3 de SEQ ID NO: 6 comprenant au moins un motif MSP-3-c/d-like obtenu par substitution conservatrice d'au moins un acide aminé de SEQ ID NO: 27, ledit motif étant reconnu par un anticorps dirigé contre le motif MSP-3-c/d-like tel que défini dans SEQ ID NO: 27, à condition que ledit polypeptide antigénique ne soit pas un fragment de MSP3-1 de SEQ ID NO: 2 ou un fragment de MSP-3-2 de SEQ ID NO: 4.

**3.** Polypeptide antigénique selon la revendication 2, comprenant au moins un motif MSP-3-c/d-like présentant au moins 90 % d'identité avec le motif tel que défini dans SEQ ID NO: 27.

**4.** Polypeptide antigénique selon l'une quelconque des revendications 1 à 3 comprenant en outre au moins un motif MSP-3-b-like ayant une séquence choisie parmi les séquences de SEQ ID NOs: 17 à 24.

**5.** Composition polypeptidique antigénique comprenant un motif MSP-3-c/d-like tel que défini dans l'une quelconque des revendications 1 à 4 et au moins un autre motif MSP-3-c/d-like choisi dans le groupe constitué de :

a. SEQ ID NO: 25, SEQ ID NO: 26, SEQ ID NO: 28, SEQ ID NO: 29, SEQ ID NO: 30 ;
b. un motif MSP-3-c/d-like obtenu par substitution conservatrice d'au moins un acide aminé dans une séquence choisie dans le groupe consistant en ayant une séquence de SEQ ID NO: 25, SEQ ID NO: 26, SEQ ID NO: 28, SEQ ID NO: 29 ou SEQ ID NO: 30, ledit motif étant reconnu par un anticorps dirigé contre n'importe quel motif MSP-3-c/d-like tel que défini dans SEQ ID NO: 25 à SEQ ID NO: 30, et
c. un motif MSP-3-c/d-like présentant au moins 90 % d'identité avec SEQ ID NO: 25, SEQ ID NO: 26, SEQ ID NO: 28, SEQ ID NO: 29 et SEQ ID NO: 30 ;

**6.** Composition de polypeptide antigénique selon la revendication 5 comprenant un motif MSP-3-c/d-like tel que défini dans SEQ ID NO: 27 et au moins un autre motif MSP-3-c/d-like choisi dans le groupe constitué de SEQ ID NO: 25, SEQ ID NO: 26, SEQ ID NO: 28, SEQ ID NO: 29 et SEQ ID NO: 30.

**7.** Composition de polypeptide antigénique selon la revendication 5 ou 6, dans laquelle lesdits au moins deux motifs MSP-3-c/d-like différents sont portés par des molécules distinctes.

**8.** Composition de polypeptide antigénique selon la revendication 5 ou 6, dans laquelle lesdits au moins deux motifs MSP-3-c/d-like différents sont portés par une molécule unique.

**9.** Composition polypeptidique antigénique selon l'une quelconque des revendications 5 à 8, comprenant en outre un polypeptide antigénique comprenant au moins 10 résidus d'acides aminés consécutifs provenant de la région R0 de GLURP.

**10.** Composition polypeptidique antigénique selon l'une quelconque des revendications 5 à 7, qui est un mixotope comprenant des peptides synthétiques comprenant la séquence :

$$L\text{-}X_1\text{-}X_2\text{-}X_3\text{-}X_4\text{-}X_3\text{-}X_5\text{-}X_6\text{-}X_7\text{-}D\text{-}X_8\text{-}X_9\text{-}X_{10}\text{-}I\text{-}X_{11}\text{-}X_{12}\text{-}X_{13}\text{-}X_{14}\text{-}X_{15}\text{-}X_{16}$$

(SEQ ID NO: 33), dans laquelle :

$X_1$ = E, ou S ;
$X_2$ = L, H, S ou Q ;
$X_3$ = I, V ou L ;
$X_4$ = K, N, Y ou P ;
$X_5$ = T, S ou P ;
$X_6$ = S ou L ;
$X_7$ = K, W ou S ;
$X_8$ = E, K, R ou I ;
$X_9$ = E ou N ;
$X_{10}$ = D, N ou Q ;
$X_{11}$ = I, V, S, P ou A ;
$X_{12}$ = K, D ou N ;
$X_{13}$ = H ou E ;
$X_{14}$ = N ou S ;
$X_{15}$ = E ou D ; et

$X_{16}$ = D ou Q.

**11.** Composition polypeptidique antigénique selon la revendication 10, qui est un mélange d'au moins 50, au moins 100, ou au moins 500 peptides de séquences différentes.

**12.** Polypeptide antigénique selon l'une quelconque des revendications 1 à 4, ou la composition polypeptidique antigénique selon l'une quelconque des revendications 5 à 11, dans lequel(laquelle) une molécule lipidique est liée à au moins une partie des molécules polypeptidiques.

**13.** Polypeptide antigénique ou composition polypeptidique selon la revendication 12, dans lequel(laquelle) la molécule lipidique est un résidu palmitoylysylamide C-terminal.

**14.** Polypeptide antigénique selon l'une quelconque des revendications 1 à 4, ou la composition polypeptidique antigénique selon l'une quelconque des revendications 5 à 13, dans lequel(laquelle) au moins une partie des molécules polypeptidiques sont liées à un support.

**15.** Polypeptide antigénique ou composition polypeptidique selon la revendication 14, dans lequel(laquelle) le support est des particules virales, ou des billes de nitrocellulose ou de polystyrène, ou un polymère biodégradable.

**16.** Polypeptide antigénique ou composition polypeptidique selon la revendication 15, dans lequel(laquelle) ledit polymère biodégradable est des lipophosphoglycanes ou de l'acide (poly-L) lactique.

**17.** Composition immunogène comprenant, comme immunogène, un polypeptide antigénique selon l'une quelconque des revendications 1 à 4 et 12 à 16, ou une composition polypeptidique selon l'une quelconque des revendications 5 à 15.

**18.** Vaccin contre le paludisme comprenant, comme immunogène, un polypeptide antigénique selon l'une quelconque des revendications 1 à 4 et 12 à 16, ou une composition polypeptidique selon l'une quelconque des revendications 5 à 15, en association avec un véhicule pharmaceutique convenable.

**19.** Composition immunogène selon la revendication 17 ou le vaccin selon la revendication 18, comprenant en outre au moins un antigène choisi parmi LSA-1, LSA-3, LSA-5, SALSA, STARP, TRAP, PfEXP1, CS, MSP1, MSP2, MSP4, MSP5, AMA-1, SERP et GLURP.

**20.** Composition immunogène ou le vaccin selon l'une quelconque des revendications 17 à 20, qui est formulé(e) pour une injection intradermique ou intramusculaire.

**21.** Composition immunogène ou vaccin selon la revendication 20, comprenant entre 1 et 100 µg d'immunogène par dose d'injection.

**22.** Composition immunogène ou vaccin selon la revendication 20, comprenant entre 2 et 50 µg d'immunogène par dose d'injection.

**23.** Composition immunogène ou vaccin selon l'une quelconque des revendications 17 à 22, comprenant en outre du SBAS2 et/ou de l'alun et/ou un Montanide comme adjuvant.

**24.** Utilisation d'un polypeptide antigénique selon l'une quelconque des revendications 1 à 4 et 12 à 16, ou d'une composition polypeptidique selon l'une quelconque des revendications 5 à 16, pour la préparation d'une composition vaccinale contre le paludisme.

**25.** Anticorps synthétique ou recombinant dirigé contre le motif MSP-3-c/d-like d'un polypeptide antigénique tel que défini selon l'une quelconque des revendications 1 à 3.

**26.** Groupe d'anticorps dirigés contre une composition polypeptidique selon l'une quelconque des revendications 5 à 16.

**27.** Anticorps selon la revendication 25, ou un groupe d'anticorps selon la revendication 26, dans lequel lesdits anticorps sont des anticorps humains ou humanisés.

**28.** Utilisation d'une composition comprenant un anticorps ou un groupe d'anticorps selon l'une quelconque des revendications 25 à 27, pour la préparation d'un médicament contre le paludisme.

**29.** Médicament pour une immunothérapie passive du paludisme, comprenant un anticorps ou un groupe d'anticorps selon l'une quelconque des revendications 25 à 27.

**30.** Médicament selon la revendication 29, comprenant en outre des anticorps dirigés contre au moins un antigène choisi parmi LSA-1, LSA-3, LSA-5, SALSA, STARP, TRAP, PfEXP1, CS, MSP1, MSP2, MSP4, MSP5, AMA-1, SERP et GLURP.

**31.** Procédé pour le diagnostic *in vitro* du paludisme chez un sujet susceptible d'être infecté par P. *falciparum,* qui comprend la mise en contact d'un échantillon biologique provenant dudit sujet avec un polypeptide antigénique selon l'une quelconque des revendications 1 à 4, dans des conditions permettant la formation de complexes antigène/anticorps entre ledit peptide ou polypeptide antigénique et les anticorps éventuellement présents dans l'échantillon biologique, et la détection *in vitro* des complexes antigène/anticorps éventuellement formés.

**32.** Procédé selon la revendication 31, dans lequel le diagnostic *in vitro* est réalisé par un dosage ELISA.

**33.** Procédé selon la revendication 31 ou 32, dans lequel l'échantillon biologique est en outre porté au contact d'un ou plusieurs peptides antigéniques provenant d'autres antigènes choisis parmi LSA-1, LSA-3, LSA-5, SALSA, STARP, TRAP, PfEXP1, CS, MSP-3-1, MSP-3-2, MSP-3-5, MSP-3-6, MSP1, MSP2, MSP4, MSP5, AMA-1, SERP et GLURP.

**34.** Kit pour le diagnostic *in vitro* du paludisme, comprenant au moins un polypeptide antigénique tel que défini selon l'une quelconque des revendications 1 à 4.

**35.** Kit selon la revendication 34, dans lequel le peptide ou polypeptide antigénique est lié à un support.

**36.** Kit selon la revendication 34 ou 35, comprenant en outre des réactifs pour permettre la formation de complexes antigène/anticorps entre ledit peptide ou polypeptide antigénique et les anticorps éventuellement présents dans un échantillon biologique, et des réactifs permettant la détection *in vitro* des complexes antigène/anticorps éventuellement formés.

**37.** Procédé pour le diagnostic *in vitro* du paludisme chez un sujet susceptible d'être infecté par *P. falciparum,* qui comprend la mise en contact d'un échantillon biologique provenant dudit sujet avec des anticorps tels que définis selon l'une quelconque des revendications 25 à 27, dans des conditions permettant la formation de complexes antigène/anticorps entre lesdits anticorps et les antigènes spécifiques de *P. falciparum* éventuellement présents dans l'échantillon biologique, et la détection *in vitro* des complexes antigène/anticorps éventuellement formés.

**38.** Kit pour le diagnostic *in vitro* du paludisme, comprenant des anticorps tels que définis selon l'une quelconque des revendications 25 à 27.

**39.** Kit selon la revendication 38, comprenant en outre des réactifs pour permettre la formation de complexes antigène/anticorps entre lesdits anticorps et des antigènes provenant des protéines de la famille MSP-3 éventuellement présents dans un échantillon biologique, et des réactifs permettant la détection *in vitro* des complexes antigène/anticorps éventuellement formés.

**40.** Séquence nucléotidique recombinante codant pour un polypeptide antigénique tel que défini selon l'une quelconque des revendications 1 à 4.

**41.** Séquence nucléotidique recombinante selon la revendication 40, comprenant une séquence codant pour une protéine de fusion comprenant plusieurs motifs MSP-3-c/d-like, dans laquelle au moins deux desdits motifs sont choisis parmi les motifs de SEQ ID NOs: 25 à 30.

**42.** Vecteur de clonage et/ou d'expression recombinant, comprenant une séquence nucléotidique selon l'une quelconque des revendications 40 à 41.

**43.** Vecteur de clonage et/ou d'expression recombinant selon la revendication 42, dans lequel la séquence nucléotidique est sous le contrôle d'un promoteur et d'éléments régulateurs d'expression dans une cellule hôte, lesdits promoteur et éléments régulateurs étant homologues ou hétérologues vis-à-vis de la cellule hôte.

**44.** Utilisation d'un vecteur d'expression selon la revendication 43, pour la préparation d'un médicament destiné à l'immunisation génétique contre *Plasmodium falciparum*.

**45.** Vaccin à ADN comprenant une séquence nucléotidique selon l'une quelconque des revendications 40 à 41.

**46.** Cellule hôte recombinante, qui est transformée par le vecteur de la revendication 42 ou 43.

**47.** Cellule hôte de la revendication 46, qui est une bactérie, une levure, une cellule d'insecte, ou une cellule de mammifère.

**48.** Polypeptide antigénique selon l'une quelconque des revendications 1 à 4 et 12 à 16, ou une composition polypeptidique selon l'une quelconque des revendications 5 à 16, pour une utilisation comme composition vaccinale contre le paludisme.

**49.** Composition comprenant un anticorps ou un groupe d'anticorps selon l'une quelconque des revendications 25 à 27, pour une utilisation comme médicament contre le paludisme.

**50.** Vecteur d'expression selon la revendication 42 ou 43, pour une utilisation comme médicament destiné à une immunisation génétique contre *Plasmodium falciparum.*

Fig. 1

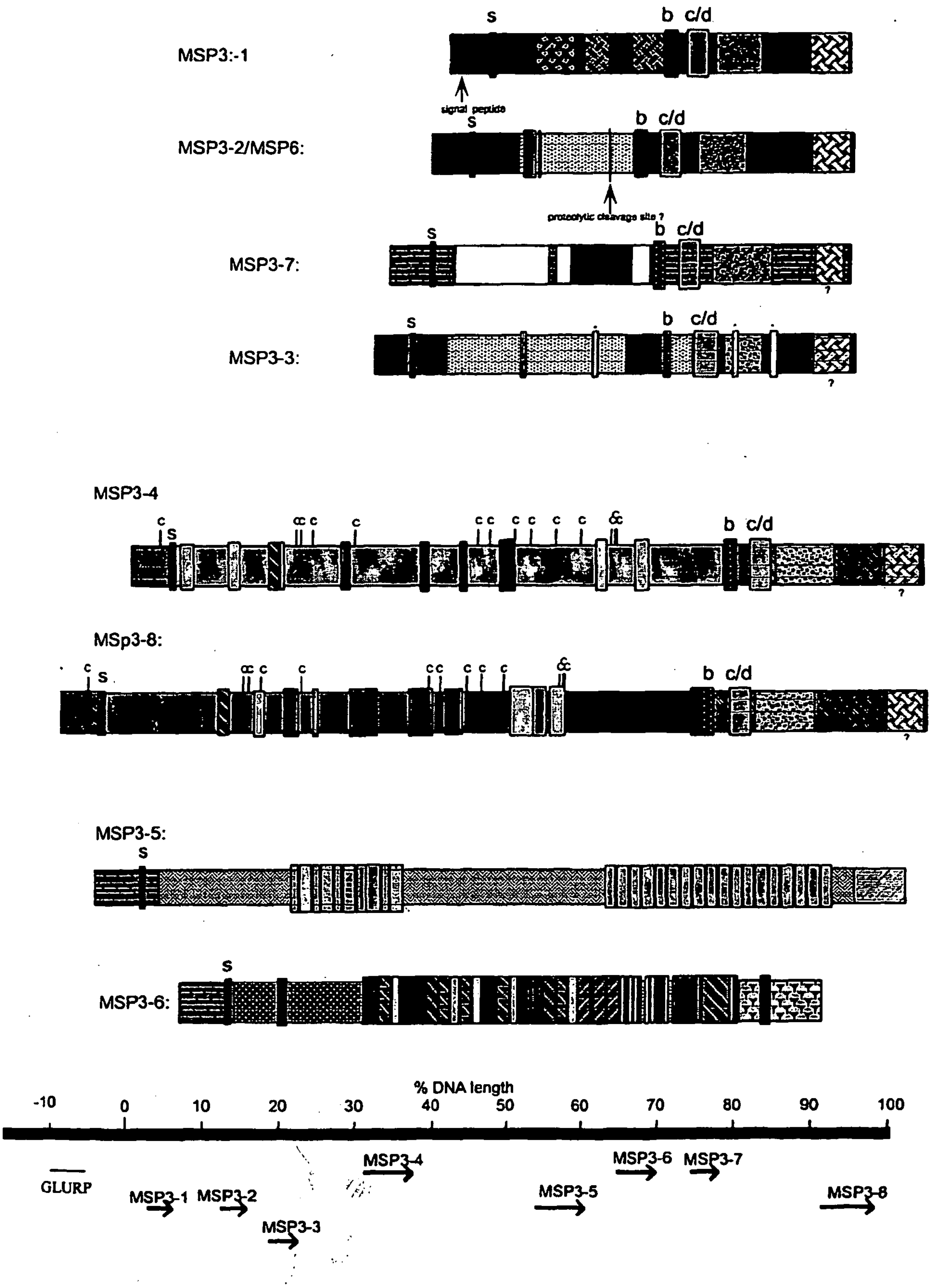
MSP3:-1
s
b c/d
signal peptide
MSP3-2/MSP6:
s
b c/d
proteolytic cleavage site ?
MSP3-7:
s
b c/d
MSP3-3:
s
b c/d
MSP3-4
c
s
cc c
c
c c c c c c
cc
b c/d
MSp3-8:
c
s
cc c
c
cc c c c
cc
b c/d
MSP3-5:
s
MSP3-6:
s
% DNA length
-10 0 10 20 30 40 50 60 70 80 90 100
GLURP
MSP3-1
MSP3-2
MSP3-3
MSP3-4
MSP3-5
MSP3-6
MSP3-7
MSP3-8

# Fig 2

## MSP3 peptides

(numbering corresponding to 3D7 strain)

MSP 3a: 167-YEKA KNAY QKANQAV LKA KEASSYD-191

MSP 3b: 184-AK EASSYDYI LGWEFGGGVP EHKKEEN-210

MSP 3c: 203-**PEHKKEENM LSHL YV SSKDK ENISKEND**-230

MSP 3d: 211-**MLSHLYV SSKDK ENISKENDDVLD EKEEEAEETEEEELEEK**-251

MSP 3e: 275-**EENDKKKEQEKEQSNEN NDQKKD MEAQN LISKN**-307

MSP 3f: 302-NLISKNQ NNNE KNVK EAA ESIMKTLAGLI KGNNQIDSTLKD LVE ELSKYFKNH

# Fig. 3

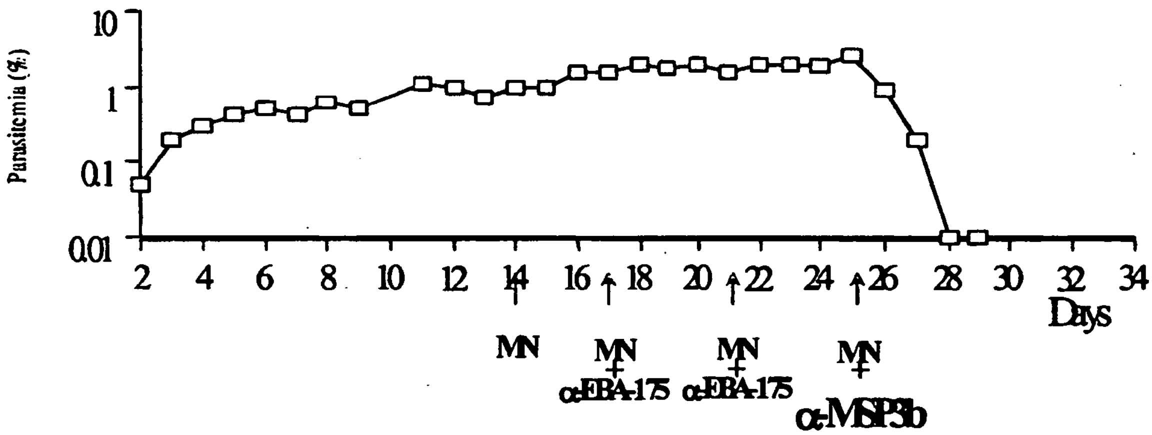
Parasitemia (%)
10
1
0.1
0.01
2 4 6 8 10 12 14 16 18 20 22 24 26 28 30 32 34
Days
MN
MN + α-EBA-175
MN + α-EBA-175
MN + α-MSP3b

Fig 4

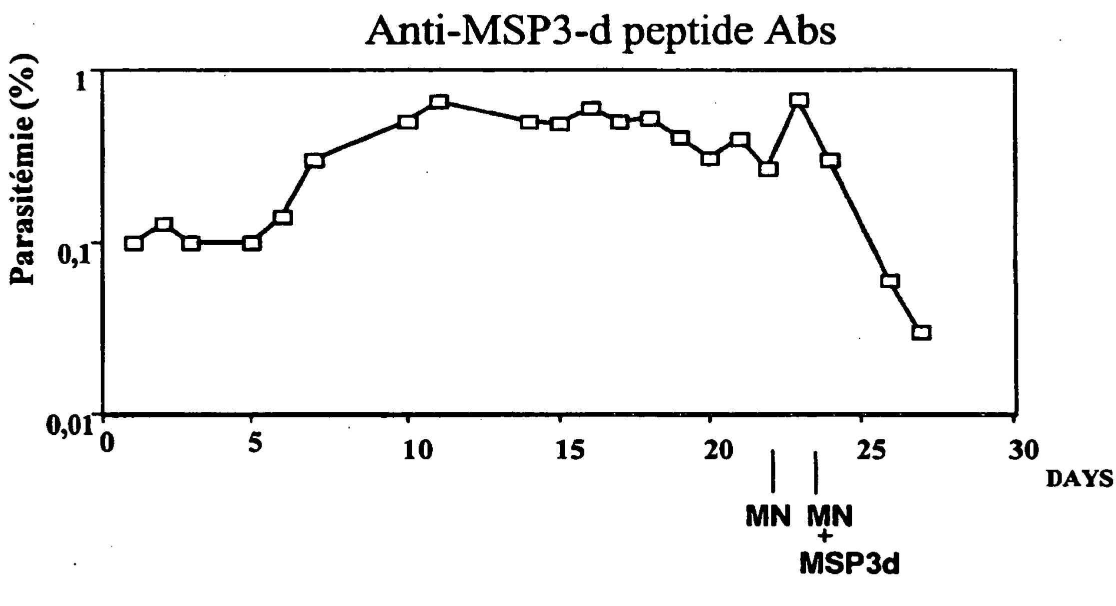
Anti-MSP3-d peptide Abs
Parasitémie (%)
1
0,1
0,01
0
5
10
15
20
25
30
DAYS
MN
MN
+
MSP3d

# Fig. 5

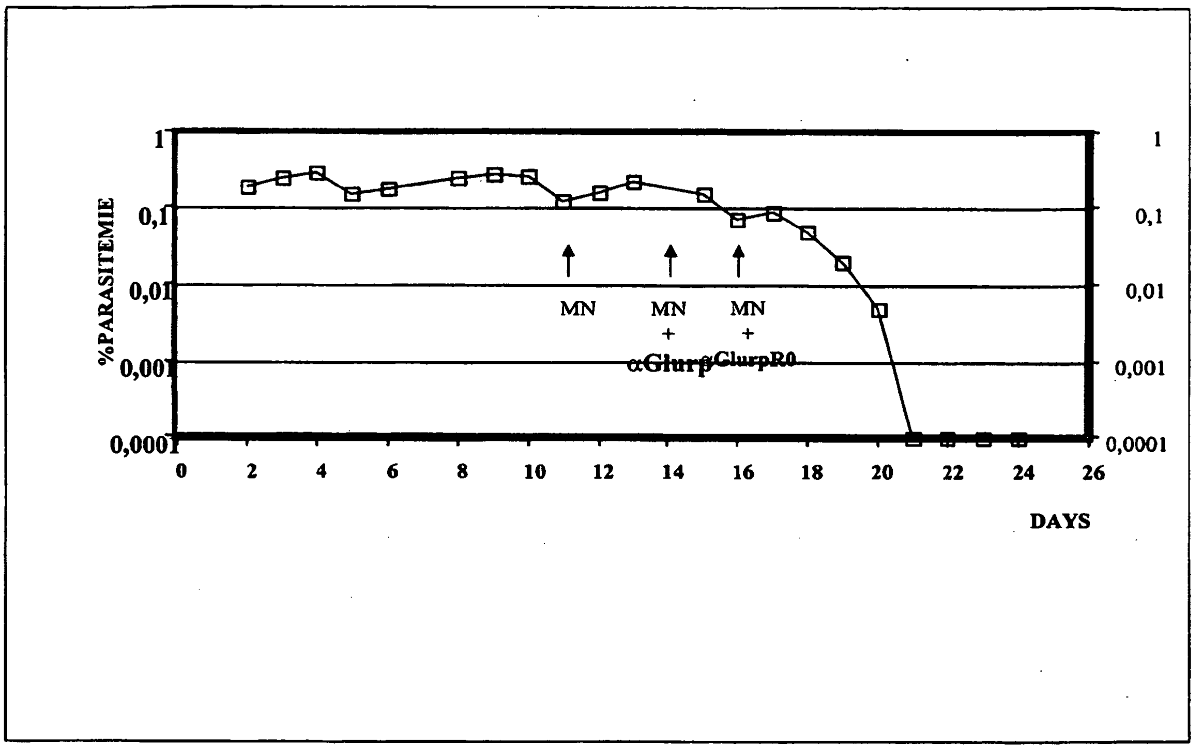
%PARASITEMIE
1
0,1
0,01
0,001
0,0001
0
2
4
6
8
10
12
14
16
18
20
22
24
26
DAYS
MN
MN
+
αGlurp
MN
+
αGlurpR0

Fig. 6

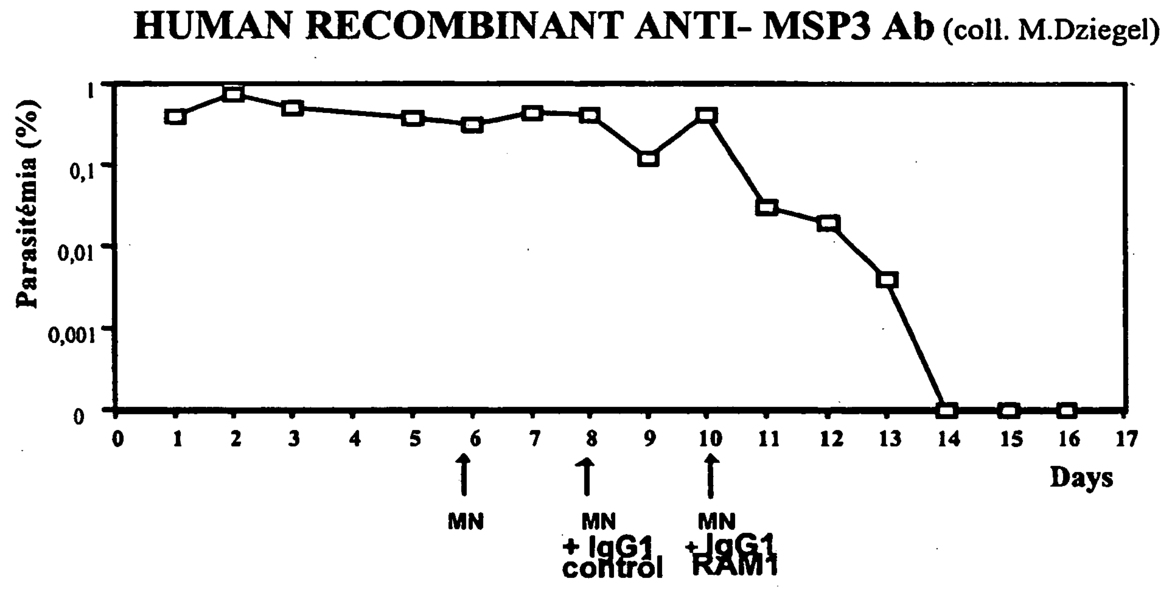
HUMAN RECOMBINANT ANTI- MSP3 Ab (coll. M.Dziegel)
Parasitémia (%)
1
0,1
0,01
0,001
0
0 1 2 3 4 5 6 7 8 9 10 11 12 13 14 15 16 17
Days
MN
MN + IgG1 control
MN + IgG1 RAM1

Fig. 7

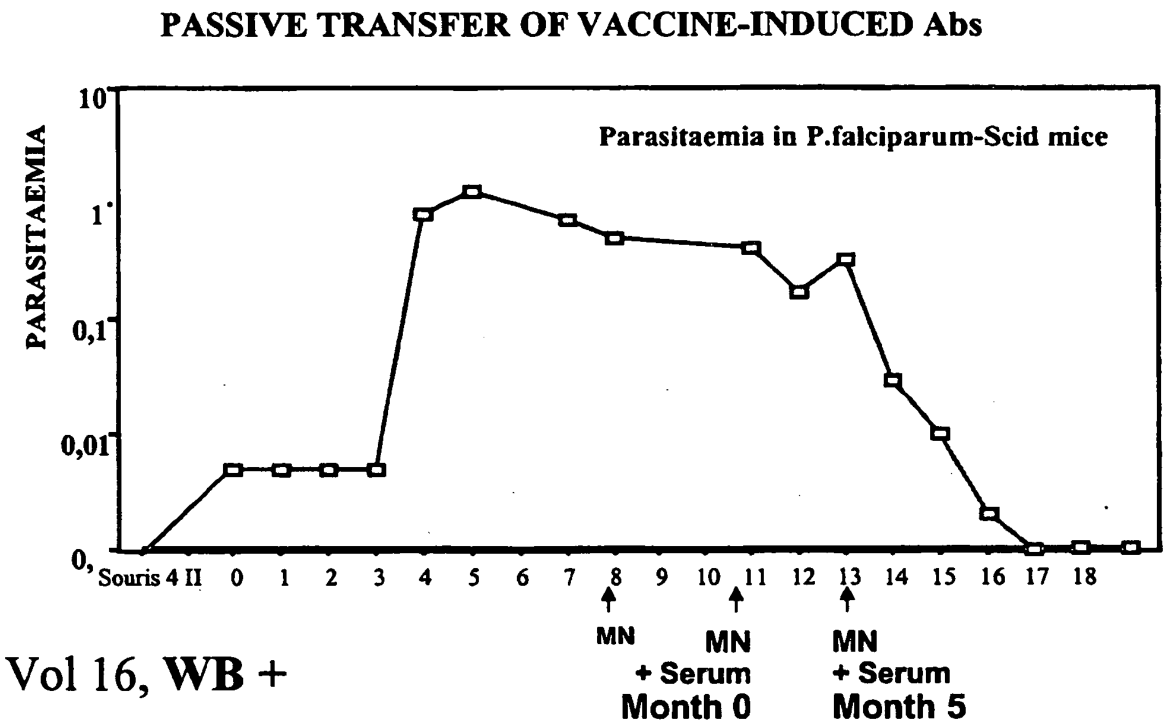
PASSIVE TRANSFER OF VACCINE-INDUCED Abs
Parasitaemia in P.falciparum-Scid mice
PARASITAEMIA
10
1
0,1
0,01
0,
Souris 4 II 0 1 2 3 4 5 6 7 8 9 10 11 12 13 14 15 16 17 18
MN
MN + Serum Month 0
MN + Serum Month 5
Vol 16, WB +

Fig. 8

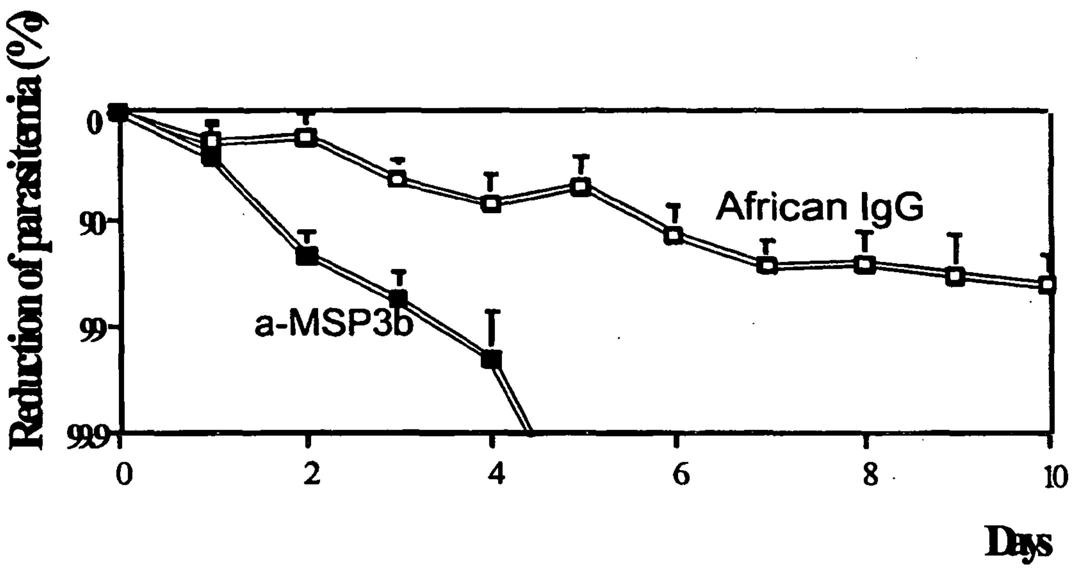


**Mean±SD from 2 groups of 6 mice**

Fig 9A

CLUSTAL W (1.82) Multiple **ClustalW alignment**

<u>Nucleotide Sequence Alignment - Viawww.ebi.ac.uk/clustalw</u>/ **MSP3-1**

CLUSTAL W (1.82) multiple sequence alignment

```
MSP3.1C  3D7     -TATGAAAAGGCAAAAAATGCTTATCAAAAAGCAAACCAAGCTGTTTTAAAAGCAAAAGA 59
MSP3.1C  B2      -TATGAAAAGGCAAAAAATGCTTATCAAAAAGCAAACCAAGCTGTTTTAAAAGCAAAAGA 59
MSP3.1C  B3      -TATGAAAAGGCAAAAAATGCTTATCAAAAAGCAAACCAAGCTGTTTTAAAAGCAAAAGA 59
MSP3.1C  B4      -TATGAAAAGGCAAAAAATGCTTATCAAAAAGCAAACCAAGCTGTTTTAAAAGCAAAAGA 59
MSP3.1C  B5      -TATGAAAAGGCAAAAAATGCTTATCAAAAAGCAAACCAAGCTGTTTTAAAAGCAAAAGA 59
MSP3.1C  D2      -TATGAAAAGGCAAAAAATGCTTATCAAAAAGCAAACCAAGCTGTTTTAAAAGCAAAAGA 59
MSP3.1C  D3      -TATGAAAAGGCAAAAAATGCTTATCAAAAAGCAAACCAAGCTGTTTTAAAAGCAAAAGA 59
MSP3.1C  D4      TTATGAAAAGGCAAAAAATGCTTATCAAAAAGCAAACCAAGCTGTTTTAAAAGCAAAAGA 60
MSP3.1C  D5      -TATGAAAAGGCAAAAAATGCTTATCAAAAAGCAAACCAAGCTGTTTTAAAAGCAAAAGA 59
                  ***********************************************************

MSP3.1C  3D7     AGCTTCTAGTTATGATTATATTTTAGGTTGGGAATTTGGAGGAGGCGTTCCAGAACACAA 119
MSP3.1C  B2      AGCTTCTAGTTATGATTATATTTTAGGTTGGGAATTTGGAGGAGGCGTTCCAGAACACAA 119
MSP3.1C  B3      AGCTTCTAGTTATGATTATATTTTAGGTTGGGAATTTGGAGGAGGCGTTCCAGAACACAA 119
MSP3.1C  B4      AGCTTCTAGTTATGATTATATTTTAGGTTGGGAATTTGGAGGAGGCGTTCCAGAACACAA 119
MSP3.1C  B5      AGCTTCTAGTTATGATTATATTTTAGGTTGGGAATTTGGAGGAGGCGTTCCAGAACACAA 119
MSP3.1C  D2      AGCTTCTAGTTATGATTATATTTTAGGTTGGGAATTTGGAGGAGGCGTTCCAGAACACAA 119
MSP3.1C  D3      AGCTTCTAGTTATGATTATATTTTAGGTTGGGAATTTGGAGGAGGCGTTCCAGAACACAA 119
MSP3.1C  D4      AGCTTCTAGTTATGATTATATTTTAGGTTGGGAATTTGGAGGAGGCGTTCCAGAACACAA 120
MSP3.1C  D5      AGCTTCTAGTTATGATTATATTTTAGGTTGGGAATTTGGAGGAGGCGTTCCAGAACACAA 119
                 ************************************************************

MSP3.1C  3D7     AAAAGAAGAAAATATGTTATCACATTTATATGTTTCTTCAAAGGATAAGGAAAATATATC 179
MSP3.1C  B2      AAAAGAAGAAAATATGTTATCACATTTATATGTTTCTTCAAAGGATAAGGAAAATATATC 179
MSP3.1C  B3      AAAAGAAGAAAATATGTTATCACATTTATATGTTTCTTCAAAGGATAAGGAAAATATATC 179
MSP3.1C  B4      AAAAGAAGAAAATATGTTATCACATTTATATGTTTCTTCAAAGGATAAGGAAAATATATC 179
MSP3.1C  B5      AAAAGAAGAAAATATGTTATCACATTTATATGTTTCTTCAAAGGATAAGGAAAATATATC 179
MSP3.1C  D2      AAAAGAAGAAAATATGTTATCACATTTATATGTTTCTTCAAAGGATAAGGAAAATATATC 179
MSP3.1C  D3      AAAAGAAGAAAATATGTTATCACATTTATATGTTTCTTCAAAGGATAAGGAAAATATATC 179
MSP3.1C  D4      AAAAGAAGAAAATATGTTATCACATTTATATGTTTCTTCAAAGGATAAGGAAAATATATC 180
MSP3.1C  D5      AAAAGAAGAAAATATGTTATCACATTTATATGTTTCTTCAAAGGATAAGGAAAATATATC 179
                 ************************************************************
```

Fig 9B

```
MSP3.1C  3D7     TAAGGAAAATGATGATGTATTAGATGAGAAGGAAGAAGAGGCAGAAGAAACAGAAGAAGA 239
MSP3.1C  B2      TAAGGAAAATGATGATCTATTAGATGAGAAGGAAGAAGAGGCAGAAGAAACAGAAGAAGA 239
MSP3.1C  B3      TAAGGAAAATGATGATGTATTAGATGAGAAGGAAGAAGAGGCAGAAGAAACAGAAGAAGA 239
MSP3.1C  B4      TAAGGAAAATGATGATGTATTAGATGAGAAGGAAGAAGAGGCAGAAGAAACAGAAGAAGA 239
MSP3.1C  B5      TAAGGAAAATGATGATGTATTAGATGAGAAGGAAGAAGAGGCAGAAGAAACAGAAGAAGA 239
MSP3.1C  D2      TAAGGAAAATGATGATGTATTAGATGAGAAGGAAGAAGAGGCAGAAGAAACAGAAGAAGA 239
MSP3.1C  D3      TAAGGAAAATGATGATGTATTAGATGAGAAGGAAGAAGAGGCAGAAGAAACAGAAGAAGA 239
MSP3.1C  D4      TAAGGAAAATGATGATGTATTAGATGAGAAGGAAGAAGAGGCAGAAGAAACAGAAGAAGA 240
MSP3.1C  D5      TAAGGAAAATGATGATGTATTAGATGAGAAGGAAGAAGAGGCAGAAGAAACAGAAGAAGA 239
                 **************** *******************************************

MSP3.1C  3D7     AGAACTTGAAGAAAAAAATGAAGAAGAAACAGAATCAGAAATAAGTGAAGATGAAGAAGA 299
MSP3.1C  B2      AGAACTTGAAGAAAAAAATGAAGAAGAAACCGAATCAGAAATAAGTGAAGATGAAGAAGA 299
MSP3.1C  B3      AGAACTTGAAGAAAAAAATGAAGAAGAAACCGAATCAGAAATAAGTGAAGATGAAGAAGA 299
MSP3.1C  B4      AGAACTTGAAGAAAAAAATGAAGAAGAAACCGAATCAGAAATAAGTGAAGATGAAGAAGA 299
MSP3.1C  B5      AGAACTTGAAGAAAAAAATGAAGAAGAAACCGAATCAGAAATAAGTGAAGATGAAGAAGA 299
MSP3.1C  D2      GGAACTTGAAGAAAAAAATGAAGAAGAAACAGAATCAGAAATAAGTGAAGATGAAGAAGA 299
MSP3.1C  D3      AGAACTTGAAGAAAAAAATGAAGAAGAAACAGAATCAGAAATAAGTGAAGATGAAGAAGA 299
MSP3.1C  D4      AGAACTTGAAGAAAAAAATGAAGAAGAAACAGAATCAGAAATAAGTGAAGATGAAGAAGA 300
MSP3.1C  D5      AGAACTTGAAGAAAAAAATGAAGAAGAAACCGAATCAGAAATAAGTGAAGATGAAGAAGA 299
                 ****************************** *****************************

MSP3.1C  3D7     AGAAGAAGAAGAAGAAGAA---AAGGAAGAAGAAAATGACAAAAAAAAGAACAAGAAAA 356
MSP3.1C  B2      AGAAGAAGAAGAAAAGGAAGAAGA---------------------AAAAGAGCAAGCAAA 338
MSP3.1C  B3      AGAAGAAGAAGAAAAGGAAGAAGA---------------------AAAAGAGCAAGCAAA 338
MSP3.1C  B4      AGAAGAAGAAGAAAAGGAAGAAGA---------------------AAAAGAGCAAGCAAA 338
MSP3.1C  B5      AGAAGAAGAAGAAAAGGAAGAAGA---------------------AAAAGAGCAAGCAAA 338
MSP3.1C  D2      AGAAGAAGAAGAAGAA------AAGGAAGAAGAAAATGACAAAAAAAAGAACAAGAAAA 353
MSP3.1C  D3      AGAAGAAGAAGAAGAAGAA---AAGGAAGAAGAAAATGACAAAAAAAAGAACAAGAAAA 356
MSP3.1C  D4      AGAAGAAGAAGAAGAAGAAGAAAGGAAGAAGAAAATGACAAAAAAAAGAACAAGAAAA 360
MSP3.1C  D5      AGAAGAAGAAGAAGAAGAAGAAAAGGAAGAAGAGAATGACAAAAAAAAGAACAAGAAAA 359
                 ************* *                    ****** **** ***

MSP3.1C  3D7     AGAACAAAGTAATGAAAATAATGATCAAAAAAAAGATATGGAAGCACAGAATTTAATTTC 416
MSP3.1C  B2      AGAACAAAGTAACGAAAATAATGATCAAAAAAAAGATATGGAAGCTCAGAATTTAATTTC 398
MSP3.1C  B3      AGAACAAAGTAACGAAAATAATGATCAAAAAAAAGATATGGAAGCTCAGAATTTAATTTC 398
MSP3.1C  B4      AGAACAAAGTAACGAAAATAATGATCAAAAAAAAGATATGGAAGCTCAGAATTTAATTTC 398
```

Fig 9C

```
MSP3.1C  B5     AGAACAAAGTAACGAAAATAATGATCAAAAAAAAGATATGGAAGCTCAGAATTTAATTTC 398
MSP3.1C  D2     AGAACAAAGTAATGAAAATAATGATCAAAAAAAAGATATGGAAGCACAGAATTTAATTTC 413
MSP3.1C  D3     AGAACAAAGTAATGAAAATAATGATCAAAAAAAAGATATGGAAGCTCAGAATTTAATTTC 416
MSP3.1C  D4     AGAACAAAGTAGTGAAAATAATGATCAAAAAAAAGATATGGAAGCTCAGAATTTAATTTC 420
MSP3.1C  D5     AGAACAAAGTAACGAAAATAATGATCAAAAAAAAGATATGGAAGCTCAGAATTTAATTTC 419
                ***********  ******************************** **************

MSP3.1C  3D7    TAAAAACCAGAATAATAATGAGAAAAACGTAAAAGAAGCTGCTGAAAGCATCATGAAAAC 476
MSP3.1C  B2     TAAAAACCAGAATAATAATGAGAAAAACGTAAAAGAAGCTGCTGAAAGCATAATGAAAAC 458
MSP3.1C  B3     TAAAAACCAGAATAATAATGAGAAAAACGTAAAAGAAGCTGCTGAAAGCATAATGAAAAC 458
MSP3.1C  B4     TAAAAACCAGAATAATAATGAGAAAAACGTAAAAGAAGCTGCTGAAAGCATAATGAAAAC 458
MSP3.1C  B5     TAAAAACCAGAATAATAATGAGAAAAACGTAAAAGAAGCTGCTGAAAGCATAATGAAAAC 458
MSP3.1C  D2     TAAAAACCAGAATAATAATGAGAAAAACGTAAAAGAAGCTGCTGAAAGCATAATGAAAAC 473
MSP3.1C  D3     TAAAAACCAGAATAATAATGAGAAAAACGTAAAAGAAGCTGCTGAAAGCATAATGAAAAC 476
MSP3.1C  D4     TAAAAACCAGAATAATAATGAGAAAAACGTAAAAGAAGCTGCTGAAAGCATAATGAAAAC 480
MSP3.1C  D5     TAAAAACCAGAATAATAATGAGAAAAACGTAAAAGAAGCTGCTGAAAGCATAATGAAAAC 479
                *************************************************** ********

MSP3.1C  3D7    TTTAGCTGGTTTAATCAAGGGAAATAATCAAATAGATTCTACCTTAAAAGATTTAGTAGA 536
MSP3.1C  B2     TTTAGCTGGTTTAATCAAGGGAAATAATCAAATAGATTCTACCTTAAAAGATTTAGTAGA 518
MSP3.1C  B3     TTTAGCTGGTTTAATCAAGGGAAATAATCAAATAGATTCTACCTTAAAAGATTTAGTAGA 518
MSP3.1C  B4     TTTAGCTGGTTTAATCAAGGGAAATAATCAAATAGATTCTACCTTAAAAGATTTAGTAGA 518
MSP3.1C  B5     TTTAGCTGGTTTAATCATGGGAAATAATCAAATAGATTCTACCTTAAAAGATTTAGTAGA 518
MSP3.1C  D2     TTTAGCTGGTTTAATCAAGGGAAATAATCAAATAGATTCTACCTTAAAAGATTTAGTAGA 533
MSP3.1C  D3     TTTAGCTGGTTTAATCAAGGGAAATAATCAAATAGATTCTACCTTAAAAGATTTAGTAGA 536
MSP3.1C  D4     TTTAGCTGGTTTAATCAAGGGAAATAATCAAATAGATTCTACCTTAAAAGATTTAGTAGA 540
MSP3.1C  D5     TTTAGCTGGTTTAATCAAGGGAAATAATCAAATAGATTCTACCTTAAAAGATTTAGTAGA 539
                ***************** ******************************************

MSP3.1C  3D7    AGAATTATCCAAATATTTTAAAAATCATTAA 567
MSP3.1C  B2     AGAATTATCCAAATATTTTAAAAATCATT-- 547
MSP3.1C  B3     AGAATTATCCAAATATTTTAAAAATCATTAA 549
MSP3.1C  B4     AGAATTATCCAAATATTTTAAAAATCATTAA 549
MSP3.1C  B5     AGAATTATCCCAATATTTTAAAAATCATTAA 549
MSP3.1C  D2     AGAATTATCCAAATATTTTAAAAATCATTAA 564
MSP3.1C  D3     AGAATTATCCAAATATTTTAAAAATCATTAA 567
MSP3.1C  D4     AGAATTATCCAAATATTTTAAAAATCATTAA 571
MSP3.1C  D5     AGAATTATCCAAATATTTTAAAAATCATTAA 570
```

fig 9D

```
*********** ******************
```

**ClustalW alignment MSP6FL Nucleotide sequences** (Www.ebi.ac.uk/clustalw/) MSP3-2

```
CLUSTAL W (1.82) multiple sequence alignment

MSP3.2FL 3D7      ATGAATAAGATTTATAATATTACTTTTCTTTTCATTCTTTTAAACTTATATATAAATGAA 60
MSP3.2FL B1       ATGAATAAGATTTATAATATTACTTTTCTTTTCATTCTTTTAAACTTATATATAAATGAA 60
MSP3.2FL B3       ATGAATAAGATTTATAATATTACTTTTCTTTTCATTCTTTTAAACTTATATATAAATGAA 60
MSP3.2FL B4       ATGAATAAGATTTATAATATTACTTTTCTTTTCATTCTTTTAAACTTATATATAAATGAA 60
MSP3.2FL B5       ATGAATAAGATTTATAATATTACTTTTCTTTTCATTCTTTTAAACTTATATATAAATGAA 60
MSP3.2FL D1       ATGAATAAGATTTATAATATTACTTTTCTTTTCATTCTTTTAAACTTATATATAAATGAA 60
MSP3.2FL D2       ATGAATAAGATTTATAATATTACTTTTCTTTTCATTCTTTTAAACTTATATATAAATGAA 60
MSP3.2FL D3       ATGAATAAGAATTATAATATTACTTTTCTTTTCATTCTTTTAAACTTATATATAAATGAA 60
MSP3.2FL D4       ATGAATAAGATTTATAATATTACTCTTCTCTTCATTCTTTTAAACTTATATATAAATGAA 60
MSP3.2FL D5       ATGAATAAGATTTATAATATTACTTTTCTTTTCATTCTTTTAAACTTATATATAAATGAA 60
                  ********** ************* **** ******************************

MSP3.2FL 3D7      AATAACTTTATCAGAAATGAACTTATAAACGAAAAAAACCATAATTTAAGAAATGGTTCA 120
MSP3.2FL B1       AATAACTTTATCAGAAATGAACTTATAAACGAAAAAAACCATAATTTAAGAAATGGTTCA 120
MSP3.2FL B3       AATAACTTTATCAGAAATGAACTTATAAACGAAAAAAACCATAATTTAAGAAATGGTTCA 120
MSP3.2FL B4       AATAACTTTATCAGAAATGAACTTATAAACGAAAAAAACCATAATTTAAGAAATGGTTCA 120
MSP3.2FL B5       AATAACTTTATCAGAAATGAACTTATAAACGAAAAAAACCATAATTTAAGAAATGGTTCA 120
MSP3.2FL D1       AATAACTTTATCAGAAATGAACTTATAAACGAAAAAAACCATAATTTAAGAAATGGTTCA 120
MSP3.2FL D2       AATAACTTTATCAGAAATGAACTTATAAACGAAAAAAACCATAATTTAAGAAATGGTTCA 120
MSP3.2FL D3       AATAACTTTATCAGAAATGAACTTATAAACGAAAAAAACCATAATTTAAGAAATGGTTCA 120
MSP3.2FL D4       AATAACTTTATCAGAAATGAACTTATAAACGAAAAAAACCATAATTTAAGAAATGGTTCA 120
MSP3.2FL D5       AATAACTTTATCAGAAATGAACTTATAAACGAAAAAAACCATAATTTAAGAAATGGTTCA 120
                  ************************************************************

MSP3.2FL 3D7      ATGTATAATAACGATAAAATATTAAGTAAAAATGAAGTAGATACTAATATAGAAAGTAAC 180
MSP3.2FL B1       ATGTATAATAACGATAAAATATTAAGTAAAAATGAAGTAGATACTAATATAGAAAGTAAC 180
MSP3.2FL B3       ATGTATAATAACGATAAAATATTAAGTAAAAATGAAGTAGATACTAATATAGAAAGTAAC 180
MSP3.2FL B4       ATGTATAATAACGATAAAATATTAAGTAAAAATGAAGTAGATACTAATATAGAAAGTAAC 180
MSP3.2FL B5       ATGTATAATAACGATAAAATATTAAGTAAAAATGAAGTAGATACTAATATAGAAAGTAAC 180
MSP3.2FL D1       ATGTATAATAACGATAAAATATTAAGTAAAAATGAAGTAGATACTAATATAGAAAGTAAC 180
MSP3.2FL D2       ATGTATAATAACGATAAAATATTAAGTAAAAATGAAGTAGATACTAATATAGAAAGTAAC 180
```

Fig 9E

```
MSP3.2FL D3     ATGTATAATAACGATAAAATATTAAGTAAAAATGAAGTAGATACTAATATAGAAAGTAAC 180
MSP3.2FL D4     ATGTATAATAACGATAAAATATTAAGTAAAAATGAAGTAGATACTAATATAGAAAGTAAC 180
MSP3.2FL D5     ATGTATAATAACGATAAAATATTAAGTAAAAATGAAGTAGATACTAATATAGAAAGTAAC 180
                ************************************************************

MSP3.2FL 3D7    GAAAATAGTATTCACGAATCTGGACATAAGATTGATGGGGAAGAAGTTTTAAAAGCTAAT 240
MSP3.2FL B1     GAAAATAGTATTCACGAATCTGGACATAAGATTGATGGGGAAGAAGTTTTAAAAGCTAAT 240
MSP3.2FL B3     GAAAATAGTATTCACGAATCTGGACATAAGATTGATGGGGAAGAAGTTTTAAAAGCTAAT 240
MSP3.2FL B4     GAAAATAGTATTCACGAATCTGGACATAAGATTGATGGGGAAGAAGTTTTAAAAGCTAAT 240
MSP3.2FL B5     GAAAATAGTATTCACGAATCTGGACATAAGATTGATGGGGAAGAAGTTTTAAAAGCTAAT 240
MSP3.2FL D1     GAAAATAGTATTCACGAATCTGGACATAAGATTGATGGGGAAGAAGTTTTAAAAGCTAAT 240
MSP3.2FL D2     GAAAATAGTATTCACGAATCTGGACATAAGATTGATGGGGAAGAAGTTTTAAAAGCTAAT 240
MSP3.2FL D3     GAAAATAGTATTCACGAATCTGGACATAAGATTGATGGGGAAGAAGTTTTAAAAGCTAAT 240
MSP3.2FL D4     GAAAATAGTATTCACGAATCTGGACATAAGATTGATGGGGAAGAAGTTTTAAAAGCTAAT 240
MSP3.2FL D5     GAAAATAGTATTCACGAATCTGGACATAAGATTGATGGGGAAGAAGTTTTAAAAGCTAAT 240
                ************************************************************

MSP3.2FL 3D7    ---------------GTAGATGATATAACATACAAAAAAAAAAATGTTGATGATTCAGAA 285
MSP3.2FL B1     ---------------GTAGATGATATAACATACAAAAAAAAAAATGTTGATGATTCAGAA 285
MSP3.2FL B3     ---------------GTAGATGATATAACATACAAAAAAAAAAATGTTGATGATTCAGAA 285
MSP3.2FL B4     ---------------GTAGATGATATAACATACAAAAAAAAAAATGTTGATGATTCAGAA 285
MSP3.2FL B5     ---------------GTAGATGATATAACATACAAAAAAAAAAATGTTGATGATTCAGAA 285
MSP3.2FL D1     ---------------GTAGATGATATAACATACAAAAAAAAAAATGTTGATGATTCAGAA 285
MSP3.2FL D2     ---------------GTAGATGATATAACATACAAAAAAAAAAATGTTGATGATTCAGAA 285
MSP3.2FL D3     ---------------GTAGATGATATAACATACAAAAAAAAAAATGTTGATGATTCAGAA 285
MSP3.2FL D4     CAAGAACAAGCTAATGTAGAAGATATAACATACAAAAAAAAAAATGTTGATGATTCAGAA 300
MSP3.2FL D5     ---------------GTAGATGATATAACATACAAAAAAAAAAATGTTGATGATTCAGAA 285
                               ***** ***************************************

MSP3.2FL 3D7    ATTCCTTTTTCTGGTTATGATATACAAGCAACATATCAATTTCCTTCTACATCAGGAGGA 345
MSP3.2FL B1     ATTCCTTTTTCTGGTTATGATATACAAGCAACATATCAATTTCCTTCTACATCAGGAGGA 345
MSP3.2FL B3     ATTCCTTTTTCTGGTTATGATATACAAGCAACATATCAATTTCCTTCTACATCAGGAGGA 345
MSP3.2FL B4     ATTCCTTTTTCTGGTTATGATATACAAGCAACATATCAATTTCCTTCTACATCAGGAGGA 345
MSP3.2FL B5     ATTCCTTTTTCTGGTTATGATATACAAGCAACATATCAATTTCCTTCTACATCAGGAGGA 345
MSP3.2FL D1     ATTCCTTTTTCTGGTTATGATATACAAGCAACATATCAATTTCCTTCTACATCAGGAGGA 345
MSP3.2FL D2     ATTCCTTTTTCTGGTTATGATATACAAGCAACATATCAATTTCCTTCTACATCAGGAGGA 345
MSP3.2FL D3     ATTCCTTTTTCTGGTTATGATATACAAGCAACATATCAATTTCCTTCTACATCAGGAGGA 345
MSP3.2FL D4     ATTCCTTTTTCTGGTTCTGATATACAGGCAACATATCAATTTCCTCCTGCACCAGGAAGA 360
```

fig 9F

```
MSP3.2FL D5     ATTCCTTTTTCTGGTTATGATATACAAGCAACATATCAATTTCCTTCTACATCAGGAGGA 345
                **************** ********* ****************** ** ** ***** **

MSP3.2FL 3D7    AATAATGTAATTCCACTTCCTATAAAACAAAGTGGAGAAAATCAATATACTGTTACATCT 405
MSP3.2FL B1     AATAATGTAATTCCACTTCCTATAAAACAAAGTGGAGAAAATCAATATACTGTTACATCT 405
MSP3.2FL B3     AATAATGTAATTCCACTTCCTATAAAACAAAGTGGAGAAAATCAATATACTGTTACATCT 405
MSP3.2FL B4     AATAATGTAATTCCACTTCCTATAAAACAAAGTGGAGAAAATCAATATACTGTTACATCT 405
MSP3.2FL B5     AATAATGTAATTCCACTTCCTATAAAACAAAGTGGAGAAAATCAATATACTGTTACATCT 405
MSP3.2FL D1     AATAATGTAATTCCACTTCCTATAAAACAAAGTGGAGAAAATCAATATACTGTTACATCT 405
MSP3.2FL D2     AATAATGTAATTCCACTTCCTATAAAACAAAGTGGAGAAAATCAATATACTGTTACATCT 405
MSP3.2FL D3     AATAATGTAATTCCACTTCCTATAAAACAAAGTGGAGAAAATCAATATACTGTTACATCT 405
MSP3.2FL D4     ATTA---TAAATCC---TCGTACTGGAGGAAATACTGTAATTCCAC---CTCCTAGA--- 408
MSP3.2FL D5     AATAATGTAATTCCACTTCCTATAAAACAAAGTGGAGAAAATCAATATACTGTTACATCT 405
                * **   *** ***   ** **     *  ** *  * ** ** *    **  ** *

MSP3.2FL 3D7    ATATCAGGTATTCAAAAGGGAGCAAATGGTTTAACTGGTGCAACAGAAAATATTACACAA 465
MSP3.2FL B1     ATATCAGGTATTCAAAAGGGAGCAAATGGTTTAACTGGTGCAACAGAAAATATTACACAA 465
MSP3.2FL B3     ATATCAGGTATTCAAAAGGGAGCAAATGGTTTAACTGGTGCAACAGAAAATATTACACAA 465
MSP3.2FL B4     ATATCAGGTATTCAAAAGGGAGCAAATGGTTTAACTGGTGCAACAGAAAATATTACACAA 465
MSP3.2FL B5     ATATCAGGTATTCAAAAGGGAGCAAATGGTTTAACTGGTGCAACAGAAAATATTACACAA 465
MSP3.2FL D1     ATATCAGGTATTCAAAAGGGAGCAAATGGTTTAACTGGTGCAACAGAAAATATTACACAA 465
MSP3.2FL D2     ATATCAGGTATTCAAAAGGGAGCAAATGGTTTAACTGGTGCAACAGAAAATATTACACAA 465
MSP3.2FL D3     ATATCAGGTATTCAAAAGGGAGCAAATGGTTTAACTGGTGCAACAGAAAATATTACACAA 465
MSP3.2FL D4     AGACTAAGTGGACTAGAAGGTGTTTCTTATCCACATGTATCTACATCTTCTAATCAATCA 468
MSP3.2FL D5     ATATCAGGTATTCAAAAGGGAGCAAATGGTTTAACTGGTGCAACAGAAAATATTACACAA 465
                * *  * **   * * * ** *    *  *  * **   * ***     ** *  *  *

MSP3.2FL 3D7    GTTGTACAAGCAAACTCTGAAACAAATAAAAATCCTACTTCTCA--------TAGTAATA 517
MSP3.2FL B1     GTTGTACAAGCAAACTCTGAAACAAATAAAAATCCTACTTCTCA--------TAGTAATA 517
MSP3.2FL B3     GTTGTACAAGCAAACTCTGAAACAAATAAAAATCCTACTTCTCA--------TAGTAATA 517
MSP3.2FL B4     GTTGTACAAGCAAACTCTGAAACAAATAAAAATCCTACTTCTCA--------TAGTAATA 517
MSP3.2FL B5     GTTGTACAAGCAAACTCTGAAACAAATAAAAATCCTACTTCTCA--------TAGTAATA 517
MSP3.2FL D1     GTTGTACAAGCAAACTCTGAAACAAATAAAAATCCTACTTCTCA--------TAGTAATA 517
MSP3.2FL D2     GTTGTACAAGCAAACTCTGAAACAAATAAAAATCCTACTTCTCA--------TAGTAATA 517
MSP3.2FL D3     GTTGTACAAGCAAACTCTGAAACAAATAAAAATCCTACTTCTCA--------TAGTAATA 517
MSP3.2FL D4     CATCCTCAA--AGAGCCAACATCGGAGGAATATCCCATTTATCAGGGACACGTGGAATTC 526
MSP3.2FL D5     GTTGTACAAGCAAACTCTGAAACAAATAAAAATCCTACTCCTGG--------TAGTAAAA 517
                  *  ***  * *  *   * *  *  ** **** * *  *          * * *
```

Fig 9G

```
MSP3.2FL 3D7    GTACTACAACTTCTCTGAATAATAATATACTTGGATGGGAATTTGGAGGAGGTGCTCCTC 577
MSP3.2FL B1     GTACTACAACTTCTCTGAATAATAATATACTTGGATGGGAATTTGGAGGAGGTGCTCCTC 577
MSP3.2FL B3     GTACTACAACTTCTCTGAATAATAATATACTTGGATGGGAATTTGGAGGAGGTGCTCCTC 577
MSP3.2FL B4     GTACTACAACTTCTCTGAATAATAATATACTTGGATGGGAATTTGGAGGAGGTGCTCCTC 577
MSP3.2FL B5     GTACTACAACTTCTCTGAATAATAATATACTTGGATGGGAATTTGGAGGAGGTGCTCCTC 577
MSP3.2FL D1     GTACTACAACTTCTCTGAATAATAATATACTTGGATGGGAATTTGGAGGAGGTGCTCCTC 577
MSP3.2FL D2     GTACTACAACTTCTCTGAATAATAATATACTTGGATGGGAATTTGGAGGAGGTGCTCCTC 577
MSP3.2FL D3     GTACTACAACTTCTCTGAATAATAATATACTTGGATGGGAATTTGGAGGAGGTGCCCCTC 577
MSP3.2FL D4     ATACTTATTCATCTCTGGATAGTAATATACTTGGATGGGAATTTGGAGGAGGTGCTCCTC 586
MSP3.2FL D5     GTACTACAACTTCTCTGAATAATAATATACTTGGATGGGAATTTGGAGGAGGTGCTCCTC 577
                 ****    * ****** *** ********************************* ****

MSP3.2FL 3D7    AAAATGGAGCTGCAGAAGATAAAAAGACAGAATATTTACTAGAACAAATAAAAATTCCAT 637
MSP3.2FL B1     AAAATGGAGCTGCAGAAGATAAAAAGACAGAATATTTACTAGAACAAATAAAGATTCCAT 637
MSP3.2FL B3     AAAATGGAGCTGCAGAAGATAAAAAGACCGAATATTTACTAGAACAAATAAAAATTCCAT 637
MSP3.2FL B4     AAAATGGAGCTGCAGAAGATAAAAAGACAGAATATTTACTAGAACAAATAAAAATTCCAT 637
MSP3.2FL B5     AAAATGGAGCTGCAGAAGATAAAAAGACAGAATATTTACTAGAACAAATAAAAATTCCAT 637
MSP3.2FL D1     AAAATGGAGCTGCAGAAGATAAAAAGACCGAATATTTACTAGAACAAATAAAAATTCCAT 637
MSP3.2FL D2     AAAATGGAGCTGCAGAAGATAAAAAGACAGAATATTTACTAGAACAAATAAAAATTCCAT 637
MSP3.2FL D3     AAAATGGAGCTGCAGAAGATAAAAAGACAGAATATTTACTAGAACAAATAAAAATTCCAT 637
MSP3.2FL D4     AAAATGGAGCTGCAGAAGGTAAAAAGACAGAATATTTACTAGAACAAATAAAAATTCCAT 646
MSP3.2FL D5     AAAATGGAGCTGCAGAAGATAAAAAGACAGAATATTTACTAGAACAAATAAAAATTCCAT 637
                ****************** ********* *********************** *******

MSP3.2FL 3D7    CATGGGATAGAAATAACATCCCCGATGAGAATGAACAAGTAATAGAGGACCCTCAAGAAG 697
MSP3.2FL B1     CATGGGATAGAAATAACATCCCCGATGAGAATGAACAAGTAAAAGAGGACCCTCAAGAAG 697
MSP3.2FL B3     CATGGGATAAAAATAACATCCCCGATGAGAATGAACAAGTAAAAGAGGACCCTCAAGAAG 697
MSP3.2FL B4     CATGGGATAGAAATAACATCCCCGATGAGAATGAACAAGTAAAAGAGGACCCTCAAGAAG 697
MSP3.2FL B5     CATGGGATAGAAATAACATCCCCGATGAGAATGAACAAGTAAAAGAGGACCCTCAAGAAG 697
MSP3.2FL D1     CATGGGATAGAAATAACATCCCCGATGAGAATGAACAAGTAAAAGAGGACCCTCAAGAAG 697
MSP3.2FL D2     CATGGGATAGAAATAACATCCCCGATGAGAATGAACAAGTAAAAGAGGACCCTCAAGAAG 697
MSP3.2FL D3     CATGGGATAGAAATAACATCCCCGATGAGAATGAACAAGTAAAAGAGGACCCTCAAGAAG 697
MSP3.2FL D4     CATGGGATAGAAATAACATCCCCGATGAGAATGAACAAGTAAAAGAGGAACCTCAAGAAG 706
MSP3.2FL D5     CATGGGATAGAAATAACATCCCCGATGAGAATGAACAAGTAATAGAGGACCCTCAAGAAG 697
                ********* ******************************* ******* **********

MSP3.2FL 3D7    ATAATAAAGATGAAGATGAAGATGAAGA------AACAGAAACAGAAAATTTGGAAACAG 751
```

fig 9H

```
MSP3.2FL B1     ATAATAAAGATGAAGATGAAGATGAAGA------AACAGAAACAGAAAATTTGGAAACAG 751
MSP3.2FL B3     ATAATAAAGATGAAGATGAAGATGAAGG------AACAGAAACAGAAAATTTGGAAACAG 751
MSP3.2FL B4     ATAATAAAGATGAAGATGAAGATGAAGA------AACAGAAACAGAAAATTTGGAAACAG 751
MSP3.2FL B5     ATAATAAAGATGAAGATGAAGATGAAGA------AACAGAAACAGAAAATTTGGAAACAG 751
MSP3.2FL D1     ATAATAAAGATGAAGATGAAGATGAAGA------AACAGAAACAGAAAATTTGGAAACAG 751
MSP3.2FL D2     ATAATAAAGATGAAGATGAAGATGAAGA------AACAGAAACAGAAAATTTGGAAACAG 751
MSP3.2FL D3     ATAATAAAGATGAAGATGAAGATGAAGA------AACAGAAACAGAAAATTTGGAAACAG 751
MSP3.2FL D4     ATAATAAAGATGAAGATGAAGATGAAGATGAAGAAACAGAAACAGAAAATTTGGAAACAG 766
MSP3.2FL D5     ATAATAAAGATGAAGATGAAGATGAAGA------AACAGAAACAGAAAATTTGGAAACAG 751
                ***************************       **************************

MSP3.2FL 3D7    AAGATGATAATAATGAAGAGATAGAAGAAAATGAAGAAGATGACATAGATGAAGAAAGTG 811
MSP3.2FL B1     AAGATGATAATAATGAAGAGATAGAAGAAAATGAAGAAGATGACATAGATGAAGAAAGTG 811
MSP3.2FL B3     AAGATGATAATAATGAAGAGATAGAAGAAAATGAAGAAGATGACATAGATGAAGAAAGTG 811
MSP3.2FL B4     AAGATGATAATAATGAAGAGATAGAAGAAAATGAAGAAGATGACATAGATGAAGAAAGTG 811
MSP3.2FL B5     AAGATGATAATAATGAAGAGATAGAAGAAAATGAAGAAGATGACATAGATGAAGAAAGTG 811
MSP3.2FL D1     AAGATGATAATAATGAAGAGATAGAAGAAAATGAAGAAGATGACATAGATGAAGAAAGTG 811
MSP3.2FL D2     AAGATGATAATAATGAAGAGATAGAAGAAAATGAAGAAGATGACATAGATGAAGAAAGTG 811
MSP3.2FL D3     AAGATGATAATAATGAAGAGATAGAAGAAAATGAAGAAGATGACATAGATGAAGAAAGTG 811
MSP3.2FL D4     AAGATGATAATAATGAAGAGATAGAAGAAAATGAAGAAGATGACATAGATGAAGAAAGTG 826
MSP3.2FL D5     AAGATGATAATAATGAAGAGATAGAAGAAAATGAAGAAGATGACATAGATGAAGAAAGTG 811
                ************************************************************

MSP3.2FL 3D7    TAGAAGAAAAGGAAGAAGAGGAAGAAAAAAAAGGAAGAAGAAGAAAAAAGGAAGAAAAAA 871
MSP3.2FL B1     TAGAAGAAAAGGAAGAAGAGGAAGAAAAAAAAGGAAGAAGAAGAAAAAAGGAAGAAAAAA 871
MSP3.2FL B3     TAGAAGAAAAGGAAGAAGAGGAAGAAAAAAAAGGAAGAAGAAGAAAAAAGGAAGAAAAAA 871
MSP3.2FL B4     TAGAAGAAAAGGAAGAAGAGGAAGAAAAAAAAGGAAGAAGAAGAAAAAAGGAAGAAAAAA 871
MSP3.2FL B5     TAGAAGAAAAGGAAGAAGAGGAAGAAAAAAAAGGAAGAAGAAGAAAAAAGGAAGAAAAAA 871
MSP3.2FL D1     TAGAAGAAAAGGAAGAAGAGGAAGAAAAAAAAGGAAGAAGAAGAAAAAAGGAAGAAAAAA 871
MSP3.2FL D2     TAGAAGAAAAGGAAGAAGAGGAAGAAAAAAAAGGAAGAAGAAGAAAAAAGGAAGAAAAAA 871
MSP3.2FL D3     TAGAAGAAAAGGAAGAAGAGGAAGAAAAAAAGGGAAGAAGAAGAAAAAAGGAAGAAAAAA 871
MSP3.2FL D4     TAGAAGAAAAGGAAGAAGAGGAAGAAAAAAAAGGAAGAAGAAGAGAAAAGGAAGAAAAAA 886
MSP3.2FL D5     TAGAAGAAAAGGAAGAAGAGGAAGAAAAAAAAGGAAGAAGAAGAAAAAAGGAAGAAAAAA 871
                ****************************** ************ ****************

MSP3.2FL 3D7    AAGAAGAAAAAAAACCAGACAATGAAATTACAAATGAAGTTAAAGAGGAACAAAAATATA 931
MSP3.2FL B1     AAGAAGAAAAAAACCCAGACAATGAAATTACAAATGAAGTTAAAGAGGAACAAAAATATA 931
MSP3.2FL B3     AAGAAGAAAAAAAACCAGACAATGAAATTACAAATGAAGTTAAAGAGGAACAAAAATATA 931
```

Fig 9I

```
MSP3.2FL B4     AAGAAGAAAAAAAACCAGACAATGAAATTACAAATGAAGTTAAAGAGGAACAAAAATATA 931
MSP3.2FL B5     AAGAAGAAAAAAAACCAGACAATGAAATTACAAATGAAGTTAAAGAGGAACAAAAATATA 931
MSP3.2FL D1     AAGAAGAAAAAAAACCAGACAATGAAATTACAAATGAAGTTAAAGAGGAACAAAAATATA 931
MSP3.2FL D2     AAGAAGAAAAAAAACCAGACAATGAAATTACAAATGAAGTTAAAGAGGAACAAAAATATA 931
MSP3.2FL D3     AAGAAGAAAAAAAACCAGACAATGAAATTACAAATGAAGTTAAAGAGGAACAAAAATATA 931
MSP3.2FL D4     AAGAAGAAAAAAAACCAGACAATGAAATTACAAATGAAGTTAAAGAGGAACAAAAATATA 946
MSP3.2FL D5     AAGAAGAAAAAAAACCAGACAATGAAATTACAAATGAAGTTAAAGAGGAACAAAAATATA 931
                ************* **********************************************

MSP3.2FL 3D7    GTTCACCAAGTGATATAAATGCCCAAAATTTAATTTCTAATAAGAATAAAAAGAATGATG 991
MSP3.2FL B1     GTTCACCAAGTGATATAAATGCCCAAAATTTAATTTCTAATAAGAATAAAAAGAATGATG 991
MSP3.2FL B3     GTTCACCAAGTGATATAAATGCCCAAAATTTAATTTCTAATAAGAATAAAAAGAATGATG 991
MSP3.2FL B4     GTTCACCAAGTGATATAAATGCCCAAAATTTAATTTCTAATAAGAATAAAAAGAATGATG 991
MSP3.2FL B5     GTTCACCAAGTGATATAAATGCCCAAAATTTAATTTCTAATAAGAATAAAAAGAATGATG 991
MSP3.2FL D1     GTTCACCAAGTGATATAAATGCCCAAAATTTAATTTCTAACAAGAATAAAAAGAATGATG 991
MSP3.2FL D2     GTTCACCAAGTGATATAAATGCCCAAAATTTAATTTCTAATAAGAATAAAAAGAATGATG 991
MSP3.2FL D3     GTTCACCAAGTGATATAAATGCCCAAAATTTAATTTCTAATAAGAATAAAAAGAATGATG 991
MSP3.2FL D4     GTTCACCAAGTGATATAAATGCCCAAAATTTAATTTCTAATAAGAATAAAAAGAATGATG 1006
MSP3.2FL D5     GTTCACCAAGTGATATAAATGCCCAAAATTTAATTTCTAATAAGAATAAAAAGAATGATG 991
                **************************************** *******************

MSP3.2FL 3D7    AAACAAAAAAGACTGCTGAAAATATAGTTAAAACATTGGTTGGATTATTTAATGAAAAAA 1051
MSP3.2FL B1     AAACAAAAAAGACTGCTGAAAATATAGTTAAAACATTGGTTGGATTATTTAATGAAAAAA 1051
MSP3.2FL B3     AAACGAAAAAGACTGCTGAAAATATAGTTAAAACATTGGTTGGATTATTTAATGAAAAAA 1051
MSP3.2FL B4     AAACAAAAAAGACTGCTGAAAATATAGTTAAAACATTGGTTGGATTATTTAATGAAAAAA 1051
MSP3.2FL B5     AAACAAAAAAGACTGCTGAAAATATAGTTAAAACATTGGTTGGATTATTTAATGAAAAAA 1051
MSP3.2FL D1     AAACAAAAAAGACTGCTGAAAATATAGTTAAAACATTGGTTGGATTATTTAATGAAAAAA 1051
MSP3.2FL D2     AAACAAAAAAGACTGCTGAAAATATAGTTAAAACATTGGTTGGATTATTTAATGAAAAAA 1051
MSP3.2FL D3     AAACAAAAAAGACTGCTGAAAATATAGTTAAAACATTGGTTGGATTATTTAATGAAAAAA 1051
MSP3.2FL D4     AAACAAAAAAGACTGCTGAAAATATAGTTAAAACATTGGTTGGATTATTTAATGAAAAAA 1066
MSP3.2FL D5     AAACAAAAAAGACTGCTGAAAATATAGTTAAAACATTGGTTGGATTATTTAATGAAAAAA 1051
                **** *******************************************************

MSP3.2FL 3D7    ATGAGATAGATTCTACTATAAATAATTTAGTACAAGAAATGATCCATCTATTTAGTAATA 1111
MSP3.2FL B1     ATGAGATAGATTCTACTATAAATAATTTAGTACAAGAAATGATCCATCTATTTAGTAATA 1111
MSP3.2FL B3     ATGAGATAGATTCTACTATAAATAATTTAGTACAAGAAATGATCCATCTATTTAGTAATA 1111
MSP3.2FL B4     ATGAGATAGATTCTACTATAAATAATTTAGTACAAGAAATGATCCATCTATTTAGTAATA 1111
MSP3.2FL B5     ATGAGATAGATTCTACTATAAATAATTTAGTACAAGAAATGATCCATCTATTTAGTAATA 1111
```

Fig 9J

```
MSP3.2FL D1      ATGAGATAGATTCTACTATAAATAATTTAGTACAAGAAATGATCCATCTATTTAGTAATA 1111
MSP3.2FL D2      ATGAGATAGATTCTACTATAAATAATTTAGTACAAGAAATGATCCATCTATTTAGTAATA 1111
MSP3.2FL D3      ATGAGATAGATTCTACTATAAATAATTTAGTACAAGAAATGATCCATCTATTTAGTAATA 1111
MSP3.2FL D4      ATGGGATAGATTCTACTATAAATAATTTAGTACAAGAAATGATCCATCTATTTAGTAATA 1126
MSP3.2FL D5      ATGAGATAGATTCTACTATAAATAATTTAGTACAAGAAATGATCCATCTATTTAGTAATA 1111
                 *** ********************************************************

MSP3.2FL 3D7     ATTAA 1116
MSP3.2FL B1      ATTAA 1116
MSP3.2FL B3      ATTAA 1116
MSP3.2FL B4      ATTAA 1116
MSP3.2FL B5      ATTAA 1116
MSP3.2FL D1      ATTAA 1116
MSP3.2FL D2      ATTAA 1116
MSP3.2FL D3      ATTAA 1116
MSP3.2FL D4      ATTAA 1131
MSP3.2FL D5      ATTAA 1116
                 *****
```

**<u>ClustalW alignment ORF2C Nucleotide sequences</u>** <u>(Www.ebi.ac.uk/clustalw/)</u> **MSP3-3**

```
CLUSTAL W (1.82) multiple sequence alignment


MSP3.3C 3D7      TATGAGAAGAAAAATGAAAATAAAAATGTATCAAATGTAGATTCAAAAACAAAATCAAAT 60
MSP3.3C B1       TATGAGAAGAAAAATAAAAATAAAAATGTATCAAATGTAGATTCAAAAACAAAATCAAAT 60
MSP3.3C B2       -ATGAGAAGAAAAATGAAAATAAAAATGTATCAAATGTAGATTCAAAAACAAAATCAAAT 59
MSP3.3C B5       TATGAGAAGAAAAATGAAAATAAAAATGTATCAAATGTAGATTCAAAAACAAAATCAAAT 60
MSP3.3C D1       TATGAGAAGAAAAATGAAAATAAAAATGTATCAAATGTAGATTCAAAAACAAAATCAAAT 60
MSP3.3C D2       TATGAGAAGAAAAATGAAAATAAAAATGTATCAAATGTAGATTCAAAAACAAAATCAAAT 60
MSP3.3C D4       TATGAGAAGAAAAATGAAAATAAAAATGTATCAAATGTAGATTCAAAAACAAAATCAAAT 60
MSP3.3C D5       TATGAGAAGAAAAATGAAAATAAAAATGTATCAAATGTAGATTCAAAAACAAAATCAAAT 60
                  ************** ********************************************

MSP3.3C 3D7      GAAAAAGGAAGACCTCCTACATATTCTCCTATTCTGGATGATGGGATAGAATTTAGTGGT 120
MSP3.3C B1       GAAAAAGGAAGACCTCCTACATATTCTCCTATTCTGGATGATGGGATAGAATTTAGTGGT 120
MSP3.3C B2       GAAAAAGGAAGACCTCCTACATATTCTCCTATTCTGGATGATGGGATAGAATTTAGTGGT 119
```

Fig 9K

```
MSP3.3C B5     GAAAAAGGAAGACCTCCTACATATTCTCCTATTCTGGATGATGGGATAGAATTTAGTGGT 120
MSP3.3C D1     GAAAAAGGAAGACCTCCTACATATTCTCCTATTCTGGATGATGGGATAGAATTTAGTGGT 120
MSP3.3C D2     GAAAAAGGAAGACCTCCTACATATTCTCCTATTCTGGATGATGGGATAGAATTTAGTGGT 120
MSP3.3C D4     GAAAAAGGAAGACCTCCTACATATTCTCCTATTCTGGATGATGGGATAGAATTTAGTGGT 120
MSP3.3C D5     GAAAAAGGAAGACCTCCTACATATTCTCCTATTCTGGATGATGGGATAGAATTTAGTGGT 120
               ************************************************************

MSP3.3C 3D7    GGTTTATATTTTAATGAGAAAAAGTCGACTGAAGAAAATAAACAAAAAAATGTTTTAGAA 180
MSP3.3C B1     GGTTTATATTTTAATGAGAAAAAGTCGACTGAAGAAAATAAACAAAAAAATGTTTTAGAA 180
MSP3.3C B2     GGTTTATATTTTAATGAGAAAAAGTCGACTGAAGAAAATAAACAAAAAAATGTTTTAGAA 179
MSP3.3C B5     GGTTTATATTTTAATGAGAAAAAGTCGACTGAAGAAAATAAACAAAAAAATGTTTTAGAA 180
MSP3.3C D1     GGTTTATATTTTAATGAGAAAAAGTCGACTGAAGAAAATAAACAAAAAAATGTTTTAGAA 180
MSP3.3C D2     GGTTTATATTTTAATGAGAAAAAGTCGACTGAAGAAAATAAACAAAAAAATGTTTTAGAA 180
MSP3.3C D4     GGTTTATATTTTAATGAGAAAAAGTCGACTGAAGAAAATAAACAAAAAAATGTTTTAGAA 180
MSP3.3C D5     GGTTTATATTTTAATGAGAAAAAGTCGACTGAAGAAAATAAACAAAAAAATGTTTTAGAA 180
               ************************************************************

MSP3.3C 3D7    TCAGTAAATTTAACATCGTGGGATAAAGAGGATATTGTTAAGGAAAATGAGGATGTTAAA 240
MSP3.3C B1     TCAGTAAATTTAACATCGTGGGATAAAGAGGATATTGTTAAGGAAAATGAGGATGTTAAA 240
MSP3.3C B2     TCAGTAAATTTAACATCGTGGGATAAAGAGGATATTGTTAAGGAAAATGAGGATGTTAAA 239
MSP3.3C B5     TCAGTAAATTTAACATCGTGGGATAAAGAGGATATTGTTAAGGAAAATGAGGATGTTAAA 240
MSP3.3C D1     TCAGTAAATTTAACATCGTGGGATAAAGAGGATATTGTTAAGGAAAATGAGGATGTTAAA 240
MSP3.3C D2     TCAGTAAATTTAACATCGTGGGATAAAGAGGATATTGTTAAGGAAAATGAGGATGTTAAA 240
MSP3.3C D4     TCAGTAAATTTAACATCGTGGGATAAAGAGGATATTGTTAAGGAAAATGAGGATGTTAAA 240
MSP3.3C D5     TCAGTAAATTTAACATCGTGGGATAAAGAGGATATTGTTAAGGAAAATGAGGATGTTAAA 240
               ************************************************************

MSP3.3C 3D7    GATGAAAAGGATGAAGATGATGAAGAAGAAGAAGAAAAATACGAAAACGAAATTATAAAG 300
MSP3.3C B1     GATGAAAAGGATGAAGATGATGAAGAAGAAGAAGAAAAATACGAAAACGAAATTATAAAG 300
MSP3.3C B2     GATGAAAAGGATGAAGATGATGAAGAAGAAGAAGAAAAATACGAAAACGAAATTATAAAG 299
MSP3.3C B5     GATGAAAAGGATGAAGATGATGAAGAAGAAGAAGAAAAATACGAAAACGAAATTATAAAG 300
MSP3.3C D1     GATGAAAAGGATGAAGATGATGAAGAAGAAGAAGAAAAATACGAAAACGAAATTATAAAG 300
MSP3.3C D2     GATGAAAAGGATGAAGATGATGAAGAAGAAGAAGAAAAATACGAAAACGAAATTATAAAG 300
MSP3.3C D4     GATGAAAAGGATGAAGATGATGAAGAAGAAGAAGAAAAATACGAAAACGAAATTATAAAG 300
MSP3.3C D5     GATGAAAAGGATGAAGATGATGAAGAAGAAGAAGAAAAATACGAAAACGAAATTATAAAG 300
               ************************************************************

MSP3.3C 3D7    CAACCAGAAGACATATTGGATGAGGAAGAAGTATTAGAAGAAGAAATATTAGAAGAAAAT 360
```

Fig 9L

```
MSP3.3C B1     CAACCAGAAGACATATTGGATGAGGAAGAAGTATTAGAAGAAGAAATATTAGAAGAAAAT 360
MSP3.3C B2     CAACCAGAAGACATATTGGATGAGGAAGAAGTATTAGAAGAAGAAATATTAGAAGAAAAT 359
MSP3.3C B5     CAACCAGAAGACATATTGGATGAGGAAGAAGTATTAGAAGAAGAAATATTAGAAGAAAAT 360
MSP3.3C D1     CAACCAGAAGACATATTGGATGAGGAAGAAGTATTAGAAGAAGAAATATTAGAAGAAAAT 360
MSP3.3C D2     CAACCAGAAGACATATTGGATGAGGAAGAAGTATTAGAAGAAGAAATATTAGAAGAAAAT 360
MSP3.3C D4     CAACCAGAAGACATATTGGATGAGGAAGAAGTATTAGAAGAAGAAATATTAGAAGAAAAT 360
MSP3.3C D5     CAACCAGAAGACATATTGGATGAGGAAGAAGTATTAGAAGAAGAAATATTAGAAGAAAAT 360
               ************************************************************

MSP3.3C 3D7    AAAAATGATACAGTAGATACAAGTGATTTAGAAAAGAAAAATATACCAGATTTATCAAAC 420
MSP3.3C B1     AAAAATGATACAGTAGATACAAGTGATTTAGAAAAGAAAAATATACCAGATTTATCAAAC 420
MSP3.3C B2     AAAAATGATACAGTAGATACAAGTGATTTAGAAAAGAAAAATATACCAGATTTATCAAAC 419
MSP3.3C B5     AAAAATGATACAGTAGATACAAGTGATTTAGAAAAGAAAAATATACCAGATTTATCAAAC 420
MSP3.3C D1     AAAAATGATACAGTAGATACAAGTGATTTAGAAAAGAAAAATATACCAGATTTATCAAAC 420
MSP3.3C D2     AAAAATGATACAGTAGATACAAGTGATTTAGAAAAGAAAAATATACCAGATTTATCAAAC 420
MSP3.3C D4     AAAAATGATACAGTAGATACAAGTGATTTAGAAAAGAAAAATATACCAGATTTATCAAAC 420
MSP3.3C D5     AAAAATGATACAGTAGATACAAGTGATTTAGAAAAGAAAAATATACCAGATTTATCAAAC 420
               ************************************************************

MSP3.3C 3D7    GATAATAATTATTATAGTTTAATTTATAAGAACTATAAGGATAATGATAAATCAGAAAAA 480
MSP3.3C B1     GATAATAATTATTATAGTTTAATTTATAAGAACTATAAGGGTAATGATAAATCAGAAAAA 480
MSP3.3C B2     GATAATAATTATTATAGTTTAATTTATAAGAACTATAAGGATAATGATAAATCAGAAAAA 479
MSP3.3C B5     GATAATAATTATTATAGTTTAATTTATAAGAACTATAAGGATAATGATAAATCAGAAAAA 480
MSP3.3C D1     GATAATAATTATTATAGTTTAATTTATAAGAACTATAAGGATAATGATAAATCAGAAAAA 480
MSP3.3C D2     GATAATAATTATTATAGTTTAATTTATAAGAACTATAAGGATAATGATAAATCAGAAAAA 480
MSP3.3C D4     GATAATAATTATTATAGTTTAATTTATAAGAACTATAAGGATAATGATAAATCAGAAAAA 480
MSP3.3C D5     GATAATAATTATTATAGTTTAATTTATAAGAACTATAAGGATAATGATAAATCAGAAAAA 480
               **************************************** *******************

MSP3.3C 3D7    ACTGCACAAACATTAATCACAGCTCTGATAAGTTTATTAAATGGAAAAAATGAATTAGAT 540
MSP3.3C B1     ACTGCACAAACATTAATCACAGCTCTGATAAGTTTATTAAATGGAAAAAATGAATTAGAT 540
MSP3.3C B2     ACTGCACAAACATTAATCACAGCTCTGATAAGTTTATTAAATGGAAAAAATGAATTAGAT 539
MSP3.3C B5     ACTGCACAAACATTAATCACAGCTCTGATAAGTTTATTAAATGGAAAAAATGAATTAGAT 540
MSP3.3C D1     ACTGCACAAACATTAATCACAGCTCTGATAAGTTTATTAAATGGAAAAAATGAATTAGAT 540
MSP3.3C D2     ACTGCACAAACATTAATCACAGCTCTGATAAGTTTATTAAATGGAAAAAATGAATTAGAT 540
MSP3.3C D4     ACTGCACAAACATTAATCACAGCTCTGATAAGTTTATTAAATGGAAAAAATGAATTAGAT 540
MSP3.3C D5     ACTGCACAAACATTAATCACAGCTCTGATAAGTTTATTAAATGGAAAAAATGAATTAGAT 540
               ************************************************************
```

Fig 9M

```
MSP3.3C 3D7    GCTACCATAAGAAGATTAAAACATAGGTTTATGGAATTTTTACATATAATTAA 594
MSP3.3C B1     GCTACCATAAGAAGATTAAAACATAGGTTTATGGAATTTTTACATATAATTAA 594
MSP3.3C B2     GCTACCATAAGAAGATTAAAACATAGGTTTATGGAATTTTTACATATAATTAA 593
MSP3.3C B5     GCTACCATAAGAAGATTAAAACATAGGTTTATGGAATTTTTACATATAATTAA 594
MSP3.3C D1     GCTACCATAAGAAGATTAAAACATAGGTTTATGGAATTTTTACATATAATTAA 594
MSP3.3C D2     GCTACCATAAGAAGATTAAAACATAGGTTTATGGAATTTTTACATATAATTAA 594
MSP3.3C D4     GCTACCATAAGGAGATTAAAACATAGGTTTATGGAATTTTTACATATAATTAA 594
MSP3.3C D5     GCTACCATAAGAAGATTAAAACATAGGTTTATGGAATTTTTACATATAATTAA 594
               *********** *****************************************
```

**ClustalW alignment ORF3C Nucleotide sequences** (Www.ebl.ac.uk/clustalw/) **MSP3-4**

```
CLUSTAL W (1.82) multiple sequence alignment

MSP3.4C 3D7    GATAATGTAAACTCTGTAACGCAAAGAGGAAATAATAACTACAACAATAATTTAGAGCGT 60
MSP3.4C B4     -ATAATGTAAACTCTGTAACGCAAAGAGGAAATAATAACTACAACAATAATTTAGAGCGT 59
MSP3.4C B1     GATAATGTAAACTCTGTAACGCAAAGAGGAAATAATAACTACAACAATAATTTAGAGCGT 60
MSP3.4C B5     GATAATGTAAACTCTGTAACGCAAAGAGGAAATAATAACTACAACAATAATTTAGAGCGT 60
MSP3.4C D4     GATAATGTAAACTCTGTAACGCAAAGAGGAAATAATAACTACAACAATAATTTAGAGCGT 60
MSP3.4C D5     GATAATGTAAACTCTGTAACGCAAAGAGGAAATAATAACTACAACAATAATTTAGAGCGT 60
MSP3.4C D2     GATAATGTAAACTCTGTAACGCAAAGAGGAAATAATAACTACAACAATAATTTAGAGCGT 60
MSP3.4C D1     GATAATGTAAACTCTGTAACGCAAAGAGGAAATAATAACTACAACAATAATTTAGAGCGT 60
                ***********************************************************

MSP3.4C 3D7    GGATTGGGTTCTGGTGCTCTTCCTGGTACAAATATTATTACTGAAGAAAAATATTCTCTA 120
MSP3.4C B4     GGATTGGGTTCTGGTGCTCTTCCTGGTACAAATATTATTACTGAAGAAAAATATTCTCTA 119
MSP3.4C B1     GGATTGGGTTCTGGTGCTCTTCCTGGTACAAATATTATTACTGAAGAAAAATATTCTCTA 120
MSP3.4C B5     GGATTGGGTTCTGGTGCTCTTCCTGGTACAAATATTATTACTGAAGAAAAATATTCTCTA 120
MSP3.4C D4     GGATTGGGTTCTGGTGCTCTTCCTGGTACAAATATTATTACTGAAGAAAAATATTCTCTA 120
MSP3.4C D5     GGATTGGGTTCTGGTGCTCTTCCTGGTACAAATATTATTACTGAAGAAAAATATTCTCTA 120
MSP3.4C D2     GGATTGGGTTCTGGTGCTCTTCCTGGTACAAATATTATTACTGAAGAAAAATATTCTCTA 120
MSP3.4C D1     GGATTGGGTTCTGGTGCTCTTCCTGGTACAAATATTATTACTGAAGAAAAATATTCTCTA 120
               ************************************************************

MSP3.4C 3D7    GAATTAATAAAATTAACATCAAAGGATGAAGAAGATATTATAAAGCATAATGAGGATGTG 180
```

Fig 9N

```
MSP3.4C  B4    GAATTAATAAAATTAACATCAAAGGATGAAGAAGATATTATAAAGCATAATGAGGATGTG 179
MSP3.4C  B1    GAATTAATAAAATTAACATCAAAGGATGAAGAAGATATTATAAAGCATAATGAGGATGTG 180
MSP3.4C  B5    GAATTAATAAAATTAACATCAAAGGATGAAGAAGATATTATAAAGCATAATGAGGATGTG 180
MSP3.4C  D4    GAATTAATAAAATTAACATCAAAGGATGAAGAAGATATTATAAAGCATAATGAGGATGTG 180
MSP3.4C  D5    GAATTAATAAAATTAACATCAAAGGATGAAGAAGATATTATAAAGCATAATGAGGATGTG 180
MSP3.4C  D2    GAATTAATAAAATTAACATCAAAGGATGAAGAAGATATTATAAAGCATAATGAGGATGTG 180
MSP3.4C  D1    GAATTAATAAAATTAACATCAAAGGATGAAGAAGATATTATAAAGCATAATGAGGATGTG 180
               ************************************************************

MSP3.4C  3D7   AGAGAAGAAATAGAAGAACAACAAGAAGACATCGAGGAAGATGAAGAAGAATTGGAAAAT 240
MSP3.4C  B4    AGAGAAGAAATAGAAGAACAACAAGAAGACATCGAGGAAGATGAAGAAGAATTGGAAAAT 239
MSP3.4C  B1    AGAGAAGAAATAGAAGAACAACAAGAAGACATCGAGGAAGATGAAGAAGAATTGGAAAAT 240
MSP3.4C  B5    AGAGAAGAAATAGAAGAACAACAAGAAGACATCGAGGAAGATGAAGAAGAATTGGAAAAT 240
MSP3.4C  D4    AGAGAAGAAATAGAAGAACAACAAGAAGACATCGAGGAAGATGAAGAAGAATTGGAAAAT 240
MSP3.4C  D5    AGAGAAGAAATAGAAGAACAACAAGAAGACATCGAGGAAGATGAAGAAGAATTGGAAAAT 240
MSP3.4C  D2    AGAGAAGAAATAGAAGAACAACAAGAAGACATCGAGGAAGATGAAGAAGAATTGGAAAAT 240
MSP3.4C  D1    AGAGAAGAAATAGAAGAACAACAAGAAGACATCGAGGAAGATGAAGAAGAATTGGAAAAT 240
               ************************************************************

MSP3.4C  3D7   GAAGGAGAAGAAACAAAAGAAGAAGATGATGAAGAAAAGAATGAAACAAATGATACGGAA 300
MSP3.4C  B4    GAAGGAGAAGAAACAAAAGAAGAAGATGATGAAGAAAAGAATGAAACAAATGATACGGAA 299
MSP3.4C  B1    GAAGGAGAAGAAACAAAAGAAGAAGATGATGAAGAAAAGAATGAAACAAATGATACGGAA 300
MSP3.4C  B5    GAAGGAGAAGAAACAAAAGAAGAAGATGATGAAGAAAAGAATGAAACAAATGATACGGAA 300
MSP3.4C  D4    GAAGGAGAAGAAACAAAAGAAGAAGATGATGAAGAAAAGAATGAAACAAATGATACGGAA 300
MSP3.4C  D5    GAAGGAGAAGAAACAAAAGAAGAAGATGATGAAGAAAAGAATGAAACAAATGATACGGAA 300
MSP3.4C  D2    GAAGGAGAAGAAACAAAAGAAGAAGATGATGAAGAAAAGAATGAAACAAATGATACGGAA 300
MSP3.4C  D1    GAAGGAGAAGAAACAAAAGAAGAAGATGATGAAGAAAAGGATGAAACAAATGATACGGAA 300
               *************************************** ********************

MSP3.4C  3D7   GATACGGACGATACTGAAGATACGGAAGATATAGAAGAGGAAAATAAGGAAAAAGAACTC 360
MSP3.4C  B4    GATACGGACGATACTGAAGATACGGAAGATATAGAAGAGGAAAATAAGGAAAAAGAACTC 359
MSP3.4C  B1    GATACGGACGATACTGAAGATACGGAAGATATAGAAGAGGAAAATAAGGAAAAAGAACTC 360
MSP3.4C  B5    GATACGGACGATACTGAAGATACGGAAGATATAGAAGAGGAAAATAAGGAAAAAGAACTC 360
MSP3.4C  D4    GATACGGACGATACTGAAGATACGGAAGATATAGAAGAGGAAAATAAGGAAAAAGAACTC 360
MSP3.4C  D5    GATACGGACGATACTGAAGATACGGAAGATATAGAAGAGGAAAATAAGGAAAAAGAACTC 360
MSP3.4C  D2    GATACGGACGATACTGAAGATACGGAAGATATAGAAGAGGAAAATAAGGAAAAAGAACTC 360
MSP3.4C  D1    GATACGGACGATACTGAAGATACGGAAGATATAGAAGAGGAAAATAAGGAAAAAGAACTC 360
               ************************************************************
```

Fig 90

```
MSP3.4C  3D7    AGTAATCAACAACAAAGTGAAAAAAAAAGTATTTCAAAAGTTGACGAAGATTCATATCGA 420
MSP3.4C  B4     AGTAATCAACAACAAAGTGAAAAAAAAAGTATTTCAAAAGTTGACGAAGATTCATATCGA 419
MSP3.4C  B1     AGTAATCAACAACAAAGTGAAAAAAAAAGTATTTCAAAAGTTGACGAAGATTCATATCGA 420
MSP3.4C  B5     AGTAATCAACAACAAAGTGAAAAAAAAAGTATTTCAAAAGTTGACGAAGATTCATATCGA 420
MSP3.4C  D4     AGTAATCAACAACAAAGTGAAAAAAAAAGTATTTCAAAAGTTGACGAAGATTCATATCGA 420
MSP3.4C  D5     AGTAATCAACAACAAAGTGAAAAAAAAAGTATTTCAAAAGTTGACGAAGATTCATATCGA 420
MSP3.4C  D2     AGTAATCAACAACAAAGCGAAAAAAAAAGTATTTCAAAAGTTGACGAAGATTCATATCGA 420
MSP3.4C  D1     AGTAATCAACAACAAAGTGAAAAAAAAAGTATTTCAAAAGTTGACGAAGATTCATATCGA 420
                ***************** ******************************************

MSP3.4C  3D7    ATACTATCAGTAAGTTATAAGGACAATAATGAAGTAAAAAATGTTGCTGAATCTATAGTG 480
MSP3.4C  B4     ATACTATCAGTAAGTTATAAGGACAATAATGAAGTAAAAAATGTTGCTGAATCTATAGTG 479
MSP3.4C  B1     ATACTATCAGTAAGTTATAAGGACAATAATGAAGTAAAAAATGTTGCTGAATCTATAGTG 480
MSP3.4C  B5     ATACTATCAGTAAGTTATAAGGACAATAATGAAGTAAAAAATGTTGCTGAATCTATAGTG 480
MSP3.4C  D4     ATACTATCAGTAAGTTATAAGGACAATAATGAAGTAAAAAATGTTGCTGAATCTATAGTG 480
MSP3.4C  D5     ATACTATCAGTAAGTTATAAGGACAATAATGAAGTAAAAAATGTTGCTGAATCTATAGTG 480
MSP3.4C  D2     ATACTATCAGTAAGTTATAAGGACAATAATGAAGTAAAAAATGTTGCTGAATCTATAGTG 480
MSP3.4C  D1     ATACTATCAGTAAGTTATAAGGACAATAATGAAGTAAAAAATGTTGCTGAATCTATAGTG 480
                ************************************************************

MSP3.4C  3D7    AAAAAACTATTTAGTTTATTTAATGATAATAATAATTTGGAAACTATTTTTAAGGGTTTG 540
MSP3.4C  B4     AAAAAACTATTTAGTTTATTTAATGATAATAATAATTTGGAAACTATTTTTAAGGGTTTG 539
MSP3.4C  B1     AAAAAACTATTTAGTTTATTTAATGATAATAATAATTTGGAAACTATTTTTAAGGGTTTG 540
MSP3.4C  B5     AAAAAACTATTTAGTTTATTTAATGATAATAATAATTTGGAAACTATTTTTAAGGGTTTG 540
MSP3.4C  D4     AAAAAACTATTTAGTTTATTTAATGATAATAATAATTTGGAAACTATTTTTAAGGGTTTG 540
MSP3.4C  D5     AAAAAACTATTTAGTTTATTTAATGATAATAATAATTTGGAAACTATTTTTAAGGGTTTG 540
MSP3.4C  D2     AAAAAACTATTTAGTTTATTTAATGATAATAATAATTTGGAAACTATTTTTAAGGGTTTG 540
MSP3.4C  D1     AAAAAACTATTTAGTTTATTTAATGATAATAATAATTTGGAAACTATTTTTAAGGGTTTG 540
                ************************************************************

MSP3.4C  3D7    ACAGAAGATATGACAGATTTATTTCAAAAATAA 573
MSP3.4C  B4     ACAGAAGATATGACAGATTTATTTCAAAAATAA 572
MSP3.4C  B1     ACAGAAGATATGACAGATTTATTTCAAAAATAA 573
MSP3.4C  B5     ACAGAAGATATGACAGATTTATTTCAAAAATAA 573
MSP3.4C  D4     ACAGAAGATATGACAGATTTATTTCAAAAATAA 573
MSP3.4C  D5     ACAGAAGATATGACAGATTTATTTCAAAAATAA 573
MSP3.4C  D2     ACAGAAGATATGACAGATTTATTTCAAAAATAA 573
```

```
MSP3.4C  D1          ACAGAAGATATGACAGATTTATTTCAAAAATAA 573
                     *********************************
```

**<u>ClustalW alignment ORF6C Nucleotide sequences</u> (<u>Www.ebi.ac.uk/clustalw/)</u> MSP3-7**

```
CLUSTAL W (1.82) multiple sequence alignment

MSP3.7C  3D7        CCTGAAGGACCAAGAGCAAACAATAGAAATGAAAATAATCAAAATACAGATCCATATAAC 60
MSP3.7C  B1         CCTGAAGGACCAAGAGCAAACAATAGAAATGAAAATAATCAAAATACGGATCCATATAAC 60
MSP3.7C  B2         CCTGAAGGACCAAGAGCAAACAATAGAAATGAAAATAATCAAAATACAGATCCATATAAC 60
MSP3.7C  B3         CCTGAAGGACCAAGAGCAAACAATAGAAATGAAAATAATCAAAATACAGATCCATATAAC 60
MSP3.7C  B4         CCTGAAGGACCAAGAGCAAACAATAGAAATGAAAATAATCAAAATACAGATCCATATAAC 60
MSP3.7C  B5         CCTGAAGGACCAAGAGCAAACAATAGAAATGAAAATAATCAAAATACAGATCCATATAAC 60
MSP3.7C  D1         CCTGAAGGACCAAGAGCAAACAATAGAAATGAAAATAATCAAAATACAGATCCATATAAC 60
MSP3.7C  D2         CCTGAAGGACCAAGAGCAAACAATAGAAATGAAAATAATCAAAATACAGATCCATATAAC 60
MSP3.7C  D5         CCTGAAGGACCAAGAGCAAACAATAGAAATGAAAATAATCAAAATACAGATCCATATAAC 60
MSP3.7C  addtional  CCTGAAGGACCAAGAGCAAACAATAGAAATGAAAATAATCAAAATACAGATCCATATAAC 60
                    *********************************************** ************

MSP3.7C  3D7        CACTATTTTGCATGGGAAATTGGAGGTGGTGCTCCAACGTATAAACCCGAGAACAATAAG 120
MSP3.7C  B1         CACTATTTTGCATGGGAAATTGGAGGTGGTGCTCCAACGTATAAACCCGAGAACAATAAG 120
MSP3.7C  B2         CACTATTTTGCATGGGAAATTGGAGGTGGTGCTCCAACGTATAAACCCGAGAACAATAAG 120
MSP3.7C  B3         CACTATTTTGCATGGGAAATTGGAGGTGGTGCTCCAACGTATAAACCCGAGAACAATAAG 120
MSP3.7C  B4         CACTATTTTGCATGGGAAATTGGAGGTGGTGCTCCAACGTATAAACCCGAGAACAATAAG 120
MSP3.7C  B5         CACTATTTTGCATGGGAAATTGGAGGTGGTGCTCCAACGTATAAACCCGAGAACAATAAG 120
MSP3.7C  D1         CACTATTTTGCATGGGAAATTGGAGGTGGTGCTCCAACGTATAAACCCGAGAACAATAAG 120
```

Fig 9Q

```
MSP3.7C   D2          CACTATTTTGCATGGGAAATTGGAGGTGGTGCTCCAACGTATAAACCCGAGAACAATAAG 120
MSP3.7C   D5          CACTATTTTGCATGGGAAATTGGAGGTGGTGCTCCAACGTATAAACCCGAGAACAATAAG 120
MSP3.7C   addtional   CACTATTTTGCATGGGAAATTGGAGGTGGTGCTCCAACGTATAAACCCGAGAACAATAAG 120
                      ************************************************************

MSP3.7C   3D7         AACGATAATATTTTGCTAGAACACGTAAAAATTACCTCGTGGGATAAAGAAGATATAATT 180
MSP3.7C   B1          AACGATAATATTTTGCTAGAACACGTAAAAATTACCTCGTGGGATAAAGAAGATATAATT 180
MSP3.7C   B2          AACGATAATATTTTGCTAGAACACGTAAAAATTACCTCGTGGGATAAAGAAGATATAATT 180
MSP3.7C   B3          AACGATAATATTTTGCTAGAACACGTAAAAATTACCCCGTGGGATAAAGAAGATATAATT 180
MSP3.7C   B4          AACGATAATATTTTGCTAGAACACGTAAAAATTACCTCGTGGGATAAAGAAGATATAATT 180
MSP3.7C   B5          AACGATAATATTTTGCTAGAACACGTAAAAATTACCTCGTGGGATAAAGAAGATATAATT 180
MSP3.7C   D1          AACGATAATATTTTGCTAGAACACGTAAAAATTACCTCGTGGGATAAAGAAGATATAATT 180
MSP3.7C   D2          AACGATAATATTTTGCTAGAACACGTAAAAATTACCTCGTGGGATAAAGAAGATATAATT 180
MSP3.7C   D5          AACGATAATATTTTGCTAGAACACGTAAAAATTACCTCGTGGGATAAAGAAGATATAATT 180
MSP3.7C   addtional   AACGATAATATTTTGCTAGAACACGTAAAAATTACCTCGTGGGATAAAGAAGATATAATT 180
                      ************************************ ***********************

MSP3.7C   3D7         AAAGAAAATGAAGACACAAAACGCGAAGTTCAAGAAACTGAAGACACTGACGAAACTGAA 240
MSP3.7C   B1          AAAGAAAATGAAGACACAAAACGCGAAGTTCAAGAAACTGAAGACACTGACGAAACTGAA 240
MSP3.7C   B2          AAAGAAAATGAAGACACAAAACGCGAAGTTCAAGAAACTGAAGACACTGACGAAACTGAA 240
MSP3.7C   B3          AAAGAAAATGAAGACACAAAACGCGAAGTTCAAGAAACTGAAGACACTGACGAAACTGAA 240
MSP3.7C   B4          AAAGAAAATGAAGACACAAAACGCGAAGTTCAAGAAACTGAAGACACTGACGAAACTGAA 240
MSP3.7C   B5          AAAGAAAATGAAGACACAAAACGCGAAGTTCAAGAAACTGAAGACACTGACGAAACTGAA 240
MSP3.7C   D1          AAAGAAAATGAAGACACAAAACGCGAAGTTCAAGAAACTGAAGACACTGACGAAACTGAA 240
MSP3.7C   D2          AAAGAAAATGAAGACACAAAACGCGAAGTTCAAGAAACTGAAGACACTGACGAAACTGAA 240
MSP3.7C   D5          AAAGAAAATGAAGACACAAAACGCGAAGTTCAAGAAACTGAAGACACTGACGAAACTGAA 240
MSP3.7C   addtional   AAAGAAAATGAAGACACAAAACGCGAAGTTCAAGAAACTGAAGACACTGACGAAACTGAA 240
                      ************************************************************

MSP3.7C   3D7         GATACTGACGAAACTGAAGAAACAGAAGATATGGAAGATGAAAACGAAATTGTGGAAGAT 300
MSP3.7C   B1          GATACTGACGAAACTGAAGAAACAGAAGATATGGAAGATGAAAACGAAATTGTGGAAGAT 300
MSP3.7C   B2          GATACTGACGAAACTGAAGAAACAGAAGATATGGAAGATGAAAACGAAATTGTGGAAGAT 300
MSP3.7C   B3          GATACTGACGAAACTGAAGAAACAGAAGATATGGAAGATGAAAACGAAATTGTGGAAGAT 300
MSP3.7C   B4          GATACTGACGAAACTGAAGAAACAGAAGATATGGAAGATGAAAACGAAATTGTGGAAGAT 300
MSP3.7C   B5          GATACTGACGAAACTGAAGAAACAGAAGATATGGAAGATGAAAACGAAATTGTGGAAGAT 300
MSP3.7C   D1          GATACTGACGAAACTGAAGAAACAGAAGATATGGAAGATGAAAACGAAATTGTGGAAGAT 300
MSP3.7C   D2          GATACTGACGAAACTGAAGAAACAGAAGATATGGAAGATGAAAACGAAATTGTGGAAGAT 300
MSP3.7C   D5          GATACTGACGAAACTGAAGAAACAGAAGATATGGAAGATGAAAACGAAATTGTGGAAGAT 300
```

Fig 9R

```
MSP3.7C   addtional   GATACTGACGAAACTGAAGAAACAGAAGATATGGAAGATGAAAACGAAATTGTGGAAGAT 300
                      ************************************************************

MSP3.7C   3D7         CAATTACAAGAAAATGAAGATGATGAGGATAATGTAAATTTAGAAGATATTAATAAAAAT 360
MSP3.7C   B1          CAATTACAAGAAAATGAAGATGATGAGGATAATGTAAATTTAGAAGATATTAATAAAAAT 360
MSP3.7C   B2          CAATTACAAGAAAATGAAGATGATGAGGATAATGTAAATTTAGAAGATATTAATAAAAAT 360
MSP3.7C   B3          CAATTACAAGAAAATGAAGATGATGAGGATAATGTAAATTTAGAAGATATTAATAAAAAT 360
MSP3.7C   B4          CAATTACAAGAAAATGAAGATGATGAGGATAATGTAAATTTAGAAGATATTAATAAAAAT 360
MSP3.7C   B5          CAATTACAAGAAAATGAAGATGATGAGGATAATGTAAATTTAGAAGATATTAATAAAAAT 360
MSP3.7C   D1          CAATTACAAGAAAATGAAGATGATGAGGATAATGTAAATTTAGAAGATATTAATAAAAAT 360
MSP3.7C   D2          CAATTACAAGAAAATGAAGATGATGAGGATAATGTAAATTTAGAAGATATTAATAAAAAT 360
MSP3.7C   D5          CAATTACAAGAAAATGAAGATGATGAGGATAATGTAAATTTAGAAGATATTAATAAAAAT 360
MSP3.7C   addtional   CAATTACAAGAAAATGAAGATGATGAGGATAATGTAAATTTAGAAGATATTAATAAAAAT 360
                      ************************************************************

MSP3.7C   3D7         ACTAGAAATGATATATTTGAAGAACAAATAAAATTAGATTCTACGCAAGATGACAAAGCT 420
MSP3.7C   B1          ACTAGAAATGATATATTTGAAGAACAAATAAAATTAGATTCTACGCAAGATGACAAAGCT 420
MSP3.7C   B2          ACTAGAAATGATATATTTGAAGAACAAATAAAATTAGATTCTACGCAAGATGACAAAGCT 420
MSP3.7C   B3          ACTAGAAATGATATATTTGAAGAACAAATAAAATTAGATTCTACGCAAGATGACAAAGCT 420
MSP3.7C   B4          ACTAGAAATGATATATTTGAAGAACAAATAAAATTAGATTCTACGCAAGATGACAAAGCT 420
MSP3.7C   B5          ACTAGAAATGATATATTTGAAGAACAAATAAAATTAGATTCTACGCAAGATGACAAAGCT 420
MSP3.7C   D1          ACTAGAAATGATATATTTGAAGAACAAATAAAATTAGATTCTACGCAAGATGACAAAGCT 420
MSP3.7C   D2          ACTAGAAATGATATATTTGAAGAACAAATAAAATTAGATTCTACGCAAGATGACAAAGCT 420
MSP3.7C   D5          ACTAGAAATGATATATTTGAAGAACAAATAAAATTAGATTCTACGCAAGATGACAAAGCT 420
MSP3.7C   addtional   ACTAGAAATGATATATTTGAAGAACAAATAAAATTAGATTCTACGCAAGATGACAAAGCT 420
                      ************************************************************

MSP3.7C   3D7         CAAAAATTAATTTCTAATGAATATAAAAAAACTGAAGAAAAAAAATCATTAGAAGATCAT 480
MSP3.7C   B1          CAAAAATTAATTTCTAATGAATATAAAAAAACTGAAGAAAAAAAATCATTAGAAGATCAT 480
MSP3.7C   B2          CAAAAATTAATTTCTAATGAATATAAAAAAACTGAAGAAAAAAAATCATTAGAAGATCAT 480
MSP3.7C   B3          CAAAAATTAATTTCTAATGAATATAAAAAAACTGAAGAAAAAAAATCATTAGAAGATCAT 480
MSP3.7C   B4          CAAAAATTAATTTCTAATGAATATAAAAAAACTGAAGAAAAAAAATCATTAGAAGATCAT 480
MSP3.7C   B5          CAAAAATTAATTTCTAATGAATATAAAAAAACTGAAGAAAAAAAATCATTAGAAGATCAT 480
MSP3.7C   D1          CAAAAATTAATTTCTAATGAATATAAAAAAACTGAAGAAAAAAAATCATTAGAAGATCAT 480
MSP3.7C   D2          CAAAAATTAATTTCTAATGAATATAAAAAAACTGAAGAAAAAAAATCATTAGAAGATCAT 480
MSP3.7C   D5          CAAAAATTAATTTCTAATGAATATAAAAAAACTGAAGAAAAAAAATCATTAGAAGATCAT 480
```

Fig 9S

```
MSP3.7C    addtional    CAAAAATTAATTTCTAATGAATATAAAAAAACTGAAGAAAAAAAATCATTAGAAGATCAT 480
                        ************************************************************

MSP3.7C    3D7          GTAAATCTATTATTTAATTTTTTACAAACAAATAACCAACTAGATCCTTCACTAAAAGAT 540
MSP3.7C    B1           GTAAATCTATTATTTAATTTTTTACAAACAAATAACCAACTAGATCCTTCACTAAAAGAT 540
MSP3.7C    B2           GTAAATCTATTATTTAATTTTTTACAAACAAATAACCAACTAGATCCTTCACTAAAAGAT 540
MSP3.7C    B3           GTAAATCTATTATTTAATTTTTTACAAACAAATAACCAACTAGATCCTTCACTAAAAGAT 540
MSP3.7C    B4           GTAAATCTATTATTTAATTTTTTACAAACAAATAACCAACTAGATCCTTCACTAAAAGAT 540
MSP3.7C    B5           GTAAATCTATTATTTAATTTTTTACAAACAAATAACCAACTAGATCCTTCACTAAAAGAT 540
MSP3.7C    D1           GTAAATCTATTATTTAATTTTTTACAAACAAATAACCAACTAGATCCTTCACTAAAAGAT 540
MSP3.7C    D2           GTAAATCTATTATTTAATTTTTTACAAACAAATAACCAACTAGATCCTTCACTAAAAGAT 540
MSP3.7C    D5           GTAAATCTATTATTTAATTTTTTACAAACAAATAACCAACTAGATCCTTCACTAAAAGAT 540
MSP3.7C    addtional    GTAAATCTATTATTTAATTTTTTACAAACAAATAACCAACTAGATCCTTCACTAAAAGAT 540
                        ************************************************************


MSP3.7C    3D7          TTAGAAAATGAGTTAACTTTTTTTTTAAATAACTATTGA 579
MSP3.7C    B1           TTAGAAAATGAGTTAACTTTTTTTTTAAATAACTATTGA 579
MSP3.7C    B2           TTAGAAAATGAGTTAACTTTTTTTTTAAATAACTATTGA 579
MSP3.7C    B3           TTAGAAAATGAGTTAACTTTTTTTTTAAATAACTATTGA 579
MSP3.7C    B4           TTAGAAAATGAGTTAACTTTTTTTTTAAATAACTATTGA 579
MSP3.7C    B5           TTAGAAAATGAGTTAACTTTTTTTTTAAATAATTATTGA 579
MSP3.7C    D1           TTAGAAAATGAGTTAACTTTTTTTTTAAATAACTATTGA 579
MSP3.7C    D2           TTAGAAAATGAGTTAACTTTTTTTTTAAATAACTATTGA 579
MSP3.7C    D5           TTAGAAAATGAGTTAACTTTTTTTTTAAATAACTATTGA 579
MSP3.7C    addtional    TTAGAAAATGAGTTAACTTTTTTTTTAAATAACTATTGA 579
                        ********************************* ******
```

**ClustalW alignment ORF7C Nucleotide sequences** (Www.ebl.ac.uk/clustalw/) **MSP3-8**

CLUSTAL W (1.82) multiple sequence alignment excluding MSP3.8C B2

Fig 9T

```
MSP3.8C    3D7    CATGAAAGTAATGTTGGTAGCATCCAAGAAGTAAACCAAGGTAGCGTGAGCGAAGAATCA 60
MSP3.8C    B1     CATGAAAGTAATGTTGGTAGCATCCAAGAAGTAAACCAAGGTAGCGTGAGCGAAGAATCA 60
MSP3.8C    B4     CATGAAAGTAATGTTGGTAGCATCCAAGAAGTAAACCAAGGTAGCGTGAGCGAAGAATCA 60
MSP3.8C    B5     CATGAAAGTAATGTTGGTAGCATCCAAGAAGTAAACCAAGGTAGCGTGAGCGAAGAATCA 60
MSP3.8C    D1     CATGAAAGTAATGTTGGTAGCATCCAAGAAGTAAACCAAGGTAGCGTGAGCGAAGAATCA 60
MSP3.8C    D2     CATGAAAGTAATGTTGGTAGCATCCAAGAAGTAAACCAAGGTAGCGTGAGCGAAGAATCA 60
MSP3.8C    D4     CATGAAAGTAATGTTGGTAGCATCCAAGAAGTAAACCAAGGTAGCGTGAGCGAAGAATCA 60
                  ************************************************************

MSP3.8C    3D7    CATTCTAAAACTATAGATCCTTCTAAGATTGACGACCGTTTGGAATTAAGTAGTGGGTCA 120
MSP3.8C    B1     CATTCTAAAACTATAGATCCTTCTAAGATTGACGACCGTTTGGAATTAAGTAGTGGGTCA 120
MSP3.8C    B4     CATTCTAAAACTATAGATCCTTCTAAGATTGACGACCGTTTGGAATTAAGTAGTGGGTCA 120
MSP3.8C    B5     CATTCTAAAACTATAGATCCTTCTAAGATTGACGACCGTTTGGAATTAAGTAGTGGGTCA 120
MSP3.8C    D1     CATTCTAAAACTATAGATCCTTCTAAGATTGACGACCGTTTGGAATTAAGTAGTGGGTCA 120
MSP3.8C    D2     CATTCTAAAACTATAGATCCTTCTAAGATTGACGACCGTTTGGAATTAAGTAGTGGGTCA 120
MSP3.8C    D4     CATTCTAAAACTATAGATCCTTCTAAGATTGACGACCGTTTGGAATTAAGTAGTGGGTCA 120
                  ************************************************************

MSP3.8C    3D7    TCATCTCTTGAACAACACTCTAAGGAAGATGTAAAAAAGGGATGTGCTTTAGAATTGGTA 180
MSP3.8C    B1     TCATCTCTTGAACAACACTCTAAGGAAGATGTAAAAAAGGGAAGTGCTTTAGAATTGGTA 180
MSP3.8C    B4     TCATCTCTTGAACAACACTCTAAGGAAGATGTAAAAAAGGGAAGTGCTTTAGAATTGGTA 180
MSP3.8C    B5     TCATCTCTTGAACAACACTCTAAGGAAGATGTAAAAAAGGGAAGTGCTTTAGAATTGGTA 180
MSP3.8C    D1     TCATCTCTTGAACAACACTCTAAGGAAGATGTAAAAAAGGGAAGTGCTTTAGAATTGGTA 180
MSP3.8C    D2     TCATCTCTTGAACAACACTCTAAGGAAGATGTAAAAAAGGGAAGTGCTTTAGAATTGGTA 180
MSP3.8C    D4     TCATCTCTTGAACAACACTCTAAGGAAGATGTAAAAAAGGGAAGTGCTTTAGAATTGGTA 180
                  ****************************************** *****************

MSP3.8C    3D7    CCTTTATCTTTATCGGATATTGAACAGATAGCTAATGAAAGCGAAGATGTACTGGAAGAG 240
MSP3.8C    B1     CCTTTATCTTTATCGGATATTGAACAGATAGCTAATGAAAGCGAAGATGTACTGGAAGAG 240
MSP3.8C    B4     CCTTTATCTTTATCGGATATTGAACAGATAGCTAATGAAAGCGAAGATGTACTGGAAGAG 240
MSP3.8C    B5     CCTTTATCTTTATCGGATATTGAACAGATAGCTAATGAAAGCGAAGATGTACTGGAAGAG 240
MSP3.8C    D1     CCTTTATCTTTATCGGATATTGAACAGATAGCTAATGAAAGCGAAGATGTACTGGAAGAG 240
MSP3.8C    D2     CCTTTATCTTTATCGGATATTGAACAGATAGCTAATGAAAGCGAAGATGTACTGGAAGAG 240
MSP3.8C    D4     CCTTTATCTTTATCGGATATTGAACAGATAGCTAATGAAAGCGAAGATGTACTGGAAGAG 240
                  ************************************************************

MSP3.8C    3D7    ATAGAAGAAGAAATTAATACAGATGGGGAAATAGAATATATAACAGAAGAAGAAATAAAA 300
```

Fig 9U

```
MSP3.8C   B1    ATAGAAGAAGAAATTAATACAGATGGGGAAATAGAATATATAACAGAAGAAGAAATAAAA 300
MSP3.8C   B4    ATAGAAGAAGAAATTAATACAGATGGGGAAATAGAATATATAACAGAAGAAGAAATAAAA 300
MSP3.8C   B5    ATAGAAGAAGAAATTAATACAGATGGGGAAATAGAATATATAACAGAAGAAGAAATAAAA 300
MSP3.8C   D1    ATAGAAGAAGAAATTAATACAGATGGGGAAATAGAATATATAACAGAAGAAGAAATAAAA 300
MSP3.8C   D2    ATAGAAGAAGAAATTAATACAGATGGGGAAATAGAATATATAACAGAAGAAGAAATAAAA 300
MSP3.8C   D4    ATAGAAGAAGAAATTAATACAGATGGGGAAATAGAATATATAACAGAAGAAGAAATAAAA 300
                ************************************************************

MSP3.8C   3D7   GAAGATATAGAAGAAGAAACAGAAGAAGATATAGAAGAAGAAACAGAAGAAGAAACAGAA 360
MSP3.8C   B1    GAAGATATAGAAGAAGAAATAAAAGAAGATATAGAAGAAGAAACAGAAGAAGATATAGAA 360
MSP3.8C   B4    GAAGATATAGAAGAAGAAACAGAAGAAGATATAGAAGAAGAAACAGAAGAAGAAACAGAA 360
MSP3.8C   B5    GAAGATATAGAAGAAGAAATAAAAGAAGATATAGAAGAAGAAACAGAAGAAGATATAGAA 360
MSP3.8C   D1    GAAGATATAGAAGAAGAAACAGAAGAAGATATAGAAGAAGAAACAGAAGAAGAAACAGAA 360
MSP3.8C   D2    GAAGATATAGAAGAAGAAACAGAAGAAGATATAGAAGAAGAAACAGAAGAAGAAACAGAA 360
MSP3.8C   D4    GAAGATATAGAAGAAGAAACAGAAGAAGATATAGAAGAAGAAACAGAAGAAGAAACAGAA 360
                ****************** * ******************************** * ****

MSP3.8C   3D7   GAAGAAACAGAAGAAGAAGCAGA------------TGAAGAAACAGTAAAAGAAATAGAA 408
MSP3.8C   B1    GAAGAAACAGAAGAAGAAACAGAAGAAGAAGCAGATGAAGAAACAGTAAAAGAAATAGAA 420
MSP3.8C   B4    GAAGAAACAGAAGAAGAAGCAGA------------TGAAGAAACAGTAAAAGAAATAGAA 408
MSP3.8C   B5    GAAGAAACAGAAGAAGAAACAGAAGAAGAAGCAGATGAAGAAACAGTAAAAGAAATAGAA 420
MSP3.8C   D1    GAAGAAACAGAAGAAGAAACAGAAGAAGAAGCAGATGAAGAAACAGTAAAAGAAATAGAA 420
MSP3.8C   D2    GAAGAAACAGAAGAAGAAACAG------------------------TAAAAGAAATAGAA 396
MSP3.8C   D4    GAAGAAACAGAAGAAGAAGCAGA------------TGAAGAAACAGTAAAAGAAATAGAA 408
                ****************** ***                      **************

MSP3.8C   3D7   GACAAACCAGAACAAGAAATTAAAAATAAATCGCTAGAAGAAAAACAAATAGATAAAAAT 468
MSP3.8C   B1    GACAAACCAGAACAAGAAATTAAAAATAAATCGCTAGAAGAAAAACAAATAGATAAAAAT 480
MSP3.8C   B4    GACAAACCAGAACAAGAAATTAAAAATAAATCGCTAGAAGAAAAACAAATAGATAAAAAT 468
MSP3.8C   B5    GACAAACCAGAACAAGAAATTAAAAATAAATCGCTAGAAGAAAAGCAAATAGATAAAAAT 480
MSP3.8C   D1    GACAAACCAGAACAAGAAATTAAAAATAAATCGCTAGAAGAAAAACAAATAGATAAAAAT 480
MSP3.8C   D2    GACAAACCAGAACAAGAAATTAAAAATAAATCGCTAGAAGAAAAACAAATAGATAAAAAT 456
MSP3.8C   D4    GACAAACCAGAACAAGAAATTAAAAATAAATCGCTAGAAGAAAAACAAATAGATAAAAAT 468
                ******************************************** ***************

MSP3.8C   3D7   ACAGATACCAGTGAAAAGAAAGGATTTAATAATTCAGAAAAAGATGAAAAAGCTCGAAAT 528
```

Fig 9V

```
MSP3.8C    B1     ACAGATACCAGTGAAAAGAAAGGATTTAATAATTCAGAAAAAGATGAAAAAGCTCGAAAT 540
MSP3.8C    B4     ACAGATACCAGTGAAAAGAAAGGATTTAATAATTCAGAAAAAGATGAAAAAGCTCGAAAT 528
MSP3.8C    B5     ACAGATACCAGTGAAAAGAAAGGATTTAATAATTCAGAAAAAGATGAAAAAGCTCGAAAT 540
MSP3.8C    D1     ACAGATACCAGTGAAAAGAAAGGATTTAATAATTCAGAAAAAGATGAAAAAGCTCGAAAT 540
MSP3.8C    D2     ACAGATACCAGTGAAAAGAAAGGATTTAATAATTCAGAAAAAGATGAAAAAGCTCGAAAT 516
MSP3.8C    D4     ACAGATACCAGTGAAAAGAAAGGATTTAATAATTCAGAAAAAGATGAAAAAGCTCGAAAT 528
                  ************************************************************

MSP3.8C    3D7    TTAATTTCTAAAAATTATAAAAATTATAATGAACTAGATAAAAACGTTCATACTTTAGTA 588
MSP3.8C    B1     TTAATTTCTAAAAATTATAAAAATTATAATGAACTAGATAAAAACGTTCATACTTTAGTA 600
MSP3.8C    B4     TTAACTTCTAAAAATTATAAAAATTATAATGAACTAGATAAAAACGTTCATACTTTAGTA 588
MSP3.8C    B5     TTAATTTCTAAAAATTATAAAAATTATAATGAACTAGATAAAAACGTTCATACTTTAGTA 600
MSP3.8C    D1     TTAATTTCTAAAAATTATAAAAATTATAATGAACTAGATAAAAACGTTCATACTTTAGTA 600
MSP3.8C    D2     TTAATTTCTAAAAATTATAAAAATTATAATGAACTAGATAAAAACGTTCATACTTTAGTA 576
MSP3.8C    D4     TTAACTTCTAAAAATTATAAAAATTATAATGAACTAGATAAAAACGTTCATACTTTAGTA 588
                  **** *******************************************************

MSP3.8C    3D7    AATTCAATTATTAGTTTATTAGAAGAAGGTAATGGAAGTGATTCTACCTTGAATAGTTTA 648
MSP3.8C    B1     AATTCAATTATTAGTTTATTAGAAGAAGGTAATGGAAGTGATTCTACCTTGAATAGTTTA 660
MSP3.8C    B4     AATTCAATTATTAGTTTATTAGAAGAAGGTAATGGAAGTGATTCTACCTTGAATAGTTTA 648
MSP3.8C    B5     AATTCAATTATTAGTTTATTAGAAGAAGGTAATGGAAGTGATTCTACCTTGAATAGTTTA 660
MSP3.8C    D1     AATTCAATTATTAGTTTATTAGAAGAAGGTAATGGAAGTGATTCTACCTTGAATAGTTTA 660
MSP3.8C    D2     AATTCAATTATTAGTTTATTAGAAGAAGGTAATGGAAGTGATTCTACCTTGAATAGTTTA 636
MSP3.8C    D4     AATTCAATTATTAGTTTATTAGAAGAAGGTAATGGAAGTGATTCTACCTTGAATAGTTTA 648
                  ************************************************************

MSP3.8C    3D7    TCAAAAGATATTACAAATTTATTTAAAAATTAA 681
MSP3.8C    B1     TCAAAAGATATTACAAATTTATTTAAAAATTAA 693
MSP3.8C    B4     TCAAAAGATATTACAAATTTATTTAAAAATTAA 681
MSP3.8C    B5     TCAAAAGATATTACAAATTTATTTAAAAATTAA 693
MSP3.8C    D1     TCAAAAGATATTACAAATTTATTTAAAAATTAA 693
MSP3.8C    D2     TCAAAAGATATTACAAATTTATTTAAAAATTAA 669
MSP3.8C    D4     TCAAAAGATATTACAAATTTATTTAAAAATTAA 681
                  *********************************
```

Fig 10 A

**CLUSTAL W (1.82) Multiple ClustalW alignment MSP3-1**

**AA Sequence Alignment - Viawww.ebi.ac.uk/clustalw/**

```
CLUSTAL W (1.82) multiple sequence alignment

MSP3.1C 3D7     YEKAKNAYQKANQAVLKAKEASSYDYILGWEFGGGVPEHKKEENMLSHLYVSSKDKENIS 60
MSP3.1C B2      YEKAKNAYQKANQAVLKAKEASSYDYILGWEFGGGVPEHKKEENMLSHLYVSSKDKENIS 60
MSP3.1C B3      YEKAKNAYQKANQAVLKAKEASSYDYILGWEFGGGVPEHKKEENMLSHLYVSSKDKENIS 60
MSP3.1C B4      YEKAKNAYQKANQAVLKAKEASSYDYILGWEFGGGVPEHKKEENMLSHLYVSSKDKENIS 60
MSP3.1C B5      YEKAKNAYQKANQAVLKAKEASSYDYILGWEFGGGVPEHKKEENMLSHLYVSSKDKENIS 60
MSP3.1C D2      YEKAKNAYQKANQAVLKAKEASSYDYILGWEFGGGVPEHKKEENMLSHLYVSSKDKENIS 60
MSP3.1C D3      YEKAKNAYQKANQAVLKAKEASSYDYILGWEFGGGVPEHKKEENMLSHLYVSSKDKENIS 60
MSP3.1C D4      YEKAKNAYQKANQAVLKAKEASSYDYILGWEFGGGVPEHKKEENMLSHLYVSSKDKENIS 60
MSP3.1C D5      YEKAKNAYQKANQAVLKAKEASSYDYILGWEFGGGVPEHKKEENMLSHLYVSSKDKENIS 60
                ************************************************************

MSP3.1C 3D7     KENDDVLDEKEEEAEETEEEELEEKNEEETESEISEDEEEEE-EEEEKEEENDKKKEQEK 119
MSP3.1C B2      KENDDVLDEKEEEAEETEEEELEEKNEEETESEISEDEEEEE---EEKEEE----KEQAK 113
MSP3.1C B3      KENDDVLDEKEEEAEETEEEELEEKNEEETESEISEDEEEEE---EEKEEE----KEQAK 113
MSP3.1C B4      KENDDVLDEKEEEAEETEEEELEEKNEEETESEISEDEEEEE---EEKEEE----KEQAK 113
MSP3.1C B5      KENDDVLDEKEEEAEETEEEELEEKNEEETESEISEDEEEEE---EEKEEE----KEQAK 113
MSP3.1C D2      KENDDVLDEKEEEAEETEEEELEEKNEEETESEISEDEEEEE-EEE-KEEENDKKKEQEK 118
MSP3.1C D3      KENDDVLDEKEEEAEETEEEELEEKNEEETESEISEDEEEEE-EEEEKEEENDKKKEQEK 119
MSP3.1C D4      KENDDVLDEKEEEAEETEEEELEEKNEEETESEISEDEEEEEEEEEEREEENDKKKEQEK 120
MSP3.1C D5      KENDDVLDEKEEEAEETEEEELEEKNEEETESEISEDEEEEEEEEEEKEEENDKKKEQEK 120
                ******************************************   * :***    *** *

MSP3.1C 3D7     EQSNENNDQKKDMEAQNLISKNQNNNEKNVKEAAESIMKTLAGLIKGNNQIDSTLKDLVE 179
MSP3.1C B2      EQSNENNDQKKDMEAQNLISKNQNNNEKNVKEAAESIMKTLAGLIKGNNQIDSTLKDLVE 173
MSP3.1C B3      EQSNENNDQKKDMEAQNLISKNQNNNEKNVKEAAESIMKTLAGLIKGNNQIDSTLKDLVE 173
MSP3.1C B4      EQSNENNDQKKDMEAQNLISKNQNNNEKNVKEAAESIMKTLAGLIKGNNQIDSTLKDLVE 173
MSP3.1C B5      EQSNENNDQKKDMEAQNLISKNQNNNEKNVKEAAESIMKTLAGLIMGNNQIDSTLKDLVE 173
MSP3.1C D2      EQSNENNDQKKDMEAQNLISKNQNNNEKNVKEAAESIMKTLAGLIKGNNQIDSTLKDLVE 178
MSP3.1C D3      EQSNENNDQKKDMEAQNLISKNQNNNEKNVKEAAESIMKTLAGLIKGNNQIDSTLKDLVE 179
MSP3.1C D4      EQSSENNDQKKDMEAQNLISKNQNNNEKNVKEAAESIMKTLAGLIKGNNQIDSTLKDLVE 180
MSP3.1C D5      EQSNENNDQKKDMEAQNLISKNQNNNEKNVKEAAESIMKTLAGLIKGNNQIDSTLKDLVE 180
                ***.***************************************** **************

MSP3.1C 3D7     ELSKYFKNH 188
MSP3.1C B2      ELSKYFKNH 182
MSP3.1C B3      ELSKYFKNH 182
MSP3.1C B4      ELSKYFKNH 182
MSP3.1C B5      ELSQYFKNH 182
MSP3.1C D2      ELSKYFKNH 187
MSP3.1C D3      ELSKYFKNH 188
MSP3.1C D4      ELSKYFKNH 189
MSP3.1C D5      ELSKYFKNH 189
```

Fig 10 B

```
***:*****
```

**ClustalW alignment AA sequences MSP6FL (Full Length)** (www.ebl.ac.uk/clustalw/) MSP3-2

```
CLUSTAL W (1.82) multiple sequence alignment

MSP3.2FL  3D7     MNKIYNITFLFILLNLYINENNFIRNELINEKNHNLRNGSMYNNDKILSKNEVDTNIESN 60
MSP3.2FL  B1      MNKIYNITFLFILLNLYINENNFIRNELINEKNHNLRNGSMYNNDKILSKNEVDTNIESN 60
MSP3.2FL  B3      MNKIYNITFLFILLNLYINENNFIRNELINEKNHNLRNGSMYNNDKILSKNEVDTNIESN 60
MSP3.2FL  B4      MNKIYNITFLFILLNLYINENNFIRNELINEKNHNLRNGSMYNNDKILSKNEVDTNIESN 60
MSP3.2FL  B5      MNKIYNITFLFILLNLYINENNFIRNELINEKNHNLRNGSMYNNDKILSKNEVDTNIESN 60
MSP3.2FL  D1      MNKIYNITFLFILLNLYINENNFIRNELINEKNHNLRNGSMYNNDKILSKNEVDTNIESN 60
MSP3.2FL  D2      MNKIYNITFLFILLNLYINENNFIRNELINEKNHNLRNGSMYNNDKILSKNEVDTNIESN 60
MSP3.2FL  D3      MNKNYNITFLFILLNLYINENNFIRNELINEKNHNLRNGSMYNNDKILSKNEVDTNIESN 60
MSP3.2FL  D4      MNKIYNITLLFILLNLYINENNFIRNELINEKNHNLRNGSMYNNDKILSKNEVDTNIESN 60
MSP3.2FL  D5      MNKIYNITFLFILLNLYINENNFIRNELINEKNHNLRNGSMYNNDKILSKNEVDTNIESN 60
                  *** ****:*******************************************************

MSP3.2FL  3D7     ENSIHESGHKIDGEEVLKAN-----VDDITYKKKNVDDSEIPFSGYDIQATYQFPSTS-- 113
MSP3.2FL  B1      ENSIHESGHKIDGEEVLKAN-----VDDITYKKKNVDDSEIPFSGYDIQATYQFPSTS-- 113
MSP3.2FL  B3      ENSIHESGHKIDGEEVLKAN-----VDDITYKKKNVDDSEIPFSGYDIQATYQFPSTS-- 113
MSP3.2FL  B4      ENSIHESGHKIDGEEVLKAN-----VDDITYKKKNVDDSEIPFSGYDIQATYQFPSTS-- 113
MSP3.2FL  B5      ENSIHESGHKIDGEEVLKAN-----VDDITYKKKNVDDSEIPFSGYDIQATYQFPSTS-- 113
MSP3.2FL  D1      ENSIHESGHKIDGEEVLKAN-----VDDITYKKKNVDDSEIPFSGYDIQATYQFPSTS-- 113
MSP3.2FL  D2      ENSIHESGHKIDGEEVLKAN-----VDDITYKKKNVDDSEIPFSGYDIQATYQFPSTS-- 113
MSP3.2FL  D3      ENSIHESGHKIDGEEVLKAN-----VDDITYKKKNVDDSEIPFSGYDIQATYQFPSTS-- 113
MSP3.2FL  D4      ENSIHESGHKIDGEEVLKANQEQANVEDITYKKKNVDDSEIPFSGSDIQATYQFPPAPGR 120
MSP3.2FL  D5      ENSIHESGHKIDGEEVLKAN-----VDDITYKKKNVDDSEIPFSGYDIQATYQFPSTS-- 113
                  ********************     *:****************** *********.:.

MSP3.2FL  3D7     ------GGNNVIPLPIKQSGENQYTVTSISGIQKGANGLTGATENITQVVQANSETNKNP 167
MSP3.2FL  B1      ------GGNNVIPLPIKQSGENQYTVTSISGIQKGANGLTGATENITQVVQANSETNKNP 167
MSP3.2FL  B3      ------GGNNVIPLPIKQSGENQYTVTSISGIQKGANGLTGATENITQVVQANSETNKNP 167
MSP3.2FL  B4      ------GGNNVIPLPIKQSGENQYTVTSISGIQKGANGLTGATENITQVVQANSETNKNP 167
MSP3.2FL  B5      ------GGNNVIPLPIKQSGENQYTVTSISGIQKGANGLTGATENITQVVQANSETNKNP 167
MSP3.2FL  D1      ------GGNNVIPLPIKQSGENQYTVTSISGIQKGANGLTGATENITQVVQANSETNKNP 167
MSP3.2FL  D2      ------GGNNVIPLPIKQSGENQYTVTSISGIQKGANGLTGATENITQVVQANSETNKNP 167
MSP3.2FL  D3      ------GGNNVIPLPIKQSGENQYTVTSISGIQKGANGLTGATENITQVVQANSETNKNP 167
MSP3.2FL  D4      IINPRTGGNTVIPPPRRLSGLEGVSYPHVS---------TSSNQSHPQRANICGISHLSG 171
```

Fig 10 C

```
MSP3.2FL  D5     ------GGNNVIPLPIKQSGENQYTVTSISGIQKGANGLTGATENITQVVQANSETNKNP 167
                       ***.*** * : ** :  : . :*         *.:.:. .* .: .. :: .

                                                                     C term
                                                                     region
MSP3.2FL  3D7    TSHSNSTTTSLNNNILGWEFGGGAPQNGAAEDKKTEYLLEQIKIPSWDRNNIPDENEQVI 227
MSP3.2FL  B1     TSHSNSTTTSLNNNILGWEFGGGAPQNGAAEDKKTEYLLEQIKIPSWDRNNIPDENEQVK 227
MSP3.2FL  B3     TSHSNSTTTSLNNNILGWEFGGGAPQNGAAEDKKTEYLLEQIKIPSWDKNNIPDENEQVK 227
MSP3.2FL  B4     TSHSNSTTTSLNNNILGWEFGGGAPQNGAAEDKKTEYLLEQIKIPSWDRNNIPDENEQVK 227
MSP3.2FL  B5     TSHSNSTTTSLNNNILGWEFGGGAPQNGAAEDKKTEYLLEQIKIPSWDRNNIPDENEQVK 227
MSP3.2FL  D1     TSHSNSTTTSLNNNILGWEFGGGAPQNGAAEDKKTEYLLEQIKIPSWDRNNIPDENEQVK 227
MSP3.2FL  D2     TSHSNSTTTSLNNNILGWEFGGGAPQNGAAEDKKTEYLLEQIKIPSWDRNNIPDENEQVK 227
MSP3.2FL  D3     TSHSNSTTTSLNNNILGWEFGGGAPQNGAAEDKKTEYLLEQIKIPSWDRNNIPDENEQVK 227
MSP3.2FL  D4     TRG-IHTYSSLDSNILGWEFGGGAPQNGAAEGKKTEYLLEQIKIPSWDRNNIPDENEQVK 230
MSP3.2FL  D5     TPGSKSTTTSLNNNILGWEFGGGAPQNGAAEDKKTEYLLEQIKIPSWDRNNIPDENEQVI 227
                 *     * :**:.******************.****************:**********

MSP3.2FL  3D7    EDPQEDNKDEDEDE--ETETENLETEDDNNEEIEENEEDDIDEESVEEKEEEEEKKEEEE 285
MSP3.2FL  B1     EDPQEDNKDEDEDE--ETETENLETEDDNNEEIEENEEDDIDEESVEEKEEEEEKKEEEE 285
MSP3.2FL  B3     EDPQEDNKDEDEDE--GTETENLETEDDNNEEIEENEEDDIDEESVEEKEEEEEKKEEEE 285
MSP3.2FL  B4     EDPQEDNKDEDEDE--ETETENLETEDDNNEEIEENEEDDIDEESVEEKEEEEEKKEEEE 285
MSP3.2FL  B5     EDPQEDNKDEDEDE--ETETENLETEDDNNEEIEENEEDDIDEESVEEKEEEEEKKEEEE 285
MSP3.2FL  D1     EDPQEDNKDEDEDE--ETETENLETEDDNNEEIEENEEDDIDEESVEEKEEEEEKKEEEE 285
MSP3.2FL  D2     EDPQEDNKDEDEDE--ETETENLETEDDNNEEIEENEEDDIDEESVEEKEEEEEKKEEEE 285
MSP3.2FL  D3     EDPQEDNKDEDEDE--ETETENLETEDDNNEEIEENEEDDIDEESVEEKEEEEEKREEEE 285
MSP3.2FL  D4     EEPQEDNKDEDEDEDEETETENLETEDDNNEEIEENEEDDIDEESVEEKEEEEEKKEEEE 290
MSP3.2FL  D5     EDPQEDNKDEDEDE--ETETENLETEDDNNEEIEENEEDDIDEESVEEKEEEEEKKEEEE 285
                 *:************  ****************************************:****

MSP3.2FL  3D7    KKEEKKEEKKPDNEITNEVKEEQKYSSPSDINAQNLISNKNKKNDETKKTAENIVKTLVG 345
MSP3.2FL  B1     KKEEKKEEKNPDNEITNEVKEEQKYSSPSDINAQNLISNKNKKNDETKKTAENIVKTLVG 345
MSP3.2FL  B3     KKEEKKEEKKPDNEITNEVKEEQKYSSPSDINAQNLISNKNKKNDETKKTAENIVKTLVG 345
MSP3.2FL  B4     KKEEKKEEKKPDNEITNEVKEEQKYSSPSDINAQNLISNKNKKNDETKKTAENIVKTLVG 345
MSP3.2FL  B5     KKEEKKEEKKPDNEITNEVKEEQKYSSPSDINAQNLISNKNKKNDETKKTAENIVKTLVG 345
MSP3.2FL  D1     KKEEKKEEKKPDNEITNEVKEEQKYSSPSDINAQNLISNKNKKNDETKKTAENIVKTLVG 345
MSP3.2FL  D2     KKEEKKEEKKPDNEITNEVKEEQKYSSPSDINAQNLISNKNKKNDETKKTAENIVKTLVG 345
MSP3.2FL  D3     KKEEKKEEKKPDNEITNEVKEEQKYSSPSDINAQNLISNKNKKNDETKKTAENIVKTLVG 345
MSP3.2FL  D4     KKEEKKEEKKPDNEITNEVKEEQKYSSPSDINAQNLISNKNKKNDETKKTAENIVKTLVG 350
MSP3.2FL  D5     KKEEKKEEKKPDNEITNEVKEEQKYSSPSDINAQNLISNKNKKNDETKKTAENIVKTLVG 345
                 *********:**************************************************
```

Fig 10 D

```
MSP3.2FL  3D7      LFNEKNEIDSTINNLVQEMIHLFSNN 371
MSP3.2FL  B1       LFNEKNEIDSTINNLVQEMIHLFSNN 371
MSP3.2FL  B3       LFNEKNEIDSTINNLVQEMIHLFSNN 371
MSP3.2FL  B4       LFNEKNEIDSTINNLVQEMIHLFSNN 371
MSP3.2FL  B5       LFNEKNEIDSTINNLVQEMIHLFSNN 371
MSP3.2FL  D1       LFNEKNEIDSTINNLVQEMIHLFSNN 371
MSP3.2FL  D2       LFNEKNEIDSTINNLVQEMIHLFSNN 371
MSP3.2FL  D3       LFNEKNEIDSTINNLVQEMIHLFSNN 371
MSP3.2FL  D4       LFNEKNGIDSTINNLVQEMIHLFSNN 376
MSP3.2FL  D5       LFNEKNEIDSTINNLVQEMIHLFSNN 371
                   ****** *******************
```

**<u>ClustalW alignment AA sequences ORF2C</u>** (<u>www.ebl.ac.uk/clustalw/</u>) **<u>MSP3-3</u>**

CLUSTAL W (1.82) multiple sequence alignment

```
MSP3.3C  3D7       YEKKNENKNVSNVDSKTKSNEKGRPPTYSPILDDGIEFSGGLYFNEKKSTEENKQKNVLE 60
MSP3.3C  B1        YEKKNKNKNVSNVDSKTKSNEKGRPPTYSPILDDGIEFSGGLYFNEKKSTEENKQKNVLE 60
MSP3.3C  B2        YEKKNENKNVSNVDSKTKSNEKGRPPTYSPILDDGIEFSGGLYFNEKKSTEENKQKNVLE 60
MSP3.3C  B5        YEKKNENKNVSNVDSKTKSNEKGRPPTYSPILDDGIEFSGGLYFNEKKSTEENKQKNVLE 60
MSP3.3C  D1        YEKKNENKNVSNVDSKTKSNEKGRPPTYSPILDDGIEFSGGLYFNEKKSTEENKQKNVLE 60
MSP3.3C  D2        YEKKNENKNVSNVDSKTKSNEKGRPPTYSPILDDGIEFSGGLYFNEKKSTEENKQKNVLE 60
MSP3.3C  D4        YEKKNENKNVSNVDSKTKSNEKGRPPTYSPILDDGIEFSGGLYFNEKKSTEENKQKNVLE 60
MSP3.3C  D5        YEKKNENKNVSNVDSKTKSNEKGRPPTYSPILDDGIEFSGGLYFNEKKSTEENKQKNVLE 60
                   *****:******************************************************

MSP3.3C  3D7       SVNLTSWDKEDIVKENEDVKDEKDEDDEEEEEKYENEIIKQPEDILDEEEVLEEEILEEN 120
MSP3.3C  B1        SVNLTSWDKEDIVKENEDVKDEKDEDDEEEEEKYENEIIKQPEDILDEEEVLEEEILEEN 120
MSP3.3C  B2        SVNLTSWDKEDIVKENEDVKDEKDEDDEEEEEKYENEIIKQPEDILDEEEVLEEEILEEN 120
MSP3.3C  B5        SVNLTSWDKEDIVKENEDVKDEKDEDDEEEEEKYENEIIKQPEDILDEEEVLEEEILEEN 120
MSP3.3C  D1        SVNLTSWDKEDIVKENEDVKDEKDEDDEEEEEKYENEIIKQPEDILDEEEVLEEEILEEN 120
MSP3.3C  D2        SVNLTSWDKEDIVKENEDVKDEKDEDDEEEEEKYENEIIKQPEDILDEEEVLEEEILEEN 120
MSP3.3C  D4        SVNLTSWDKEDIVKENEDVKDEKDEDDEEEEEKYENEIIKQPEDILDEEEVLEEEILEEN 120
MSP3.3C  D5        SVNLTSWDKEDIVKENEDVKDEKDEDDEEEEEKYENEIIKQPEDILDEEEVLEEEILEEN 120
                   ************************************************************
```

Fig 10 E

```
MSP3.3C  3D7     KNDTVDTSDLEKKNIPDLSNDNNYYSLIYKNYKDNDKSEKTAQTLITALISLLNGKNELD 180
MSP3.3C  B1      KNDTVDTSDLEKKNIPDLSNDNNYYSLIYKNYKGNDKSEKTAQTLITALISLLNGKNELD 180
MSP3.3C  B2      KNDTVDTSDLEKKNIPDLSNDNNYYSLIYKNYKDNDKSEKTAQTLITALISLLNGKNELD 180
MSP3.3C  B5      KNDTVDTSDLEKKNIPDLSNDNNYYSLIYKNYKDNDKSEKTAQTLITALISLLNGKNELD 180
MSP3.3C  D1      KNDTVDTSDLEKKNIPDLSNDNNYYSLIYKNYKDNDKSEKTAQTLITALISLLNGKNELD 180
MSP3.3C  D2      KNDTVDTSDLEKKNIPDLSNDNNYYSLIYKNYKDNDKSEKTAQTLITALISLLNGKNELD 180
MSP3.3C  D4      KNDTVDTSDLEKKNIPDLSNDNNYYSLIYKNYKDNDKSEKTAQTLITALISLLNGKNELD 180
MSP3.3C  D5      KNDTVDTSDLEKKNIPDLSNDNNYYSLIYKNYKDNDKSEKTAQTLITALISLLNGKNELD 180
                 *********************************.**************************

MSP3.3C  3D7     ATIRRLKHRFMEFFTYN 197
MSP3.3C  B1      ATIRRLKHRFMEFFTYN 197
MSP3.3C  B2      ATIRRLKHRFMEFFTYN 197
MSP3.3C  B5      ATIRRLKHRFMEFFTYN 197
MSP3.3C  D1      ATIRRLKHRFMEFFTYN 197
MSP3.3C  D2      ATIRRLKHRFMEFFTYN 197
MSP3.3C  D4      ATIRRLKHRFMEFFTYN 197
MSP3.3C  D5      ATIRRLKHRFMEFFTYN 197
                 *****************
```

**ClustalW alignment AA sequences ORF3C (www.ebi.ac.uk/clustalw/) MSP3-4**

```
CLUSTAL W (1.82) multiple sequence alignment

MSP3.4C  3D7     DNVNSVTQRGNNNYNNNLERGLGSGALPGTNIITEEKYSLELIKLTSKDEEDIIKHNEDV 60
MSP3.4C  B1      DNVNSVTQRGNNNYNNNLERGLGSGALPGTNIITEEKYSLELIKLTSKDEEDIIKHNEDV 60
MSP3.4C  B4      DNVNSVTQRGNNNYNNNLERGLGSGALPGTNIITEEKYSLELIKLTSKDEEDIIKHNEDV 60
MSP3.4C  B5      DNVNSVTQRGNNNYNNNLERGLGSGALPGTNIITEEKYSLELIKLTSKDEEDIIKHNEDV 60
MSP3.4C  D1      DNVNSVTQRGNNNYNNNLERGLGSGALPGTNIITEEKYSLELIKLTSKDEEDIIKHNEDV 60
MSP3.4C  D2      DNVNSVTQRGNNNYNNNLERGLGSGALPGTNIITEEKYSLELIKLTSKDEEDIIKHNEDV 60
MSP3.4C  D4      DNVNSVTQRGNNNYNNNLERGLGSGALPGTNIITEEKYSLELIKLTSKDEEDIIKHNEDV 60
MSP3.4C  D5      DNVNSVTQRGNNNYNNNLERGLGSGALPGTNIITEEKYSLELIKLTSKDEEDIIKHNEDV 60
                 ************************************************************

MSP3.4C  3D7     REEIEEQQEDIEEDEEELENEGEETKEEDDEEKNETNDTEDTDDTEDTEDIEEENKEKEL 120
MSP3.4C  B1      REEIEEQQEDIEEDEEELENEGEETKEEDDEEKNETNDTEDTDDTEDTEDIEEENKEKEL 120
MSP3.4C  B4      REEIEEQQEDIEEDEEELENEGEETKEEDDEEKNETNDTEDTDDTEDTEDIEEENKEKEL 120
```

Fig 10 F

```
MSP3.4C  B5      REEIEEQQEDIEEDEEELENEGEETKEEDDEEKNETNDTEDTDDTEDTEDIEEENKEKEL 120
MSP3.4C  D1      REEIEEQQEDIEEDEEELENEGEETKEEDDEEKDETNDTEDTDDTEDTEDIEEENKEKEL 120
MSP3.4C  D2      REEIEEQQEDIEEDEEELENEGEETKEEDDEEKNETNDTEDTDDTEDTEDIEEENKEKEL 120
MSP3.4C  D4      REEIEEQQEDIEEDEEELENEGEETKEEDDEEKNETNDTEDTDDTEDTEDIEEENKEKEL 120
MSP3.4C  D5      REEIEEQQEDIEEDEEELENEGEETKEEDDEEKNETNDTEDTDDTEDTEDIEEENKEKEL 120
                 *********************************:**************************

MSP3.4C  3D7     SNQQQSEKKSISKVDEDSYRILSVSYKDNNEVKNVAESIVKKLFSLFNDNNNLETIFKGL 180
MSP3.4C  B1      SNQQQSEKKSISKVDEDSYRILSVSYKDNNEVKNVAESIVKKLFSLFNDNNNLETIFKGL 180
MSP3.4C  B4      SNQQQSEKKSISKVDEDSYRILSVSYKDNNEVKNVAESIVKKLFSLFNDNNNLETIFKGL 180
MSP3.4C  B5      SNQQQSEKKSISKVDEDSYRILSVSYKDNNEVKNVAESIVKKLFSLFNDNNNLETIFKGL 180
MSP3.4C  D1      SNQQQSEKKSISKVDEDSYRILSVSYKDNNEVKNVAESIVKKLFSLFNDNNNLETIFKGL 180
MSP3.4C  D2      SNQQQSEKKSISKVDEDSYRILSVSYKDNNEVKNVAESIVKKLFSLFNDNNNLETIFKGL 180
MSP3.4C  D4      SNQQQSEKKSISKVDEDSYRILSVSYKDNNEVKNVAESIVKKLFSLFNDNNNLETIFKGL 180
MSP3.4C  D5      SNQQQSEKKSISKVDEDSYRILSVSYKDNNEVKNVAESIVKKLFSLFNDNNNLETIFKGL 180
                 ************************************************************

MSP3.4C  3D7     TEDMTDLFQK 190
MSP3.4C  B1      TEDMTDLFQK 190
MSP3.4C  B4      TEDMTDLFQK 190
MSP3.4C  B5      TEDMTDLFQK 190
MSP3.4C  D1      TEDMTDLFQK 190
MSP3.4C  D2      TEDMTDLFQK 190
MSP3.4C  D4      TEDMTDLFQK 190
MSP3.4C  D5      TEDMTDLFQK 190
                 **********
```

**<u>ClustalW alignment AA sequences ORF6C</u> (<u>www.ebi.ac.uk/clustalw/</u>) MSP3-7**

CLUSTAL W (1.82) multiple sequence alignment

```
MSP3.7C  3D7     PEGPRANNRNENNQNTDPYNHYFAWEIGGGAPTYKPENNKNDNILLEHVKITSWDKEDII 60
MSP3.7C  B1      PEGPRANNRNENNQNTDPYNHYFAWEIGGGAPTYKPENNKNDNILLEHVKITSWDKEDII 60
MSP3.7C  B2      PEGPRANNRNENNQNTDPYNHYFAWEIGGGAPTYKPENNKNDNILLEHVKITSWDKEDII 60
MSP3.7C  B3      PEGPRANNRNENNQNTDPYNHYFAWEIGGGAPTYKPENNKNDNILLEHVKITPWDKEDII 60
MSP3.7C  B4      PEGPRANNRNENNQNTDPYNHYFAWEIGGGAPTYKPENNKNDNILLEHVKITSWDKEDII 60
MSP3.7C  B5      PEGPRANNRNENNQNTDPYNHYFAWEIGGGAPTYKPENNKNDNILLEHVKITSWDKEDII 60
MSP3.7C  D1      PEGPRANNRNENNQNTDPYNHYFAWEIGGGAPTYKPENNKNDNILLEHVKITSWDKEDII 60
```

Fig 10 G

```
MSP3.7C   D2          PEGPRANNRNENNQNTDPYNHYFAWEIGGGAPTYKPENNKNDNILLEHVKITSWDKEDII 60
MSP3.7C   D5          PEGPRANNRNENNQNTDPYNHYFAWEIGGGAPTYKPENNKNDNILLEHVKITSWDKEDII 60
MSP3.7C   addtional   PEGPRANNRNENNQNTDPYNHYFAWEIGGGAPTYKPENNKNDNILLEHVKITSWDKEDII 60
                      ****************************************************.*******

MSP3.7C   3D7         KENEDTKREVQETEDTDETEDTDETEETEDMEDENEIVEDQLQENEDDEDNVNLEDINKN 120
MSP3.7C   B1          KENEDTKREVQETEDTDETEDTDETEETEDMEDENEIVEDQLQENEDDEDNVNLEDINKN 120
MSP3.7C   B2          KENEDTKREVQETEDTDETEDTDETEETEDMEDENEIVEDQLQENEDDEDNVNLEDINKN 120
MSP3.7C   B3          KENEDTKREVQETEDTDETEDTDETEETEDMEDENEIVEDQLQENEDDEDNVNLEDINKN 120
MSP3.7C   B4          KENEDTKREVQETEDTDETEDTDETEETEDMEDENEIVEDQLQENEDDEDNVNLEDINKN 120
MSP3.7C   B5          KENEDTKREVQETEDTDETEDTDETEETEDMEDENEIVEDQLQENEDDEDNVNLEDINKN 120
MSP3.7C   D1          KENEDTKREVQETEDTDETEDTDETEETEDMEDENEIVEDQLQENEDDEDNVNLEDINKN 120
MSP3.7C   D2          KENEDTKREVQETEDTDETEDTDETEETEDMEDENEIVEDQLQENEDDEDNVNLEDINKN 120
MSP3.7C   D5          KENEDTKREVQETEDTDETEDTDETEETEDMEDENEIVEDQLQENEDDEDNVNLEDINKN 120
MSP3.7C   addtional   KENEDTKREVQETEDTDETEDTDETEETEDMEDENEIVEDQLQENEDDEDNVNLEDINKN 120
                      ************************************************************

MSP3.7C   3D7         TRNDIFEEQIKLDSTQDDKAQKLISNEYKKTEEKKSLEDHVNLLFNFLQTNNQLDPSLKD 180
MSP3.7C   B1          TRNDIFEEQIKLDSTQDDKAQKLISNEYKKTEEKKSLEDHVNLLFNFLQTNNQLDPSLKD 180
MSP3.7C   B2          TRNDIFEEQIKLDSTQDDKAQKLISNEYKKTEEKKSLEDHVNLLFNFLQTNNQLDPSLKD 180
MSP3.7C   B3          TRNDIFEEQIKLDSTQDDKAQKLISNEYKKTEEKKSLEDHVNLLFNFLQTNNQLDPSLKD 180
MSP3.7C   B4          TRNDIFEEQIKLDSTQDDKAQKLISNEYKKTEEKKSLEDHVNLLFNFLQTNNQLDPSLKD 180
MSP3.7C   B5          TRNDIFEEQIKLDSTQDDKAQKLISNEYKKTEEKKSLEDHVNLLFNFLQTNNQLDPSLKD 180
MSP3.7C   D1          TRNDIFEEQIKLDSTQDDKAQKLISNEYKKTEEKKSLEDHVNLLFNFLQTNNQLDPSLKD 180
MSP3.7C   D2          TRNDIFEEQIKLDSTQDDKAQKLISNEYKKTEEKKSLEDHVNLLFNFLQTNNQLDPSLKD 180
MSP3.7C   D5          TRNDIFEEQIKLDSTQDDKAQKLISNEYKKTEEKKSLEDHVNLLFNFLQTNNQLDPSLKD 180
MSP3.7C   addtional   TRNDIFEEQIKLDSTQDDKAQKLISNEYKKTEEKKSLEDHVNLLFNFLQTNNQLDPSLKD 180
                      ************************************************************

MSP3.7C   3D7         LENELTFFLNNY 192
MSP3.7C   B1          LENELTFFLNNY 192
MSP3.7C   B2          LENELTFFLNNY 192
MSP3.7C   B3          LENELTFFLNNY 192
MSP3.7C   B4          LENELTFFLNNY 192
MSP3.7C   B5          LENELTFFLNNY 192
MSP3.7C   D1          LENELTFFLNNY 192
MSP3.7C   D2          LENELTFFLNNY 192
MSP3.7C   D5          LENELTFFLNNY 192
```

Fig 10 H

```
MSP3.7C   addtional      LENELTFFLNNY 192
                         ************
```

Fig 10 I

**ClustalW alignment AA sequences ORF7C (www.ebi.ac.uk/clustalw/) MSP3-8**

```
CLUSTAL W (1.82) multiple sequence alignment excluding MSP3.8C     B2

MSP3.8C    3D7     HESNVGSIQEVNQGSVSEESHSKTIDPSKIDDRLELSSGSSSLEQHSKEDVKKGCALELV 60
MSP3.8C    B1      HESNVGSIQEVNQGSVSEESHSKTIDPSKIDDRLELSSGSSSLEQHSKEDVKKGSALELV 60
MSP3.8C    B4      HESNVGSIQEVNQGSVSEESHSKTIDPSKIDDRLELSSGSSSLEQHSKEDVKKGSALELV 60
MSP3.8C    B5      HESNVGSIQEVNQGSVSEESHSKTIDPSKIDDRLELSSGSSSLEQHSKEDVKKGSALELV 60
MSP3.8C    D1      HESNVGSIQEVNQGSVSEESHSKTIDPSKIDDRLELSSGSSSLEQHSKEDVKKGSALELV 60
MSP3.8C    D2      HESNVGSIQEVNQGSVSEESHSKTIDPSKIDDRLELSSGSSSLEQHSKEDVKKGSALELV 60
MSP3.8C    D4      HESNVGSIQEVNQGSVSEESHSKTIDPSKIDDRLELSSGSSSLEQHSKEDVKKGSALELV 60
                   ******************************************************.*****

MSP3.8C    3D7     PLSLSDIEQIANESEDVLEEIEEEINTDGEIEYITEEEIKEDIEEETEEDIEEETEEETE 120
MSP3.8C    B1      PLSLSDIEQIANESEDVLEEIEEEINTDGEIEYITEEEIKEDIEEEIKEDIEEETEEDIE 120
MSP3.8C    B4      PLSLSDIEQIANESEDVLEEIEEEINTDGEIEYITEEEIKEDIEEETEEDIEEETEEETE 120
MSP3.8C    B5      PLSLSDIEQIANESEDVLEEIEEEINTDGEIEYITEEEIKEDIEEEIKEDIEEETEEDIE 120
MSP3.8C    D1      PLSLSDIEQIANESEDVLEEIEEEINTDGEIEYITEEEIKEDIEEETEEDIEEETEEETE 120
MSP3.8C    D2      PLSLSDIEQIANESEDVLEEIEEEINTDGEIEYITEEEIKEDIEEETEEDIEEETEEETE 120
MSP3.8C    D4      PLSLSDIEQIANESEDVLEEIEEEINTDGEIEYITEEEIKEDIEEETEEDIEEETEEETE 120
                   ********************************************* :*********: *

MSP3.8C    3D7     EETEEEAD----EETVKEIEDKPEQEIKNKSLEEKQIDKNTDTSEKKGFNNSEKDEKARN 176
MSP3.8C    B1      EETEEETEEEADEETVKEIEDKPEQEIKNKSLEEKQIDKNTDTSEKKGFNNSEKDEKARN 180
MSP3.8C    B4      EETEEEAD----EETVKEIEDKPEQEIKNKSLEEKQIDKNTDTSEKKGFNNSEKDEKARN 176
MSP3.8C    B5      EETEEETEEEADEETVKEIEDKPEQEIKNKSLEEKQIDKNTDTSEKKGFNNSEKDEKARN 180
MSP3.8C    D1      EETEEETEEEADEETVKEIEDKPEQEIKNKSLEEKQIDKNTDTSEKKGFNNSEKDEKARN 180
MSP3.8C    D2      EETEEET--------VKEIEDKPEQEIKNKSLEEKQIDKNTDTSEKKGFNNSEKDEKARN 172
MSP3.8C    D4      EETEEEAD----EETVKEIEDKPEQEIKNKSLEEKQIDKNTDTSEKKGFNNSEKDEKARN 176
                   ******.        *********************************************

MSP3.8C    3D7     LISKNYKNYNELDKNVHTLVNSIISLLEEGNGSDSTLNSLSKDITNLFKN 226
MSP3.8C    B1      LISKNYKNYNELDKNVHTLVNSIISLLEEGNGSDSTLNSLSKDITNLFKN 230
MSP3.8C    B4      LTSKNYKNYNELDKNVHTLVNSIISLLEEGNGSDSTLNSLSKDITNLFKN 226
MSP3.8C    B5      LISKNYKNYNELDKNVHTLVNSIISLLEEGNGSDSTLNSLSKDITNLFKN 230
MSP3.8C    D1      LISKNYKNYNELDKNVHTLVNSIISLLEEGNGSDSTLNSLSKDITNLFKN 230
MSP3.8C    D2      LISKNYKNYNELDKNVHTLVNSIISLLEEGNGSDSTLNSLSKDITNLFKN 222
MSP3.8C    D4      LTSKNYKNYNELDKNVHTLVNSIISLLEEGNGSDSTLNSLSKDITNLFKN 226
```

Fig. 11 A

MSP3.1 Vs MSP3.2:

```
                   10        20        30        40        50        60        70        80        90
SEQ ID No 2  MKSFINITLSLFLLHLYIYINNVASKEIVKKYNLNLRNAILNNNSQIENEENVNTTITGNDFSGGEFLWPGYTEELKAKKASEDAEKAANDAENASK
SEQ ID No 4  MNKIYNITFLFILLNLYINENNFIRNELINEKNHNLRNGSMYNNDKILSKNEVDTNIESNENS---------IHESGHKIDGEEVLKANVDDITYKK
                   10        20        30        40        50        60                 70        80

        100       110       120          130       140       150       160       170       180
190
EAEEAAKEAVNLKESDKSYTKAKEACTAASKA---KKAVETALKAKDDAEKSSKADSISTKTKEYAEKAKNAYEKAKNAYQKANQAVLKAKEASSYD
KNVDDSEIPFSGYDIQATYQFPSTSGGNNVIPLPIKQSGENQYTVTSISGIQKGANGLTGATENITQVVQANSETNKNPTSHSN-----STTTSLNN
90       100       110       120       130       140       150       160       170
180

       200                 210       220       230            240       250       260       270
YILGWEFGGGVP-----EHKKEENMLSHLYVSSKDKENISKENDDVLDE------KEEEAEETEEEELEEKNEEETESEISEDEEEEEEE-EEKEEE
NILGWEFGGGAPQNGAAEDKKTEYLLEQIKIPSWDRNNIPDENEQVIEDPQEDNKDEDEDEETETENLETEDDNNEEIEENEEDDIDEESVEEKEEE
        190       200       210       220       230       240       250       260       270

    280                 290            300       310       320       330       340       350
```

Fig 11B

```
NDKKKEQEKEQ------------SNENNDQKK-----DMEAQNLISKNQNNNEKNVKEAAESIMKTLAGLIKGNNQIDSTLKDLVEELSKYFKNH
||•• •|•• |            |•• ||||•     •||•••••••|||||•| | • |••|•|••• ••|| |•|••••|||••|•|    • •|
EEKKEEEEKKEEKKEEKKPDNEITNEVKEEQKYSSPSDINAQNLISNKNKKND-ETKKTAENIVKTLVGLFNEKNEIDSTINNLVQEMIHLFSNN
  |         |         |         |         |         |         |         |         |         |
 280       290       300       310       320       330       340       350       360       370
```

Fig 11 C

MSP3.3 Vs MSP3.1:

```
SEQ ID No:6 MKKIVNIIF--YILYLYIYKRNLVQNENVNKSNLRKGLSTNNSENGIKSLKDEDEHINIIGDDFSA--FSYGGYPIYETTGSLGTGVESVKAIDGES
SEQ ID No:2 MKSFINITLSLFLLHLYIYINNVASKEIVKKYNLN--LRNAILNNNSQIENEENVNTTITGNDFSGGEFLWPGYT-----------EELKA-----

GTSMDSKPKENKISTEPGADQVSIGLVNESDSSLENDKKKKENVKKEMLGTEKEGSPDSHDSSKEKLNLNDNSKWSDFLKNIVTFGGFGPTVVHDVS
----------------------------------KKASEDAEKAANDAEN-ASKEAEEAAKEAVNLKESDK--SYTKAKEACTA-ASKAKKAVE

DTLSDISKDEVTQKTTKDIGSTLLDFFLPLPTKNTNTYEK-KNENKNVSNVDSKTKSNEKGRPPTYSPILDDGIEFSGGLYFNEKKSTEENKQKNVL
TALKAKDDAEKSSKADS-ISTKTKEY----AEKAKNAYEKAKNAYQKANQAVLKAKE-----ASSYDYIL--GWEFGGGVP-------EHKKEENML

ESVNLTSWDKEDIVKENEDVKDEKDEDDEE-EEEKYENEIIKQPEDILDEEEVLEEEILEENKNDTVDTSDLEKKNIPDLSNDNN----YYSLIYKN
```

Fig. 11 D

```
 |  ||• •••|• •••|•• •••|•| •• •••  •|    | •  | •|• •••  ••|•||  •    | •| || |••||      |•• ••
SHLYVSSKDKENISKENDDVLDEKEEEAEETEEEELEEKNEEETESEISEDE--EEEEEEEEKEEENDKKKEQEKEQSNENNDQKKDMEAQNLISKN
    |    |    |    |    |      |    |    |    |
  220  230  240  250  260    270  280  290  300
```

```
380       390       400       410       420
|         |         |         |         |
YKDNDKSEK-TAQTLITALISLLNGKNELDATIRRLKHRFMEFFTYN
||•|•| • |•|||| |• |•||•|•||•|•|| • | | |• |
QNNNEKNVKEAAESIMKTLAGLIKGNNQIDSTLKDLVEELSKYFKNH
|         |         |         |         |
310       320       330       340       350
```

Fig. 11 E

MSP3.3 Vs MSP3.2:

```
              10        20        30          40        50        60        70        80        90
SEQ ID No 6 MKKIVNIIF-YILY-LYIYKRNLVQNENVNKSN--LRKGLSTNNSENGIKSLKDEDEHINIIGDDFSAFSYGGYPIYETTGSLGTGVESVKAIDGES
            •|•• •• • |•• ••• •|||•• |• • ••|•                                    ||•|     •|       •
SEQ ID No 4 MNKIYNITFLFILLNLYINENNFIRNELINEKNHNLRNG------------------------------------SMYNNDKILSKN---------EV
              10        20        30                                    40        50

      100       110       120       130       140       150       160       170       180
190
GTSMDSKPKENKISTEPGADQVSIGLVNESDSSLENDKKKKENVKKEMLGTEKEGSPDSHDSSKEKLNLNDNSKWSDFLKNIVTFGGFGPTVVHDVS
|•|||•| |                 ||••| || | • ••                 |• | • •||•|         | •|•|| ||
DTNIESNENS----------------IHESGHKIDGEEVLKANV--------------DDITYKKKNVDDSE---------IPFSGYDIQATYQFP
      60                         70        80                     90                      100
110

      200       210       220       230       240       250       260       270       280
DTLSDISKDEVTQKTTKDIGSTLLDFFLPLPTKNTNTYEKKNEN-KNVSNVDSKTKSNEKGRPPTYSPILDDGI---EFSGGLYFNEKKSTEENKQK
 • ||| | • || •| || • |• •• |• | |• •| • ||| | | •|| • ••|•• || ||•||•
STSGGNNVIPLPIKQSGENQYTVTS--ISGIQKGANGLTGATENITQVVQANSETNKNPTSHSNSTTTSLNNNILGWEFGGGAP--QNGAAEDKKTE
       120       130       140       150       160       170       180       190       200

      290       300       310       320       330       340       350       360
```

Fig. 11 F

```
NVLESVNLTSWDKEDIVKENEDV-----KDEKDEDDEEE--------EEKYENEIIKQPEDILDEEEVLEEEILEENKNDTVDTSDLEKKNIPD---
YLLEQIKIPSWDRNNIPDENEQVIEDPQEDNKDEDEDEETETENLETEDDNNEEIEENEEDDIDEESVEEKEEEEEKKEEEEKKEEKKEEKKPDNEI
        210       220       230       240       250       260       270       280       290
300

             370                 380       390       400       410       420
---LSNDNNYYS--------LIYKNYKDNDKSEKTAQTLITALISLLNGKNELDATIRRLKHRFMEFFTYN
TNEVKEEQKYSSPSDINAQNLISNKNKKNDETKKTAENIVKTLVGLFNEKNEIDSTINNLVQEMIHLFSNN
             310       320       330       340       350       360       370
```

Fig. 11 G

MSP3.4 Vs MSP3.1:

```
          10        20        30        40        50        60        70        80        90
Seq id no 8  MKKIYSIFFSLFILNLHIYIKNIKCNDLINYNDSNLRNGLLNNSLDLTNGLNNKDNSFIDSKIEEHENKSYQNKDNNISIVGQDVPITSVYSSKIIN
Seq id no 2  MKSFINITLSLFLLHLYIYINNVASKEIVKKYNLNLRNAILNN---------------NSQIENEEN-----------------------------
          10        20        30        40                  50

100       110       120 b-like? 130       140       150       160       170       180       190
ANDLEGNSIDDTKGLSVTNSGFDDGSAFGGGLPFSGYSPLQGNHNKCPDENFCKGIKNVLSCPPKNSTGRNGDWISVAVKESSTTNKGVLVPPRRTK
--------VNTT----ITGNDFS-----GGEFLWPGYT---------------EELKAKKASEDAEKAANDAE---NASKEAEEAAKEAVN------
                     60              70                         80        90            100

290 200       210       220       230       240       250       260       270       280
LCLRNINKVWHRIKDEKNFKEEFVKVALGESNALMKHYKEKNLNALTAIKYGFSDMGDIIKGTDLIDYQITKNINRALDKILRNETSNDKIKKRVDW
--LKESDKSYTKAKE----------------------------------------------------ACTAASKAKKAVETALKAKDDAEKS------
    110       120                                                              130       140

   300       310       320       330       340       350       360       370       380
WEANKSAFWDAFMCGYKVHIGNKPCPEHDNMDRIPQYLRWFREWGTYVCSEYKNKFEDVIKLCNIQQFTNQDDSQLLEISKKDKCKEALKHYEEWVN
```

Fig 11 I

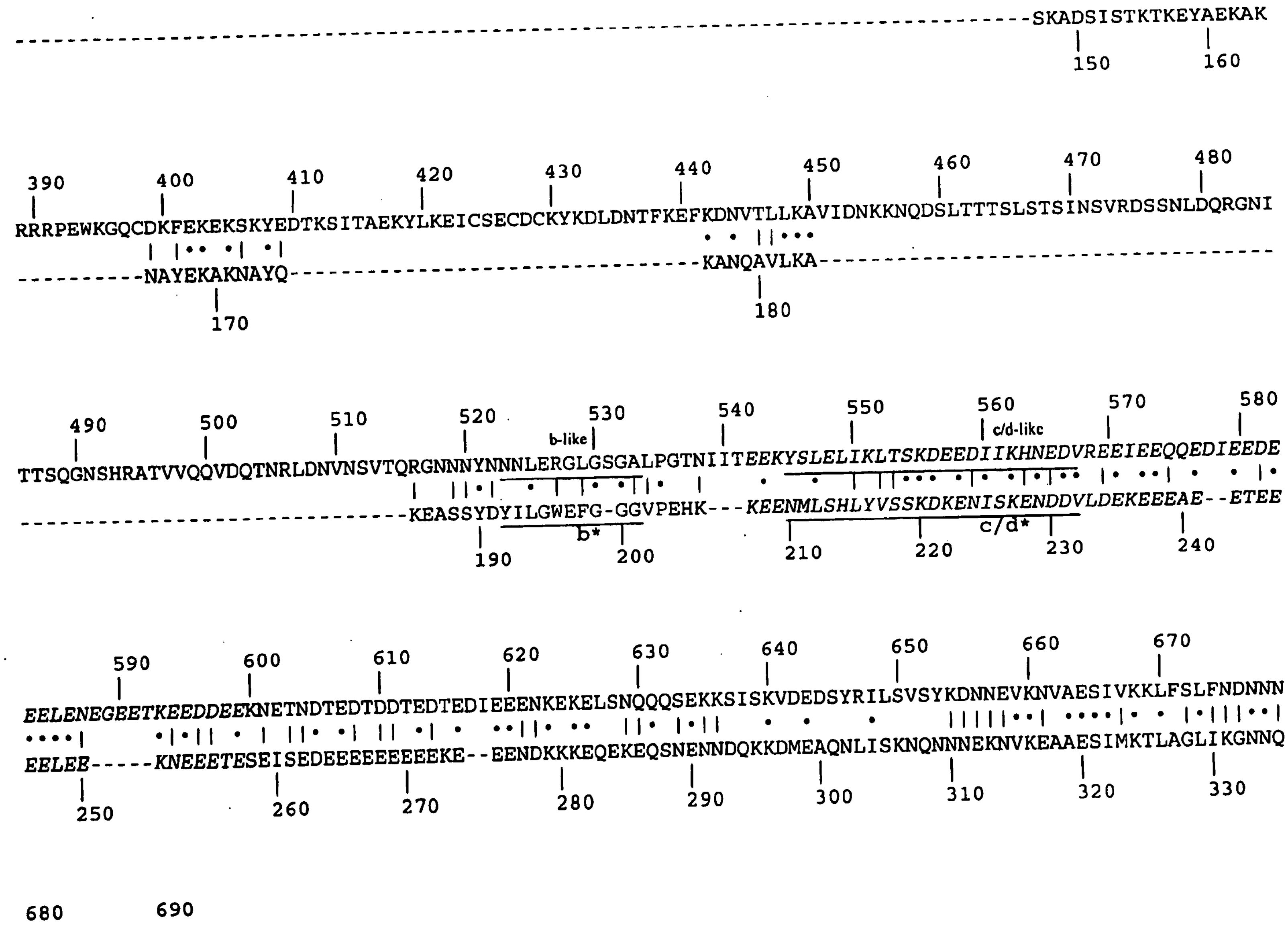

-----SKADSISTKTKEYAEKAK
150 160
390 400 410 420 430 440 450 460 470 480
RRRPEWKGQCDKFEKEKSKYEDTKSITAEKYLKEICSECDCKYKDLDNTFKEFKDNVTLLKAVIDNKKNQDSLTTTSLSTSINSVRDSSNLDQRGNI
----------NAYEKAKNAYQ-----KANQAVLKA-----
170 180
490 500 510 520 530 540 550 560 570 580
b-like
c/d-like
TTSQGNSHRATVVQQVDQTNRLDNVNSVTQRGNNNYNNNLERGLGSGALPGTNIITEEKYSLELIKLTSKDEEDIIKHNEDVREEIEEQQEDIEEDE
-----KEASSYDYILGWEFG-GGVPEHK---KEENMLSHLYVSSKDKENISKENDDVLDEKEEEAE--ETEE
b*
c/d*
190 200 210 220 230 240
590 600 610 620 630 640 650 660 670
EELENEGEETKEEDDEEKNETNDTEDTDDTEDTEDIEEENKEKELSNQQQSEKKSISKVDEDSYRILSVSYKDNNEVKNVAESIVKKLFSLFNDNNN
EELEE-----KNEEETESEISEDEEEEEEEEKE--EENDKKKEQEKEQSNENNDQKKDMEAQNLISKNQNNNEKNVKEAAESIMKTLAGLIKGNNQ
250 260 270 280 290 300 310 320 330
680 690

Fig 11 I

```
|               |
LETIFKGLTEDMTDLFQK-
||| |•|• •|||  •||
IDSTLKDLVEELSKYFKNH
    |         |
   340       350
```

Fig 11 J

MSP3.4 Vs MSP3.2:

```
          10        20        30        40        50        60        70        80        90
MKKIYSIFFSLFILNLHIYIKN---IKCNDLINYNDSNLRNGLLNNS---LDLTNGLNNKDNSFIDSKIEEHENKSYQNKDNNISIVGQDVPITSV-
MNKIYNITF-LFIL-LNLYINENNFIR-NELINEKNHNLRNGSMYNNDKIL-------SKNE--VDTNIESNEN-SIHESGHKID--GEEVLKANVD
            10        20        30        40                50        60        70        80

       100       110       120       130       140       150       160       170       180
---YSSKIINANDLEGNSIDDTKGLSVTNSGFDDGSAFGGGLPFSGYSPLQGNHNKCPDENFCKGIKNVLSCPPKNSTGRNGDWISVAVKESSTTNK
DITYKKK----------NVDDSE-----------------IPFSGYD-IQATYQ------F-----------PSTSGGNNV--IPLPIKQS-----
                  90                         100                        110         120

280     190       200       210       220       230       240       250       260       270
GVLVPPRRTKLCLRNINKVWHRIKDEKNFKEEFVKVALGESNALMKHYKEKNLNALTAIKYGFSDMGDIIKGTD-LIDYQITKNINRALDKILRNET
-------------------------------------GEN-----QY------TVTSI-------SGIQKGANGLTG--ATENITQVV------QA
                                              130                    140         150

             290       300       310       320       330       340       350       360       370
SNDKIKKRVDWWEANKSAFWDAFMCGYKVHIGNKPCPEHDNMDRIPQYLRWFREWGTYVCSEYKNKFEDVIKLCNIQQFTNQDDSQLLEISKKDKCK
```

Fig 11 K

```
NS----------ETNKNP-----------------------------------------------------TSHSNS------------
160                                                                       170

 380       390       400       410       420       430       440       450       460       470
EALKHYEEWVNRRRPEWKGQCDKFEKEKSKYEDTKSITAEKYLKEICSECDCKYKDLDNTFKEFKDNVTLLKAVIDNKKNQDSLTTTSLSTSINSVR
-------------------------------------------------------------------------------TTTSLN-------
                                                                                     180

  480       490       500       510       520       530         540          550       560
DSSNLDQRGNITTSQGNSHRATVVQQVDQTNRLDNVNSVTQRGNNNYNNNLERGLGSGALP--GTNIITEEK---YSLELIKLTSKDEEDIIKHNED
-----------------------------------------------NNILGWEFGGGA-PQNGA---AEDKKTEYLLEQIKIPSWDRNNIPDENEQ
                                                       190            200       210       220

 570        580       590         600       610         620       630         640        650
VREEIEEQQEDI-EEDEEELENEGE--ETKEEDDEEKNETNDTEDTDD--TEDTEDIEEENKEKELSNQQQSE-KKS---ISK-V-DEDSYRILS--
V---IEDPQEDNKDEDEDE-ETETENLETEDDNNEEIEE-NEEDDIDEESVEEKEE-EEEKKEEEEKKEEKKEEKKPDNEITNEVKEEQKYSSPSDI
      230       240        250       260        270       280       290       300       310
```

Fig 11 L

```
            660       670       680       690
              |         |         |         |
-----VSYKD--NNEVKNVAESIVKKLFSLFNDNNNLETIFKGLTEDMTDLF-QK
     |• •|  •|• •| ••|••• • |•••||•|||| || • ||• |•• ||
NAQNLISNKNKKNDETKKTAENIVKTLVGLFNEKNEIDSTINNLVQEMIHLFSNN
   |         |         |         |         |         |
  320       330       340       350       360       370
```

Fig. 11 H

MSP3.7 Vs MSP3.1 :

```
seq id n° 14  MNKFLNIIFYIFLILNFSFFQSNATSKEIQKDEQKNLRNGSSINNNKNIENKNDNIETQYEASEYIEKQNDILNMYNDEKEKNNNNSLDTNVTKNTV
seq id n° 2   MKSFINITLSLFL-LHLYIYINNVASKEIVKKYNLNLRNAI-LNNNSQIEN-EENVNTTITGNDF--SGGEFLWPGYTEELKAKKASEDAEKAANDA

              IDNSNKFQSIEDNNVYNKGIFVGTGIKLNDSQTTSDNYKNERYQIDDEKLKYGGSFDTIFSGFVNLLTPSSPTQNDGSTGRNVPPPSEPNVDTPDPP
              ENASKEAEEAAKE-----------AVNLKESDKSYTKAKE---------------------------ACTAASKAKKAVETALKAKDDAEKSSKADS

              TAPAPVKVPEDAKLS-SSPRPEGPRANNRNENNQNTDPYNHYFAWEIGGGAPTYKPENNKNDNILLEHVKITSWDKEDIIKENEDTKREVQETEDTD
              ISTKTKEYAEKAKNAYEKAKNAYQKANQAVLKAKEASSYDYILGWEFGGGVPEHKKEEN-----MLSHLYVSSKDKENISKENDDV---LDEKEEEA

              ETEDTDETEETEDMEDENEIVEDQLQENEDDEDNVNLEDINKNTRNDIFEEQIKLDSTQDDKAQKLISNEYKKTEE--KKSLEDHVNLLFNFLQTNN
```

Fig 11 N

```
•  | |• •• || • •|•• ••| |•|•||• |   •| |•|  ||  | | | •  |•  ••|•••|| || •   • | •  || •  ||| ••
EETEEEELEEKNEEETESEISEDEEEEEEEEEKE---EENDKKKEQEKEQSNENNDQKKDMEAQNLISKNQNNNEKNVKEAAESIMKTLAGLIKGNN
           |         |         |             |         |         |         |         |         |
          250       260       270           280       290       300       310       320       330

  390       400
   |         |
QLDPSLKDLENELTFFLNNY
•|•||•••• |••| |||•
QIDSTLKDLVEELSKYFKNH
    |         |
   340       350
```

Fig 11 o

MSP3.7 Vs MSP3.2:

```
MNKFLNIIFYIFLILNFSFFQSNATSKEIQKDEQKNLRNGSSINNNKNIENKNDNIETQYEASEYIEKQNDILNMYNDEKEKNNNNSLDTNVTKNTV
MNKIYNITF-LFILLNLYINENNFIRNELINEKNHNLRNGSMYNNDKILSK--NEVDTNIESNE------------NSIHESGHKIDGEEVLKANV

IDNSNKFQSIEDNNVYNKGIFVGTGIKLNDSQTTSDNYKNERYQIDDEKLKYGGSFDTIFSGFVNLLTPSSPTQNDGSTGRNVPPPSEPNVDTPDPP
DDITYKKKNVDDSEI----PFSGYDIQ------------ATYQFPSTS---GG------NNVIPL-----PIKQSGENQYTVTSISGIQKGANGLT

TAPAPVKVPEDAKLSSSPRPEGPRANNRNENNQNTDPYNHYFAWEIGGGAPTYKPENNKNDNILLEHVKITSWDKEDIIKENEDTKREVQETEDTDE
GATENITQVVQAN------SETNKNPTSHSNSTTTSLNNNILGWEFGGGAPQNGAAEDKKTEYLLEQIKIPSWDRNNIPDENEQVIEDPQEDNKDED

TEDTDETEETEDMED---------ENEIVEDQLQENEDDEDNVNLEDINKNTRNDI---------FEEQIKLDSTQDDKAQKLISNEYKKTEE-KKS
```

Fig 11P

```
||  •••| •  |•         •||• •| ||•|•||•|| | •| | | |||        •| •  •  • |••|••••  •• |• ••|
EDEETETENLETEDDNNEEIEENEEDDIDEESVEEKEEEEEKKEEEEKKEEKKEEKKPDNEITNEVKEEQKYSSPSDINAQNLISNKNKKNDETKKT
|         |         |         |         |         |         |         |         |         |
240       250       260       270       280       290       300       310       320       330

370       380       390       400
|         |         |         |
LEDHVNLLFNFLQTNNQLDPSLKDLENELTFFLNNY
 •| •| •  ||| |•||•|||||• |•| |||•
AENIVKTLVGLFNEKNEIDSTINNLVQEMIHLFSNN
    |         |         |         |
    340       350       360       370
```

Fig 11 Q

MSP3.8 Vs MSP3.1:

```
            10        20        30        40        50        60        70        80        90
             |         |         |         |         |         |         |         |         |
n°16 MIYILSIVFYIFFLHIDIYVNIYSTCFVVNEGNPNLRNNIINDDELKGKAYNNTIDANNQNIEYNKNLKHNVNSSHISKFSDIMDQEDKGDNENSHD
     •  |||• | |•|••| ••|•                •| | | |•                        •• ••||            •|||| | |••|| |
n°2  MKSFINITLSLFLLHLYIYIN-----------------NVASKEIVK---------------KYNLNLRNA-----------ILNNNSQIENEENVN
             |         |                          |                                          |         |
            10        20                         30                                         40        50

     100       110       120       130       140       150       160       170       180       190
      |         |         |         |         |         |         |         |         |         |
     IKFEEKKNINKSLDAESNYGINEISITGNDSNSDNSNQNIFPDGSELAGGIPRSIYTINLGFNKCPTEEICKDFSNLPQCRKNVHERNNWLGSSVKN
                              |•••••           •||•  •                                            • • |
     T------------------------TITGND----------FSGGEFL------------------------------------------WPGYT---
                                   |                                                               |
                                  60                                                              70

         200       210       220       230       240       250       260       270       280
290       |         |         |         |         |         |         |         |         |         |
     FSSDNKGVLVPPRRQSLCLRITLQDFRTKKKKEGDFEKFIYSYASSEARKLRTIHNNNLEKAHQAIRYSFADIGNIIRGDDMMDTPTSKETITYLEK
                              |||||••  •|                     •••
     ------------------------EELKAKKASEDA----------------------EKAA-----------------------------------
                                 |
                                80

          300       310       320       330       340       350       360       370       380
           |         |         |         |         |         |         |         |         |
     VLKIYNENNDKPKDAKKWWTENRHHVWEAMMCGYQSAQKDNQCTGYGNIDDIPQFLRWFREWGTYVCEESEKNMNTLKAVCFPKQPRTEANPALTVH
```

Fig 11R

```
-----NDAENASKEA------------------------------------------------------EEAAKEAVNLK-----------------
     90                                                              100       110

390      400       410       420       430       440       450       460       470       480
ENEMCSSTLKKYEEWYNKRKTEWTEQSIKYNNDKINYTDIKTLSPSEYLIEKCPECKCTKKNLQDVFELTFDGKALLEKLKKEESPVSNSVNALPEP
------------------------------ESDK-SYTKAK---------EACTAASKAKKAV-----------------------------------
                                         120                130

   490       500       510       520       530       540       550       560       570  ? ? ?  580
GQITLPDPSLKQTTQQENQPVVETPVTTAVINEHQGQTEPNKGDNNNERENHESNVGSIQEVNQGSVSEESHSKTIDPSKIDDRLELSSGSSSLEQH
------ETALKAKDDAEKSSKADSISTKT------------KEYAEKAKNAYEKAKNAYQKANQAVL------KAKEASSYDYILGWEFGGGVPEHK
              140       150                 160       170       180             190       200

      590       600       610       620       630       640       650       660       670
SKEDVKKGCALELVPLSLSDIEQIANESEDVLEEIEEEINTDGEIEYITEEEIKEDIEEETEEDIEEETEEETEEETEEEADEETVKEIEDKPEQEI
KEENM-----LSHLYVSSKDKENISKENDDVLDEKEEEA-----------------EETEEEELEEKNEEETESEISEDEEEEE--EEEEKEEEND
 210                 220       230       240                       250       260         270
```

Fig 11 S

```
680       690       700       710       720       730       740       750       760
|         |         |         |         |         |         |         |         |
KNKSLEEKQIDKNTDTSEKKGFNNSEKDEKARNLISKNYKNYN-ELDKNVHTLVNSIISLLEEGNGSDSTLNSLSKDITNLFKN-
•|•   •|| ||| | |||•       ••  •|•••••• |• | ||    ||||||| |•|   •   ••••| •  |||| •••
KKK---EQEKEQSNENNDQK------KDMEAQNLISKNQNNNEKNVKEAAESIMKTLAGLIKGNNQIDSTLKDLVEELSKYFKNH
 |          |               |         |         |         |         |         |
 280        290             300       310       320       330       340       350
```

Fig 11 T

MSP3.8 Vs MSP3.2:

```
             10        20        30        40         50        60        70        80        90
              |         |         |         |          |         |         |         |         |
n°16  MIYILSIVFYIFFLHIDIYVNIYSTCFVVNEGNPNLRNNIINDDE--LKGKAYNNTIDANNQNIEYNKNLKHNVNSSHISKFSDIMDQEDKGDNENS
      • • |• • |||•|| • • |   ||•• • ••••  | |||   •  |  |  •||•|||•|   | •|||| || •              •
n°4   MNKIYNITFLFILLNLYINENNFIRNELINEKNHNLRNGSMYNNDKILSKNEVDTNIESNENSIH---ESGHKIDGEEVLK-------------ANV
              |         |         |         |         |         |           |                       |
             10        20        30        40        50        60          70                      80

          100       110       120       130       140       150       160       170       180       190
            |         |         |         |         |         |         |         |         |         |
      HDIKFEEKKNINKSLDAESNYGINEISITGNDSNSDNSNQNIFPDGSELAGGIPRSIYTINLGFNKCPTEEICKDFSNLPQCRKNVHERNNWLGSSV
      |•• |  •••|| •         ••|||•                          • •|  ||
      DDITYK-KKNVDDS----------EIPFSG-------------------------YDIQATYQ--------------------------------------
               |               |
              90              100

         200       210       220       230       240       250       260       270       280
           |         |         |         |         |         |         |         |         |
      KNFSSDNKGVLVPPRRQSLCLRITLQDFRTKKKKEGDFEKFIYSYASSEARKLRTIHNNNLEKAHQAIRYSFADIGNIIRGDDMMDTPTSKETITYL
        •|• | • • •      • •       •| •|                              |•| | •| • |• | |  |•
      --FPSTSGGNNVIP------LPI---------KQSGE-----------------------------NQYTVTSISGIQKGANGLTGAT--------
          |         |                                                  |         |         |
         110       120                                                130       140       150

      290       300       310       320       330       340       350       360       370       380
      |         |         |         |         |         |         |         |         |         |
      EKVLKIYNENNDKPKDAKKWWTENRHHVWEAMMCGYQSAQKDNQCTGYGNIDDIPQFLRWFREWGTYVCEESEKNMNTLKAVCFPKQPRTEANPALT
      •|| || | •|| •||                        ||| ||  •|
```

Fig 11 C

```
ENITQVVQANSETNKNP------------------TSHSNSTTTSLN------------------------------------------
            160                      170

 390       400       410       420       430       440       450       460       470       480
VHENEMCSSTLKKYEEWYNKRKTEWTEQSIKYNNDKINYTDIKTLSPSEYLIEKCPECKCTKKNLQDVFELTFDGKALLEKLKKEESPVSNSVNALP
-------------------------------------------------------------------------------------------------

580
    490       500       510       520       530       540       550       560       570 ? ? ?
EPGQITLPDPSLKQTTQQENQPVVETPVTTAVINEHQGQTEPNKGDNNNERENHESNVGSIQEVNQGSVSEESHSKTIDPSKIDDRLELSSGSSSLE
-------------------------------------------------------------------------------------NNILGWEFGGGAPQ
                                                                                     180        190

       590       600       610       620       630       640       650       660       670
QHSKEDVKKGCALELVPLSLSDIEQIANESEDVLEEIEEEINTDGEIEYITEEEIKEDIEEETEEDIEEETEEETEEETEEEADEETVKEIEDKPEQ
NGAAEDKKTEYLLEQIKIPSWDRNNIPDENEQVIEDPQEDNKDEDEDEETETENL--ETEDDNNEEIEENEEDDIDEESVEEKEEEEEKKEEEEKKE
    200       210       220       230       240       250       260       270       280
```

Fig 11 V

```
680             690             700             710             720             730             740             750             760
 |               |               |               |               |               |               |               |               |
EIKNKSLEEKQIDKNTDTSEKKGFNNSEKDEKARNLISKNYKNYNELDKNVHTLVNSIISLLEEGNGSDSTLNSLSKDITNLF-KN
• •|    |||• ||    |•     |•  • |•|••••|| •| |•  •  | |•||||||•||•  •  •••|•|•  ||| |•• |•
EKKEEKKPDNEITNEVKEEQK---YSSPSDINAQNLISNKNKKNDETKKTAENIVKTLVGLFNEKNEIDSTINNLVQEMIHLFSNN
|         |             |         |         |         |         |         |         |
290       300           310       320       330       340       350       360       370
```

# MSP3b-like motifs

```
                    10
                     |
                    |●|
seq.4/MSP3     IL--GWEFGGGVP
seq.5/MSP6     IL--GWEFGGGAP
seq.3          YF--AWEIGGGAP
seq.1 c-term   -LER-GLGSGALP
seq.1 n-term   --DDGSAFGGGLP
seq.8          ILDDGIEFSGGLYF
seq.7 n-term   --PDGSELAGGIP
seq.7 c-term   ---RLELSSGSSLE
```

MSP3b:

| | | | | | | | | | | | |
|---|---|---|---|---|---|---|---|---|---|---|---|
| codons: | ATT | TTA | GGT | TGG | GAA | TTT | GGA | GGA | GGC | GTT | CCA |
| a.a. | I | L | G | W | E | F | G | G | G | V | P |

MSP6 equivalent:

| | | | | | | | | | | | |
|---|---|---|---|---|---|---|---|---|---|---|---|
| codons: | ATA | CTT | GGA | TGG | GAA | TTT | GGA | GGA | GGT | GCT | CCT |
| a.a. | I | L | G | W | E | F | G | G | G | A | P |

**Fig 12**

# Fig 13

## MSP3 c/d-like motifs

```
                       10
                        |
             •  | |     • ||•   ||||
seq.1        LELIKLTSKDEEDIIKHNED
seq.3        LEHVKITSWDKEDIIKENED
seq.8        LESVNLTSWDKEDIVKENED
seq.4/MSP3   LSHLYVSSKDKENISKENDD
seq.5/MSP6   LEQIKIPSWDRNNIPDENEQ
seq.7        LELVPLSLSDIEQIANESED
```

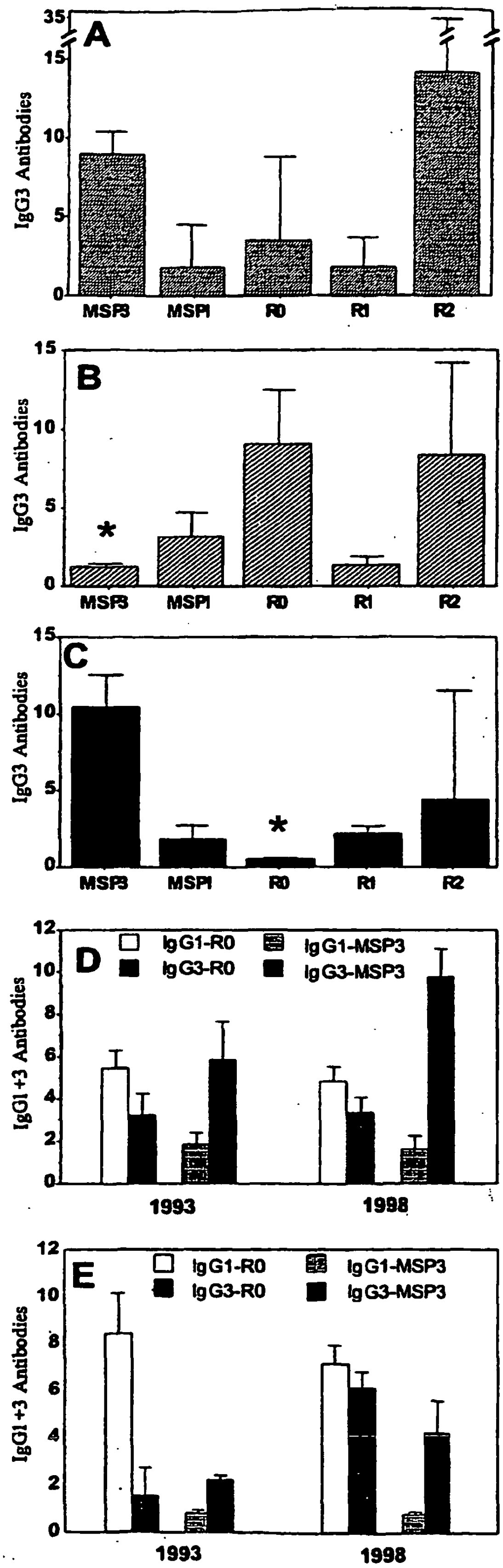
A
IgG3 Antibodies
35
15
10
5
0
MSP3
MSP1
R0
R1
R2
B
IgG3 Antibodies
15
10
5
0
*
MSP3
MSP1
R0
R1
R2
C
IgG3 Antibodies
15
10
5
0
*
MSP3
MSP1
R0
R1
R2
D
IgG1+3 Antibodies
12
10
8
6
4
2
0
IgG1-R0
IgG3-R0
IgG1-MSP3
IgG3-MSP3
1993
1998
E
IgG1+3 Antibodies
12
10
8
6
4
2
0
IgG1-R0
IgG3-R0
IgG1-MSP3
IgG3-MSP3
1993
1998

Fig. 14

Fig. 15

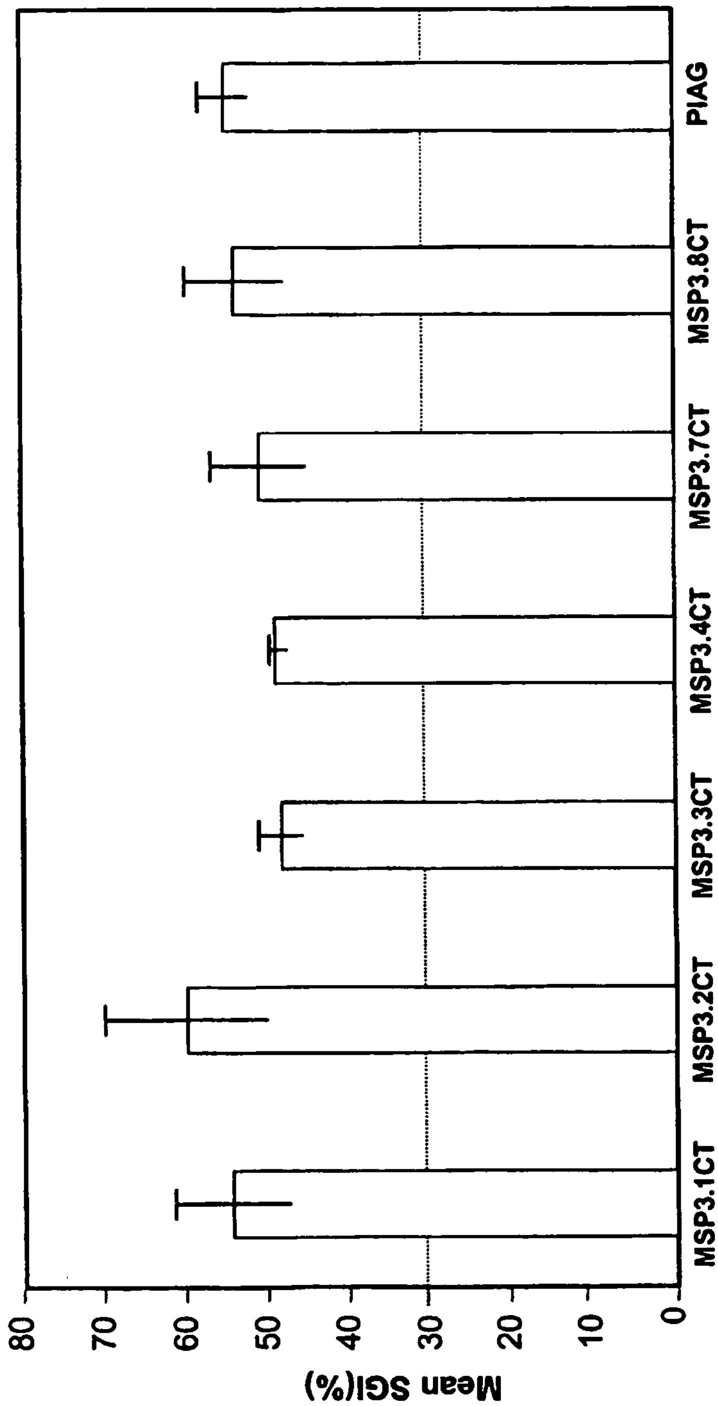
Mean SGI(%)
0
10
20
30
40
50
60
70
80
MSP3.1CT
MSP3.2CT
MSP3.3CT
MSP3.4CT
MSP3.7CT
MSP3.8CT
PIAG

Fig. 16

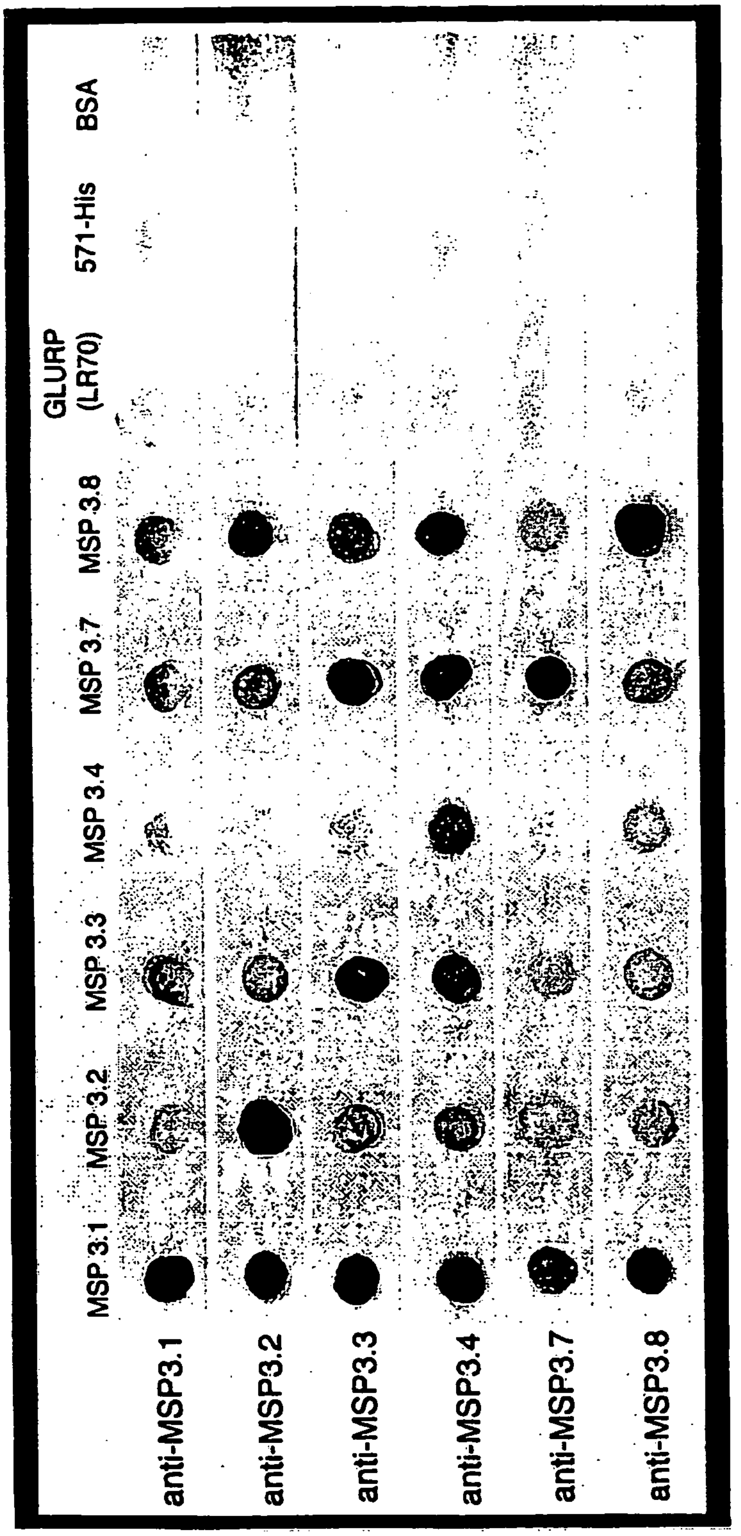
MSP 3.1
MSP 3.2
MSP 3.3
MSP 3.4
MSP 3.7
MSP 3.8
GLURP (LR70)
571-His
BSA
anti-MSP3.1
anti-MSP3.2
anti-MSP3.3
anti-MSP3.4
anti-MSP3.7
anti-MSP3.8

Fig. 17

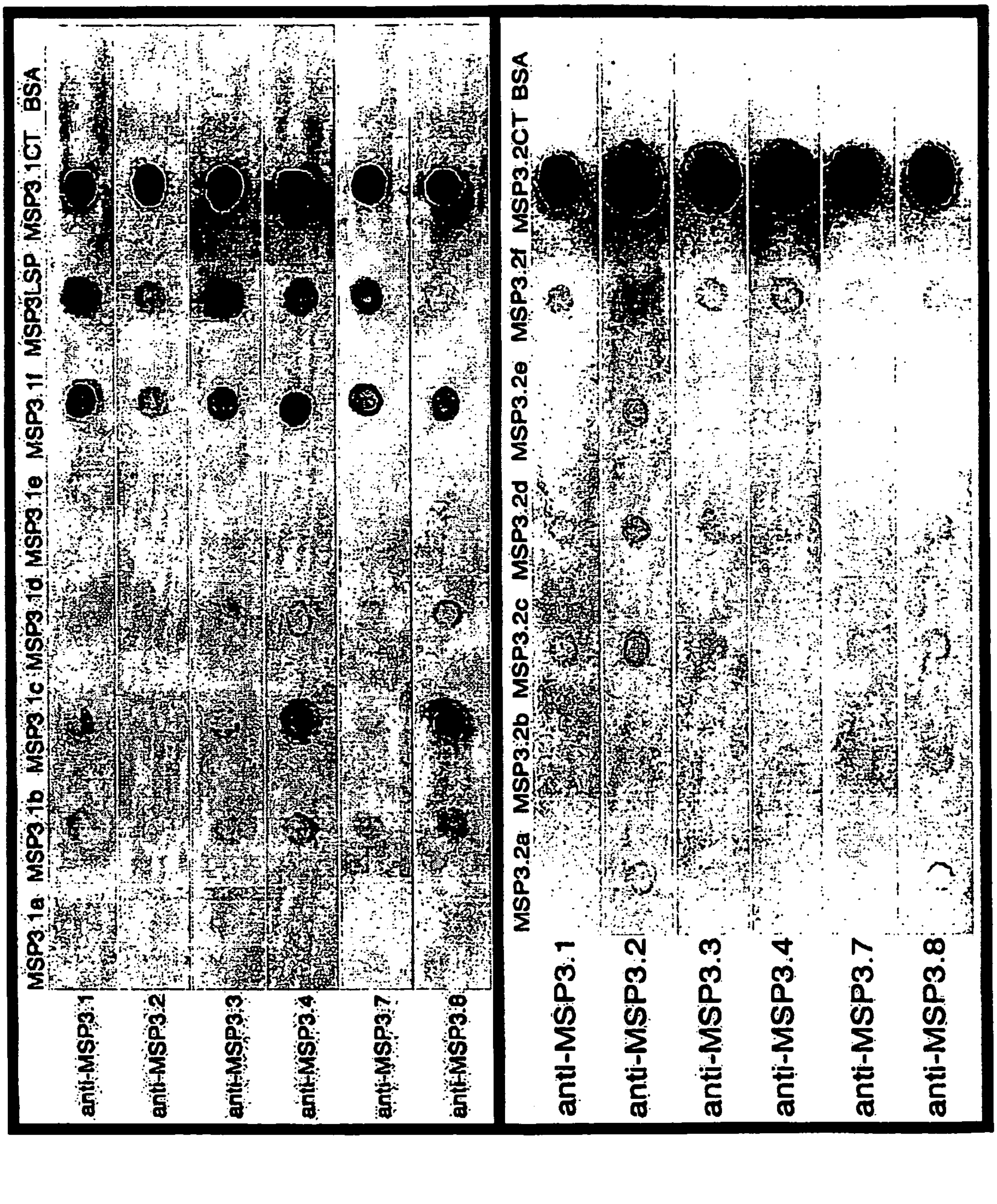
MSP3.1a
MSP3.1b
MSP3.1c
MSP3.1d
MSP3.1e
MSP3.1f
MSP3LSP
MSP3.1CT
BSA
anti-MSP3.1
anti-MSP3.2
anti-MSP3.3
anti-MSP3.4
anti-MSP3.7
anti-MSP3.8
MSP3.2a
MSP3.2b
MSP3.2c
MSP3.2d
MSP3.2e
MSP3.2f
MSP3.2CT
BSA
anti-MSP3.1
anti-MSP3.2
anti-MSP3.3
anti-MSP3.4
anti-MSP3.7
anti-MSP3.8

Fig. 18

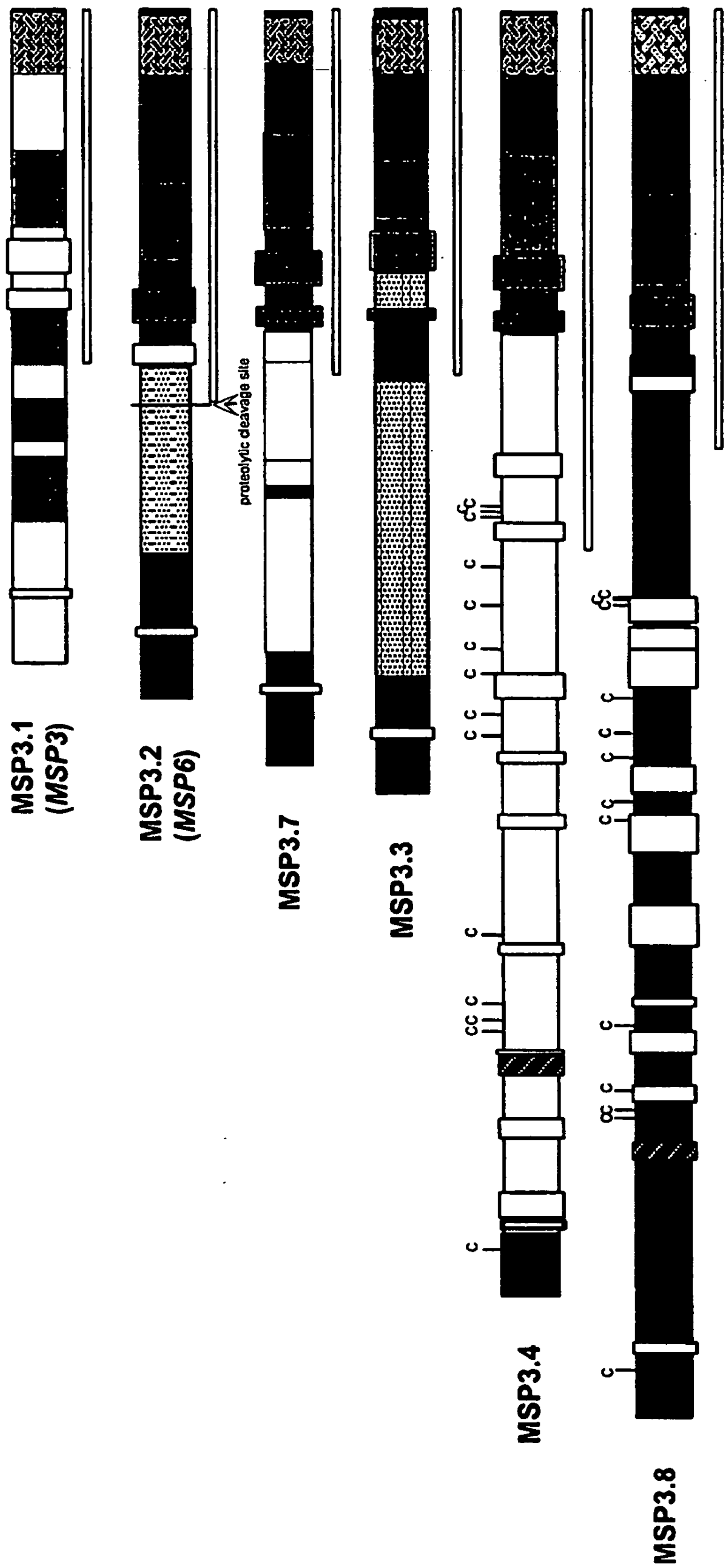
MSP3.1
(MSP3)
MSP3.2
(MSP6)
proteolytic cleavage site
MSP3.7
MSP3.3
MSP3.4
MSP3.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03016354 A


### Non-patent literature cited in the description


- **Boyum.** *Scand J. Clin. Lab. Invest.,* 1968, vol. 21, 77-89
- **Badell, E. ; C. Oeuvray et al.** Human malaria in immunocompromised mice: an in vivo model to study defense mechanisms against Plasmodium falciparum. *J Exp Med,* 2000, vol. 192 (11), 1653-60
- **Beier, J. C. ; G. F. Killeen et al.** Short report: entomologic inoculation rates and Plasmodium falciparum malaria prevalence in Africa. *Am J Trop Med Hyg,* 1999, vol. 61 (1), 109-13
- **BenMohamed, L. ; Y. Belkaid et al.** Systemic immune responses induced by mucosal administration of lipopeptides without adjuvant. *Eur J Immunol,* 2002, vol. 32 (8), 2274-81
- **Bottius, E. ; L. BenMohamed et al.** A novel Plasmodium falciparum sporozoite and liver stage antigen (SALSA) defines major B, T helper, and CTL epitopes. *J Immunol,* 1996, vol. 156 (8), 2874-84
- **Bouharoun-Tayoun, H. ; P. Druilhe.** Antibodies in falciparum malaria: what matters most, quantity or quality?. *Mem Inst Oswaldo Cruz,* 1992, vol. 87 (3), 229-34
- **Bouharoun-Tayoun, H. ; C. Oeuvray et al.** Mechanisms underlying the monocyte-mediated antibody-dependent killing of Plasmodium falciparum asexual blood stages. *J Exp Med,* 1995, vol. 182 (2), 409-18
- **Brahimi, K. ; J. L. Perignon et al.** Fast immunopurification of small amounts of specific antibodies on peptides bound to ELISA plates. *J Immunol Methods,* 1993, vol. 162 (1), 69-75
- **Dame, J. B. ; J. L. Williams et al.** Structure of the gene encoding the immunodominant surface antigen on the sporozoite of the human malaria parasite Plasmodium falciparum. *Science,* 1984, vol. 225 (4662), 593-9
- **Daubersies, P. ; A. W. Thomas et al.** Protection against Plasmodium falciparum malaria in chimpanzees by immunization with the conserved pre-erythrocytic liver-stage antigen 3. *Nat Med,* 2000, vol. 6 (11), 1258-63
- **de Stricker, K. ; J. Vuust et al.** Conservation and heterogeneity of the glutamate-rich protein (GLURP) among field isolates and laboratory lines of Plasmodium falciparum. *Mol Biochem Parasitol,* 2000, vol. 111 (1), 123-30
- **Dodoo, D. ; T. G. Theander et al.** Levels of antibody to conserved parts of Plasmodium falciparum merozoite surface protein 1 in Ghanaian children are not associated with protection from clinical malaria. *Infect Immun,* 1999, vol. 67 (5), 2131-7
- **Dodoo, D. ; M. Theisen et al.** Naturally acquired antibodies to the glutamate-rich protein are associated with protection against Plasmodium falciparum malaria. *J Infect Dis,* 2000, vol. 181 (3), 1202-5
- **Egan, A. F. ; J. Morris et al.** Clinical immunity to Plasmodium falciparum malaria is associated with serum antibodies to the 19-kDa C-terminal fragment of the merozoite surface antigen, PfMSP-1. *J Infect Dis,* 1996, vol. 173 (3), 765-9
- **Escalante, A. A. ; H. M. Grebert et al.** Polymorphism in the gene encoding the apical membrane antigen-1 (AMA-1) of Plasmodium falciparum. X. Asembo Bay Cohort Project. *Mol Biochem Parasitol,* 2001, vol. 113 (2), 279-87
- **Fidock, D. A. ; E. Bottius et al.** Cloning and characterization of a novel Plasmodium falciparum sporozoite surface antigen, STARP. *Mol Biochem Parasitol,* 1994, vol. 64 (2), 219-32
- **Gras-Masse, H. ; B. Georges et al.** Convergent peptide libraries, or mixotopes, to elicit or to identify specific immune responses. *Curr Opin Immunol,* 1999, vol. 11 (2), 223-8
- **Guerih-Marchand, C. ; P. Druilhe et al.** A liver-stage-specific antigen of Plasmodium falciparum characterized by gene cloning. *Nature,* 1987, vol. 329 (6135), 164-7
- **Huber, W. ; I. Felger et al.** Limited sequence polymorphism in the Plasmodium falciparum merozoite surface protein 3. *Mol Biochem Parasitol,* 1997, vol. 87 (2), 231-4
- **Khusmith, S. ; P. Druilhe.** Cooperation between antibodies and monocytes that inhibit in vitro proliferation of Plasmodium falciparum. *Infect Immun,* 1983, vol. 41 (1), 219-23

- **Knapp, B. ; E. Hundt et al.** Molecular cloning, genomic structure and localization in a blood stage antigen of Plasmodium falciparum characterized by a serine stretch. *Mol Biochem Parasitol,* 1989, vol. 32 (1), 73-83
- **Lunel, F. ; P. Druilhe.** Effector cells involved in nonspecific and antibody-dependent mechanisms directed against Plasmodium falciparum blood stages in vitro. *Infect Immun,* 1989, vol. 57 (7), 2043-9
- **Marshall, V. M. ; W. Tieqiao et al.** Close linkage of three merozoite surface protein genes on chromosome 2 of Plasmodium falciparum. *Mol Biochem Parasitol,* 1998, vol. 94 (1), 13-25
- **McColl, D. J. ; R. F. Anders.** Conservation of structural motifs and antigenic diversity in the Plasmodium falciparum merozoite surface protein-3 (MSP-3). *Mol Biochem Parasitol,* 1997, vol. 90 (1), 21-31
- **Miller, L. H. ; T. Roberts et al.** Analysis of sequence diversity in the Plasmodium falciparum merozoite surface protein-1 (MSP-1). *Mol Biochem Parasitol,* 1993, vol. 59 (1), 1-14
- **Oeuvray, C. ; H. Bouharoun-Tayoun et al.** Merozoite surface protein-3: a malaria protein inducing antibodies that promote Plasmodium falciparum killing by cooperation with blood monocytes. *Blood,* 1994, vol. 84 (5), 1594-602
- **Oeuvray, C. ; M. Theisen et al.** Cytophilic immunoglobulin responses to Plasmodium falciparum glutamate-rich protein are correlated with protection against clinical malaria in Dielmo, Senegal. *Infect Immun,* 2000, vol. 68 (5), 2617-20
- **Peterson, M. G. ; V. M. Marshall et al.** Integral membrane protein located in the apical complex of Plasmodium falciparum. *Mol Cell Biol,* 1989, vol. 9 (7), 3151-4
- **Pleass, R. J. ; J. M. Woof.** Fc receptors and immunity to parasites. *Trends Parasitol,* 2001, vol. 17 (11), 545-51
- **Robson, K. J. ; J. R. Hall et al.** A highly conserved amino-acid sequence in thrombospondin, properdin and in proteins from sporozoites and blood stages of a human malaria parasite. *Nature,* 1988, vol. 335 (6185), 79-82
- **Roussillon, C.** Correlates of Immune protection in malaria. *MIM African Malaria Conference,* 14 March 1999
- **Simmons, D. ; G. Woollett et al.** A malaria protein exported into a new compartment within the host erythrocyte. *Embo J,* 1987, vol. 6 (2), 485-91
- **Soe, S. ; A. Khin Saw et al.** Premunition against Plasmodium falciparum in a malaria hyperendemic village in Myanmar. *Trans R Soc Trop Med Hyg,* 2001, vol. 95 (1), 81-4
- **Theisen, M. ; D. Dodoo et al.** Selection of glutamate-rich protein long synthetic peptides for vaccine development: antigenicity and relationship with clinical protection and immunogenicity. *Infect Immun,* 2001, vol. 69 (9), 5223-9
- **Theisen, M. ; S. Soe et al.** Identification of a major B-cell epitope of the Plasmodium falciparum glutamate-rich protein (GLURP), targeted by human antibodies mediating parasite killing. *Vaccine,* 2000, vol. 19 (2-3), 204-12
- **Theisen, M. ; S. Soe et al.** The glutamate-rich protein (GLURP) of Plasmodium falciparum is a target for antibody-dependent monocyte-mediated inhibition of parasite growth in vitro. *Infect Immun,* 1998, vol. 66 (1), 11-7
- **Thomas, A. W. ; D. A. Carr et al.** Sequence comparison of allelic forms of the Plasmodium falciparum merozoite surface antigen MSA2. *Mol Biochem Parasitol,* 1990, vol. 43 (2), 211-20
- **Trucco, C. ; D. Fernandez-Reyes et al.** The merozoite surface protein 6 gene codes for a 36 kDa protein associated with the Plasmodium falciparum merozoite surface protein-1 complex. *Mol Biochem Parasitol,* 2001, vol. 112 (1), 91-101
- **Tun-Lin, W. ; M. M. Thu et al.** Hyperendemic malaria in a forested, hilly Myanmar village. *J Am Mosq Control Assoc,* 1995, vol. 11 (4), 401-7

SEQUENCE LISTING

<110> INSTITUT PASTEUR

<120> MSP-3-LIKE FAMILY OF GENES

<130> 85767 - AD/VMA/VG

<140> New European Patent Application
<141> 2003-10-24

<160> 30

<170> PatentIn version 3.1

<210> 1
<211> 1065
<212> DNA
<213> Plasmodium Falciparum

<220>
<221> misc_feature
<222> (1)..(1065)
<223> MSP 3.1

<400> 1

```
atgaaaagtt ttataaatat tactctttca ttatttttgt tacatttata tatttatata     60
aataatgttg ctagtaaaga aattgtaaaa aaatataatc ttaacttaag aaatgcaata    120
ttgaataata attctcaaat agaaaatgaa gaaaatgtaa atactacaat tactggtaat    180
gattttagtg gtggagaatt tttgtggcct ggttatacgg aagaattaaa agctaaaaaa    240
gcttccgaag atgctgaaaa agctgctaat gatgctgaaa atgcttcaaa agaggcagaa    300
gaagctgcta aagaagcagt aaatttaaag gaatctgata aatcttatac aaaagcaaaa    360
gaagcatgta cagctgcttc aaaggcaaag aaagctgttg aaactgcttt aaaggcaaaa    420
gatgatgctg aaaaatcttc aaaagctgat agtatttcta caaaaacaaa agaatatgct    480
gaaaaagcaa aaaatgctta tgaaaaggca aaaaatgctt atcaaaaagc aaaccaagct    540
gttttaaaag caaaagaagc ttctagttat gattatattt taggttggga atttggagga    600
ggcgttccag aacacaaaaa agaagaaaat atgttatcac atttatatgt ttcttcaaag    660
gataaggaaa atatatctaa ggaaaatgat gatgtattag atgagaagga agaagaggca    720
gaagaaacag aagaagaaga acttgaagaa aaaaatgaag aagaaacaga atcagaaata    780
agtgaagatg aagaagaaga agaagaagaa gaagaaaagg aagaagaaaa tgacaaaaaa    840
aaagaacaag aaaaagaaca aagtaatgaa aataatgatc aaaaaaaaga tatggaagca    900
cagaatttaa tttctaaaaa ccagaataat aatgagaaaa acgtaaaaga agctgctgaa    960
agcatcatga aaactttagc tggtttaatc aagggaaata atcaaataga ttctacctta   1020
aaagatttag tagaagaatt atccaaatat tttaaaaatc attaa                  1065
```

<210> 2
<211> 354
<212> PRT
<213> Plasmodium Falciuparum

<220>
<221> MISC_FEATURE
<222> (1)..(354)
<223> MSP 3.1

<400> 2

```
Met Lys Ser Phe Ile Asn Ile Thr Leu Ser Leu Phe Leu Leu His Leu
1               5                   10                  15

Tyr Ile Tyr Ile Asn Asn Val Ala Ser Lys Glu Ile Val Lys Lys Tyr
            20                  25                  30

Asn Leu Asn Leu Arg Asn Ala Ile Leu Asn Asn Asn Ser Gln Ile Glu
        35                  40                  45

Asn Glu Glu Asn Val Asn Thr Thr Ile Thr Gly Asn Asp Phe Ser Gly
    50                  55                  60

Gly Glu Phe Leu Trp Pro Gly Tyr Thr Glu Glu Leu Lys Ala Lys Lys
65                  70                  75                  80

Ala Ser Glu Asp Ala Glu Lys Ala Ala Asn Asp Ala Glu Asn Ala Ser
                85                  90                  95

Lys Glu Ala Glu Glu Ala Ala Lys Glu Ala Val Asn Leu Lys Glu Ser
            100                 105                 110

Asp Lys Ser Tyr Thr Lys Ala Lys Glu Ala Cys Thr Ala Ala Ser Lys
        115                 120                 125

Ala Lys Lys Ala Val Glu Thr Ala Leu Lys Ala Lys Asp Asp Ala Glu
    130                 135                 140

Lys Ser Ser Lys Ala Asp Ser Ile Ser Thr Lys Thr Lys Glu Tyr Ala
145                 150                 155                 160

Glu Lys Ala Lys Asn Ala Tyr Glu Lys Ala Lys Asn Ala Tyr Gln Lys
                165                 170                 175

Ala Asn Gln Ala Val Leu Lys Ala Lys Glu Ala Ser Ser Tyr Asp Tyr
            180                 185                 190

Ile Leu Gly Trp Glu Phe Gly Gly Gly Val Pro Glu His Lys Lys Glu
        195                 200                 205

Glu Asn Met Leu Ser His Leu Tyr Val Ser Ser Lys Asp Lys Glu Asn
    210                 215                 220

Ile Ser Lys Glu Asn Asp Asp Val Leu Asp Glu Lys Glu Glu Glu Ala
225                 230                 235                 240
```

Glu Glu Thr Glu Glu Glu Glu Leu Glu Glu Lys Asn Glu Glu Glu Thr
245 250 255

Glu Ser Glu Ile Ser Glu Asp Glu Glu Glu Glu Glu Glu Glu Glu Glu
260 265 270

Lys Glu Glu Glu Asn Asp Lys Lys Lys Glu Gln Glu Lys Glu Gln Ser
275 280 285

Asn Glu Asn Asn Asp Gln Lys Lys Asp Met Glu Ala Gln Asn Leu Ile
290 295 300

Ser Lys Asn Gln Asn Asn Asn Glu Lys Asn Val Lys Glu Ala Ala Glu
305 310 315 320

Ser Ile Met Lys Thr Leu Ala Gly Leu Ile Lys Gly Asn Asn Gln Ile
325 330 335

Asp Ser Thr Leu Lys Asp Leu Val Glu Glu Leu Ser Lys Tyr Phe Lys
340 345 350

Asn His

<210> 3
<211> 1116
<212> DNA
<213> Plasmodium Falciparum

<220>
<221> misc_feature
<222> (1)..(1116)
<223> MSP 3.2

<400> 3

atgaataaga tttataatat tacttttctt ttcattcttt taaacttata tataaatgaa 60
aataacttta tcagaaatga acttataaac gaaaaaaacc ataatttaag aaatggttca 120
atgtataata acgataaaat attaagtaaa aatgaagtag atactaatat agaaagtaac 180
gaaaatagta ttcacgaatc tggacataag attgatgggg aagaagtttt aaaagctaat 240
gtagatgata taacatacaa aaaaaaaaat gttgatgatt cagaaattcc tttttctggt 300
tatgatatac aagcaacata tcaatttcct tctacatcag gaggaaataa tgtaattcca 360
cttcctataa aacaaagtgg agaaaatcaa tatactgtta catctatatc aggtattcaa 420
aagggagcaa atggtttaac tggtgcaaca gaaaatatta cacaagttgt acaagcaaac 480
tctgaaacaa ataaaaatcc tacttctcat agtaatagta ctacaacttc tctgaataat 540
aatatacttg gatgggaatt tggaggaggt gctcctcaaa atggagctgc agaagataaa 600
aagacagaat atttactaga acaaataaaa attccatcat gggatagaaa taacatcccc 660
gatgagaatg aacaagtaat agaggaccct caagaagata ataaagatga agatgaagat 720

gaagaaacag aaacagaaaa tttggaaaca gaagatgata ataatgaaga gatagaagaa 780
aatgaagaag atgacataga tgaagaaagt gtagaagaaa aggaagaaga ggaagaaaaa 840
aaggaagaag aagaaaaaaa ggaagaaaaa aaagaagaaa aaaaaccaga caatgaaatt 900
acaaatgaag ttaaagagga acaaaaatat agttcaccaa gtgatataaa tgcccaaaat 960
ttaatttcta ataagaataa aaagaatgat gaaacaaaaa agactgctga aaatatagtt 1020
aaaacattgg ttggattatt taatgaaaaa aatgagatag attctactat aaataattta 1080
gtacaagaaa tgatccatct atttagtaat aattaa 1116

<210> 4
<211> 371
<212> PRT
<213> Plasmodium Falciparum

<220>
<221> MISC_FEATURE
<222> (1)..(371)
<223> MSP 3.2

<400> 4

Met Asn Lys Ile Tyr Asn Ile Thr Phe Leu Phe Ile Leu Leu Asn Leu
1 5 10 15

Tyr Ile Asn Glu Asn Asn Phe Ile Arg Asn Glu Leu Ile Asn Glu Lys
20 25 30

Asn His Asn Leu Arg Asn Gly Ser Met Tyr Asn Asn Asp Lys Ile Leu
35 40 45

Ser Lys Asn Glu Val Asp Thr Asn Ile Glu Ser Asn Glu Asn Ser Ile
50 55 60

His Glu Ser Gly His Lys Ile Asp Gly Glu Glu Val Leu Lys Ala Asn
65 70 75 80

Val Asp Asp Ile Thr Tyr Lys Lys Lys Asn Val Asp Asp Ser Glu Ile
85 90 95

Pro Phe Ser Gly Tyr Asp Ile Gln Ala Thr Tyr Gln Phe Pro Ser Thr
100 105 110

Ser Gly Gly Asn Asn Val Ile Pro Leu Pro Ile Lys Gln Ser Gly Glu
115 120 125

Asn Gln Tyr Thr Val Thr Ser Ile Ser Gly Ile Gln Lys Gly Ala Asn
130 135 140

Gly Leu Thr Gly Ala Thr Glu Asn Ile Thr Gln Val Val Gln Ala Asn
145 150 155 160

Ser Glu Thr Asn Lys Asn Pro Thr Ser His Ser Asn Ser Thr Thr Thr
165 170 175

Ser Leu Asn Asn Asn Ile Leu Gly Trp Glu Phe Gly Gly Gly Ala Pro
180 185 190

Gln Asn Gly Ala Ala Glu Asp Lys Lys Thr Glu Tyr Leu Leu Glu Gln
195 200 205

Ile Lys Ile Pro Ser Trp Asp Arg Asn Asn Ile Pro Asp Glu Asn Glu
210 215 220

Gln Val Ile Glu Asp Pro Gln Glu Asp Asn Lys Asp Glu Asp Glu Asp
225 230 235 240

Glu Glu Thr Glu Thr Glu Asn Leu Glu Thr Glu Asp Asp Asn Asn Glu
245 250 255

Glu Ile Glu Glu Asn Glu Glu Asp Asp Ile Asp Glu Glu Ser Val Glu
260 265 270

Glu Lys Glu Glu Glu Glu Glu Lys Lys Glu Glu Glu Glu Lys Lys Glu
275 280 285

Glu Lys Lys Glu Glu Lys Lys Pro Asp Asn Glu Ile Thr Asn Glu Val
290 295 300

Lys Glu Glu Gln Lys Tyr Ser Ser Pro Ser Asp Ile Asn Ala Gln Asn
305 310 315 320

Leu Ile Ser Asn Lys Asn Lys Lys Asn Asp Glu Thr Lys Lys Thr Ala
325 330 335

Glu Asn Ile Val Lys Thr Leu Val Gly Leu Phe Asn Glu Lys Asn Glu
340 345 350

Ile Asp Ser Thr Ile Asn Asn Leu Val Gln Glu Met Ile His Leu Phe
355 360 365

Ser Asn Asn
370

<210> 5
<211> 1275
<212> DNA
<213> Plasmodium Falciparum

<220>
<221> misc_feature
<222> (1)..(1275)
<223> MSP 3.3

<400> 5

```
atgaaaaaaa tcgtgaatat aatattttat atcttatatt tatatatata taaaagaaac 60
ttagtacaaa acgaaaatgt aaataaatct aatctaagaa aaggattatc tactaataat 120
tcagaaaatg gaataaaaag tctaaaggat gaagatgaac atattaatat tataggagat 180
gatttttcag cattttctta tggtggttat cctatttatg aaactacagg aagtctagga 240
actggggttg aaagtgtaaa agctattgat ggggaaagtg gtacttcaat ggattctaaa 300
cctaaagaga ataaaattag tactgaacca ggagcagacc aggtgtctat tggattggtc 360
aacgaatctg atagtagttt agaaaatgat aaaaaaaaaa aagaaaacgt aaaaaaagaa 420
atgcttggta ctgaaaagga aggttctcca gatagtcatg atagttctaa ggaaaaatta 480
aatcttaacg acaattccaa atggtctgat tttcttaaaa atatcgtaac gtttggtggt 540
tttggtccta ctgtggttca tgatgttagt gataccctttt cagatatatc taaagatgaa 600
gttactcaaa aaacaactaa agatattggt agtactttac ttgacttttt tttaccatta 660
ccgactaaaa acactaatac atatgagaag aaaaatgaaa ataaaaatgt atcaaatgta 720
gattcaaaaa caaaatcaaa tgaaaaagga agacctccta catattctcc tattctggat 780
gatgggatag aatttagtgg tggtttatat tttaatgaga aaaagtcgac tgaagaaaat 840
aaacaaaaaa atgttttaga atcagtaaat ttaacatcgt gggataaaga ggatattgtt 900
aaggaaaatg aggatgttaa agatgaaaag gatgaagatg atgaagaaga agaagaaaaa 960
tacgaaaacg aaattataaa gcaaccagaa gacatattgg atgaggaaga agtattagaa 1020
gaagaaatat tagaagaaaa taaaaatgat acagtagata caagtgattt agaaaagaaa 1080
aatataccag atttatcaaa cgataataat tattatagtt taatttataa gaactataag 1140
gataatgata aatcagaaaa aactgcacaa acattaatca cagctctgat aagtttatta 1200
aatggaaaaa atgaattaga tgctaccata agaagattaa aacataggtt tatggaattt 1260
tttacatata attaa 1275
```

<210> 6
<211> 424
<212> PRT
<213> Plasmodium Falciparum

<220>
<221> MISC_FEATURE
<222> (1)..(424)
<223> MSP 3.3

<400> 6

Met Lys Lys Ile Val Asn Ile Ile Phe Tyr Ile Leu Tyr Leu Tyr Ile
1 5 10 15

Tyr Lys Arg Asn Leu Val Gln Asn Glu Asn Val Asn Lys Ser Asn Leu
20 25 30

Arg Lys Gly Leu Ser Thr Asn Asn Ser Glu Asn Gly Ile Lys Ser Leu

35 40 45

Lys Asp Glu Asp Glu His Ile Asn Ile Ile Gly Asp Asp Phe Ser Ala
50 55 60

Phe Ser Tyr Gly Gly Tyr Pro Ile Tyr Glu Thr Thr Gly Ser Leu Gly
65 70 75 80

Thr Gly Val Glu Ser Val Lys Ala Ile Asp Gly Glu Ser Gly Thr Ser
85 90 95

Met Asp Ser Lys Pro Lys Glu Asn Lys Ile Ser Thr Glu Pro Gly Ala
100 105 110

Asp Gln Val Ser Ile Gly Leu Val Asn Glu Ser Asp Ser Ser Leu Glu
115 120 125

Asn Asp Lys Lys Lys Lys Glu Asn Val Lys Lys Glu Met Leu Gly Thr
130 135 140

Glu Lys Glu Gly Ser Pro Asp Ser His Asp Ser Ser Lys Glu Lys Leu
145 150 155 160

Asn Leu Asn Asp Asn Ser Lys Trp Ser Asp Phe Leu Lys Asn Ile Val
165 170 175

Thr Phe Gly Gly Phe Gly Pro Thr Val Val His Asp Val Ser Asp Thr
180 185 190

Leu Ser Asp Ile Ser Lys Asp Glu Val Thr Gln Lys Thr Thr Lys Asp
195 200 205

Ile Gly Ser Thr Leu Leu Asp Phe Phe Leu Pro Leu Pro Thr Lys Asn
210 215 220

Thr Asn Thr Tyr Glu Lys Lys Asn Glu Asn Lys Asn Val Ser Asn Val
225 230 235 240

Asp Ser Lys Thr Lys Ser Asn Glu Lys Gly Arg Pro Pro Thr Tyr Ser
245 250 255

Pro Ile Leu Asp Asp Gly Ile Glu Phe Ser Gly Gly Leu Tyr Phe Asn
260 265 270

Glu Lys Lys Ser Thr Glu Glu Asn Lys Gln Lys Asn Val Leu Glu Ser
275 280 285

Val Asn Leu Thr Ser Trp Asp Lys Glu Asp Ile Val Lys Glu Asn Glu
290 295 300

Asp Val Lys Asp Glu Lys Asp Glu Asp Asp Glu Glu Glu Glu Glu Lys

305 310 315 320

Tyr Glu Asn Glu Ile Ile Lys Gln Pro Glu Asp Ile Leu Asp Glu Glu
325 330 335

Glu Val Leu Glu Glu Glu Ile Leu Glu Glu Asn Lys Asn Asp Thr Val
340 345 350

Asp Thr Ser Asp Leu Glu Lys Lys Asn Ile Pro Asp Leu Ser Asn Asp
355 360 365

Asn Asn Tyr Tyr Ser Leu Ile Tyr Lys Asn Tyr Lys Asp Asn Asp Lys
370 375 380

Ser Glu Lys Thr Ala Gln Thr Leu Ile Thr Ala Leu Ile Ser Leu Leu
385 390 395 400

Asn Gly Lys Asn Glu Leu Asp Ala Thr Ile Arg Arg Leu Lys His Arg
405 410 415

Phe Met Glu Phe Phe Thr Tyr Asn
420

<210> 7
<211> 2094
<212> DNA
<213> Plasmodium Falciparum

<220>
<221> misc_feature
<222> (1)..(2094)
<223> MSP 3.4

<400> 7

atgaagaaaa tatatagtat tttcttttct ttatttattt tgaatcttca tatatatata 60
aaaaatatca aatgcaatga cctaataaat tataatgatt cgaatctaag aaacggatta 120
ctaaataata gtttagattt aacaaatgga ttaaataaca aagataacag ttttattgat 180
tctaaaattg aagaacatga aaataaatct taccaaaata aagataataa tatctctatc 240
gttggacaag atgtgcctat tacatcggta tattcttcta aaattataaa tgctaatgat 300
ttagaaggaa atagtattga cgatactaaa ggtcttagtg ttactaatag tggatttgat 360
gatggtagtg cttttggtgg tggactccct ttttctggtt attctcctct acaaggaaat 420
cataataaat gtcctgatga aaatttttgt aagggtatta aaaatgtctt atcctgtcct 480
ccaaaaaatt ctactggtag aaatggggat tggattagtg tggctgttaa agaaagttca 540
actacaaata aaggtgttct tgttcccccc agaagaacaa aattatgtct aagaaatatt 600
aacaaggttt ggcatcgaat caaagacgag aaaaatttta aagaagaatt tgttaaagtt 660
gctttaggag aatcaaatgc tttaatgaaa cattataaag aaaaaaatct gaatgccctt 720
acagctataa aatatggatt ttcagatatg ggagatataa taaagggaac agacctaatt 780

gactatcaaa ttactaaaaa tataaatagg gcattagata aaatattacg taatgaaaca 840
agtaatgaca aaattaaaaa acgtgtagac tggtgggaag ctaataaaag tgcattctgg 900
gatgcattca tgtgtggata taaagttcat atcggaaata aaccatgtcc agaacatgat 960
aatatggaca gaataccaca atatcttaga tggtttagag aatggggaac atatgtttgc 1020
agcgaatata aaaataagtt tgaggatgta ataaaattat gtaatatcca acaatttaca 1080
aaccaggatg attcacaact attagaaata tcaaaaaagg ataaatgtaa agaagcatta 1140
aagcattatg aagaatgggt taatagaagg agacctgaat ggaaaggcca atgtgataaa 1200
tttgaaaaag aaaaaagtaa atatgaagat actaaaagta taactgctga aaaatattta 1260
aaagaaatat gttctgaatg tgattgtaaa tataaagatt tggataatac atttaaagaa 1320
tttaaagata acgttacact tcttaaagca gtaattgata acaaaaaaaa tcaagattct 1380
ctaacaacca cttctttatc aacgtctatt aatagtgtta gggattctag taatctagat 1440
caacgaggga atataacaac atctcaagga aattcacacc gtgcaactgt tgtgcaacaa 1500
gttgatcaaa ccaacagatt agataatgta aactctgtaa cgcaaagagg aaataataac 1560
tacaacaata atttagagcg tggattgggt tctggtgctc ttcctggtac aaatattatt 1620
actgaagaaa aatattctct agaattaata aaattaacat caaaggatga agaagatatt 1680
ataaagcata atgaggatgt gagagaagaa atagaagaac aacaagaaga catcgaggaa 1740
gatgaagaag aattggaaaa tgaaggagaa gaaacaaaag aagaagatga tgaagaaaag 1800
aatgaaacaa atgatacgga agatacggac gatactgaag atacggaaga tatagaagag 1860
gaaaataagg aaaaagaact cagtaatcaa caacaaagtg aaaaaaaaag tatttcaaaa 1920
gttgacgaag attcatatcg aatactatca gtaagttata aggacaataa tgaagtaaaa 1980
aatgttgctg aatctatagt gaaaaaacta tttagtttat ttaatgataa taataatttg 2040
gaaactattt ttaagggttt gacagaagat atgacagatt tatttcaaaa ataa 2094

<210> 8
<211> 697
<212> PRT
<213> Plasmodium Falciparum

<220>
<221> MISC_FEATURE
<222> (1)..(697)
<223> MSP 3.4

<400> 8

Met Lys Lys Ile Tyr Ser Ile Phe Phe Ser Leu Phe Ile Leu Asn Leu
1 5 10 15

His Ile Tyr Ile Lys Asn Ile Lys Cys Asn Asp Leu Ile Asn Tyr Asn
20 25 30

Asp Ser Asn Leu Arg Asn Gly Leu Leu Asn Asn Ser Leu Asp Leu Thr

35 40 45

Asn Gly Leu Asn Asn Lys Asp Asn Ser Phe Ile Asp Ser Lys Ile Glu
50 55 60

Glu His Glu Asn Lys Ser Tyr Gln Asn Lys Asp Asn Asn Ile Ser Ile
65 70 75 80

Val Gly Gln Asp Val Pro Ile Thr Ser Val Tyr Ser Ser Lys Ile Ile
85 90 95

Asn Ala Asn Asp Leu Glu Gly Asn Ser Ile Asp Asp Thr Lys Gly Leu
100 105 110

Ser Val Thr Asn Ser Gly Phe Asp Asp Gly Ser Ala Phe Gly Gly Gly
115 120 125

Leu Pro Phe Ser Gly Tyr Ser Pro Leu Gln Gly Asn His Asn Lys Cys
130 135 140

Pro Asp Glu Asn Phe Cys Lys Gly Ile Lys Asn Val Leu Ser Cys Pro
145 150 155 160

Pro Lys Asn Ser Thr Gly Arg Asn Gly Asp Trp Ile Ser Val Ala Val
165 170 175

Lys Glu Ser Ser Thr Thr Asn Lys Gly Val Leu Val Pro Pro Arg Arg
180 185 190

Thr Lys Leu Cys Leu Arg Asn Ile Asn Lys Val Trp His Arg Ile Lys
195 200 205

Asp Glu Lys Asn Phe Lys Glu Glu Phe Val Lys Val Ala Leu Gly Glu
210 215 220

Ser Asn Ala Leu Met Lys His Tyr Lys Glu Lys Asn Leu Asn Ala Leu
225 230 235 240

Thr Ala Ile Lys Tyr Gly Phe Ser Asp Met Gly Asp Ile Ile Lys Gly
245 250 255

Thr Asp Leu Ile Asp Tyr Gln Ile Thr Lys Asn Ile Asn Arg Ala Leu
260 265 270

Asp Lys Ile Leu Arg Asn Glu Thr Ser Asn Asp Lys Ile Lys Lys Arg
275 280 285

Val Asp Trp Trp Glu Ala Asn Lys Ser Ala Phe Trp Asp Ala Phe Met
290 295 300

Cys Gly Tyr Lys Val His Ile Gly Asn Lys Pro Cys Pro Glu His Asp

305 310 315 320

Asn Met Asp Arg Ile Pro Gln Tyr Leu Arg Trp Phe Arg Glu Trp Gly
325 330 335

Thr Tyr Val Cys Ser Glu Tyr Lys Asn Lys Phe Glu Asp Val Ile Lys
340 345 350

Leu Cys Asn Ile Gln Gln Phe Thr Asn Gln Asp Asp Ser Gln Leu Leu
355 360 365

Glu Ile Ser Lys Lys Asp Lys Cys Lys Glu Ala Leu Lys His Tyr Glu
370 375 380

Glu Trp Val Asn Arg Arg Arg Pro Glu Trp Lys Gly Gln Cys Asp Lys
385 390 395 400

Phe Glu Lys Glu Lys Ser Lys Tyr Glu Asp Thr Lys Ser Ile Thr Ala
405 410 415

Glu Lys Tyr Leu Lys Glu Ile Cys Ser Glu Cys Asp Cys Lys Tyr Lys
420 425 430

Asp Leu Asp Asn Thr Phe Lys Glu Phe Lys Asp Asn Val Thr Leu Leu
435 440 445

Lys Ala Val Ile Asp Asn Lys Lys Asn Gln Asp Ser Leu Thr Thr Thr
450 455 460

Ser Leu Ser Thr Ser Ile Asn Ser Val Arg Asp Ser Ser Asn Leu Asp
465 470 475 480

Gln Arg Gly Asn Ile Thr Thr Ser Gln Gly Asn Ser His Arg Ala Thr
485 490 495

Val Val Gln Gln Val Asp Gln Thr Asn Arg Leu Asp Asn Val Asn Ser
500 505 510

Val Thr Gln Arg Gly Asn Asn Asn Tyr Asn Asn Asn Leu Glu Arg Gly
515 520 525

Leu Gly Ser Gly Ala Leu Pro Gly Thr Asn Ile Ile Thr Glu Glu Lys
530 535 540

Tyr Ser Leu Glu Leu Ile Lys Leu Thr Ser Lys Asp Glu Glu Asp Ile
545 550 555 560

Ile Lys His Asn Glu Asp Val Arg Glu Glu Ile Glu Glu Gln Gln Glu
565 570 575

Asp Ile Glu Glu Asp Glu Glu Glu Leu Glu Asn Glu Gly Glu Glu Thr

580 585 590

Lys Glu Glu Asp Asp Glu Glu Lys Asn Glu Thr Asn Asp Thr Glu Asp
595 600 605

Thr Asp Asp Thr Glu Asp Thr Glu Asp Ile Glu Glu Glu Asn Lys Glu
610 615 620

Lys Glu Leu Ser Asn Gln Gln Gln Ser Glu Lys Lys Ser Ile Ser Lys
625 630 635 640

Val Asp Glu Asp Ser Tyr Arg Ile Leu Ser Val Ser Tyr Lys Asp Asn
645 650 655

Asn Glu Val Lys Asn Val Ala Glu Ser Ile Val Lys Lys Leu Phe Ser
660 665 670

Leu Phe Asn Asp Asn Asn Asn Leu Glu Thr Ile Phe Lys Gly Leu Thr
675 680 685

Glu Asp Met Thr Asp Leu Phe Gln Lys
690 695

<210> 9
<211> 2139
<212> DNA
<213> Plasmodium Falciparum

<220>
<221> misc_feature
<222> (1)..(2139)
<223> MSP 3.5

<400> 9

```
atgaaatata tattaagtat tagtcttttt ttaattcttt taaatttata taaatgtgct     60
agtatatcat gtgaaataga ttacacccct agtaccaata ttactagcaa tttaaattct    120
aatttaagaa gctgttcttc taatcttgtt ctttcttcaa atatagattc tactaaatta    180
gaaggaacat atggtgaaaa tttaaacgac agtgtatctt tgacgaataa tattaatgat    240
catacaacta acgaatcaaa tatatcaaat gtatcaaata tatcaaatga atcaaatata    300
tcaaatgaat caagtataac taaccagtca aatttatcta gcgaaacaaa tatatctagc    360
gaaacaaata tatctaacga atcaagtgta ccaaatgaaa gcagtgtcaa tgaggtacct    420
caacttgaag tacctaaaga tgcagtagaa aatcacacag aatccaaaga tgttttattg    480
aatgaaaagg aaaattttgc aaatggagtg gaaacacatg ttgatctagg atcccaagaa    540
caatattttg gatcttccta tgatatgaat atggacacag aaggaggaat aaaaaagttc    600
aaaaatgtgt ttcagtctta ttttaatcaa tccaaaggaa atagtggtac tgaaggtgat    660
ggatcaagtg tatttggatc catatttggt agcttattaa cccctataga ttcattgtta    720
gaaaaattta taggatctaa caatacaaat tcggattcta atgtgaagaa cacttctatg    780
```

```
ggaaatggac aaaataaata cgacaataat atatacttag atgaagaaga tgctttgagt    840
gatgcagaac attataatga tggtagtata agtttaggcg aagaagatga gttgagtgat    900
gcagaacatt ataatgatgg aagtataagt ttagatgaag aagatgagtt gagtgatgca    960
gaacattata atgatggagg tatatgttta ggtgaagaag atgagttaag tgatgcagaa   1020
cattataatg atggaggtat aagtttagat gaagaagatg tgttgagtga tgcagaacat   1080
tataatgatg gagatataag tttagatgaa gatgagttaa gtgatacaga aaattattat   1140
gatggaggta taagtttaga cgaatcagat gatttgagtg accccgaaag taaaacaaaa   1200
gaagataact accatttata ttattgggat gatttctatc atgaatataa accaacttat   1260
ttaaattatc atatgcatta tacactttat gaaccaaaca atttttatga tactactaat   1320
gaagaaacac acaattttta taatactact aatgaagaat cacacaattt ttacaaccct   1380
actcatgaag aatcacacaa tttttacaac cctactcatg aagaatcaca caatttttac   1440
aaccctactc atgaagaatc acacaatttt tacaacccta ctcatgaaga atcacacaat   1500
ttttacaacc ctactcatga agaatcacac aatttttaca accctactca tgaagaatca   1560
cacaattttt acaaccctac tcatgaagaa tcacacaatt tttacaaccc tactcatgaa   1620
gaatcacaca atttttacaa ccctactcat gaagaatcac acaattttta caaccctact   1680
catgaagaat cacacaattt ttacaaccct actcatgaag aatcacacaa tttttacacc   1740
cctactcatg aagaatcaca caatttttac aaccctactc atgaagaatc acacaatttt   1800
tacaacccta ctcatgaaga atcacacaat ttttacaacc ctactcatga agaatcacac   1860
aatttttaca accctactca tgaagaatca cacaattttt acaaccctac tcatgaagaa   1920
tcacacaatt tttacacccc tactcatgat gaatttaatg ttcctttaaa ttataaccat   1980
gactacgatt acaattactt tgaaaatgat aattataata ttcaaaatgt taaagacaat   2040
ttggtaaaaa aagttaatga tttcatggaa tcagataatt tattagttaa tacctttaaa   2100
ggtatagctg ggggtgttac tagtttttc ggatattaa                           2139
```

<210> 10
<211> 712
<212> PRT
<213> Plasmodium Falciparum

<220>
<221> MISC_FEATURE
<222> (1)..(712)
<223> MSP 3.5

<400> 10

```
Met Lys Tyr Ile Leu Ser Ile Ser Leu Phe Leu Ile Leu Leu Asn Leu
1               5                   10                  15

Tyr Lys Cys Ala Ser Ile Ser Cys Glu Ile Asp Tyr Thr Pro Ser Thr
            20                  25                  30
```

```
Asn Ile Thr Ser Asn Leu Asn Ser Asn Leu Arg Ser Cys Ser Ser Asn
        35                  40                  45

Leu Val Leu Ser Ser Asn Ile Asp Ser Thr Lys Leu Glu Gly Thr Tyr
    50                  55                  60

Gly Glu Asn Leu Asn Asp Ser Val Ser Leu Thr Asn Asn Ile Asn Asp
65                  70                  75                  80

His Thr Thr Asn Glu Ser Asn Ile Ser Asn Val Ser Asn Ile Ser Asn
                85                  90                  95

Glu Ser Asn Ile Ser Asn Glu Ser Ser Ile Thr Asn Gln Ser Asn Leu
            100                 105                 110

Ser Ser Glu Thr Asn Ile Ser Ser Glu Thr Asn Ile Ser Asn Glu Ser
        115                 120                 125

Ser Val Pro Asn Glu Ser Ser Val Asn Glu Val Pro Gln Leu Glu Val
    130                 135                 140

Pro Lys Asp Ala Val Glu Asn His Thr Glu Ser Lys Asp Val Leu Leu
145                 150                 155                 160

Asn Glu Lys Glu Asn Phe Ala Asn Gly Val Glu Thr His Val Asp Leu
                165                 170                 175

Gly Ser Gln Glu Gln Tyr Phe Gly Ser Ser Tyr Asp Met Asn Met Asp
            180                 185                 190

Thr Glu Gly Gly Ile Lys Lys Phe Lys Asn Val Phe Gln Ser Tyr Phe
        195                 200                 205

Asn Gln Ser Lys Gly Asn Ser Gly Thr Glu Gly Asp Gly Ser Ser Val
    210                 215                 220

Phe Gly Ser Ile Phe Gly Ser Leu Leu Thr Pro Ile Asp Ser Leu Leu
225                 230                 235                 240

Glu Lys Phe Ile Gly Ser Asn Asn Thr Asn Ser Asp Ser Asn Val Lys
                245                 250                 255

Asn Thr Ser Met Gly Asn Gly Gln Asn Lys Tyr Asp Asn Asn Ile Tyr
            260                 265                 270

Leu Asp Glu Glu Asp Ala Leu Ser Asp Ala Glu His Tyr Asn Asp Gly
        275                 280                 285

Ser Ile Ser Leu Gly Glu Glu Asp Glu Leu Ser Asp Ala Glu His Tyr
    290                 295                 300
```

Asn Asp Gly Ser Ile Ser Leu Asp Glu Glu Asp Glu Leu Ser Asp Ala
305 310 315 320

Glu His Tyr Asn Asp Gly Gly Ile Cys Leu Gly Glu Glu Asp Glu Leu
325 330 335

Ser Asp Ala Glu His Tyr Asn Asp Gly Gly Ile Ser Leu Asp Glu Glu
340 345 350

Asp Val Leu Ser Asp Ala Glu His Tyr Asn Asp Gly Asp Ile Ser Leu
355 360 365

Asp Glu Asp Glu Leu Ser Asp Thr Glu Asn Tyr Tyr Asp Gly Gly Ile
370 375 380

Ser Leu Asp Glu Ser Asp Asp Leu Ser Asp Pro Glu Ser Lys Thr Lys
385 390 395 400

Glu Asp Asn Tyr His Leu Tyr Tyr Trp Asp Asp Phe Tyr His Glu Tyr
405 410 415

Lys Pro Thr Tyr Leu Asn Tyr His Met His Tyr Thr Leu Tyr Glu Pro
420 425 430

Asn Asn Phe Tyr Asp Thr Thr Asn Glu Glu Thr His Asn Phe Tyr Asn
435 440 445

Thr Thr Asn Glu Glu Ser His Asn Phe Tyr Asn Pro Thr His Glu Glu
450 455 460

Ser His Asn Phe Tyr Asn Pro Thr His Glu Glu Ser His Asn Phe Tyr
465 470 475 480

Asn Pro Thr His Glu Glu Ser His Asn Phe Tyr Asn Pro Thr His Glu
485 490 495

Glu Ser His Asn Phe Tyr Asn Pro Thr His Glu Glu Ser His Asn Phe
500 505 510

Tyr Asn Pro Thr His Glu Glu Ser His Asn Phe Tyr Asn Pro Thr His
515 520 525

Glu Glu Ser His Asn Phe Tyr Asn Pro Thr His Glu Glu Ser His Asn
530 535 540

Phe Tyr Asn Pro Thr His Glu Glu Ser His Asn Phe Tyr Asn Pro Thr
545 550 555 560

His Glu Glu Ser His Asn Phe Tyr Asn Pro Thr His Glu Glu Ser His
565 570 575

Asn Phe Tyr Thr Pro Thr His Glu Glu Ser His Asn Phe Tyr Asn Pro
580 585 590

Thr His Glu Glu Ser His Asn Phe Tyr Asn Pro Thr His Glu Glu Ser
595 600 605

His Asn Phe Tyr Asn Pro Thr His Glu Glu Ser His Asn Phe Tyr Asn
610 615 620

Pro Thr His Glu Glu Ser His Asn Phe Tyr Asn Pro Thr His Glu Glu
625 630 635 640

Ser His Asn Phe Tyr Thr Pro Thr His Asp Glu Phe Asn Val Pro Leu
645 650 655

Asn Tyr Asn His Asp Tyr Asp Tyr Asn Tyr Phe Glu Asn Asp Asn Tyr
660 665 670

Asn Ile Gln Asn Val Lys Asp Asn Leu Val Lys Lys Val Asn Asp Phe
675 680 685

Met Glu Ser Asp Asn Leu Leu Val Asn Thr Phe Lys Gly Ile Ala Gly
690 695 700

Gly Val Thr Ser Phe Phe Gly Tyr
705 710

<210> 11
<211> 1701
<212> DNA
<213> Plasmodium Falciparum

<220>
<221> misc_feature
<222> (1)..(1701)
<223> MSP 3.6

<400> 11

```
atgttgaata tttttaatat aattttcttg ttgtttttaa taaacatata tatatgtgaa     60
gccaatggaa cactctctga aaatattgaa agtgctgaag agatagatgc tttaaaaacg    120
aatttaagaa atggatattt aaataatact tattttaatg aagaaaacaa taatttaaat    180
atagaaaatg aaataaataa tacaaattat aatgaagtaa cagaagaaac taaagaagaa    240
ttatatgata taaatgaaaa tattttccct gattattttt ttcttgatat ctttactgaa    300
aataaagaac aaaaaaatga agaagtacca atgaaaatag aagtagtaaa tgatggagaa    360
gaagtaaaaa cagaatatgt atctgaaaaa aatgaggaag tagaaaataa atcggaaact    420
gaaataggtg aagaattaac tgaaaaagta gatgaaaaag tacctgaaga agtagctgaa    480
gaattagttg aaaaagtaga tgaagaagta gctgaagaat tagttgaaaa agtagatgaa    540
aaagtagctg aagaagtaga tcaaaaagta gatgaagaag taactgaaga attaattgaa    600

aaagtagatg aagaagtaac tgaagaatta attgaaaaag tagatgaaga agttgctgaa    660
gaattaattg aaaaagtaga tgaagaagtt gctgaagaat taattgaaaa ggtagctgat    720
gaattaattg aaaaagtaga tgaagaagtt gctgaagaat taattgaaaa ggtagctgat    780
gaattagttg aaaaagtagc tgaagaatta gttgaaaaag tagatgaaga agtagctgaa    840
gaattagttg aaaaagtaga tgaaaaagta gctgaagaag tagatcaaaa agtagatgaa    900
gaagtaactg aagaattaat tgaaaaagta gatgaagaag taactgaaga attaattgaa    960
aaagtagatg aagaagttgc tgaagaatta attgaaaaag tagatgaaga agttgctgaa   1020
gaattaattg aaaaggtagc tgatgaatta gttgaaaaag tagctgaaga attagttgaa   1080
aaagtagatg aacaagtagc tgaagaatta gttgaaaaag tagatgaaca agtagctgaa   1140
gaattagttg aaaaagtaga tgaacaagta gttgaagaag tagctgaaga agtagctgaa   1200
gaagtagttg aagaaggtga aaaagtacct gaagaagtag ctgaagaagt agctgaagaa   1260
gtagctgaag aagtagctga agaagtagct gaagaattag ttgaaaaagt agatgaagaa   1320
gtagctgaaa aagtagttga agaagaaggt gaaaaagtac ctgaagaagt agttgaagaa   1380
gtagatgaag aagtagctga aaaagtagtt gaagaagaag gtgaaaaagt acttgaagaa   1440
gtaattgaag aagtagttga agaagtagcc gaagaagtag ctgaaaaagt agttgaagaa   1500
caaggtgaaa aagtaaacaa aaatgattta aatgatgcat cttccgagga aattaaggat   1560
tctagtgatt ttaaagaatc tcatgaggaa ttatttaaag ttttcctgga gttaattaat   1620
aaaaacgatt tagttaaaga aaatttaaaa aagattacaa acaatttaaa tgaaatgcat   1680
ttaagcactt tatatccata a                                             1701
```

<210> 12
<211> 566
<212> PRT
<213> Plasmodium Falciparum

<220>
<221> MISC_FEATURE
<222> (1)..(566)

<223> MSP 3.6

<400> 12

```
Met Leu Asn Ile Phe Asn Ile Ile Phe Leu Leu Phe Leu Ile Asn Ile
1               5                   10                  15

Tyr Ile Cys Glu Ala Asn Gly Thr Leu Ser Glu Asn Ile Glu Ser Ala
            20                  25                  30

Glu Glu Ile Asp Ala Leu Lys Thr Asn Leu Arg Asn Gly Tyr Leu Asn
        35                  40                  45

Asn Thr Tyr Phe Asn Glu Glu Asn Asn Asn Leu Asn Ile Glu Asn Glu
    50                  55                  60
```

Ile Asn Asn Thr Asn Tyr Asn Glu Val Thr Glu Glu Thr Lys Glu Glu
65 70 75 80

Leu Tyr Asp Ile Asn Glu Asn Ile Phe Pro Asp Tyr Phe Phe Leu Asp
85 90 95

Ile Phe Thr Glu Asn Lys Glu Gln Lys Asn Glu Glu Val Pro Met Lys
100 105 110

Ile Glu Val Val Asn Asp Gly Glu Glu Val Lys Thr Glu Tyr Val Ser
115 120 125

Glu Lys Asn Glu Glu Val Glu Asn Lys Ser Glu Thr Glu Ile Gly Glu
130 135 140

Glu Leu Thr Glu Lys Val Asp Glu Lys Val Pro Glu Glu Val Ala Glu
145 150 155 160

Glu Leu Val Glu Lys Val Asp Glu Glu Val Ala Glu Glu Leu Val Glu
165 170 175

Lys Val Asp Glu Lys Val Ala Glu Glu Val Asp Gln Lys Val Asp Glu
180 185 190

Glu Val Thr Glu Glu Leu Ile Glu Lys Val Asp Glu Glu Val Thr Glu
195 200 205

Glu Leu Ile Glu Lys Val Asp Glu Glu Val Ala Glu Glu Leu Ile Glu
210 215 220

Lys Val Asp Glu Glu Val Ala Glu Glu Leu Ile Glu Lys Val Ala Asp
225 230 235 240

Glu Leu Ile Glu Lys Val Asp Glu Glu Val Ala Glu Glu Leu Ile Glu
245 250 255

Lys Val Ala Asp Glu Leu Val Glu Lys Val Ala Glu Glu Leu Val Glu
260 265 270

Lys Val Asp Glu Glu Val Ala Glu Glu Leu Val Glu Lys Val Asp Glu
275 280 285

Lys Val Ala Glu Glu Val Asp Gln Lys Val Asp Glu Glu Val Thr Glu
290 295 300

Glu Leu Ile Glu Lys Val Asp Glu Glu Val Thr Glu Glu Leu Ile Glu
305 310 315 320

Lys Val Asp Glu Glu Val Ala Glu Glu Leu Ile Glu Lys Val Asp Glu
325 330 335

Glu Val Ala Glu Glu Leu Ile Glu Lys Val Ala Asp Glu Leu Val Glu
340 345 350

Lys Val Ala Glu Glu Leu Val Glu Lys Val Asp Glu Gln Val Ala Glu
355 360 365

Glu Leu Val Glu Lys Val Asp Glu Gln Val Ala Glu Glu Leu Val Glu
370 375 380

Lys Val Asp Glu Gln Val Val Glu Glu Val Ala Glu Glu Val Ala Glu
385 390 395 400

Glu Val Val Glu Glu Gly Glu Lys Val Pro Glu Glu Val Ala Glu Glu
405 410 415

Val Ala Glu Glu Val Ala Glu Glu Val Ala Glu Glu Val Ala Glu Glu
420 425 430

Leu Val Glu Lys Val Asp Glu Glu Val Ala Glu Lys Val Val Glu Glu
435 440 445

Glu Gly Glu Lys Val Pro Glu Glu Val Val Glu Glu Val Asp Glu Glu
450 455 460

Val Ala Glu Lys Val Val Glu Glu Glu Gly Glu Lys Val Leu Glu Glu
465 470 475 480

Val Ile Glu Glu Val Val Glu Glu Val Ala Glu Glu Val Ala Glu Lys
485 490 495

Val Val Glu Glu Gln Gly Glu Lys Val Asn Lys Asn Asp Leu Asn Asp
500 505 510

Ala Ser Ser Glu Glu Ile Lys Asp Ser Ser Asp Phe Lys Glu Ser His
515 520 525

Glu Glu Leu Phe Lys Val Phe Leu Glu Leu Ile Asn Lys Asn Asp Leu
530 535 540

Val Lys Glu Asn Leu Lys Lys Ile Thr Asn Asn Leu Asn Glu Met His
545 550 555 560

Leu Ser Thr Leu Tyr Pro
565

<210> 13
<211> 1218
<212> DNA
<213> Plasmodium Falciparum

<220>
<221> misc_feature

<222> (1)..(1218)
<223> MSP 3.7

<400> 13

atgaataagt ttttgaatat tatattttac atttttctaa tattaaattt ctctttcttc 60
caaagcaatg ccacaagtaa ggaaattcaa aaagatgaac aaaagaattt aagaaatggt 120
tcttcaataa ataataacaa aaatatagaa aataaaaatg ataatattga aactcaatat 180
gaagcttcag aatatataga aaaacaaaat gacattttaa atatgtataa tgatgaaaaa 240
gagaaaaata ataataattc attagataca aatgtaacaa aaaatactgt aattgataat 300
tcaaataaat ttcaatcaat tgaagacaat aatgtataca ataaaggtat atttgtaggt 360
actgggataa aattaaatga ttcacaaact acatctgata attacaaaaa tgaacgatat 420
caaatagacg atgaaaaatt gaagtatgga gggtcgtttg acacaatttt ttcaggtttt 480
gttaatttat taacaccatc aagtcctact caaaacgatg gatctacagg aagaaatgta 540
ccacctccta gtgaacctaa tgttgataca ccagatcctc caacagcacc cgcacctgta 600
aaggtacctg aagatgcaaa attatcaagt tctcctagac ctgaaggacc aagagcaaac 660
aatagaaatg aaaataatca aaatacagat ccatataacc actattttgc atgggaaatt 720
ggaggtggtg ctccaacgta taaacccgag aacaataaga acgataatat tttgctagaa 780
cacgtaaaaa ttacctcgtg ggataaagaa gatataatta aagaaaatga agacacaaaa 840
cgcgaagttc aagaaactga agacactgac gaaactgaag atactgacga aactgaagaa 900
acagaagata tggaagatga aaacgaaatt gtggaagatc aattacaaga aaatgaagat 960
gatgaggata atgtaaattt agaagatatt aataaaaata ctagaaatga tatatttgaa 1020
gaacaaataa aattagattc tacgcaagat gacaaagctc aaaaattaat ttctaatgaa 1080
tataaaaaaa ctgaagaaaa aaaatcatta gaagatcatg taaatctatt atttaatttt 1140
ttacaaacaa ataaccaact agatccttca ctaaaagatt tagaaaatga gttaactttt 1200
tttttaaata actattga 1218

<210> 14
<211> 405
<212> PRT
<213> Plasmodium Falciparum

<220>
<221> MISC_FEATURE
<222> (1)..(405)
<223> MSP 3.7

<400> 14

```
Met Asn Lys Phe Leu Asn Ile Ile Phe Tyr Ile Phe Leu Ile Leu Asn
1               5                   10                  15

Phe Ser Phe Phe Gln Ser Asn Ala Thr Ser Lys Glu Ile Gln Lys Asp
            20                  25                  30
```

Glu Gln Lys Asn Leu Arg Asn Gly Ser Ser Ile Asn Asn Asn Lys Asn
35 40 45

Ile Glu Asn Lys Asn Asp Asn Ile Glu Thr Gln Tyr Glu Ala Ser Glu
50 55 60

Tyr Ile Glu Lys Gln Asn Asp Ile Leu Asn Met Tyr Asn Asp Glu Lys
65 70 75 80

Glu Lys Asn Asn Asn Asn Ser Leu Asp Thr Asn Val Thr Lys Asn Thr
85 90 95

Val Ile Asp Asn Ser Asn Lys Phe Gln Ser Ile Glu Asp Asn Asn Val
100 105 110

Tyr Asn Lys Gly Ile Phe Val Gly Thr Gly Ile Lys Leu Asn Asp Ser
115 120 125

Gln Thr Thr Ser Asp Asn Tyr Lys Asn Glu Arg Tyr Gln Ile Asp Asp
130 135 140

Glu Lys Leu Lys Tyr Gly Gly Ser Phe Asp Thr Ile Phe Ser Gly Phe
145 150 155 160

Val Asn Leu Leu Thr Pro Ser Ser Pro Thr Gln Asn Asp Gly Ser Thr
165 170 175

Gly Arg Asn Val Pro Pro Pro Ser Glu Pro Asn Val Asp Thr Pro Asp
180 185 190

Pro Pro Thr Ala Pro Ala Pro Val Lys Val Pro Glu Asp Ala Lys Leu
195 200 205

Ser Ser Ser Pro Arg Pro Glu Gly Pro Arg Ala Asn Asn Arg Asn Glu
210 215 220

Asn Asn Gln Asn Thr Asp Pro Tyr Asn His Tyr Phe Ala Trp Glu Ile
225 230 235 240

Gly Gly Gly Ala Pro Thr Tyr Lys Pro Glu Asn Asn Lys Asn Asp Asn
245 250 255

Ile Leu Leu Glu His Val Lys Ile Thr Ser Trp Asp Lys Glu Asp Ile
260 265 270

Ile Lys Glu Asn Glu Asp Thr Lys Arg Glu Val Gln Glu Thr Glu Asp
275 280 285

Thr Asp Glu Thr Glu Asp Thr Asp Glu Thr Glu Glu Thr Glu Asp Met
290 295 300

Glu Asp Glu Asn Glu Ile Val Glu Asp Gln Leu Gln Glu Asn Glu Asp
305 310 315 320

Asp Glu Asp Asn Val Asn Leu Glu Asp Ile Asn Lys Asn Thr Arg Asn
325 330 335

Asp Ile Phe Glu Glu Gln Ile Lys Leu Asp Ser Thr Gln Asp Asp Lys
340 345 350

Ala Gln Lys Leu Ile Ser Asn Glu Tyr Lys Lys Thr Glu Glu Lys Lys
355 360 365

Ser Leu Glu Asp His Val Asn Leu Leu Phe Asn Phe Leu Gln Thr Asn
370 375 380

Asn Gln Leu Asp Pro Ser Leu Lys Asp Leu Glu Asn Glu Leu Thr Phe
385 390 395 400

Phe Leu Asn Asn Tyr
405

<210> 15
<211> 2289
<212> DNA
<213> Plasmodium Falciparum

<220>
<221> misc_feature
<222> (1)..(2289)
<223> MSP 3.8

<400> 15

```
atgatatata ttttatctat tgtattttat atattttttt tacatattga tatatatgta     60
aacatttatt ctacatgttt tgttgtaaat gaggggaacc ctaatttaag aaataacata    120
attaatgatg atgaactaaa ggggaaagca tataataata ctatagatgc taataaccaa    180
aatatagaat ataataaaaa cttaaagcac aatgtaaact catctcatat atctaaattt    240
tcggatatta tggatcaaga agataaagga gataatgaaa attctcatga cataaaattt    300
gaagaaaaaa aaaatattaa taaatcttta gacgctgaat ccaattatgg tattaatgaa    360
attagtatta ctggtaatga tagtaatagt gataatagta atcagaatat ttttccagat    420
ggtagtgaat tagctggagg tattcctcgt tctatatata ctattaacct tggttttaat    480
aaatgtccta ctgaagagat ttgtaaagac tttagtaatc ttccacaatg tcgaaagaat    540
gtacatgaaa gaaataattg gttgggctca agtgtaaaaa atttttcaag tgataataag    600
ggggttcttg ttcctccaag aagacaatct ttatgtttaa gaattacatt acaagatttt    660
cgtacgaaaa agaaaaagga aggagatttt gaaaaattta tttattcata tgcatcatct    720
gaagctagaa aattaagaac catacacaat aataacttag aaaaagctca tcaagctata    780
agatatagtt ttgcagatat tggaaatatt attagaggag atgacatgat ggatacacct    840
```

```
acgtcaaaag aaaccataac atatttagaa aaagtactta aaatttataa tgaaaataat    900
gataaaccaa aagatgcaaa aaaatggtgg acagaaaaca ggcatcatgt ttgggaagca    960
atgatgtgcg gatatcagag tgcgcagaaa gataaccaat gtacaggtta tggtaacatt   1020
gatgatatac cacaattttt aaggtggttc agagagtggg gaacatatgt ctgtgaagaa   1080
agcgaaaaaa atatgaacac actaaaagct gtttgctttc cgaaacagcc aagaaccgaa   1140
gcgaatcctg cattgactgt acatgaaaat gaaatgtgct catcaacttt aaaaaaatat   1200
gaagaatggt ataataaaag gaaaactgaa tggactgaac aatctattaa atataacaat   1260
gacaaaatta attatacaga tataaaaaca ttatctcctt ctgaatattt aatagaaaaa   1320
tgtcctgaat gtaaatgtac caaaaaaaat ttgcaagatg tatttgaact tacatttgat   1380
ggaaaagctt tattagaaaa gctaaaaaaa gaagaatcac ctgtgagtaa tagtgtgaat   1440
gccttacctg aaccaggtca aattacatta cctgatcctt cattaaaaca aacaacacaa   1500
caggaaaatc aacctgttgt agaaacacct gttaccacag ctgttattaa tgaacatcaa   1560
ggacaaacag aaccgaataa aggtgacaac aataatgaaa gagaaaatca tgaaagtaat   1620
gttggtagca tccaagaagt aaaccaaggt agcgtgagcg aagaatcaca ttctaaaact   1680
atagatcctt ctaagattga cgaccgtttg gaattaagta gtgggtcatc atctcttgaa   1740
caacactcta aggaagatgt aaaaaaggga tgtgctttag aattggtacc tttatcttta   1800
tcggatattg aacagatagc taatgaaagc gaagatgtac tggaagagat agaagaagaa   1860
attaatacag atggggaaat agaatatata acagaagaag aaataaaaga agatatagaa   1920
gaagaaacag aagaagatat agaagaagaa acagaagaag aaacagaaga agaaacagaa   1980
gaagaagcag atgaagaaac agtaaaagaa atagaagaca aaccagaaca agaaattaaa   2040
aataaatcgc tagaagaaaa acaaatagat aaaaatacag ataccagtga aaagaaagga   2100
tttaataatt cagaaaaaga tgaaaaagct cgaaatttaa tttctaaaaa ttataaaaat   2160
tataatgaac tagataaaaa cgttcatact ttagtaaatt caattattag tttattagaa   2220
gaaggtaatg gaagtgattc taccttgaat agtttatcaa aagatattac aaatttattt   2280
aaaaattaa                                                           2289
```

<210> 16
<211> 762
<212> PRT
<213> Plasmodium Falciparum

<220>
<221> MISC_FEATURE
<222> (1)..(762)
<223> MSP 3.8

<400> 16

```
Met Ile Tyr Ile Leu Ser Ile Val Phe Tyr Ile Phe Phe Leu His Ile
1               5                   10                  15
```

Asp Ile Tyr Val Asn Ile Tyr Ser Thr Cys Phe Val Val Asn Glu Gly
20 25 30

Asn Pro Asn Leu Arg Asn Asn Ile Ile Asn Asp Asp Glu Leu Lys Gly
35 40 45

Lys Ala Tyr Asn Asn Thr Ile Asp Ala Asn Asn Gln Asn Ile Glu Tyr
50 55 60

Asn Lys Asn Leu Lys His Asn Val Asn Ser Ser His Ile Ser Lys Phe
65 70 75 80

Ser Asp Ile Met Asp Gln Glu Asp Lys Gly Asp Asn Glu Asn Ser His
85 90 95

Asp Ile Lys Phe Glu Glu Lys Lys Asn Ile Asn Lys Ser Leu Asp Ala
100 105 110

Glu Ser Asn Tyr Gly Ile Asn Glu Ile Ser Ile Thr Gly Asn Asp Ser
115 120 125

Asn Ser Asp Asn Ser Asn Gln Asn Ile Phe Pro Asp Gly Ser Glu Leu
130 135 140

Ala Gly Gly Ile Pro Arg Ser Ile Tyr Thr Ile Asn Leu Gly Phe Asn
145 150 155 160

Lys Cys Pro Thr Glu Glu Ile Cys Lys Asp Phe Ser Asn Leu Pro Gln
165 170 175

Cys Arg Lys Asn Val His Glu Arg Asn Asn Trp Leu Gly Ser Ser Val
180 185 190

Lys Asn Phe Ser Ser Asp Asn Lys Gly Val Leu Val Pro Pro Arg Arg
195 200 205

Gln Ser Leu Cys Leu Arg Ile Thr Leu Gln Asp Phe Arg Thr Lys Lys
210 215 220

Lys Lys Glu Gly Asp Phe Glu Lys Phe Ile Tyr Ser Tyr Ala Ser Ser
225 230 235 240

Glu Ala Arg Lys Leu Arg Thr Ile His Asn Asn Asn Leu Glu Lys Ala
245 250 255

His Gln Ala Ile Arg Tyr Ser Phe Ala Asp Ile Gly Asn Ile Ile Arg
260 265 270

Gly Asp Asp Met Met Asp Thr Pro Thr Ser Lys Glu Thr Ile Thr Tyr
275 280 285

Leu Glu Lys Val Leu Lys Ile Tyr Asn Glu Asn Asn Asp Lys Pro Lys
290 295 300

Asp Ala Lys Lys Trp Trp Thr Glu Asn Arg His His Val Trp Glu Ala
305 310 315 320

Met Met Cys Gly Tyr Gln Ser Ala Gln Lys Asp Asn Gln Cys Thr Gly
325 330 335

Tyr Gly Asn Ile Asp Asp Ile Pro Gln Phe Leu Arg Trp Phe Arg Glu
340 345 350

Trp Gly Thr Tyr Val Cys Glu Glu Ser Glu Lys Asn Met Asn Thr Leu
355 360 365

Lys Ala Val Cys Phe Pro Lys Gln Pro Arg Thr Glu Ala Asn Pro Ala
370 375 380

Leu Thr Val His Glu Asn Glu Met Cys Ser Ser Thr Leu Lys Lys Tyr
385 390 395 400

Glu Glu Trp Tyr Asn Lys Arg Lys Thr Glu Trp Thr Glu Gln Ser Ile
405 410 415

Lys Tyr Asn Asn Asp Lys Ile Asn Tyr Thr Asp Ile Lys Thr Leu Ser
420 425 430

Pro Ser Glu Tyr Leu Ile Glu Lys Cys Pro Glu Cys Lys Cys Thr Lys
435 440 445

Lys Asn Leu Gln Asp Val Phe Glu Leu Thr Phe Asp Gly Lys Ala Leu
450 455 460

Leu Glu Lys Leu Lys Lys Glu Glu Ser Pro Val Ser Asn Ser Val Asn
465 470 475 480

Ala Leu Pro Glu Pro Gly Gln Ile Thr Leu Pro Asp Pro Ser Leu Lys
485 490 495

Gln Thr Thr Gln Gln Glu Asn Gln Pro Val Val Glu Thr Pro Val Thr
500 505 510

Thr Ala Val Ile Asn Glu His Gln Gly Gln Thr Glu Pro Asn Lys Gly
515 520 525

Asp Asn Asn Asn Glu Arg Glu Asn His Glu Ser Asn Val Gly Ser Ile
530 535 540

Gln Glu Val Asn Gln Gly Ser Val Ser Glu Glu Ser His Ser Lys Thr
545 550 555 560

Ile Asp Pro Ser Lys Ile Asp Asp Arg Leu Glu Leu Ser Ser Gly Ser
565 570 575

Ser Ser Leu Glu Gln His Ser Lys Glu Asp Val Lys Lys Gly Cys Ala
580 585 590

Leu Glu Leu Val Pro Leu Ser Leu Ser Asp Ile Glu Gln Ile Ala Asn
595 600 605

Glu Ser Glu Asp Val Leu Glu Glu Ile Glu Glu Glu Ile Asn Thr Asp
610 615 620

Gly Glu Ile Glu Tyr Ile Thr Glu Glu Glu Ile Lys Glu Asp Ile Glu
625 630 635 640

Glu Glu Thr Glu Glu Asp Ile Glu Glu Glu Thr Glu Glu Glu Thr Glu
645 650 655

Glu Glu Thr Glu Glu Glu Ala Asp Glu Glu Thr Val Lys Glu Ile Glu
660 665 670

Asp Lys Pro Glu Gln Glu Ile Lys Asn Lys Ser Leu Glu Glu Lys Gln
675 680 685

Ile Asp Lys Asn Thr Asp Thr Ser Glu Lys Lys Gly Phe Asn Asn Ser
690 695 700

Glu Lys Asp Glu Lys Ala Arg Asn Leu Ile Ser Lys Asn Tyr Lys Asn
705 710 715 720

Tyr Asn Glu Leu Asp Lys Asn Val His Thr Leu Val Asn Ser Ile Ile
725 730 735

Ser Leu Leu Glu Glu Gly Asn Gly Ser Asp Ser Thr Leu Asn Ser Leu
740 745 750

Ser Lys Asp Ile Thr Asn Leu Phe Lys Asn
755 760

<210> 17
<211> 11
<212> PRT
<213> Plasmodium Falciparum

<220>
<221> MISC_FEATURE
<222> (1)..(11)
<223> MSP 3-1 MSP3b-like motif

<400> 17

Ile Leu Gly Trp Glu Phe Gly Gly Gly Val Pro
1 5 10

<210> 18
<211> 11
<212> PRT
<213> Plasmodium Falciparum

<220>
<221> MISC_FEATURE
<222> (1)..(11)
<223> MSP 3-2 MSP3b-like motif

<400> 18

Ile Leu Gly Trp Glu Phe Gly Gly Gly Ala Pro
1 5 10

<210> 19
<211> 14
<212> PRT
<213> Plasmodium Falciparum

<220>
<221> MISC_FEATURE
<222> (1)..(14)
<223> MSP 3-3 MSP3b-like motif

<400> 19

Ile Leu Asp Asp Gly Ile Glu Phe Ser Gly Gly Leu Tyr Phe
1 5 10

<210> 20
<211> 11
<212> PRT
<213> Plasmodium Falciparum

<220>
<221> MISC_FEATURE
<222> (1)..(11)
<223> MSP 3-4 MSP3b-like motif

<400> 20

Leu Glu Arg Gly Leu Gly Ser Gly Ala Leu Pro
1 5 10

<210> 21
<211> 11
<212> PRT
<213> Plasmodium Falciparum

<220>
<221> MISC_FEATURE
<222> (1)..(11)
<223> MSP 3-4 MSP3b-like motif

<400> 21

Asp Asp Gly Ser Ala Phe Gly Gly Gly Leu Pro
1 5 10

<210> 22
<211> 11
<212> PRT
<213> Plasmodium Falciparum

<220>
<221> MISC_FEATURE
<222> (1)..(11)
<223> MSP 3-7 MSP3b-like motif

<400> 22

Tyr Phe Ala Trp Glu Ile Gly Gly Gly Ala Pro
1 5 10

<210> 23
<211> 11
<212> PRT
<213> Plasmodium Falciparum

<220>
<221> MISC_FEATURE
<222> (1)..(11)
<223> MSP 3-8 MSP3b-like motif

<400> 23

Arg Leu Glu Leu Ser Ser Gly Ser Ser Leu Glu
1 5 10

<210> 24
<211> 11
<212> PRT
<213> Plasmodium Falciparum

<220>
<221> MISC_FEATURE
<222> (1)..(11)
<223> MSP 3-8 MSP3b-like motif

<400> 24

Pro Asp Gly Ser Glu Leu Ala Gly Gly Ile Pro
1               5                   10

<210> 25
<211> 20
<212> PRT
<213> Plasmodium Falciparum

<220>
<221> MISC_FEATURE
<222> (1)..(20)
<223> MSP3-1 MSP3c/d-like motif

<400> 25

Leu Ser His Leu Tyr Val Ser Ser Lys Asp Lys Glu Asn Ile Ser Lys
1               5                   10                  15

Glu Asn Asp Asp
            20

<210> 26
<211> 20
<212> PRT
<213> Plasmodium Falciparum

<220>
<221> MISC_FEATURE
<222> (1)..(20)
<223> MSP 3-2 MSP3c/d-like motif

<400> 26

Leu Glu Gln Ile Lys Ile Pro Ser Trp Asp Arg Asn Asn Ile Pro Asp
1               5                   10                  15

Glu Asn Glu Gln
            20

<210> 27
<211> 20
<212> PRT
<213> Plasmodium Falciparum

<220>
<221> MISC_FEATURE
<222> (1)..(20)
<223> MSP 3-3 MSP3c/d-like motif

<400> 27

Leu Glu Ser Val Asn Leu Thr Ser Trp Asp Lys Glu Asp Ile Val Lys
1               5                   10                  15

Glu Asn Glu Asp
            20

<210> 28
<211> 20
<212> PRT
<213> Plasmodium Falciparum

<220>
<221> MISC_FEATURE
<222> (1)..(20)
<223> MSP 3-4 MSP3c/d-like motif

<400> 28

Leu Glu Leu Ile Lys Leu Thr Ser Lys Asp Glu Glu Asp Ile Ile Lys
1               5                   10                  15

His Asn Glu Asp
            20

<210> 29
<211> 20
<212> PRT
<213> Plasmodium Falciparum

<220>
<221> MISC_FEATURE
<222> (1)..(20)
<223> MSP 3-7 MSP3c/d-like motif

<400> 29

Leu Glu His Val Lys Ile Thr Ser Trp Asp Lys Glu Asp Ile Ile Lys
1               5                   10                  15

Glu Asn Glu Asp
            20

<210> 30
<211> 20
<212> PRT
<213> Plasmodium Falciparum

<220>
<221> MISC_FEATURE
<222> (1)..(20)
<223> MSP 3-8 MSP3c/d-like motif

<400> 30

```
Leu Glu Leu Val Pro Leu Ser Leu Ser Asp Ile Glu Gln Ile Ala Asn
1               5                   10                  15

Glu Ser Glu Asp
            20
```